# EUROPEAN PATENT APPLICATION

(11) **EP 4 821 559 A2**
(43) Date of publication of application: **14.10.2026**
(21) Application number: 26201928.4
(22) Date of filing: 20.01.2023
(51) Int. Cl.: A61P 35/00

(54) **MULTI-SPECIFIC ANTIBODIES IN USES THEREOF IN AVIDITY RECEPTOR CROSSLINKING AND IMMUNE MODULATION**

(30) Priority: 21.01.2022 WO PCT/CN2022/073220
(62) Document number(s) of the earlier application(s) in accordance with Art. 76 EPC: 23743980.7 / 4 466 020
(71) Applicant: Lyvgen Biopharma Holdings Limited, Grand Cayman KY11-1205 (KY)
(72) Inventor: WU, Yi, Shanghai, 201203 (CN); WANG, Jieyi, Belmont, CA 94002 (US)
(74) Representative: Cabinet Becker et Associés

(57) **Abstract**

Multi-specific, optionally multi-valent, antibodies comprising at least one antigen binding moiety in Fv format. Also provided herein are methods for making such multi-specific antibodies and uses thereof for modulating immune responses and treating diseases such as cancer.

## Description

### BACKGROUND OF THE INVENTION

Immune cell receptors such as those in the tumor necrosis factor receptor (TNFR) superfamily member or CD3 play important roles in controlling immune responses against pathogens or diseased cells, including cancer cells and pathogen infected cells. Antibodies targeting such immune cell receptors have been used for modulating immune responses and disease treatment. However, such therapeutic approaches may fail to achieve desired clinical efficacy and/or raised safety concerns. It is therefore of great interest to develop new immune therapies that are effective and safe.

### SUMMARY OF THE INVENTION

The present disclosure is based, at least in part, on the design of multi-specific, optionally multi-valent, antibody format utilizing at least one antigen-binding moiety in Fv format. The multi-specific antibodies having such a format as provided herein may bind to multiple immune cell receptors or bind to both immune cell receptors and tumor associated antigens (TAAs). For example, the binding moiety in Fv format may bind to an immune cell receptor. Since monovalent Fv fragments typically would possess relatively low binding affinity to the target antigen, especially when the Fv fragments are connected to other antibody components via peptide linkers, the multi-specific antibodies disclosed herein would be expected to result in avidity-driven crosslinking of target antigens (*e.g.*, immune cell receptors) through concurrent binding of the multiple antigen-binding moieties to their multiple target antigens so as to conditionally modulate immune responses. Such avidity-driven crosslinking of desired target receptors would lead to therapeutic activities (*e.g.*, anti-tumor cell immune responses), for example, in tumor microenvironment, while avoiding stimulation of systemic immune responses, which may cause undesired side effects.

Further, desired peptide linkers, including flexible peptide linkers and rigid peptide linkers, may be used in the multi-specific antibodies disclosed herein in some instances. Such peptide linkers may have at least the following advantageous features: (a) contribute to the functionality of the Fv fragment, (b) contributes to proper dimerization (*e.g.*, heterodimerization) of two polypeptides of a multi-specific antibody, thereby stabilizing the whole molecule; and (c) reduce binding affinity to Fc gamma receptors to reduce or eliminate Fc-mediated effector functions, either taken alone or in combination with additional mutations in heavy chain constant regions (*e.g.*, in the CH2 and CH3 regions) used in a multi-specific antibody.

It is worth noting that the advantageous features associated with the specific Fv-containing multi-specific antibody format disclosed herein are not target antigen or antigen-binding moiety specific. This multi-specific antibody format, including the one or more Fv fragments and optionally the specific peptide linker(s) disclosed herein, can be used to construct multi-specific antibodies capable of binding to any desired target antigen and would be expected to have the advantageous features disclosed herein, *e.g.*, avidity-driven crosslinking of target antigens.

Accordingly, provided herein are multi-specific antibodies, optionally in multi-valent form, that comprise at least one antigen binding moiety in Fv format (monovalent), methods for producing such multi-specific antibodies and methods of using such for modulating immune responses and for disease treatment.

In some aspects, the present disclosure features a multi-specific antibody, comprising a first binding moiety specific to a first target antigen, and a second binding moiety specific to a second target antigen. The first target antigen is a first immune cell receptor. In some instances, the first immune cell receptor can be a first T cell receptor, for example, a T cell activation receptor or a T cell checkpoint receptor. In some instances, the second target antigen is a second immune cell receptor, optionally a second T cell receptor (e.g., a T cell activation receptor or a T cell checkpoint receptor). In some examples, the second immune cell receptor is different from the first immune cell receptor. Alternatively, the second target antigen is a tumor associated antigen (TAA) (first TAA). The first binding moiety is a first Fv fragment comprising a first heavy chain variable region (V_{H}) and a first light chain variable region (V_{L}), which form a heterodimer. The first V_{H} is linked to a first flexible peptide linker and a first rigid peptide linker. The first V_{L} is linked to a second flexible peptide linker and a second rigid peptide linker. The first rigid peptide linker and the second rigid peptide linker form one or more disulfide bonds. In some instances, the second binding moiety is linked to either the first V_{H} via the first flexible peptide linker or the first V_{L} via the second flexible peptide linker.

In some embodiments, the first flexible peptide linker and the second flexible peptide linker are identical. Alternatively, the first flexible peptide linker and the second flexible peptide linker are different. In some examples, the first flexible peptide linker, the second flexible peptide linker, or both are G/S-rich peptide linkers. For example, the G/S-rich peptide linker may comprise the formula of (G_{X}S)ₙ, in which X is an integer between 1-6, inclusive, and n is an integer between 1-10, inclusive. Specific examples are provided in **Table 1**, any of which can used in the multi-specific antibodies disclosed herein.

In some embodiments, the first rigid peptide linker, the second rigid peptide linker, or both comprise the amino acid sequence of DKTHTCPPCPAPEAAGP (SEQ ID NO :21), DKTHTCPPCPAPELLGP (SEQ ID NO: 9), or DKTHTCPPCPAPELLGGP (SEQ ID NO:27). In some instances, the rigid peptide linker may comprise the just-noted sequence flanked by a G/S rich peptide linker at the N-terminus and/or the C-terminus. In some examples, the first rigid peptide linker, the second rigid peptide linker, or both comprise the (G_{X}S)ₙ motif connected to the N-terminus of DKTHTCPPCPAPEAAGP (SEQ ID NO:21), wherein X is an integer between 1-6, inclusive, and n is an integer between 1-10, inclusive. In other examples, the first rigid peptide linker, the second rigid peptide linker, or both comprise the (G_{X}S)ₙ motif connected to the N-terminus of DKTHTCPPCPAPELLGP (SEQ ID NO:9) or connected to the N-terminus of DKTHTCPPCPAPELLGGP (SEQ ID NO:27), wherein X is an integer between 1-6, inclusive, and n is an integer between 1-10, inclusive. Examples of rigid peptide linkers are provided in **Table 1**, any of which can be used in the multi-specific antibodies disclosed herein.

Any of the multi-specific antibodies disclosed herein may further comprise a third binding moiety specific to a third target antigen. In some instances, the third target antigen is identical to the second target antigen, *e.g.*, the third binding moiety is identical to the second binding moiety. Alternatively, the third binding moiety is different from the second binding moiety. For example, the third target antigen is a second TAA, which is different from the first TAA.

In some embodiments, the second binding moiety is linked to the first V_{H} via the first flexible peptide linker, and the third binding moiety is linked to the first V_{L} via the second flexible peptide linker.

In some instances, the first V_{H} is further linked to a first Fc fragment via the first rigid peptide linker. The first V_{L} may be further linked to a second Fc fragment via the second rigid peptide linker. Each Fc fragment may comprise a CH2 domain and/or a CH3 domain. In some instances, the Fc fragment(s) may be derived from an IgG1 molecule. In some instances, the first Fc fragment and the second Fc fragment may comprise mutations in the CH3 domains that enhance heterodimerization over homodimerization of the first and second Fc fragments as relative to the wild-type counterpart and/or reduce protein A binding. For example, the mutations may be knob-in-hole mutations, charged mutations, or ZW1 mutations.

In some examples, the second binding moiety and/or the third binding moiety are in single-chain variable fragment (scFv) format. Alternatively, the second binding moiety and/or the third binding moiety is in a single-domain antibody format, which optionally is a heavy-chain (VHH) format, in Fab format, or in cross Fab format. In specific examples, the second binding moiety is a Fab fragment comprising a first VH-CH1 fragment and a first VL-CL fragment. In other examples, the second binding moiety is a cross Fab fragment comprising a first VH-CL fragment and a first VL-CH1 fragment. Alternatively, or in addition, the third binding moiety is a Fab fragment comprising a second VH-CH1 fragment and a second VL-CL fragment. In other examples, the third binding moiety is a cross Fab fragment comprising a second VH-CL fragment and second VL-CH1 fragment.

In specific examples, the multi-specific antibody disclosed herein comprise: (a) a first polypeptide comprising, from N-terminus to C-terminus, the first VH-CH1 or VH-CL fragment of the second binding moiety, the first flexible peptide linker, the first V_{H}, the first rigid peptide linker, and the first Fc fragment; (b) a second polypeptide comprising, from N-terminus to C-terminus, the second VH-CH1 or VH-CL fragment of the third binding moiety, the second flexible peptide linker, the first V_{L}, the second rigid peptide linker, and the second Fc fragment; (c) a third polypeptide comprising the first VL-CL or VL-CH1 fragment of the second binding moiety; and (d) a fourth polypeptide comprising the second VL-CL or VL-CH1 fragment of the third binding moiety. In one example, the third polypeptide and the fourth polypeptide are identical.

Any of the multi-specific antibodies disclosed herein may further comprise a fourth binding moiety specific to a fourth antigen. In some embodiments, the fourth target antigen is a third immune receptor, optionally a third T cell activation receptor. In some examples, the third immune receptor is different from the first immune receptor and/or the second immune receptor. For example, the fourth target antigen is a third TAA, which optionally is different from either the first TAA or the second TAA.

In some embodiments, the fourth binding moiety is a second Fv fragment comprising a second V_{H} and a second V_{L.} The second V_{H} is linked to the first Fc fragment via a first peptide linker and the second V_{L} is linked to the second Fc fragment via a second peptide linker. In some instances, the first peptide linker is identical to the second peptide linker. In some examples, the first peptide linker, the second peptide linker, or both are G/S-rich peptide linkers. In one example, the G/S-rich peptide linkers comprise the formula of (GxS)n, in which X is an integer between 1-6, inclusive, and n is an integer between 1-10, inclusive.

In some embodiments, the multi-specific antibody may further comprise third peptide linker and a fourth peptide linker connected to the second V_{H} and second V_{L}, respectively. In some instances, the third peptide linker and the fourth peptide linker are a pair of rigid peptide linkers, optionally identical, that form one or more disulfide bonds. In some examples, the third peptide linker, the fourth peptide linker, or both comprise the amino acid sequence of DKTHTCPPCPAPEAAGP (SEQ ID NO: 21), DKTHTCPPCPAPELLGP (SEQ ID NO: 9), or DKTHTCPPCPAPELLGGP (SEQ ID NO:27). Each of the sequences may be linked to a G/S rich peptide linker. In specific examples, the third peptide linker, the fourth peptide linker, or both comprise the (G_{X}S)ₙ motif, which may be connected to the N-terminus of DKTHTCPPCPAPEAAGP (SEQ ID NO:21), DKTHTCPPCPAPELLGP (SEQ ID NO:9) or DKTHTCPPCPAPELLGGP (SEQ ID NO:27), wherein X is an integer between 1-6, inclusive, and n is an integer between 1-10, inclusive.

In specific examples, the multi-specific antibody disclosed herein may comprise: (a) a first polypeptide comprising, from N-terminus to C-terminus, the first VH-CH1 or VH-CL fragment of the second binding moiety, the first flexible peptide linker, the first V_{H}, the first rigid peptide linker, the first Fc fragment, the first peptide linker, the second V_{H}, and optionally the second peptide linker; (b) a second polypeptide comprising, from N-terminus to C-terminus, the second VH-CH1 or VH-CL fragment of the third binding moiety, the second flexible peptide linker, the first V_{L}, the second rigid peptide linker, the second Fc fragment; the third peptide linker, and second V_{L}, and optionally the fourth peptide linker; (c) a third polypeptide comprising the first VL-CL or VL-CH1 fragment of the second binding moiety; and (d) a fourth polypeptide comprising the second VL-CL or VL-CH1 fragment of the third binding moiety. In one example, the third polypeptide is identical to the fourth polypeptide.

In some instances, the multi-specific antibody disclosed herein may further comprises a first heavy chain constant region fragment linked to the first flexible peptide linker, and a second heavy chain constant region fragment linked to the second flexible peptide linker. In some examples, the first and/or the second heavy chain constant region fragment is derived from an IgG1 molecule. Each of the first and second heavy chain constant region fragment comprises a hinge domain, a CH2 domain, and a CH3 domain. The second binding moiety is linked to either the first heavy chain constant region fragment or the second heavy chain constant region fragment. In some examples, the first heavy chain constant region fragment and the second heavy chain constant region fragment comprise mutations in the CH3 domains that enhance heterodimerization over homodimerization of the first and second Fc fragments as relative to the wild-type counterpart and/or reduce protein A binding. In some instances, the mutations may be knob-in-hole mutations, charged mutations, or ZW1 mutations.

In some examples, the multi-specific antibody may further comprise a third binding moiety specific to a third target antigen. The second binding moiety may be linked to the first heavy chain constant region fragment, and the third binding moiety may be linked to the second heavy chain constant region fragment. In some examples, the third binding moiety is different from the second binding moiety. For example, the third target antigen is a second TAA, which is different from the first TAA. In other examples, the third target antigen may be identical to the second target antigen. For example, the third binding moiety is identical to the second binding moiety.

In some examples, the second binding moiety and/or the third binding moiety are in single-chain variable fragment (scFv) format. Alternatively, the second binding moiety and/or the third binding moiety are in a single-domain antibody format, which optionally is a heavy-chain (VHH) format, in Fab format, or in cross Fab format. In one example, the second binding moiety is a Fab fragment comprising a first VH-CH1 fragment and a first VL-CL fragment. In another example, the second binding moiety is a cross Fab fragment comprising a first VH-CL fragment and a first VL-CH1 fragment. Alternatively or in addition, the third binding moiety is a Fab fragment comprising a second VH-CH1 fragment and a second VL-CL fragment. In another example, the third binding moiety is a cross Fab fragment comprising a second VH-CL fragment and a second VL-CH1 fragment.

In specific examples, the multi-specific antibody disclosed herein may comprise: (a) a first polypeptide comprising, from N-terminus to C-terminus, the first VH-CH1 or VH-CL fragment of the second binding moiety, the first heavy chain constant region fragment, the first flexible peptide linker, the first V_{H}, and the first rigid peptide linker; (b) a second polypeptide comprising, from N-terminus to C-terminus, the second VH-CH1 or VH-CL fragment of the third binding moiety, the second heavy chain constant region fragment, the second flexible peptide linker, and the first V_{L}, the second rigid peptide linker; (c) a third polypeptide comprising the first VL-CL or VL-CH1 fragment of the second binding moiety; and (d) a fourth polypeptide comprising the second VL-CL or VL-CH1 fragment of the third binding moiety. In one example, the third polypeptide and the fourth polypeptide are identical.

In other aspects, the present disclosure features a multi-specific antibody, comprising a first binding moiety specific to a first target antigen, and a second binding moiety specific to a second target antigen. The first target antigen is a first immune cell receptor, which optionally is a first T cell activation receptor; and the second target antigen is (i) a second immune cell receptor, optionally a second T cell activation receptor, which is different from the first immune cell receptor, or (ii) a first tumor associated antigen (TAA). The first binding moiety is a first Fv fragment comprising a first heavy chain variable region (V_{H}) and a first light chain variable region (V_{L}). The first V_{H} is linked to a first peptide linker and a first heavy chain constant region fragment; and the first V_{L} is linked to a second peptide linker and a second heavy chain constant region fragment. The second binding moiety is connected to the first binding moiety via the first heavy chain constant region fragment or the second heavy chain constant region fragment, each of the first heavy chain constant region and the second heavy chain constant region comprises a hinge domain, a CH2 domain, and a CH3 domain. In some examples, the first and/or the second heavy chain constant region fragment is derived from an IgG1 fragment.

In some embodiments, the first peptide linker, the second peptide linker, or both are G/S-rich peptide linkers. For example, the G/S-rich peptide linkers comprise the formula of (G_{X}S)ₙ (SEQ ID NOs: 29 and 512-516), in which X is an integer between 1-6, inclusive, and n is an integer between 1-10, inclusive.

In some embodiments, the multi-specific antibody may further comprise a third binding moiety specific to a third target antigen. In some instances, the third target antigen is identical to the second target antigen. For example, the third binding moiety is identical to the second binding moiety. In some instances, the third binding moiety is different from the second binding moiety. For example, the third target antigen is a second TAA, which optionally is different from the first TAA. In some examples, the second binding moiety and/or the third binding moiety are in single-chain variable fragment (scFv) format, or in a single-domain antibody format, which optionally is a heavy-chain (VHH) format, in Fab format, or in cross Fab format. In specific examples, the binding moieties are in scFv format and/or Fab format.

In some embodiments, the first heavy chain constant region fragment and the second heavy chain constant region fragment comprise mutations in the CH3 domains that enhance heterodimerization over homodimerization of the first and second Fc fragments as relative to the wild-type counterpart and/or reduce protein A binding. Exemplary mutations include knob-in-hole mutations, charged mutations, and/or ZW1 mutations.

In any of the multi-specific antibodies disclosed herein that contain one or more heavy chain constant region fragments, such as Fc fragments, such heavy chain constant region fragments may comprise one or more mutations that alter binding activity to an Fc receptor relative to the wild-type counterpart. In specific examples, the heavy chain constant region fragments may comprise (i) a deletion at one or more of positions 236-238, (ii) an amino acid substitution at one or more of positions 239, 265, 297, 329, 330, and 332, or a combination thereof. In some examples, the heavy chain constant region fragments may comprise one or more of the following: (i) a deletion at the position 237, (ii) at least two amino acid substitutions selected from L234A, L235A and P329G, (iii) a deletion at position 237 and amino acid substitutions of D265A and N297A, (iv) amino acid substitutions S239D, A330L and I332E, and (v) a deletion at the position 237 and the amino acid substitution P329G.

Any of the multi-specific antibodies disclosed herein binds to at least one immune receptor (the first immune receptor), optionally additional immune receptors (the second immune receptor and/or the third immune receptor), which may be CD3, CD28, PD-1, PD-L1, CTLA4, CD47, or a member of the tumor necrosis factor receptor superfamily (TNFRSF). Examples of TNFRSF members include FAS, TNFRSF12A, 4-1BB/CD137, TNFRSF13B, TNFRSF13C, CD27/TNFRSF7, CD30/TNFRSF8, CD40/TNFRSF5, DR3/TNFRSF25, DR4/TNFRSF10A, DR5/TNFRSF10B, DR6/TNFRSF21, GITR/TNFRSF18, HVEM/TNFRSF14, LTβR, OX40/TNFRSF4, TROY/TNFRSF19, RELT/TNFRSF19L, TL1A/TNFSF15, TNFRSF17, TNFRSF1A, TNFRSF11B, RANK/TNFRSF11A, TNFRSF11B, NGFR, EDA2R, and TNFRSF1B, TNFRSF6B, TNFRSF10C, TNFRSF10D, or TNFRSF13A.

In some embodiments, the multi-specific antibodies disclosed herein may further bind to at least one TAA (the first TAA), optionally additional TAAs (the second and third TAAs), which may be one or more of B7H3, CD19, CD20, PSMA, HER2, CEA, BCMA, P53mut, DLL3, MET, EGFR, MAGE-A4, and PRAME.

In some embodiments, the multi-specific antibodies disclosed herein may bind to at least: (a) CD3 and CD28, (b) CD3 and CD137, (c) CD137 and PD-1, (d) CD40 and PD-1, (e) CD40 and PD-L1, (f) CD137 and GITR, (g) CD137 and PD-L1, (h) CD137 and CD40, (i) CD137 and OX40, (j) CD3 and PD-1, (k) CD3 and PD-L1, or (l) CD3 and CTLA4.

In some examples, the multi-specific antibodies disclosed herein may bind to (1) B7H3, CD3 and CD137; (2) CD19, CD3 and CD137; (3) B7H3, CD3 and CD28; (4) CD19, CD3 and CD28; (5) B7H3, CD137 and PD-1; (6) B7H3, CD40 and PD-1; (7) PMSA, CD3 and CD137; (8) B7H3 and CD3; (9) B7H3 and CD137; (10) CD19 and CD3; (11) CD19 and CD137; (12) B7H4 and CD40; (13) HER2, CD3 and CD137; (14) CEA, CD3 and CD137;(15) BCMA, CD3 and CD137; (16) P53mutant, CD3 and CD137; (17) PD-1 and CD137; (18) PD-1 and CD40; (19) B7H3 and CD40; (20) PD-L1 and CD40; (21) PD-L1 and CD3; (22) PD-L1, CD3 and CD137; (23) PD-L1, CD3 and CD28; (24) PD-L1, CD137 and B7H3; (25) PD-L1, CD40 and B7H3; (26) PD-1, CD40 and CD137; (27) PD-1, CD137 and GITR; (28) CD19, CD20, CD3 and CD137; (29) CEA and CD3; (30) CEA and CD137; (31) P53mutant and CD3; (32) P53mutant and CD137; (33) PD-L1, CD40 and CD137; (34) PD-L1 and CD137; (35) MET, EGFR and CD47; (36) BCMA and CD3; (37) BCMA and CD137; (38) PSMA and CD3; (39) HER2 and CD3; (40) HER2 and CD40; (41) HER2 and CD137; (42) PSMA and CD137; (43) HER2, MET, CD3 and CD137; (44) MAGE-A4, CD3 and CD137; (45) PRAME, CD3 and CD137; (46) HER2, MET and CD3; (47) MAGE-A4 and CD3; (48) PRAME and CD3; (49) HER2, MET and CD47; (50) B7H3, CD3 and PD-1; (51) B7H3, CD3 and PD-L1; (52) B7H3, CD3, and CTLA4; (53) PSMA, CD3 and CD137; (54) PSMA, CD3 and CD28; (55) HER2, CD3 and CD137; (56) HER2, CD3 and CD28; (57) CEA, CD3 and CD137; (58) CEA, CD3 and CD28; (59) BCMA, CD3 and CD137; (60) BCMA, CD3 and CD28; (61) CD19, CD19, CD3 and CD28.

Any of the multi-specific antibodies may comprise the same heavy chain complementary determining regions (CDRs) and the same light chain CDRs as those in one or more of the parent antibodies listed in **Table 1**. In some instances, the multi-specific antibody comprises the same V_{H} and V_{L} as those in the one or more parent antibodies.

Any of the multi-specific antibodies disclosed herein may be multi-valent. In some examples, the multi-specific antibody may be trivalent. In other examples, the multi-specific antibody may be bivalent or tetravalent.

Exemplary multi-specific antibodies disclosed herein are provided in **Table 2**.

In another aspect, provided herein is a nucleic acid or a nucleic acid set, which collectively encodes the multi-specific antibody disclosed herein. In some embodiments, the nucleic acid or nucleic acid set can be an expression vector or an expression vector set. Also provided herein is a host cell, comprising the nucleic acid or nucleic acid set encoding for the multi-specific antibody disclosed herein. In some embodiments, the host cell is a mammalian host cell.

In yet another aspect, the present disclosure features a method for producing a multi-specific antibody, comprising: (i) culturing the host cell disclosed herein under conditions allowing for expression of the antibody; and (ii) harvesting the antibody thus produced.

Further, the present disclosure features a pharmaceutical composition comprising any of the multi-specific antibodies disclosed herein or a nucleic acid or nucleic acid set encoding such, and a pharmaceutically acceptable carrier.

In other aspects, the present disclosure features a method for modulating immune responses, the method comprising administering an effective amount of the multi-specific antibody disclosed herein, a nucleic acid(s) encoding such, or a pharmaceutical composition comprising the antibody or the encoding nucleic acid(s) to a subject in need thereof. In some embodiments, the subject may be a human patient having or suspected of having cancer.

Also, within the present disclosure are any of the multi-specific antibodies disclosed herein for use in immune modulation and therapy (e.g., in cancer therapy), and uses of such multi-specific antibodies for manufacturing a medicament for the intended medical uses.

The details of one or more embodiments of the invention are set forth in the description below. Other features or advantages of the present invention will be apparent from the following drawings and detailed description of several embodiments, and also from the appended claims.

### BRIEF DESCRIPTION OF THE DRAWINGS

The following drawings form part of the present specification and are included to further demonstrate certain aspects of the present disclosure, which can be better understood by reference to the drawing in combination with the detailed description of specific embodiments presented herein.

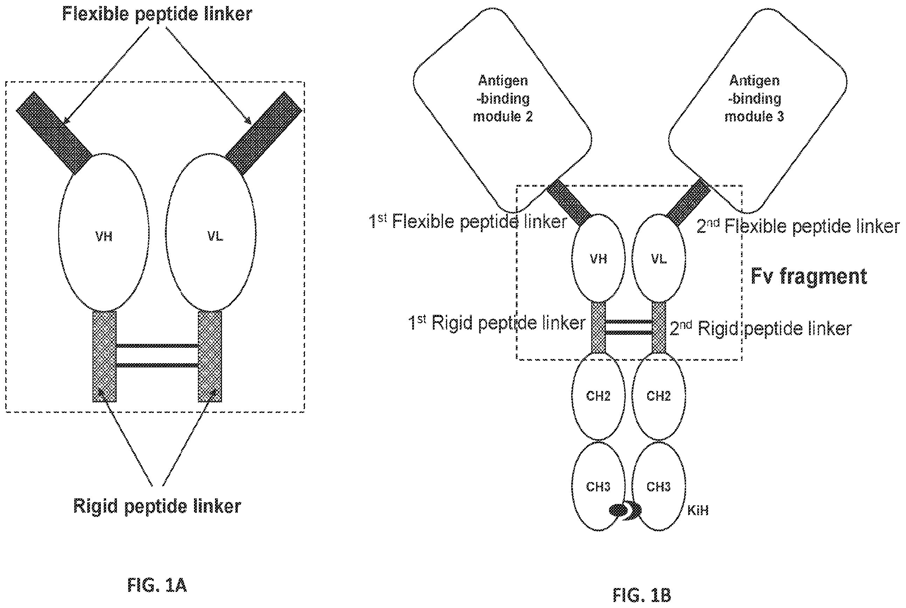

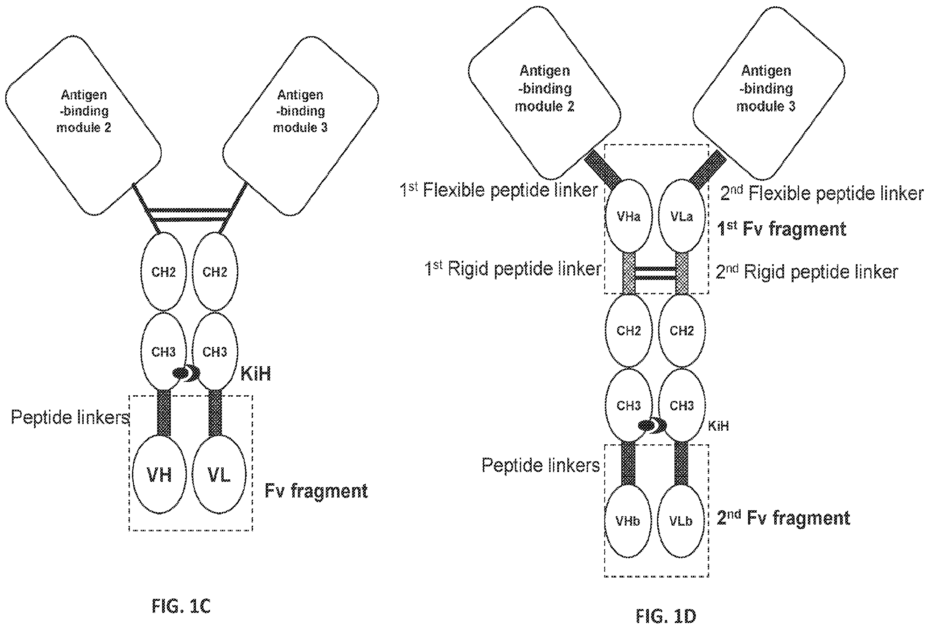

**FIGs. 1A-1D** include diagrams showing schematic design of exemplary multi-specific antibodies provided herein. **FIG. 1A**: an exemplary antigen-binding moiety in Fv format, containing a heavy chain variable domain (V_{H}) and a light chain variable domain (V_{L}), each of which is connected to a flexible peptide linker and a rigid peptide linker. **FIG. 1B**: an exemplary design of a multi-specific antibody having a central (hidden) Fv fragment. **FIG. 1C**: an exemplary design of a multi-specific antibody having a terminal Fv fragment. **FIG. 1D**: an exemplary design of a multi-specific antibody having one central Fv fragment and one terminal Fv fragment.

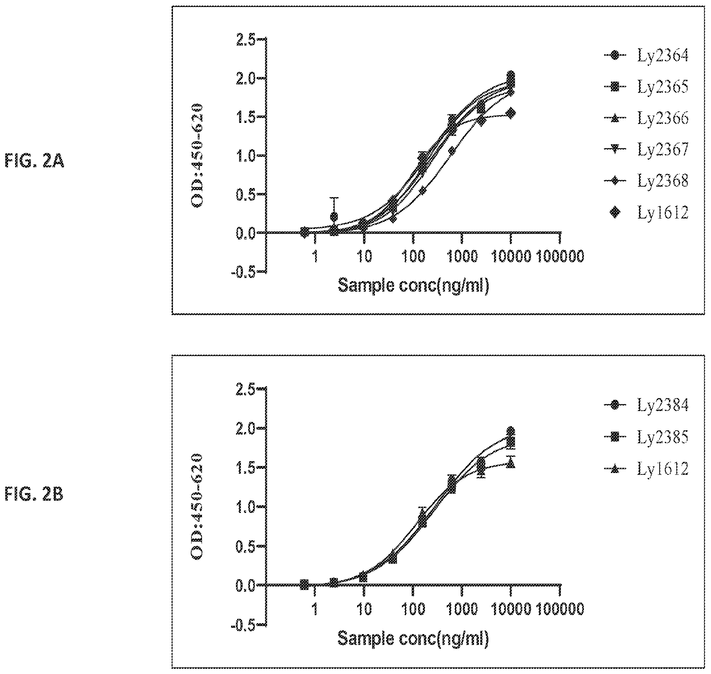

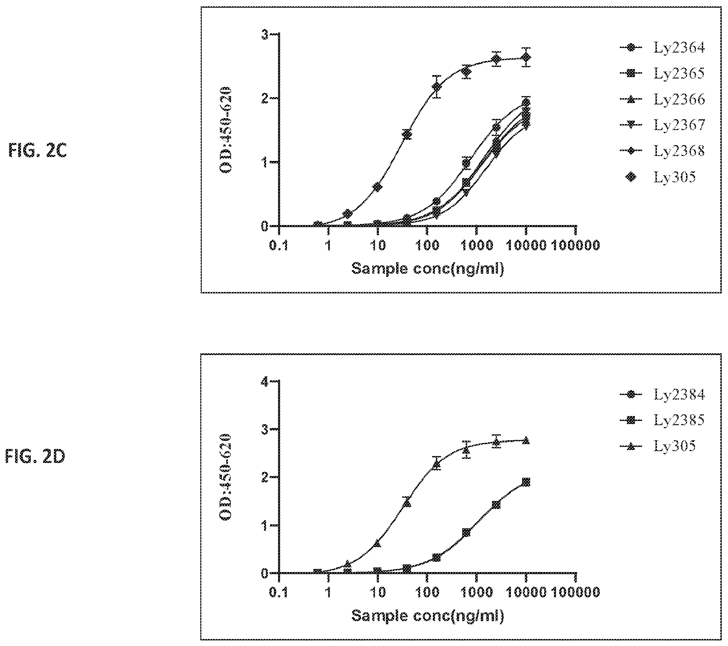

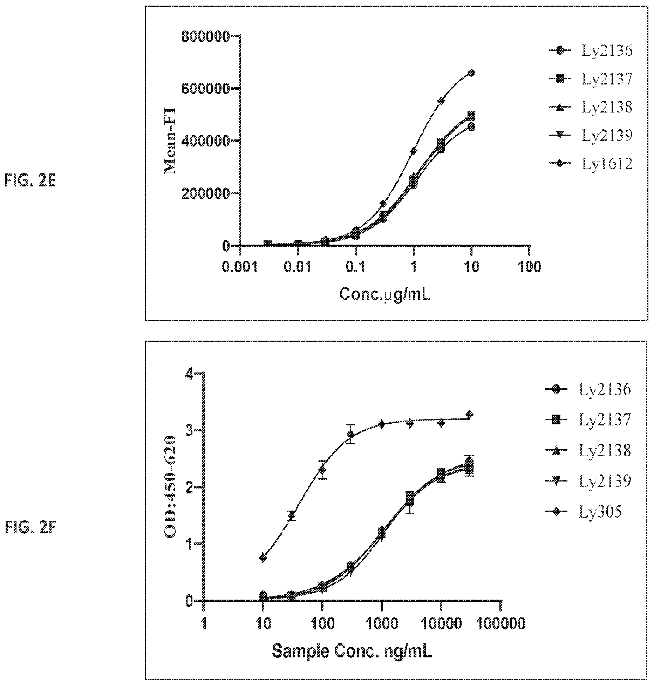

**FIGs. 2A-2B** include graphs showing characterization of multi-specific antibodies having different flexible peptide linkers. **FIG. 2A**: B7H3 binding activity of Ly2364 [having (G₄S)₅ motif (SEQ ID NO: 7) in the flexible peptide linker], Ly2365 [having (G₄S)₃ motif (SEQ ID NO: 5) in the flexible peptide linker], Ly2366 [having (G₄S)₂ motif (SEQ ID NO: 4) in the flexible peptide linker], Ly2367 (having a G₄S motif in the flexible peptide linker), Ly2368 (having no G₄S motif in the flexible peptide linker). B7H3 mAb Ly1612 was used as a positive control. **FIG. 2B**: B7H3 binding activity of Ly2384 (having a G₃S motif in the flexible peptide linker), Ly2385 (having a G₂S motif in the flexible peptide linker) and control Ly1612. **FIG. 2C**: CD3 binding activity of Ly2364, Ly2365, Ly2366, Ly2367 and Ly2368. CD3 mAb Ly305 was used as a positive control. **FIG. 2D**: CD3 binding activity of Ly2384, Ly2385 and control Ly305. **FIG. 2E**: B7H3 binding activity of Ly2136 [having (G₄S)₅ motif (SEQ ID NO: 7) in the flexible peptide linker], Ly2137 [having the (G₄S)₃ motif (SEQ ID NO: 5) in the flexible peptide linker], Ly2138 [having the (G₄S)₂ motif (SEQ ID NO: 4) in the flexible peptide linker], Ly2139 (having a G₄S motif in the flexible peptide linker) and control Ly1612**.** **FIG. 2F**: CD3 binding activity of Ly2136, Ly2137, Ly2138, Ly2139 and control Ly305.

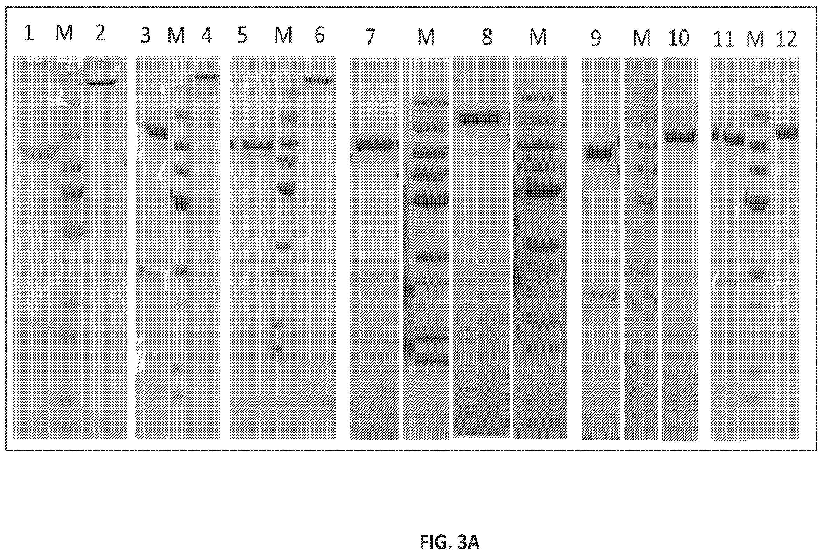

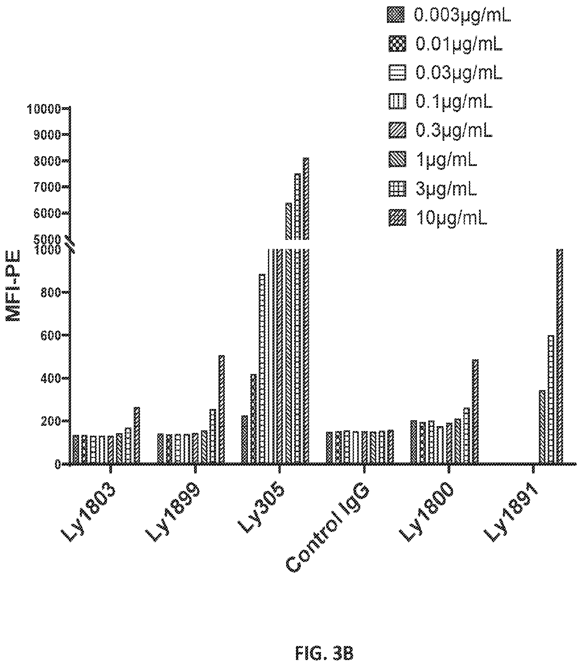

**FIGs. 3A-3B** include graphs showing characterization of multi-specific antibodies having different rigid peptide linkers. **FIG. 3A**: images showing SDS-PAGE analysis of the multi-specific antibodies with different rigid peptide linkers under both non-reduced and reduced conditions. Lanes 1 and 2: Clone Ly1891 under reduced condition and non-reduced condition, respectively. Lanes 3 and 4: Clone Ly1899 under reduced condition and non-reduced condition, respectively. Lanes 5 and 6: Clone Ly1963 under reduced condition and non-reduced condition, respectively. Lanes 7 and 8: Clone Ly1800 under reduced condition and non-reduced condition, respectively. Lanes 9 and 10: Clone Ly1803 under reduced condition and non-reduced condition, respectively. Lanes 11 and 12: Clone Ly1961 under reduced condition and non-reduced condition, respectively. M: molecular weight makers. **FIG. 3B**: a chart showing CD3 binding activity of exemplary multi-specific antibodies as indicated. Parent CD3 mAb Ly305 was used as a positive control.

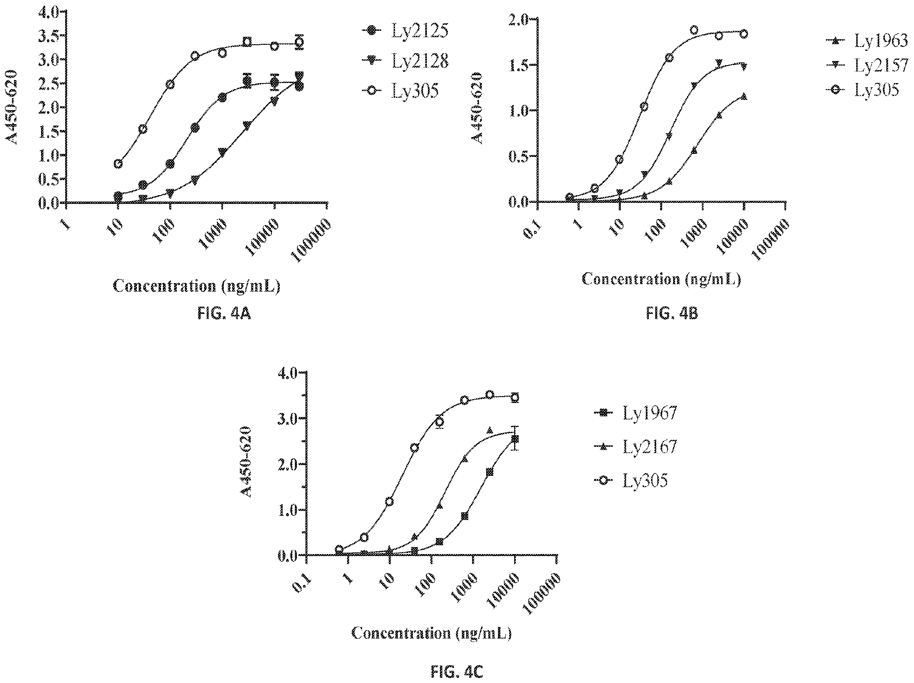

**FIGs. 4A-4C** include charts showing CD3 binding activity of exemplary multi-specific antibodies as indicated. **FIG. 4A**: CD3 binding activity of Ly2125 (having no G₄S motif in the rigid peptide linker) and Ly2128 (having a G₄S motif in the rigid peptide linker). Clone Ly305 was used as a positive control CD3 mAb**.** **FIG. 4B**: CD3 binding activity of Ly2157 (having no G₄S motif in the rigid peptide linker) and Ly1963 (having a G₄S motif in the rigid peptide linker). **FIG. 4C**: CD3 binding activity of Ly2167 (having no G₄S motif in the rigid peptide linker) and Ly1967 (having a G₄S motif in the rigid peptide linker).

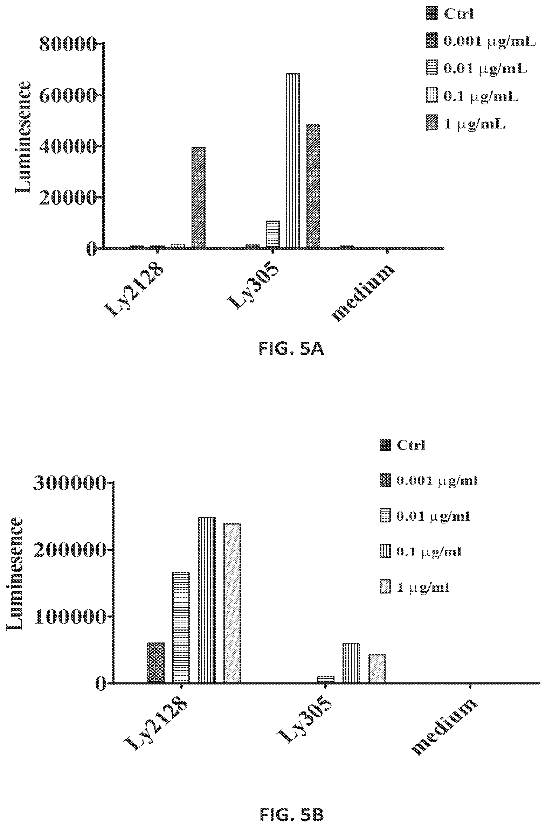

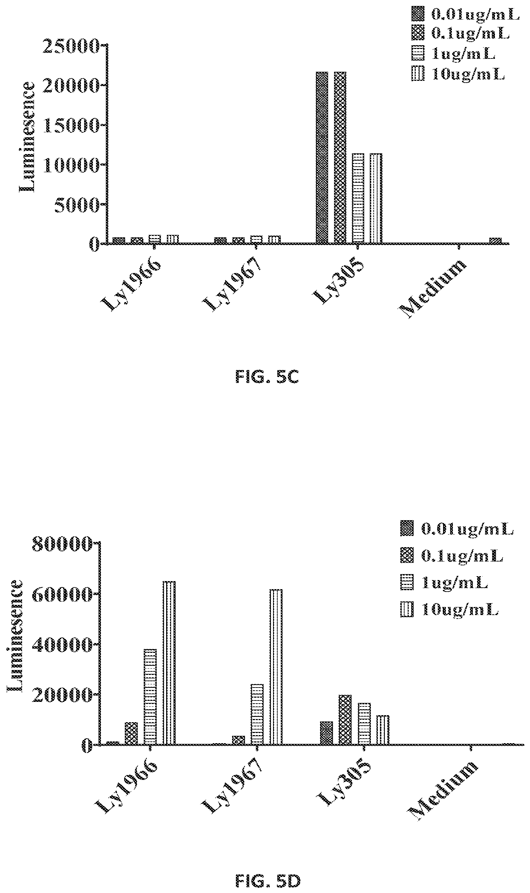

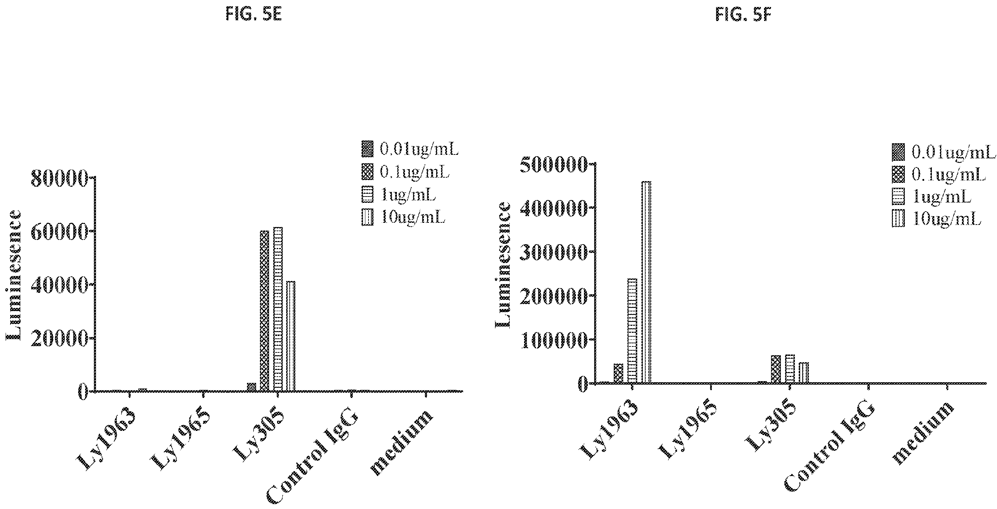

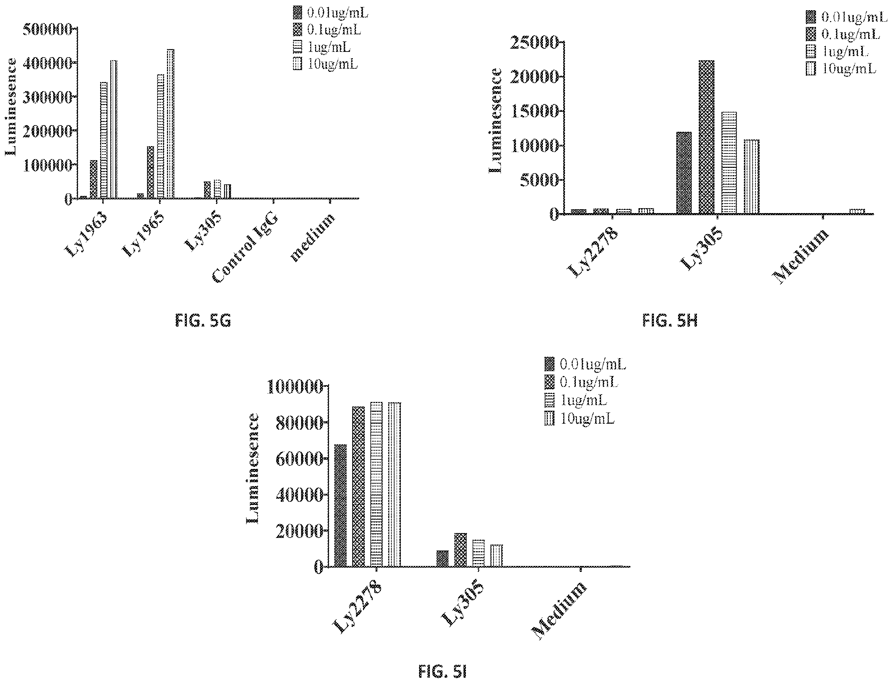

**FIGs. 5A-5I** include diagrams showing CD3 agonistic activity of exemplary multi-specific antibodies as indicated, which include a central Fv fragment that binds to CD3. The agonistic activities were measured at various concentrations when incubated alone, or in co-culture with CHO cells expressing target antigens**.** **FIG. 5A**: CD3 agonistic activity for clones Ly2128 and Ly305 (reference CD3 mAb) evaluated alone. **FIG. 5B**: CD3 agonistic activity for clones Ly2128 and Ly305 when cocultured with B7H3 overexpressing CHO cells. **FIG. 5C**: CD3 agonistic activity for clones Ly1966, Ly1967 and Ly305, evaluated alone. **FIG. 5D**: CD3 agonistic activity for clones Ly1966, Ly1967 and Ly305 when cocultured with CD137 overexpressing CHO cells. **FIG. 5E**: CD3 agonistic activity for clones Ly1963, Ly1965 and Ly305, evaluated alone. **FIG. 5F**: CD3 agonistic activity for clones Ly1963, Ly1965 and Ly305 when cocultured with CD137 overexpressing CHO cells. **FIG. 5G**: CD3 agonistic activity for clones Ly1963, Ly1965 and Ly305 when cocultured with B7H3 overexpressing CHO cells. **FIG. 5H**: CD3 agonistic activity for clones Ly2278 and Ly305, evaluated alone. **FIG. 5I**: CD3 agonistic activity for clones Ly2278 and Ly305 when cocultured with CD19 overexpressing CHO cells.

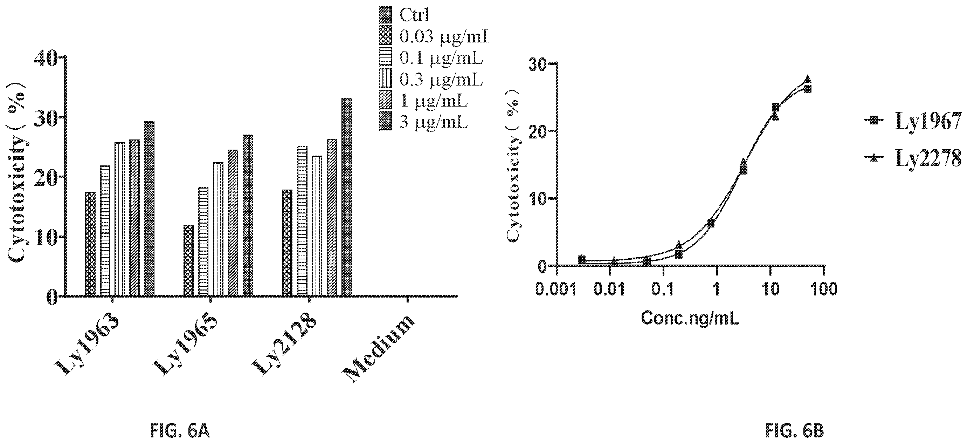

**FIGs. 6A-6B** include diagrams showing cytotoxicity assays using target tumor cells, effector PBMCs and exemplary multi-specific antibodies as indicated. Lactate Dehydrogenase (LDH) release was used as an indication of cytotoxicity. **FIG. 6A**: cytotoxicity of clones Ly1963, Ly1965 and Ly2128. **FIG. 6B**: cytotoxicity of Ly1967 and Ly2278.

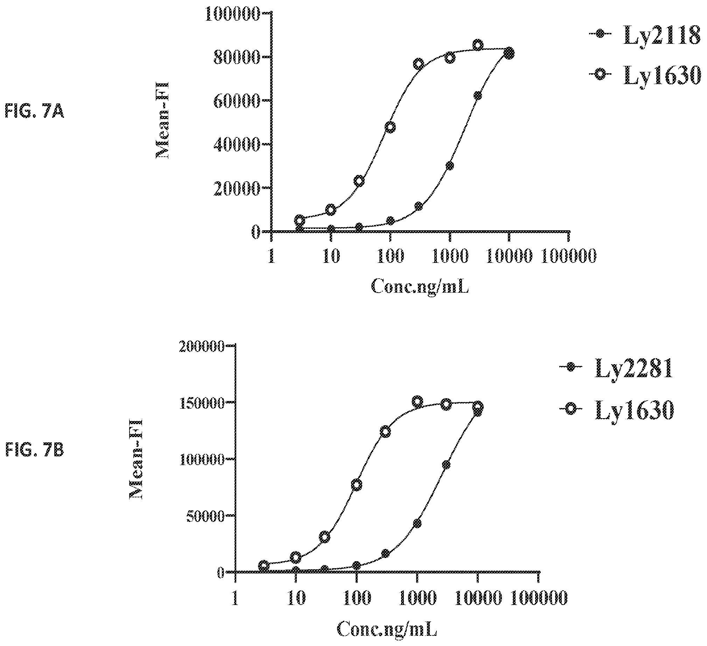

**FIGs. 7A-7B** include diagrams showing characterization of exemplary multi-specific antibodies with a central Fv fragment that binds CD137. **FIG. 7A**: CD137 binding activity of clone Ly2118 relative to parent clone Ly1630. **FIG. 7B**: CD137 binding activity of clone Ly2281 relative to parent clone Ly1630.

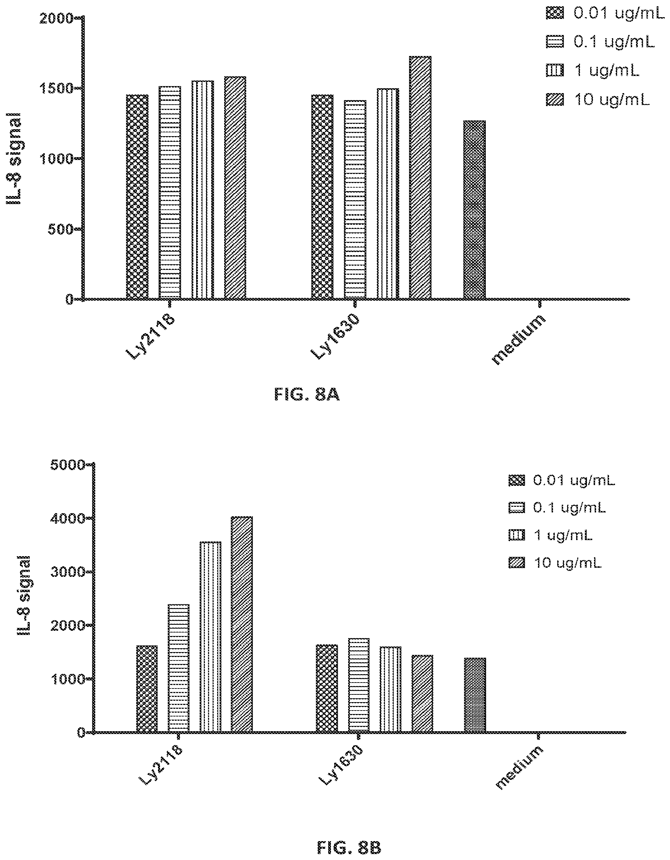

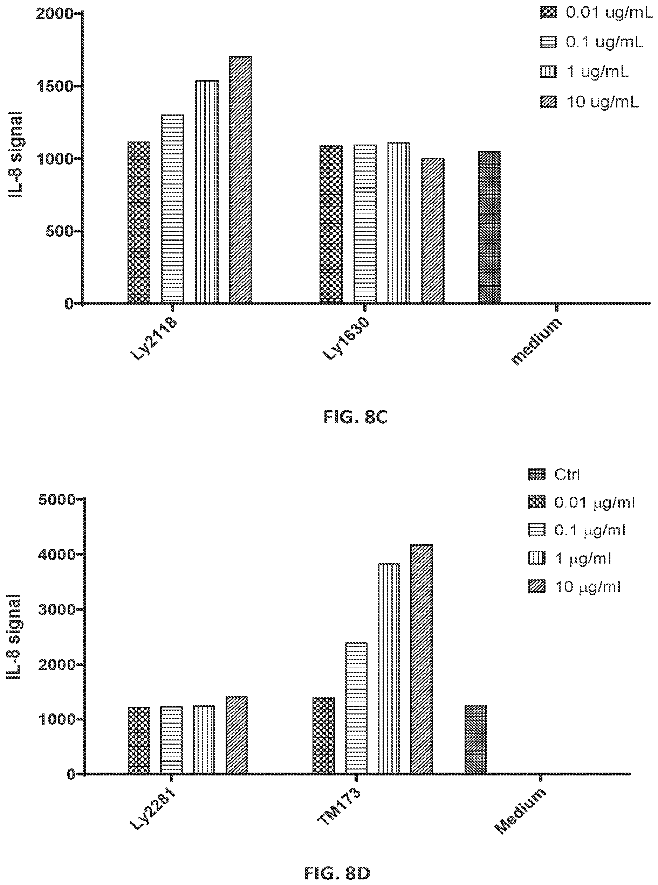

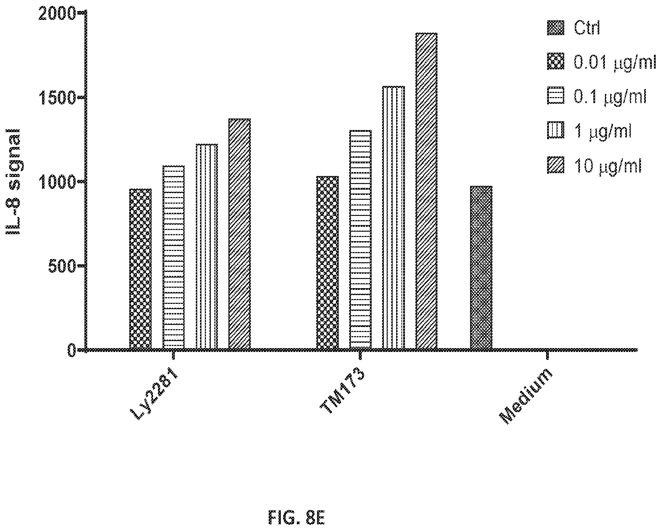

**FIGs. 8A-8E** include diagrams showing activation of human CD137 signaling as indicated by IL-8 secretion in a reporter assay by exemplary multi-specific antibodies co-cultured with CHO or Jurkat overexpressing the target antigen. Parent clone Ly1630 (anti-CD137) and a control anti-CD137 clone TM173 were used as controls. **FIG. 8A**: agonistic activity of clones Ly2118 and Ly1630 alone. **FIG. 8B**: agonistic activity of clones Ly2118 and Ly1630 cocultured with Jurkat cells overexpressing PD-1. **FIG. 8C**: agonistic activity of clones Ly2118 and Ly1630 cocultured with CHO cells overexpressing B7H3. **FIG. 8D**: agonistic activity of clones Ly2281 and TM173 alone. **FIG. 8E**: agonistic activity of clones Ly2281 and TM173 cocultured with CHO cells overexpressing PD-1.

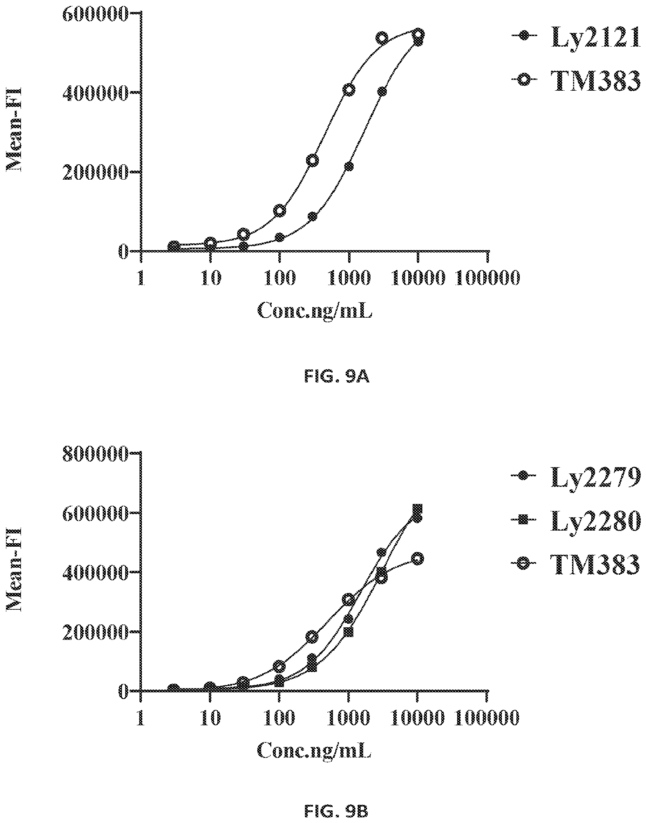

**FIGs. 9A-9B** include diagrams showing CD40 binding activity of exemplary multi-specific antibodies. **FIG. 9A**: CD40 binding activity of Ly2121 relative to the parent CD40 mAb clone TM383. **FIG. 9B**: CD40 binding activity of Ly2279 and Ly2280 relative to parent CD40 mAb clone TM383.

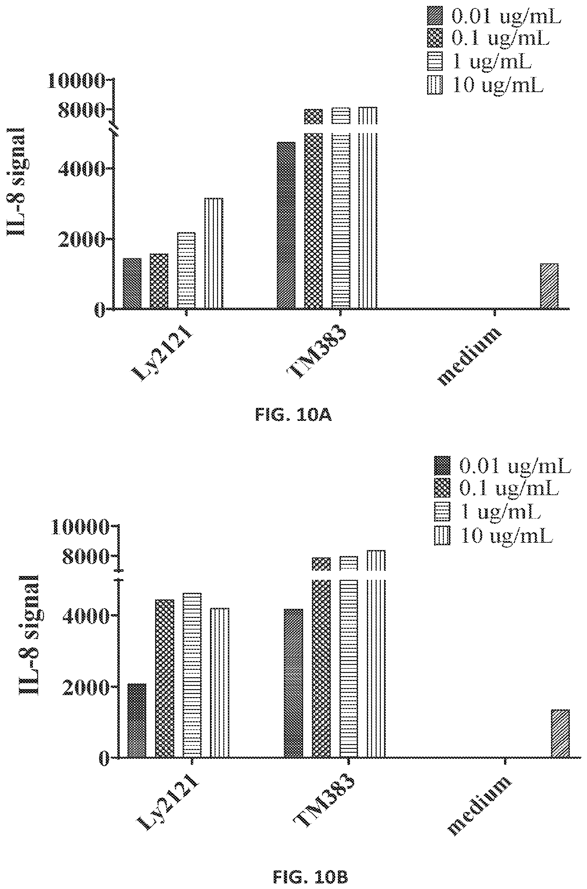

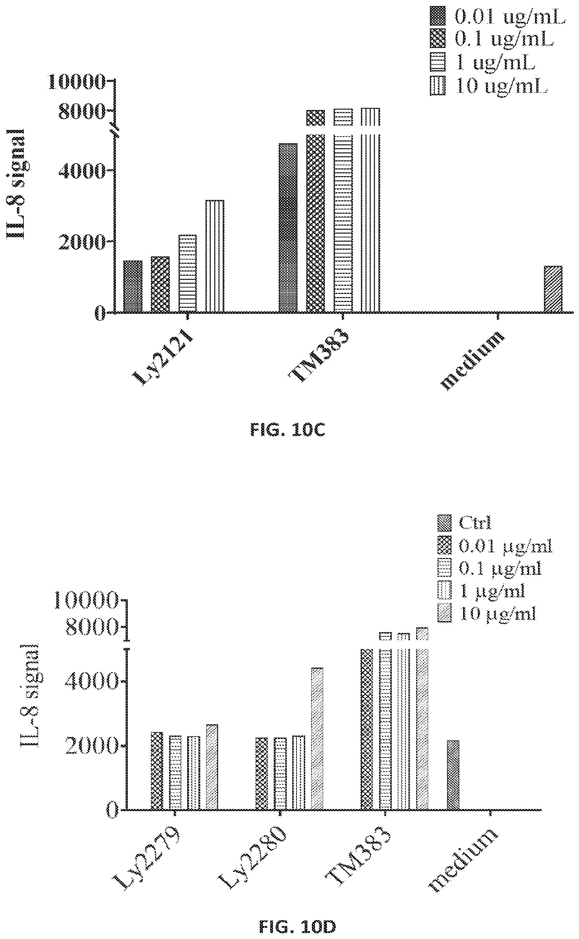

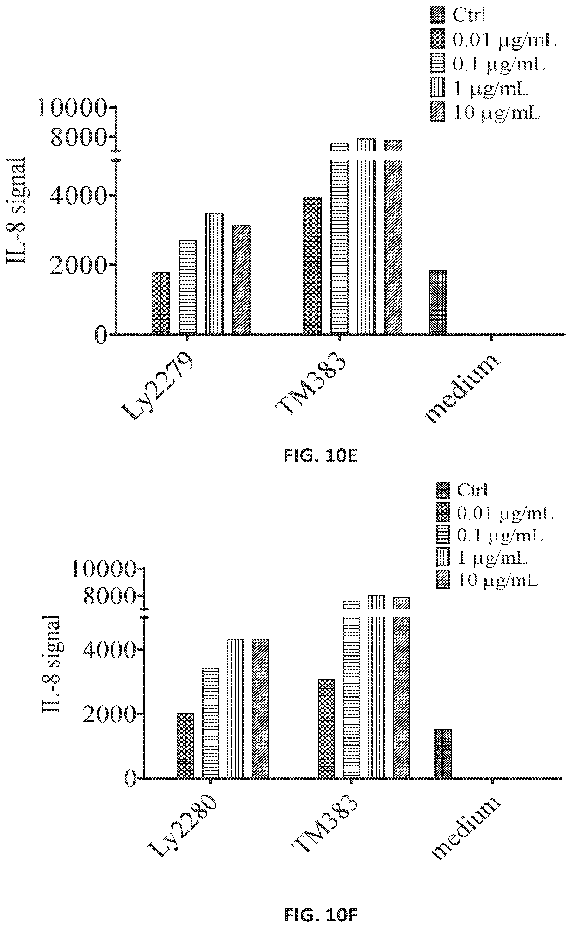

**FIGs. 10A-10F** include diagrams showing activation of human CD40 signaling as indicated by IL8 secretion in a reporter assay by exemplary multi-specific antibodies. The agonistic activity of these multi-specific antibodies was evaluated either alone or in co-culture with CHO cells overexpressing other target antigens. Clone TM383 is the parent CD40 mAb, which was used as a control. **FIG. 10A****:** activation of human CD40 signaling by clones Ly2121 relative to TM383 alone. **FIG. 10B****:** activation of human CD40 signaling by clones Ly2121 relative to TM383, in co-culture with PD-1-expressing CHO cells. **FIG. 10C**: activation of human CD40 signaling by clones Ly2121 relative to TM383, in co-culture with B7H3-expressing CHO cells. **FIG. 10D**: activation of human CD40 signaling by clones Ly2279 and Ly2280 relative to TM383 alone. **FIG. 10E**: activation of human CD40 signaling by clones Ly2279 relative to TM383, in co-culture with B7H3-expressing CHO cells. **FIG. 10F**: activation of human CD40 signaling by clones Ly2280 relative to TM383, in co-culture with PD-1-expressing CHO cells.

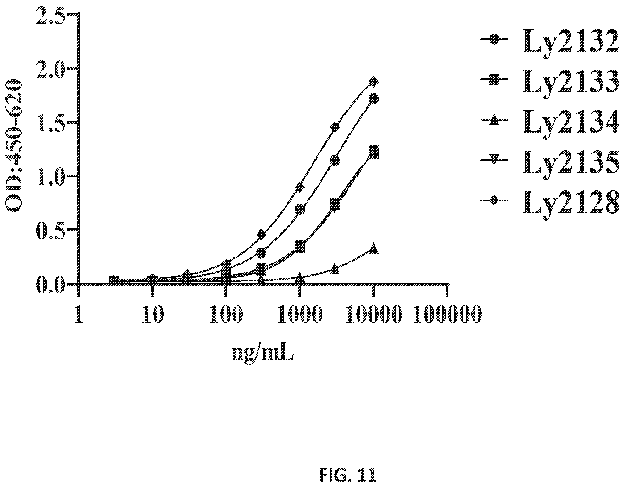

**FIG. 11** is a diagram showing CD28 binding activity of exemplary multi-specific antibodies Ly2132, Ly2133, Ly2134, Ly2135 and Ly2128, which include different peptide linkers in connection with the CD28 binding moiety.

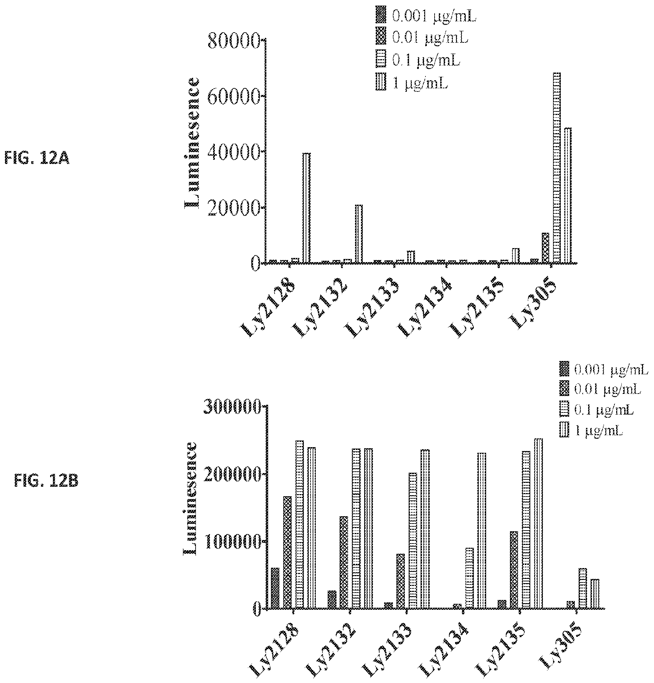

**FIGs. 12A-12B** include diagrams showing activation of human CD3/CD28 signaling. The agonistic activity of exemplary multi-specific antibodies was evaluated either alone or in co-culture with CHO cells overexpressing other target antigens. Clone Ly305 is the parent anti-CD3 antibody and used as a control. **FIG. 12A**: agonistic activity of Ly2132, Ly2133, Ly2134, Ly2135 and Ly2128 alone. **FIG. 12B**: agonistic activity of Ly2132, Ly2133, Ly2134, Ly2135 and Ly2128 in co-culture with B7H3-expressing CHO cells.

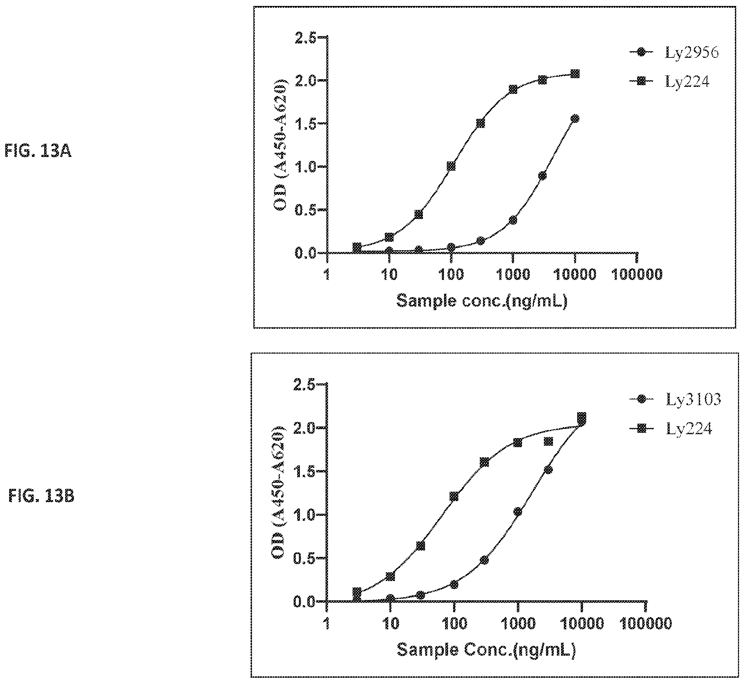

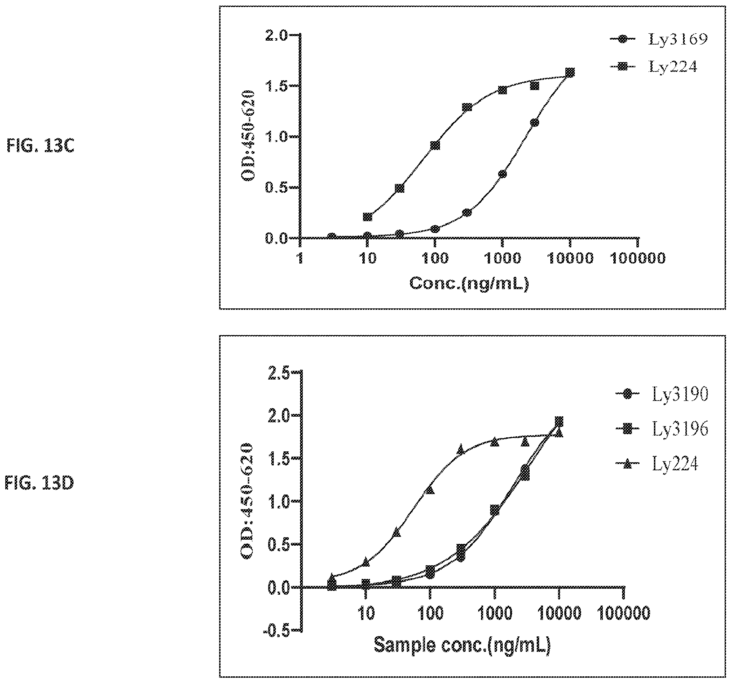

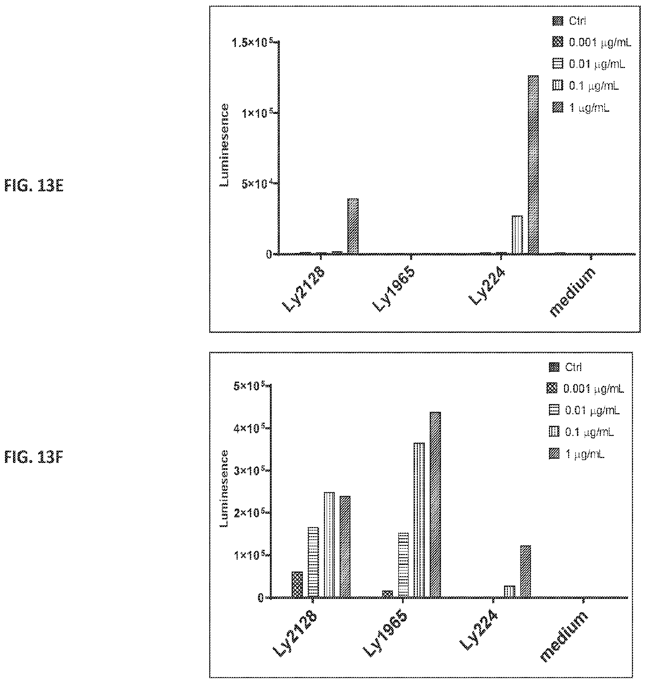

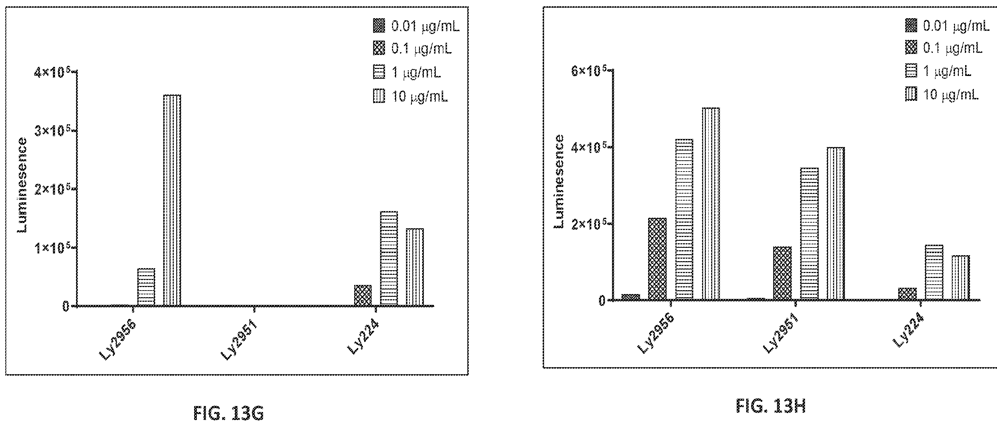

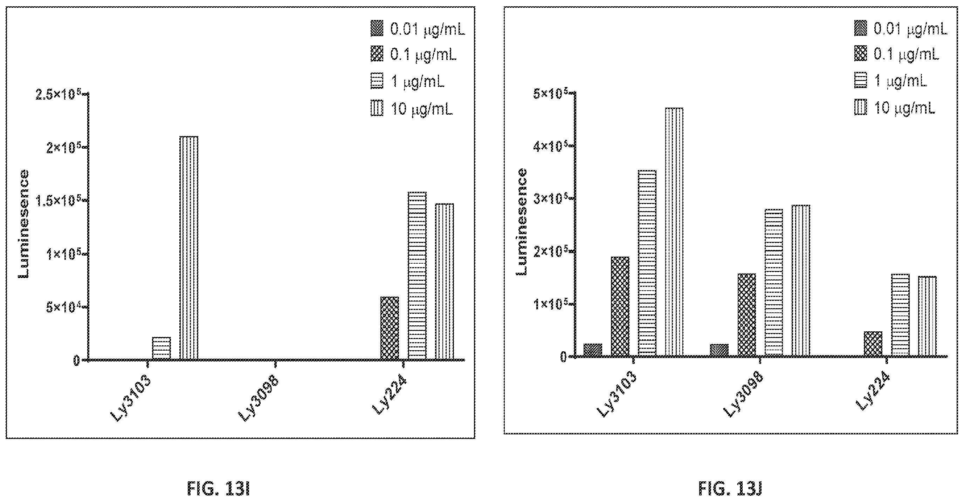

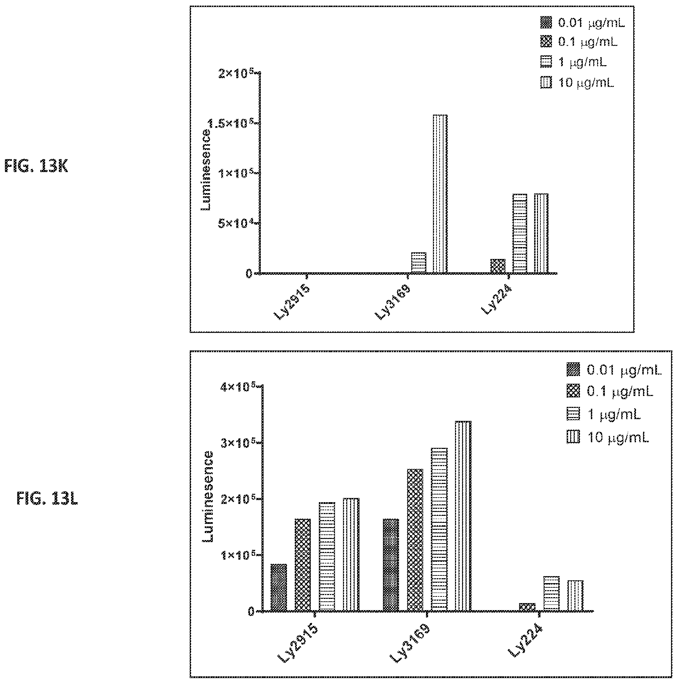

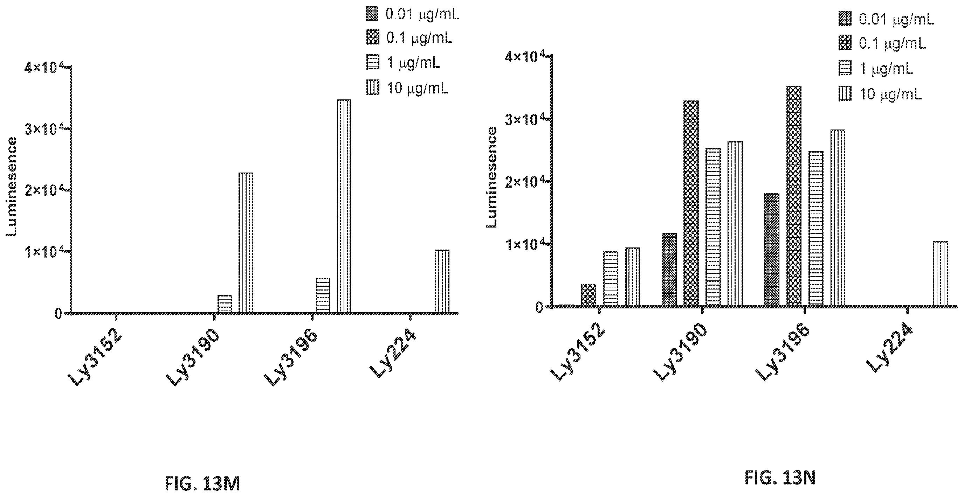

**FIGs. 13A-13N** include diagrams showing characterization of exemplary multi-specific antibodies with a terminal Fv fragment that binds CD28. **FIG. 13A**: CD28 binding activity of clone Ly2956 and parent clone Ly224. **FIG. 13B**: CD28 binding activity of clone Ly3103 and parent clone Ly224. **FIG. 13C**: CD28 binding activity of clone Ly3169 and parent clone Ly224. **FIG. 13D**: CD28 binding activity of clone Ly3190, Ly3196 and parent clone Ly224. **FIGs. 13E-13N** include diagrams showing activation of Jurkat T cell reporter. The agonistic activity of exemplary multi-specific antibodies was evaluated either alone or co-culture with additional target expressing cells. Parent anti-CD28 mAb Ly224 was used as a control. The corresponding multi-specific antibodies without CD28-binding terminal Fv fragment, Ly1965, Ly2951, Ly3098, Ly2915 and Ly3152, were also included. **FIG. 13E**: agonistic activity of clones Ly2128, Ly1965 and Ly224 alone. **FIG. 13F**: agonistic activity of clones Ly2128, Ly1965 and Ly224 cocultured with CHO cells overexpressing B7H3. **FIG. 13G**: agonistic activity of clones Ly2956, Ly2951 and Ly224 alone. **FIG. 13H**: agonistic activity of clones Ly2956, Ly2951 and Ly224 cocultured with H929 cells expressing BCMA. **FIG. 13I**: agonistic activity of clones Ly3103, Ly3098 and Ly224 alone. **FIG. 13J**: agonistic activity of clones Ly3103, Ly3098 and Ly224 cocultured with H929cells expressing BCMA. **FIG. 13K**: agonistic activity of clones Ly2915, Ly3169 and Ly224 alone. **FIG. 13L**: agonistic activity of clones Ly2915, Ly3169 and Ly224 cocultured with HEK293 cells overexpressing CEA. **FIG. 13M**: agonistic activity of clones Ly3152, Ly3190, Ly3196 and Ly224 alone. **FIG. 13N**: agonistic activity of clones Ly3152, Ly3190, Ly3196 and Ly224 cocultured with CHO cells overexpressing HER2.

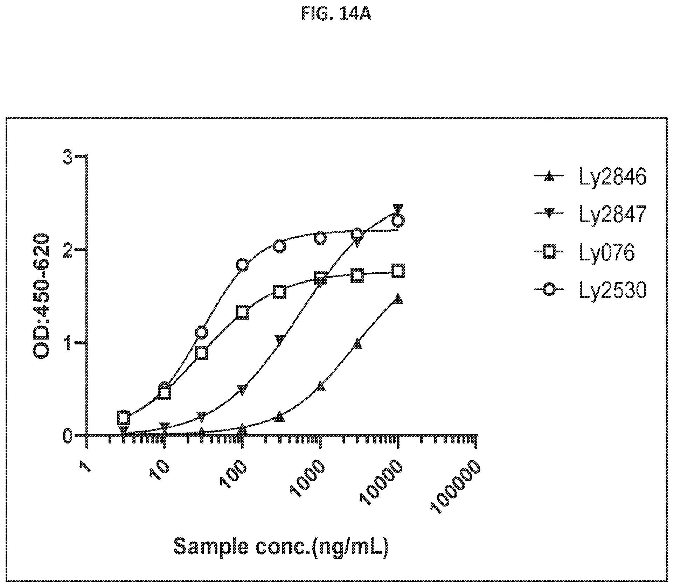

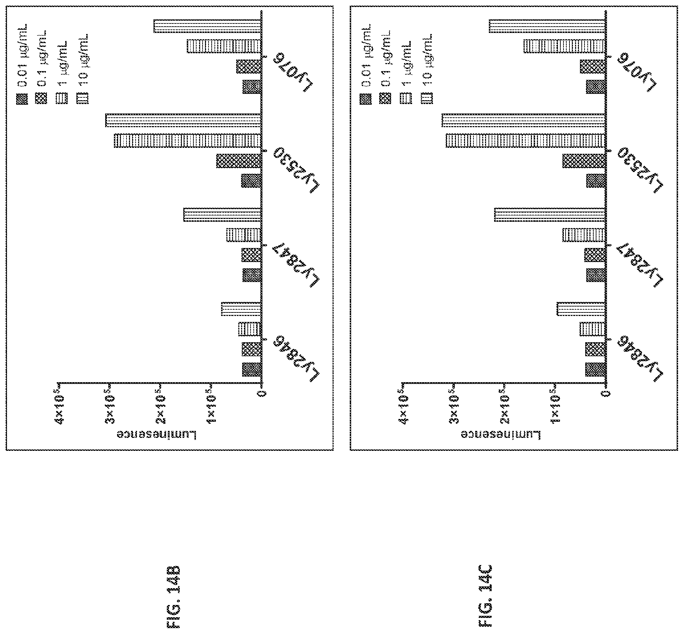

**FIGs. 14A-14C** include diagrams showing characterization of exemplary multi-specific antibodies with a terminal Fv fragment that binds PD-L1. **FIG. 14A**: PD-L1 binding activity of clone Ly2846, Ly2847 and parent clone Ly076 and Ly2530. **FIGs. 14B-14C** include diagrams showing blocking activity of human PD-1/PD-L1 signaling. The blocking activity of exemplary multi-specific antibodies was evaluated either alone or in co-culture with CHO cells overexpressing B7H3. **FIG. 14B**: blocking activity of clones Ly2846, Ly2847, Ly076 and Ly2530 alone. **FIG. 14C**: blocking activity of clones Ly2846, Ly2847, Ly076 and Ly2530 cocultured with CHO cells overexpressing B7H3.

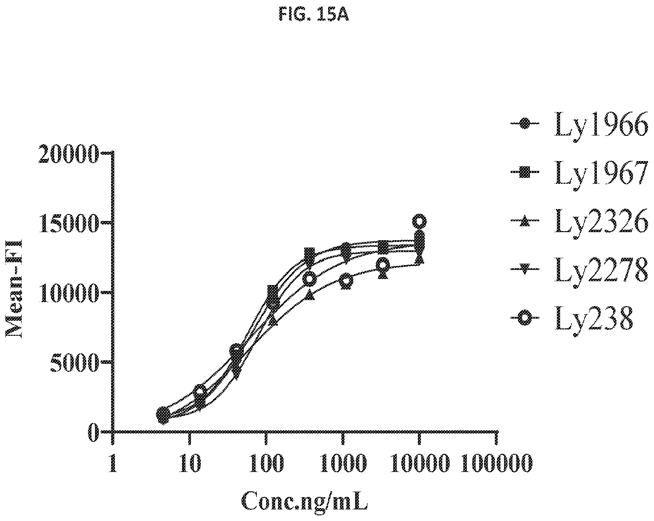

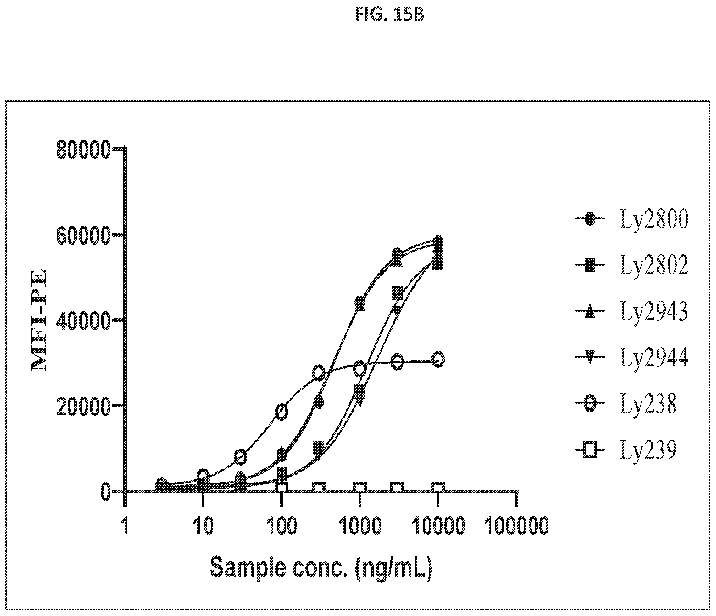

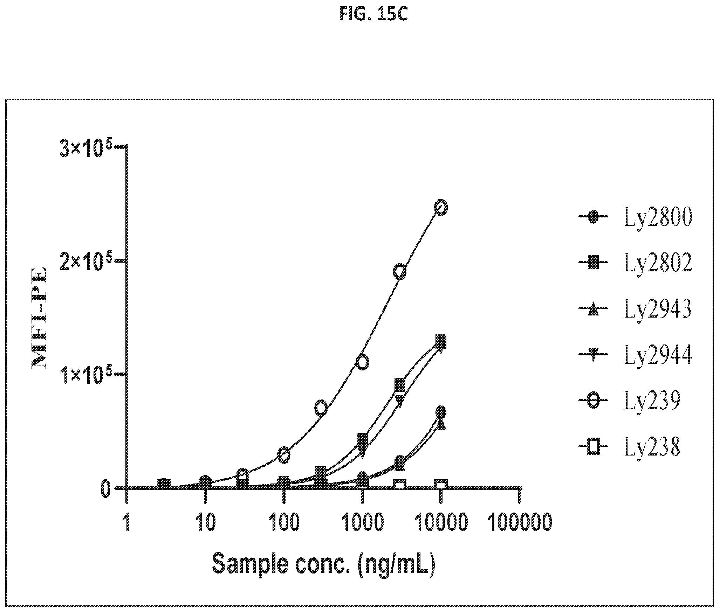

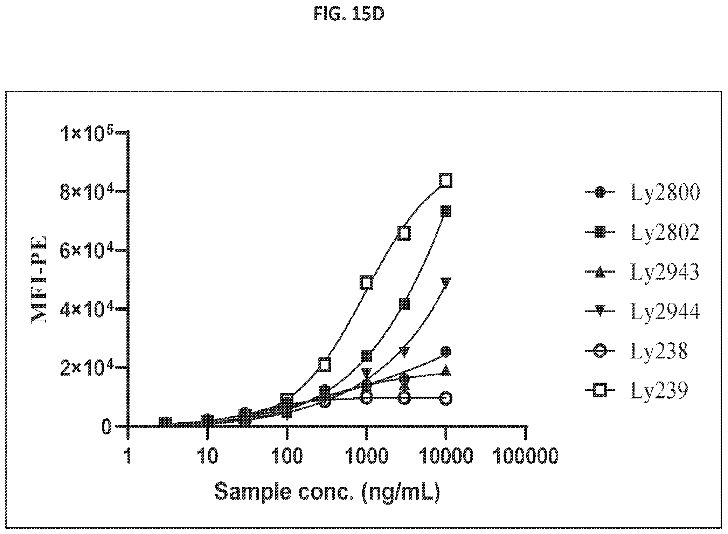

**FIGs. 15A-15D** include diagrams showing tumor antigens binding activity of exemplary multi-specific anti-CD19/CD20 antibodies. Parent anti-CD19 mAb Ly238 and parent anti-CD20 mAb Ly238 were used as controls. **FIG. 15A**: Binding of clones Ly1966, Ly1967, Ly2326, Ly2278 and Ly238 to CHO cells overexpressing CD19. **FIG. 15B**: Binding of clones Ly2800, Ly2802, Ly2943, Ly2944, Ly238 and Ly239 to CHO cells overexpressing CD19. **FIG. 15C**: Binding of clones Ly2800, Ly2802, Ly2943, Ly2944, Ly238 and Ly239 to CHO cells overexpressing CD20. **FIG. 15D**: Binding of clones Ly2800, Ly2802, Ly2943, Ly2944, Ly238 and Ly239 to Raji cells.

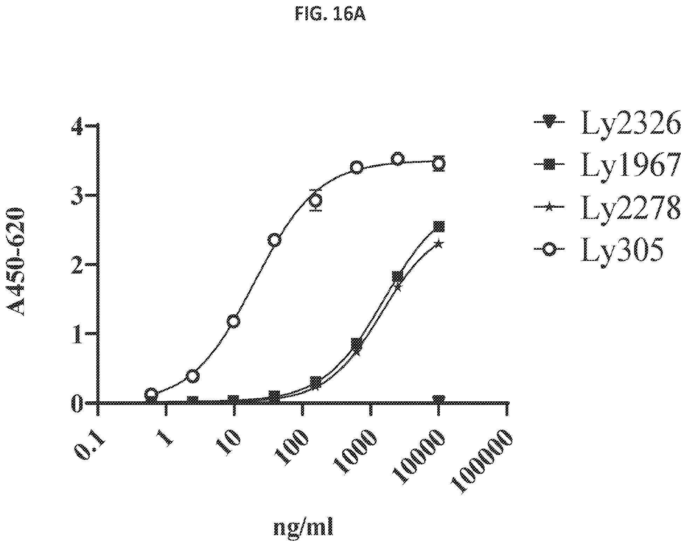

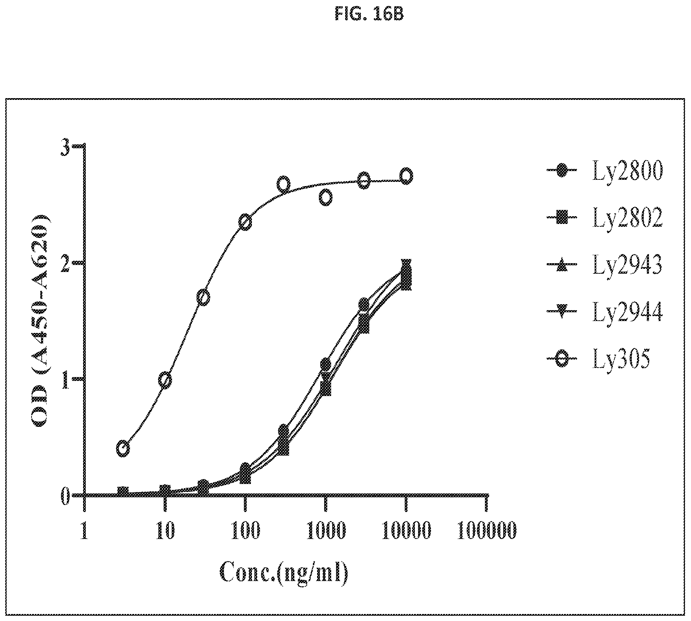

**FIGs. 16A-16B** include diagrams showing central Fv targets binding activity of exemplary multi-specific anti-CD19/CD20 antibodies. Parent anti-CD3 mAb Ly305 was used as a control. **FIG. 16A**: CD3 binding activity for clones Ly1967, Ly2326, Ly2278 and Ly305. **FIG. 16B**: CD3 binding activity for clones Ly2800, Ly2802, Ly2943, Ly2944 and Ly305.

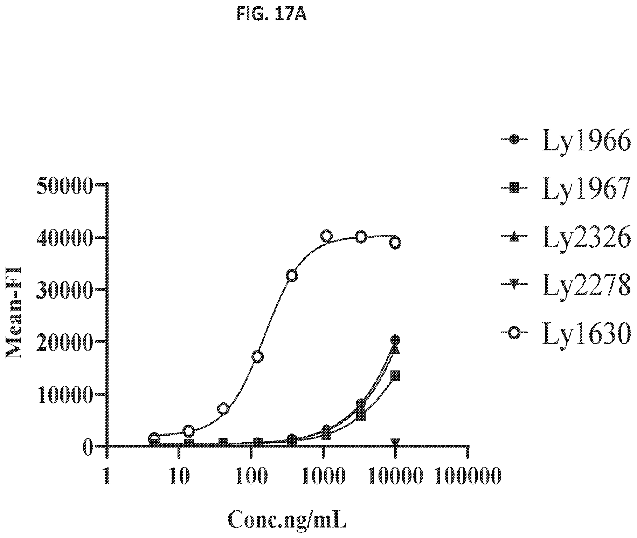

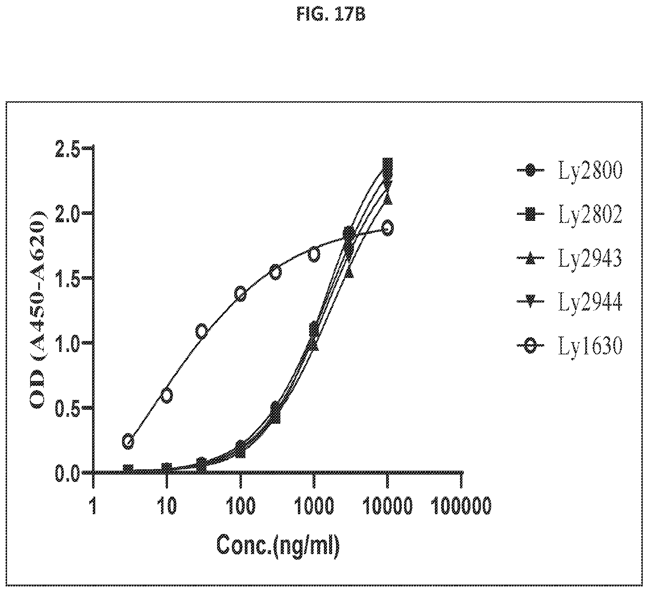

**FIGs. 17A-17B** include diagrams showing terminal Fv targets binding activity of exemplary multi-specific anti-CD19/CD20 antibodies. Parent anti-CD137 mAb Ly1630 was used as a control**.** **FIG. 17A**: CD137 binding activity for clones Ly1966, Ly1967, Ly2326, Ly2278 and Ly1630. **FIG. 17B**: CD137 binding activity for clones Ly2800, Ly2802, Ly2943, Ly2944 and Ly1630.

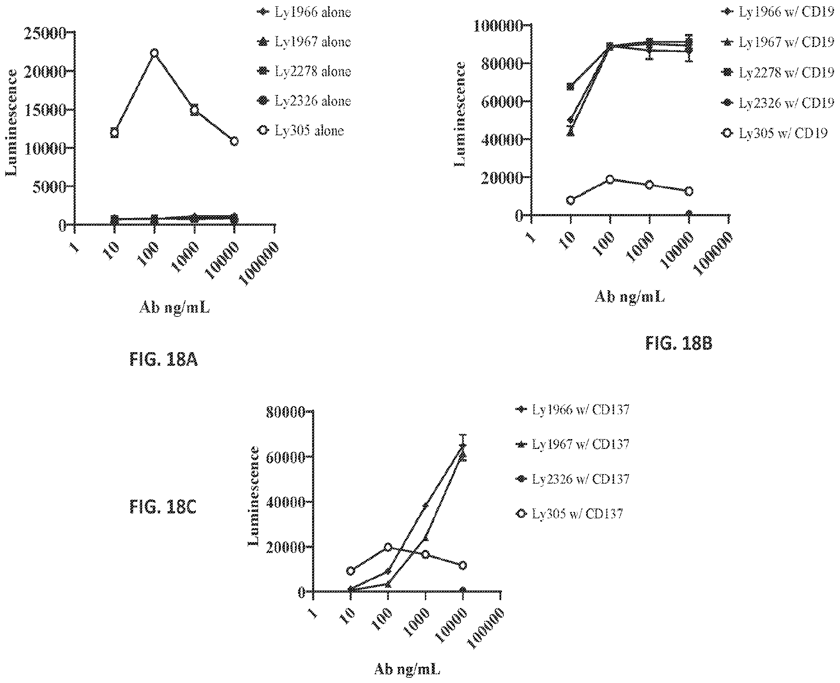

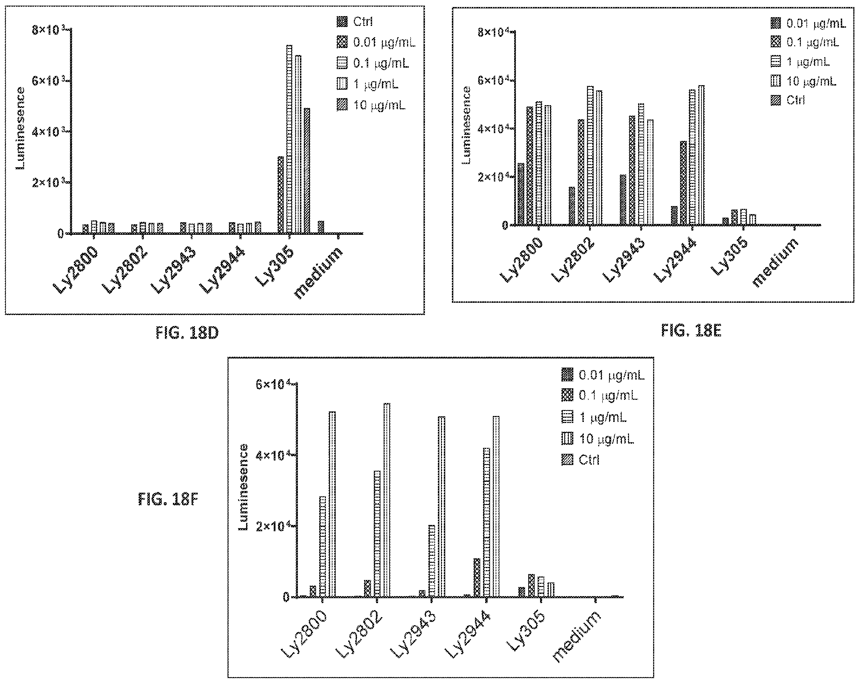

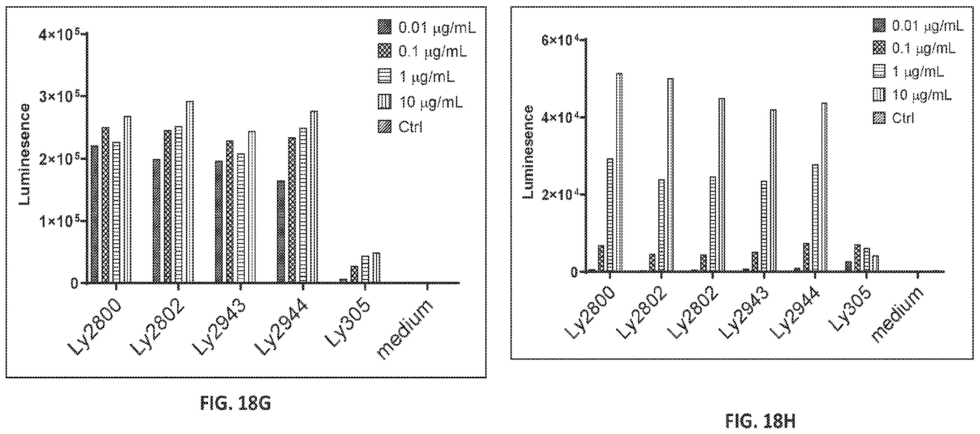

**FIGs. 18A-18H** include diagrams showing central Fv target activation activities of exemplary multi-specific anti-CD19/CD20 antibodies. Parent anti-CD3 mAb Ly305 serves as control. **FIG. 18A**: activation of human CD3 signaling for clones Ly1966, Ly1967, Ly2278, Ly2326 and Ly305 alone. **FIG. 18B**: activation of human CD3 signaling for clones Ly1966, Ly1967, Ly2278, Ly2326 and Ly305 co-cultured with CD19-expressing cells. **FIG. 18C**: activation of human CD3 signaling for clones Ly1966, Ly1967, Ly2278, Ly2326 and Ly305 co-cultured with CD137-expressing cells. **FIG. 18D**: activation of human CD3 signaling for clones Ly2800, Ly2802, Ly2943, Ly2944 and Ly305 alone. **FIG. 18E**: activation of human CD3 signaling for clones Ly2800, Ly2802, Ly2943, Ly2944 and Ly305 co-cultured with CD19-expressing cells. **FIG. 18F**: activation of human CD3 signaling for clones Ly2800, Ly2802, Ly2943, Ly2944 and Ly305 co-cultured with CD20-expressing cells. **FIG. 18G**: activation of human CD3 signaling for clones Ly2800, Ly2802, Ly2943, Ly2944 and Ly305 co-cultured with Raji cells. **FIG. 18H**: activation of human CD3 signaling for clones Ly2800, Ly2802, Ly2943, Ly2944 and Ly305 co-cultured with CD137-expressing cells.

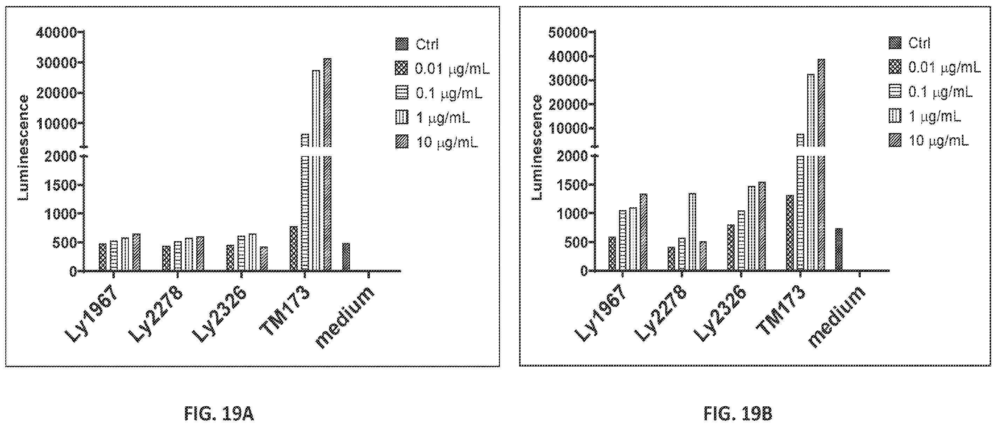

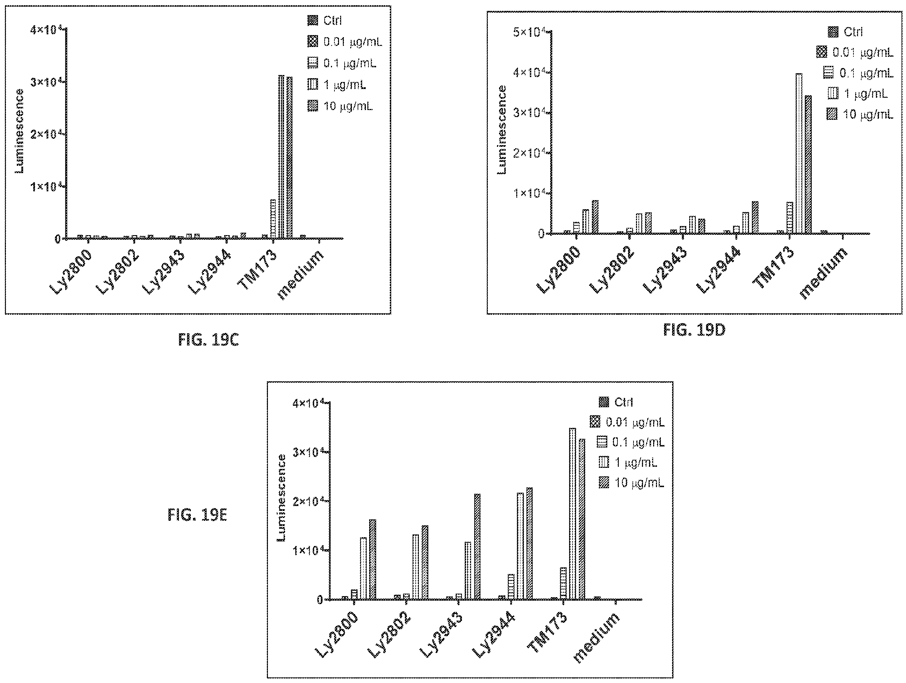

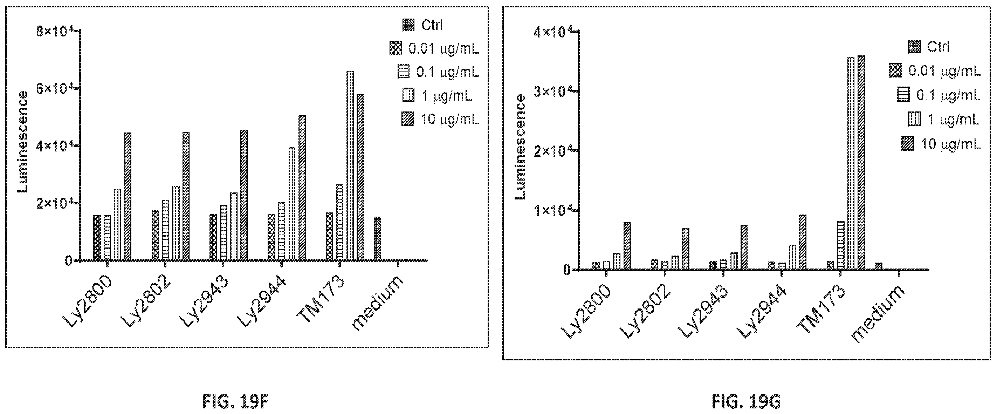

**FIGs. 19A-19G** include diagrams showing terminal Fv target activation activities of exemplary multi-specific anti-CD19/CD20 antibodies. Reference anti-CD137 mAb TM173 serves as control. **FIG. 19A**: activation of human CD137 signaling for clones Ly1967, Ly2278, Ly2326 and TM173 alone. **FIG. 19B**: activation of human CD137 signaling for clones Ly1967, Ly2278, Ly2326 and TM173 co-cultured with CD19-expressing cells. **FIG. 19C**: activation of human CD137 signaling for clones Ly2800, Ly2802, Ly2943, Ly2944 and TM173 alone. **FIG. 19D**: activation of human CD137 signaling for clones Ly2800, Ly2802, Ly2943, Ly2944 and TM173 co-cultured with CD19-expressing cells. **FIG. 19E**: activation of human CD137 signaling for clones Ly2800, Ly2802, Ly2943, Ly2944 and TM173 co-cultured with CD20-expressing cells. **FIG. 19F**: activation of human CD137 signaling for clones Ly2800, Ly2802, Ly2943, Ly2944 and TM173 co-cultured with Raji cells. **FIG. 19G**: activation of human CD137 signaling for clones Ly2800, Ly2802, Ly2943, Ly2944 and TM173 co-cultured with Jurkat cells.

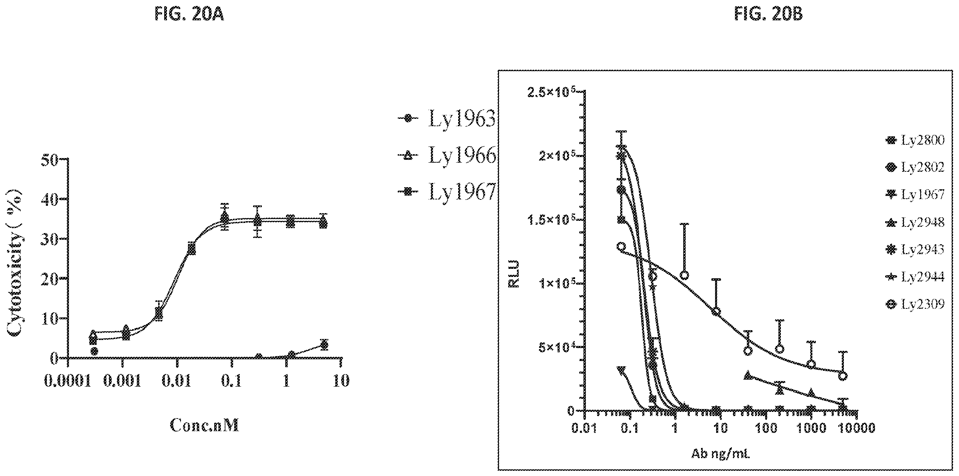

**FIGs. 20A-20B** includes diagrams showing cytotoxicity activity of exemplary multi-specific antibodies. **FIG. 20A**: Lactate dehydrogenase (LDH) release cytotoxicity assay for clones Ly1963, Ly1966 and Ly1967. **FIG. 20B**: Luciferase-transfected tumor cells cytotoxicity assay for clones Ly2800, Ly2802, Ly1967, Ly2943, Ly2944, Ly2948 and Ly2309.

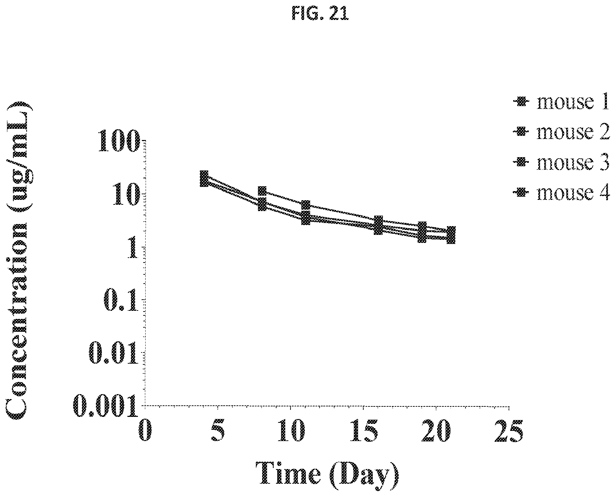

**FIG. 21** is a diagram showing serum concentrations of CD19/CD3/CD137 multi-specific antibody clone Ly1967 in mice administered intraperitoneally a single dose of 5 mg/kg.

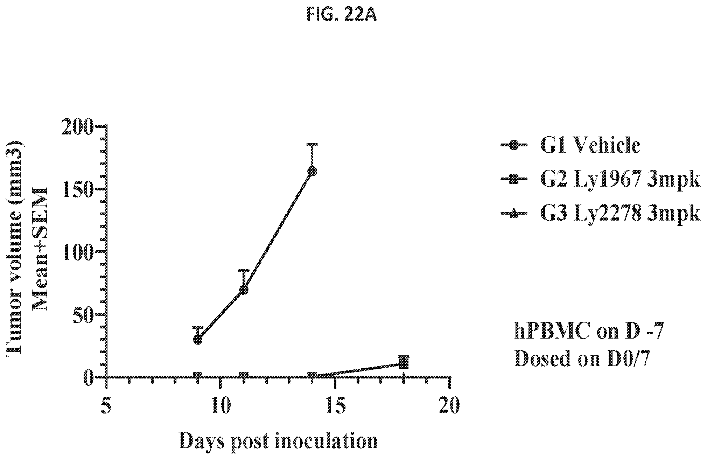

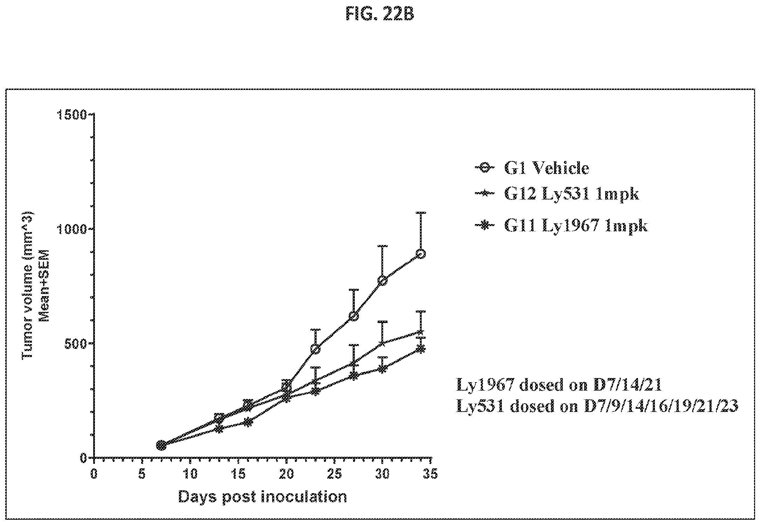

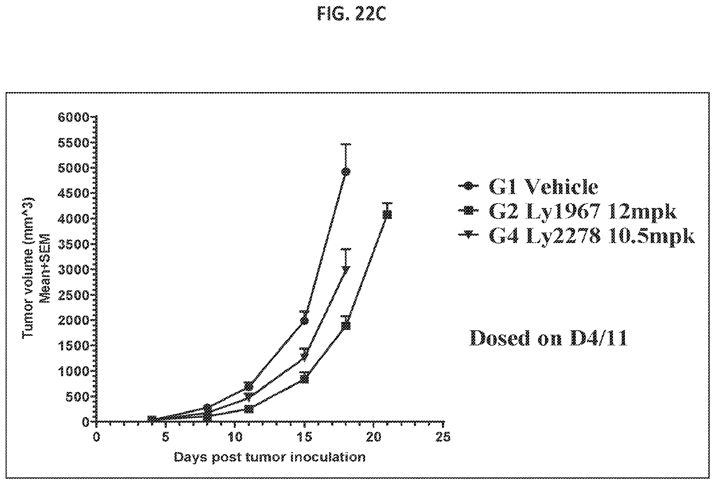

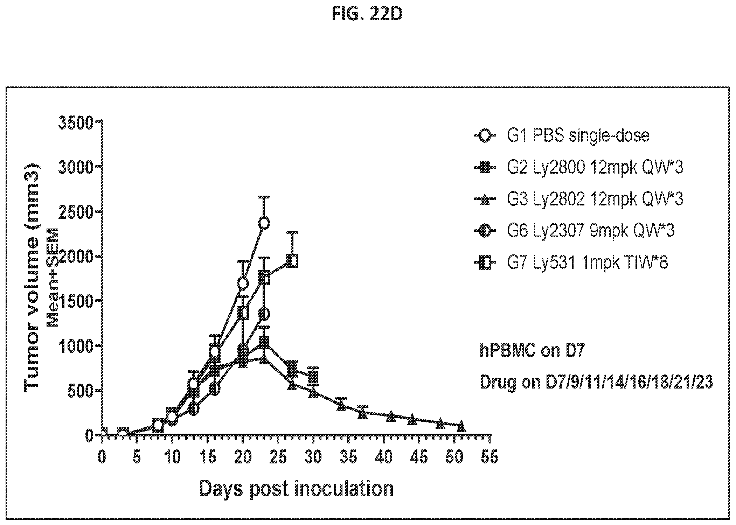

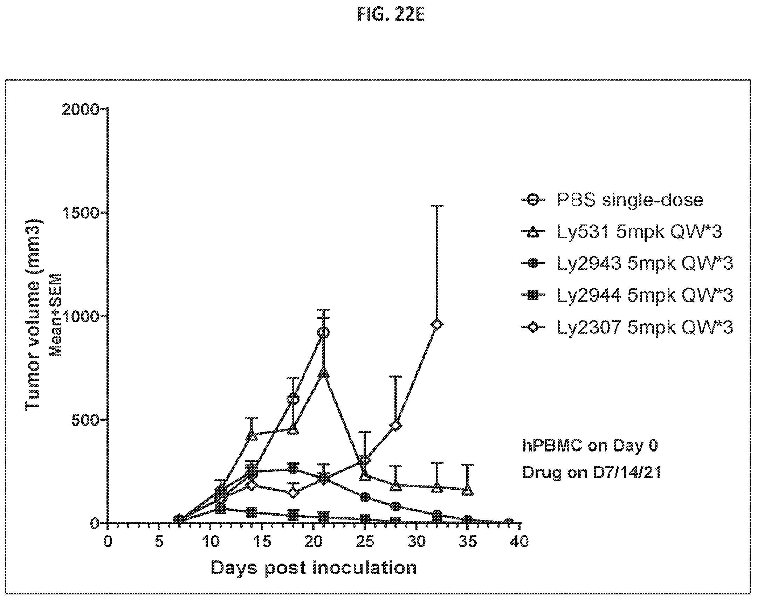

**FIGs. 22A-22C** include diagrams showing anti-tumor activity of exemplary multi-specific antibody clones**.** **FIG. 22A**: Anti-tumor activity of clones Ly1967 and Ly2278 in human PBMC engrafted mouse model bearing Raji tumor cells. **FIG. 22B**: Anti-tumor activity of Ly1967 and Ly531 in MC38-huCD19 bearing mouse models of bone marrow transplanted from CD3 and CD137 knock-in mice. **FIG. 22C**: Anti-tumor activity of Ly1967 and Ly2278 in B16F10-huCD19 bearing mouse models of bone marrow transplanted from CD3 and CD137 knock-in mice. **FIG. 22D**: Anti-tumor activity of clones Ly2800, Ly2802, Ly2307 and Ly531 in human PBMC engrafted mouse model bearing Raji tumor cells. **FIG. 22E**: Anti-tumor activity of clones Ly2943, Ly2944, Ly2307 and Ly531 in human PBMC engrafted mouse model bearing Raji tumor cells.

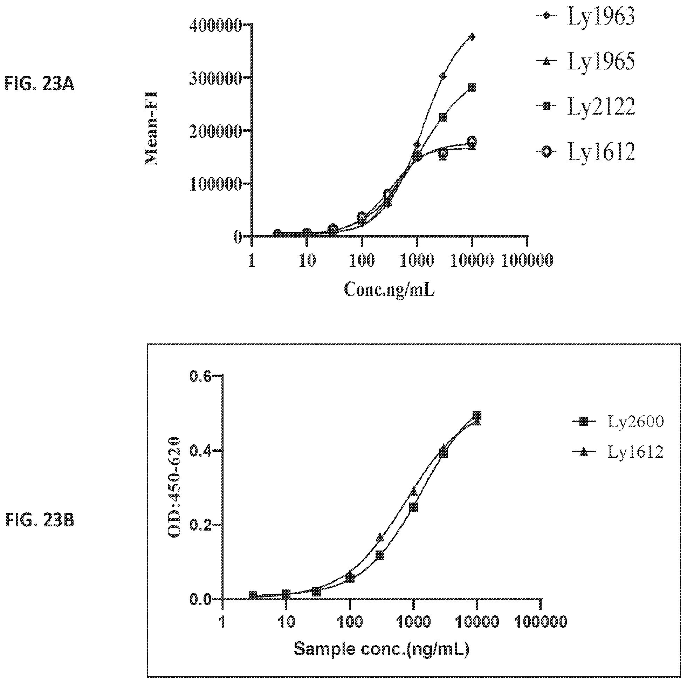

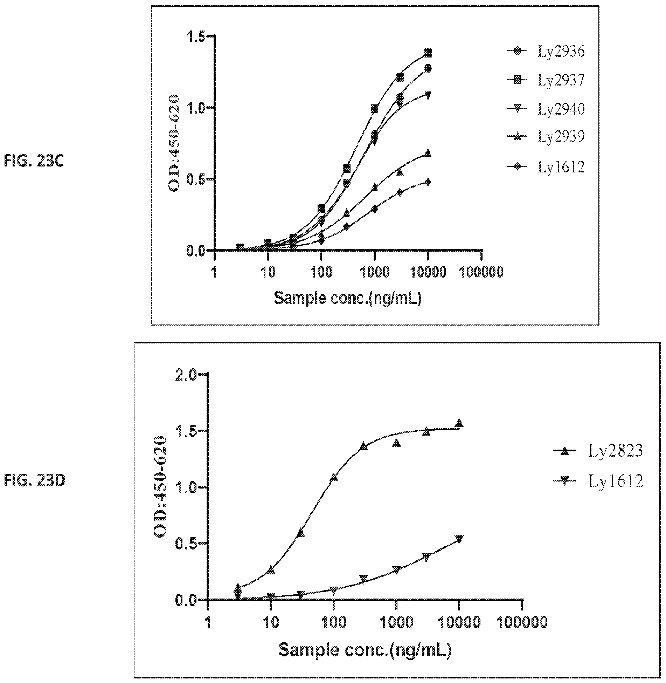

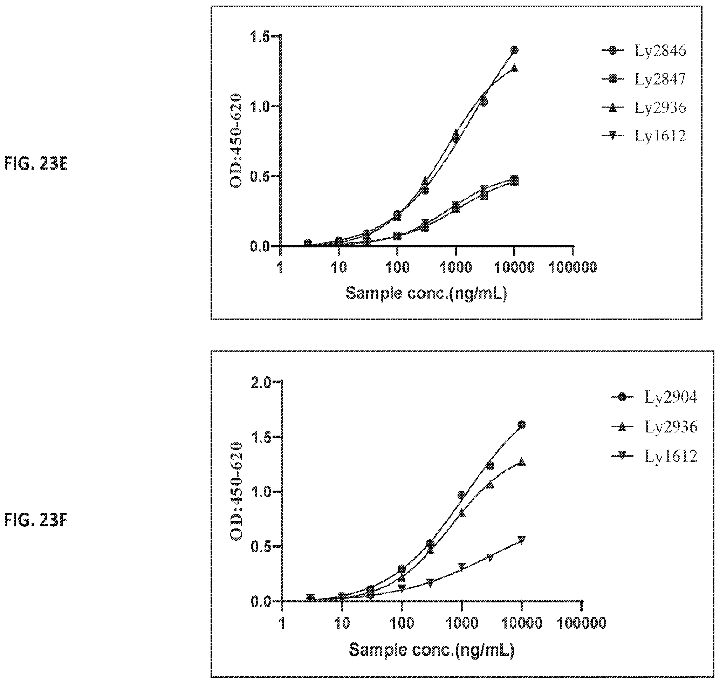

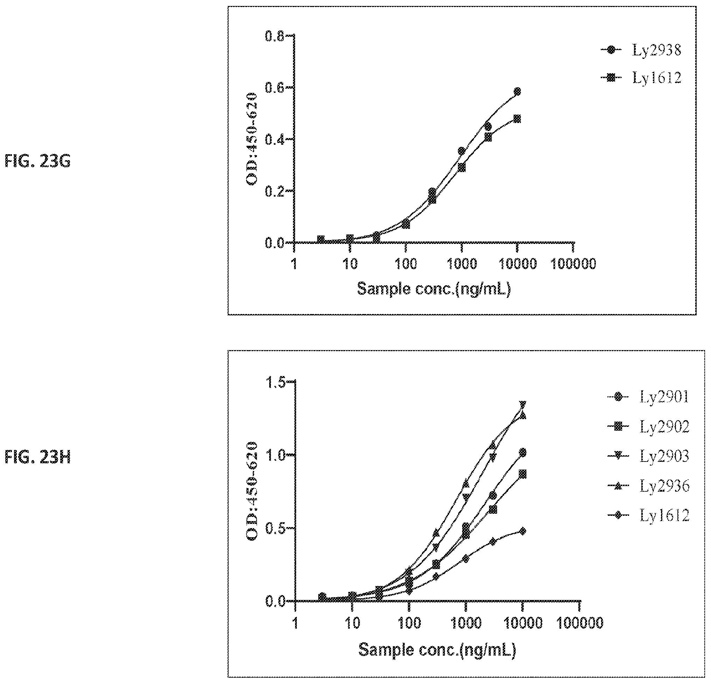

**FIGs. 23A-23H** show B7H3 binding activity of exemplary multi-specific antibodies. **FIG. 23A**: B7H3 binding activity for the clones Ly1963, Ly1965, Ly2122 and parent anti-B7H3 clone Ly1612. **FIG. 23B**: B7H3 binding activity for the clones Ly2600 and Ly1612. **FIG. 23C**: B7H3 binding activity for the clones Ly2936, Ly2937, Ly2939, Ly2940 and Ly1612. **FIG. 23D**: B7H3 binding activity for the clones Ly2823 and Ly1612. **FIG. 23E**: B7H3 binding activity for the clones Ly2846, Ly2847, Ly2936 and Ly1612. **FIG. 23F**: B7H3 binding activity for the clones Ly2904, Ly2936 and Ly1612. **FIG. 23G**: B7H3 binding activity for the clones Ly2938 and Ly1612. **FIG. 23H**: B7H3 binding activity for the clones Ly2901, Ly2902, Ly2903, Ly2936 and Ly1612.

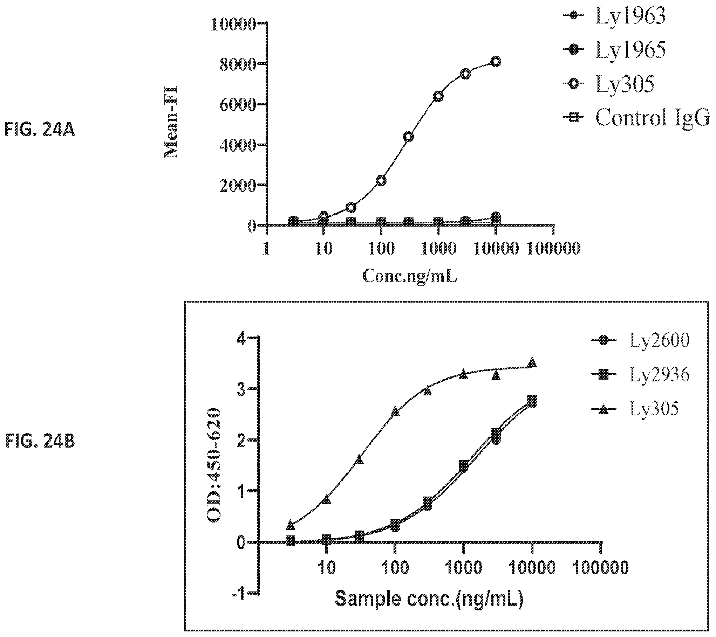

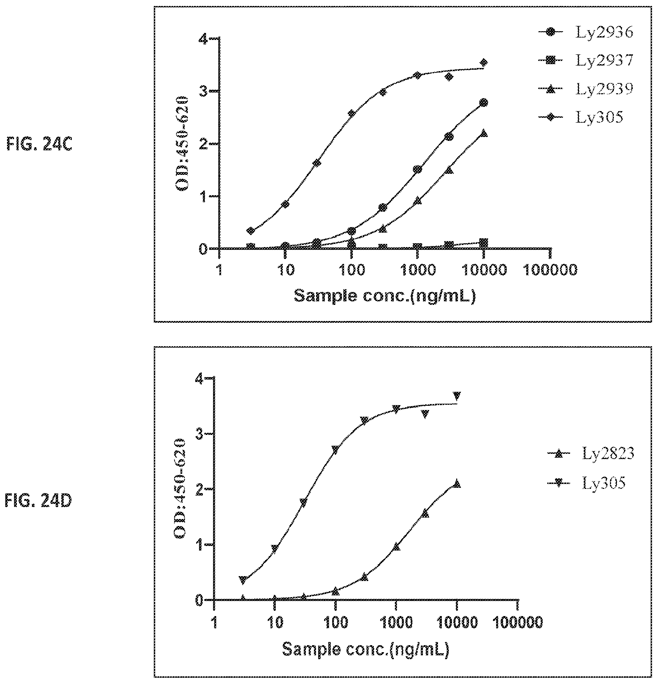

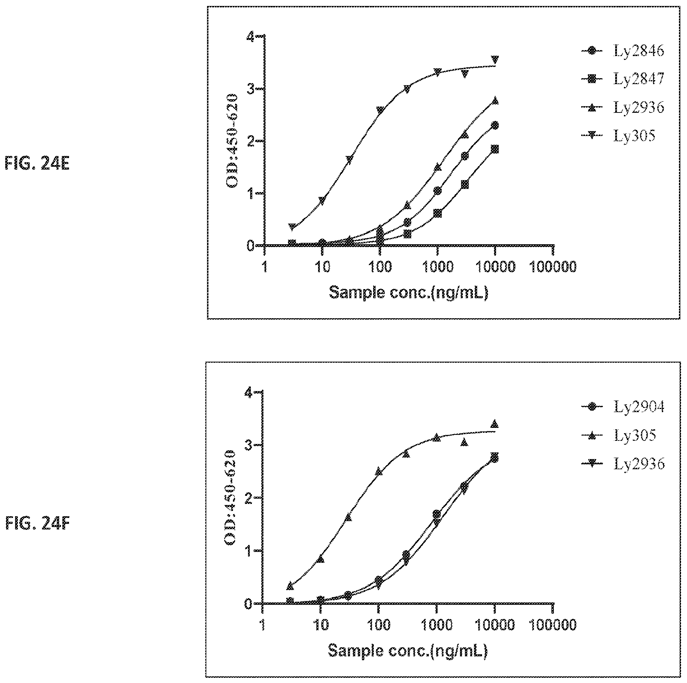

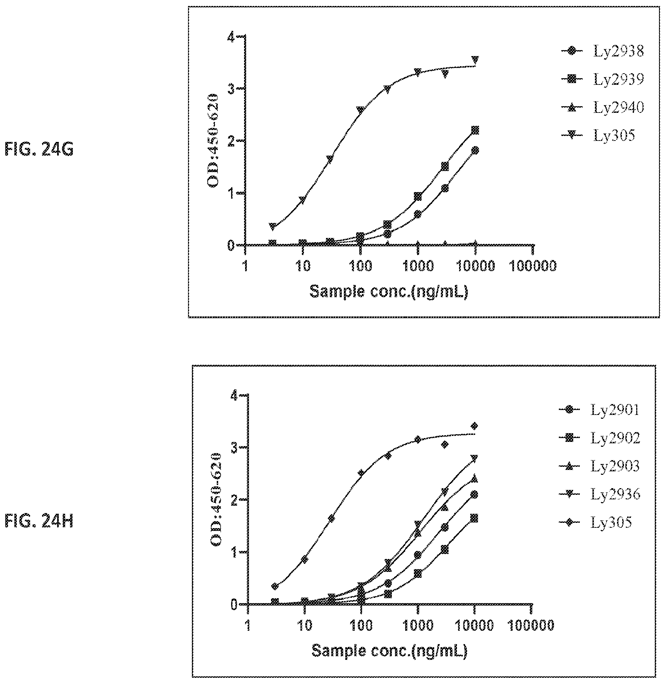

**FIGs. 24A-24H** are charts showing CD3 binding activity of multi-specific antibody clones comprising anti-B7H3 binding moiety. Parental anti-CD3 mAb Ly305 serves as control. **FIG. 24A**: CD3 binding activity for the clones Ly1963 and Ly1965. **FIG. 24B**: CD3 binding activity for the clones Ly2600 and Ly2936. **FIG. 24C**: CD3 binding activity for the clones Ly2936, Ly2937 and Ly2939. **FIG. 24D**: CD3 binding activity for the clone Ly2823. **FIG. 24E**: CD3 binding activity for the clones Ly2846, Ly2847 and Ly2936. **FIG. 24F**: CD3 binding activity for the clones Ly2904 and Ly2936. **FIG. 24G**: CD3 binding activity for the clones Ly2938, Ly2939 and Ly2940. **FIG. 24H**: CD3 binding activity for Ly2901, Ly2902, Ly2903 and Ly2936.

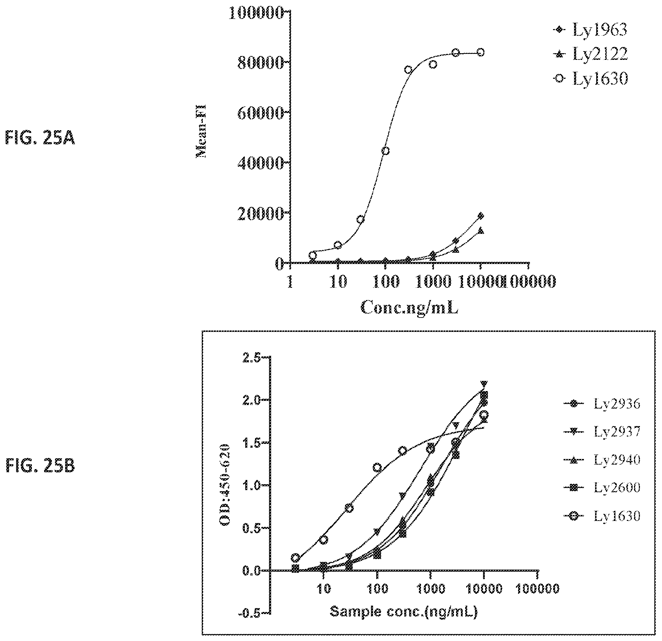

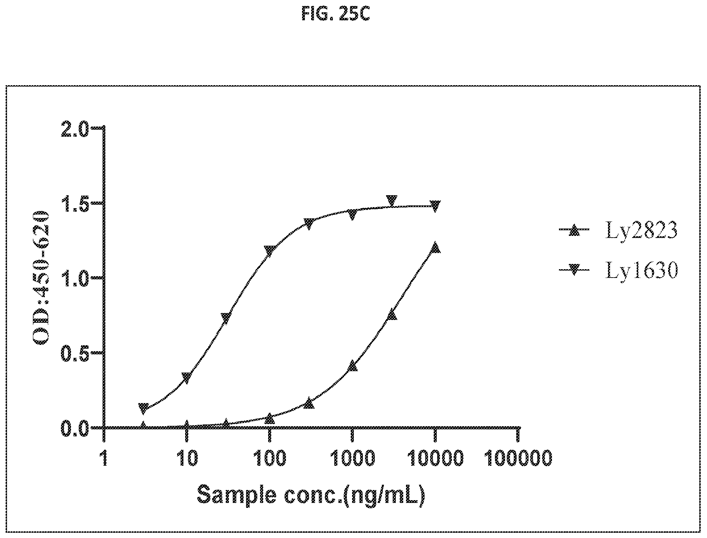

**FIGs. 25A-25C** are charts showing CD137 binding activity of exemplary B7H3 binding multi-specific antibody clones. The parent anti-CD137 clone Ly1630 serves as control. **FIG. 25A**: CD137 binding activity for the clones Ly1963 and Ly2122. **FIG. 25B**: CD137 binding activity for the clones Ly2936, Ly2937, Ly2940 and Ly2600**.** **FIG. 25C**: CD137 binding activity for the clone Ly2823.

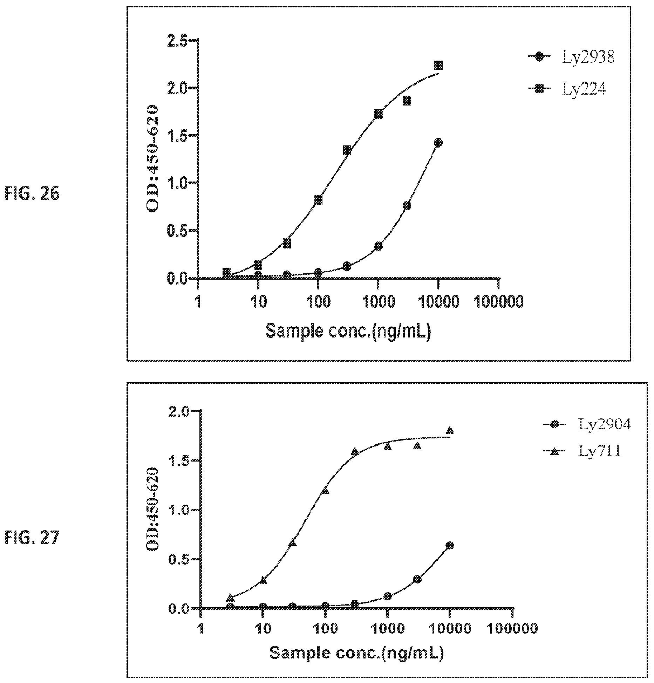

**FIG. 26** is a chart showing CD28 binding activity of exemplary anti-B7H3/CD3/CD28 clone Ly2938.
**FIG. 27** is a chart showing PD-1 binding activity of exemplary anti-B7H3/CD3/PD-1 clones Ly2904.

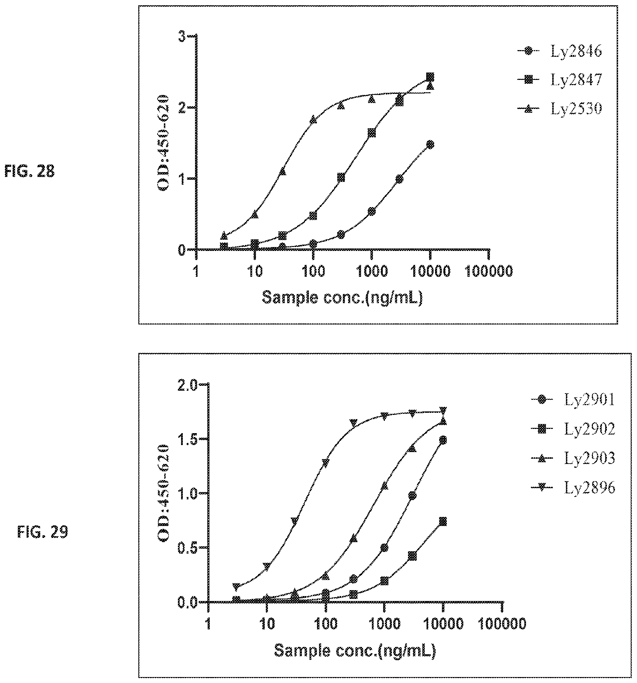

**FIG. 28** is a chart showing PD-L1 binding activity of exemplary anti-B7H3/CD3/PD-L1 clones Ly2846, Ly2847 and parental anti-PD-L1 clone Ly2530.
**FIG. 29** is a chart showing CTLA-4 binding activity of exemplary anti-B7H3/CD3/CTLA4 clones Ly2901, Ly2902, Ly2903 and reference anti-CTLA4 mAb Ly2896.

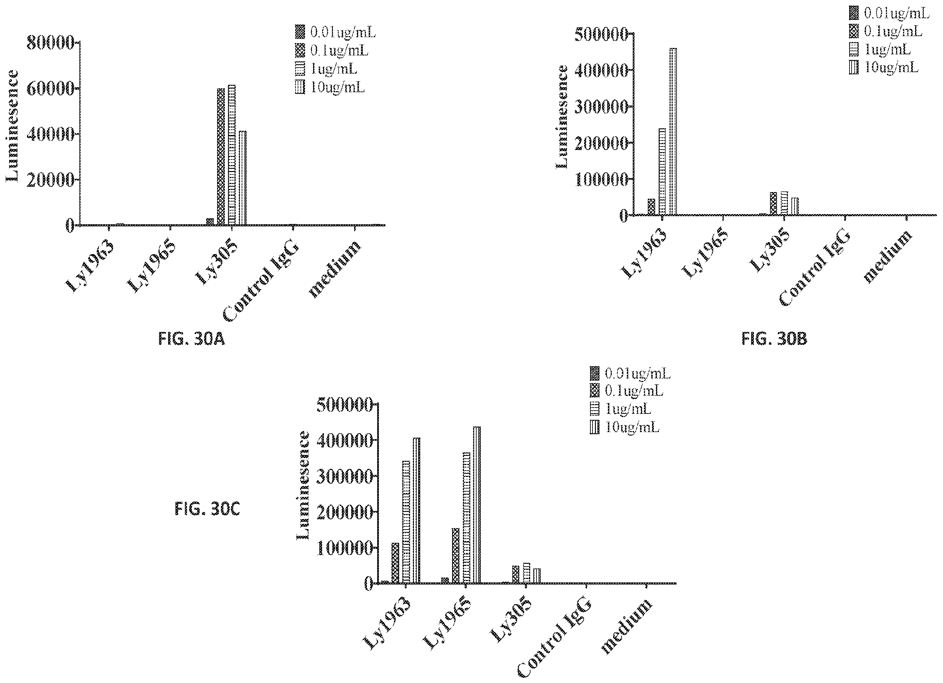

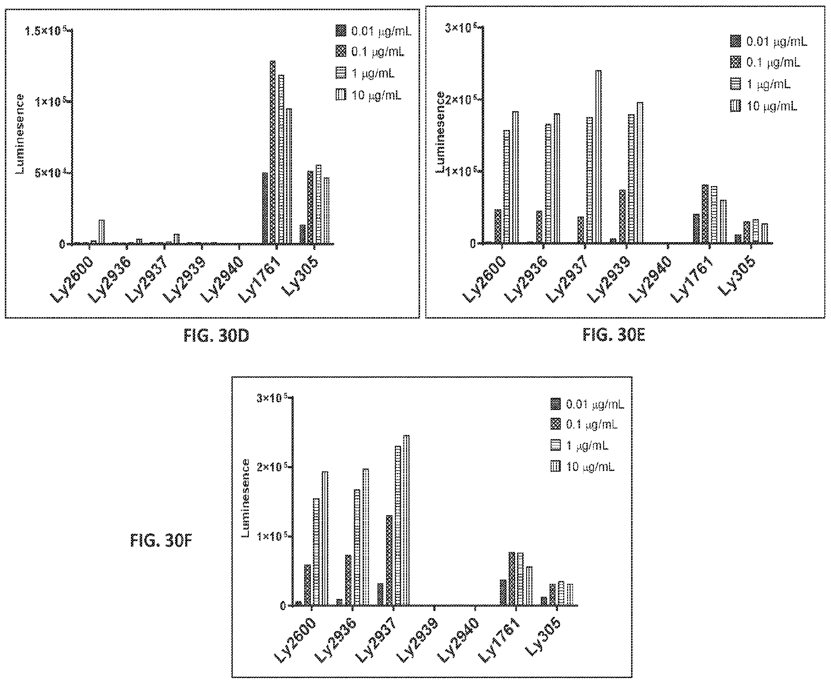

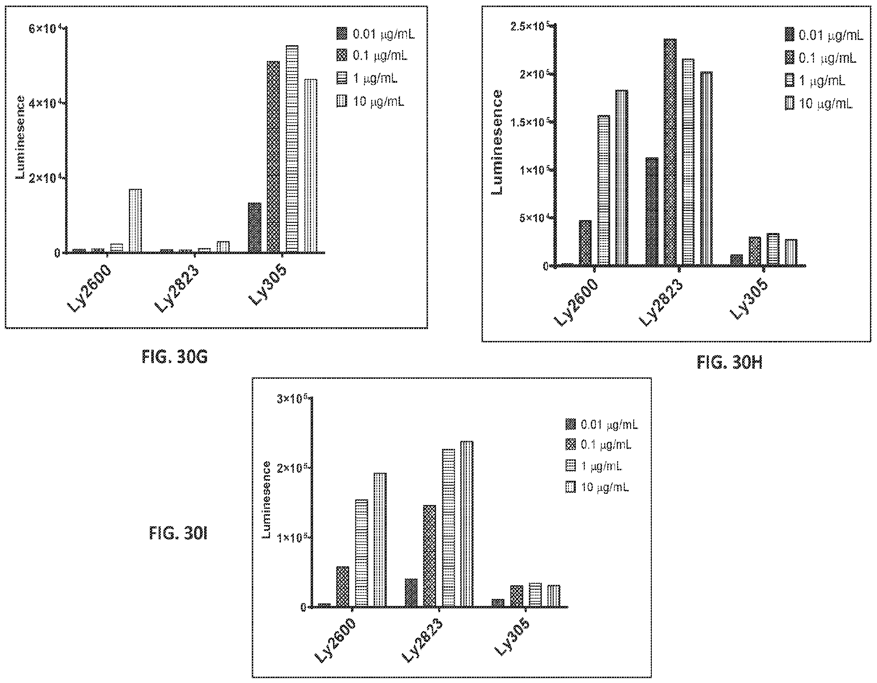

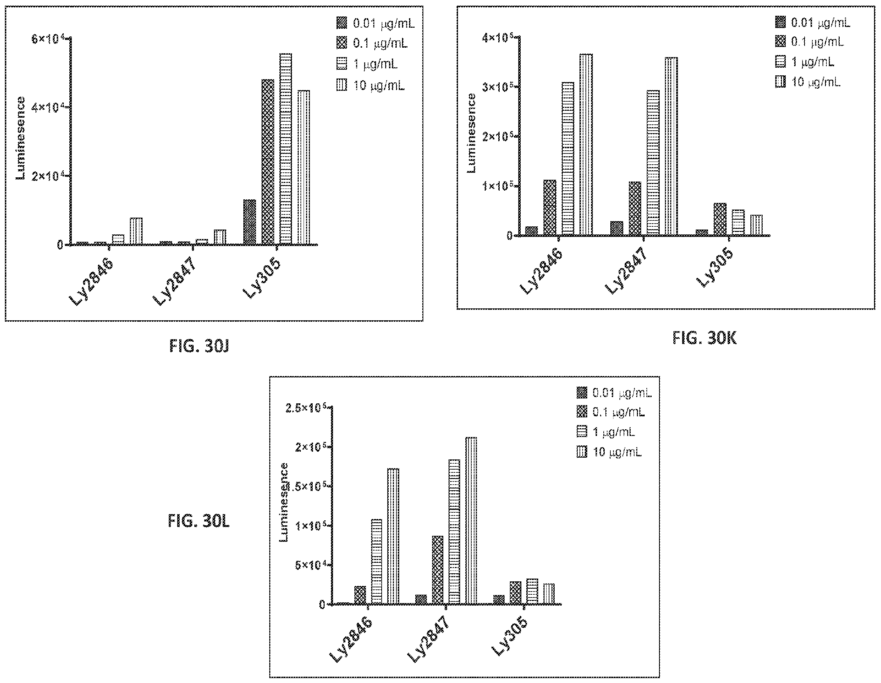

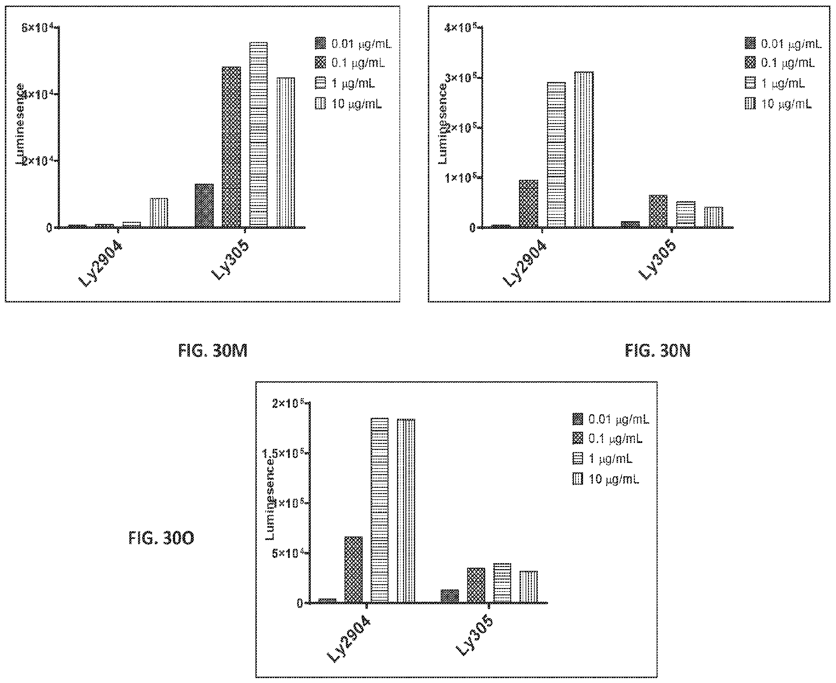

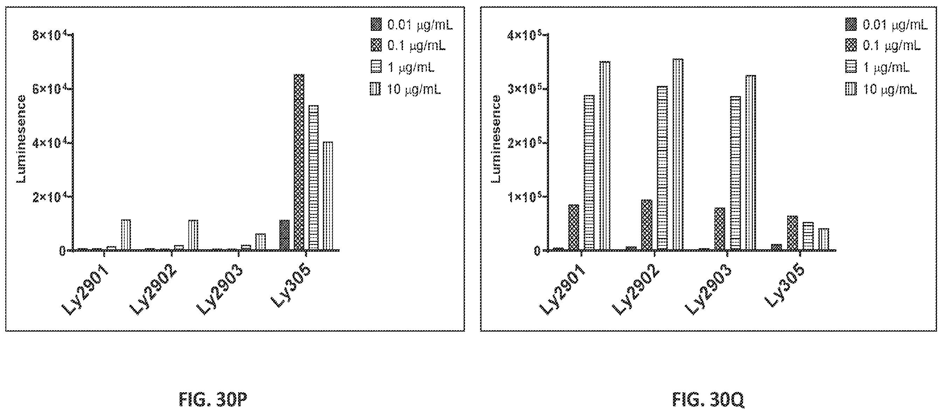

**FIGs. 30A-30Q** include charts showing activation of human CD3 signaling by exemplary multi-specific antibodies as indicated. The agonistic activity of these multi-specific antibodies was evaluated either alone or co-cultured with additional target expressing cells. **FIGs. 30A-30C**: agonistic activity of clones Ly1963, Ly1965 and parental clone Ly305 alone (**A**), co-cultured with CD137-expressing CHO cells (**B**) or B7H3-expressing CHO cells (**C**). **FIGs. 30D-30F**: agonistic activity of clones Ly2600, Ly2936, Ly2937, Ly2939, Ly2940, Ly305 and Ly1761 alone (**D**), co-cultured with B7H3-expressing CHO cells (**E**) or CD137-expressing CHO cells (**F**). **FIGs. 30G-30I**: agonistic activity of clones Ly2600, Ly2823 and Ly305 alone (**G**), co-cultured with B7H3-expressing CHO cells (**H**) or CD137-expressing CHO cells (**I**). **FIGs. 30J-30L**: agonistic activity of clones Ly2846, Ly2847 and Ly305 alone (**J**), co-cultured with B7H3-expressing CHO cells (**K**) or PD-L1-expressing CHO cells (**L**). **FIGs. 30M-30O**: agonistic activity of clones Ly2904 and Ly305 alone (**M**), co-cultured with B7H3-expressing CHO cells (**N**) or PD-1-expressing CHO cells (**O**). **FIGs. 30P-30Q**: agonistic activity of clones Ly2901, Ly2902, Ly2903 and Ly305 alone (**P**), co-cultured with B7H3-expressing CHO cells (**Q**).

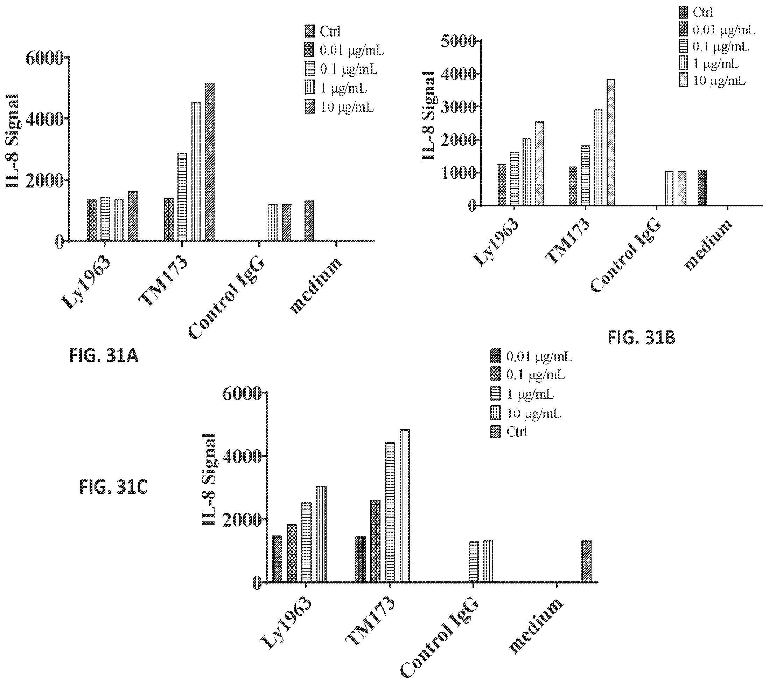

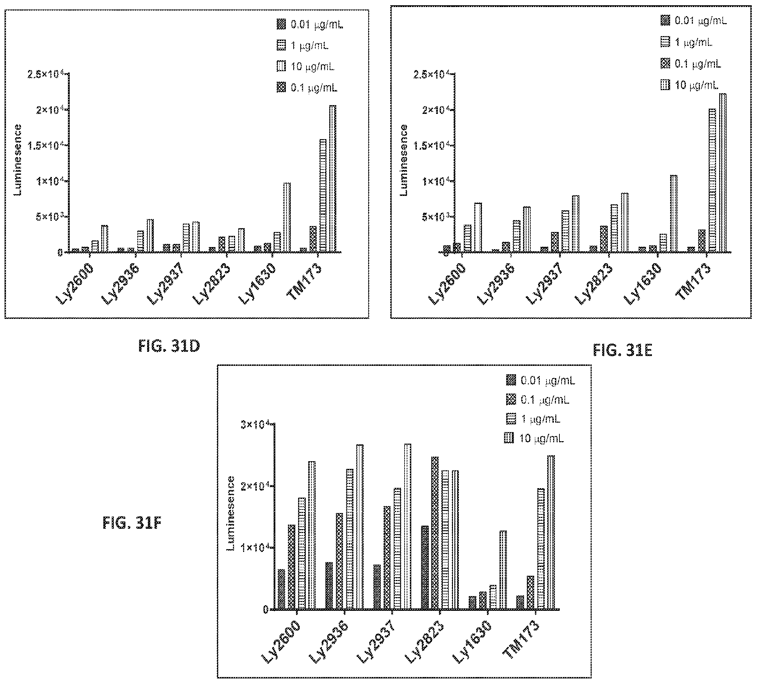

**FIGs. 31A-31Q** include charts showing activation of human CD137 signaling by exemplary multi-specific antibodies as indicated. The agonistic activity of these multi-specific antibodies was evaluated either alone or co-cultured with additional target expressing cells. The level of IL-8 or luminescence produced by reporter cells indicates CD137 activation**.** **FIGs. 31A-31C**: agonistic activity of clones Ly1963 and reference anti-CD137 mAb TM173 alone (**A**), co-cultured with B7H3-expressing CHO cells (**B**) or CD3-expressing cells (**C**). **FIGs. 31D-31F**: agonistic activity of clones Ly2600, Ly2936, Ly2937, Ly2823, parent anti-CD137 clone Ly1630 and reference anti-CD137 mAb TM173 alone (**D**), co-cultured with B7H3-expressing CHO cells (**E**) or CD3-expressing cells (**F**).

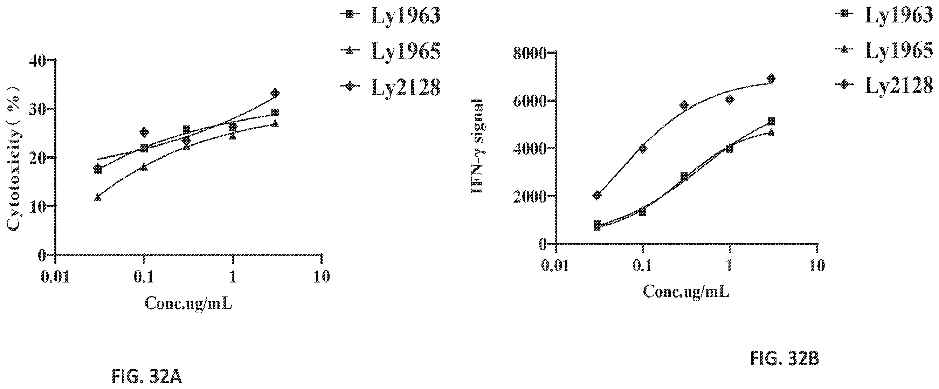

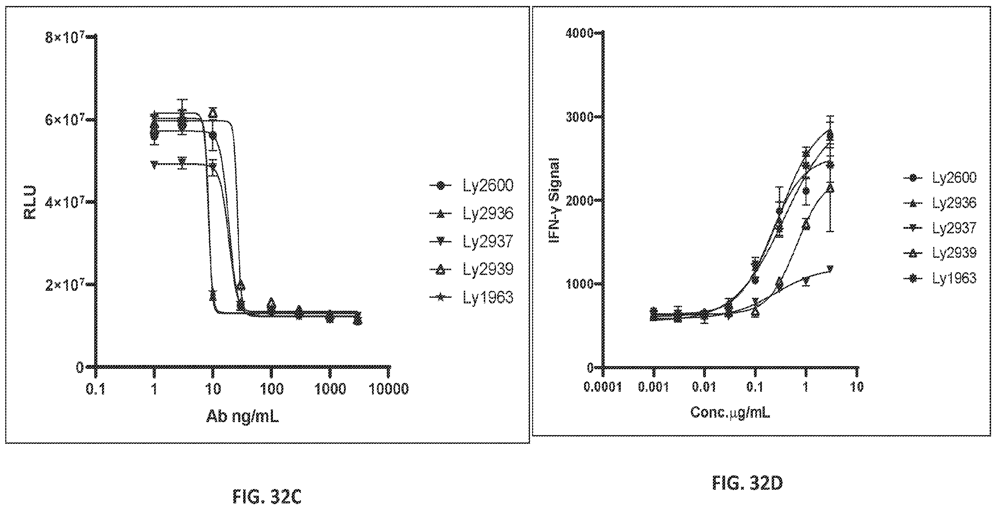

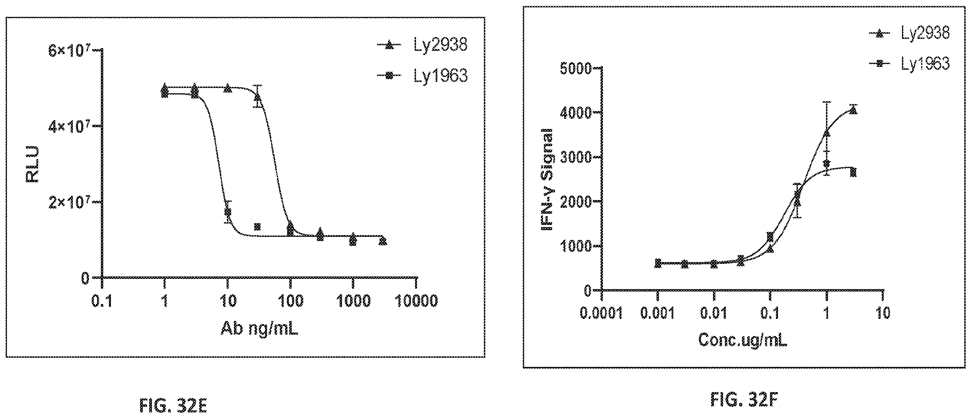

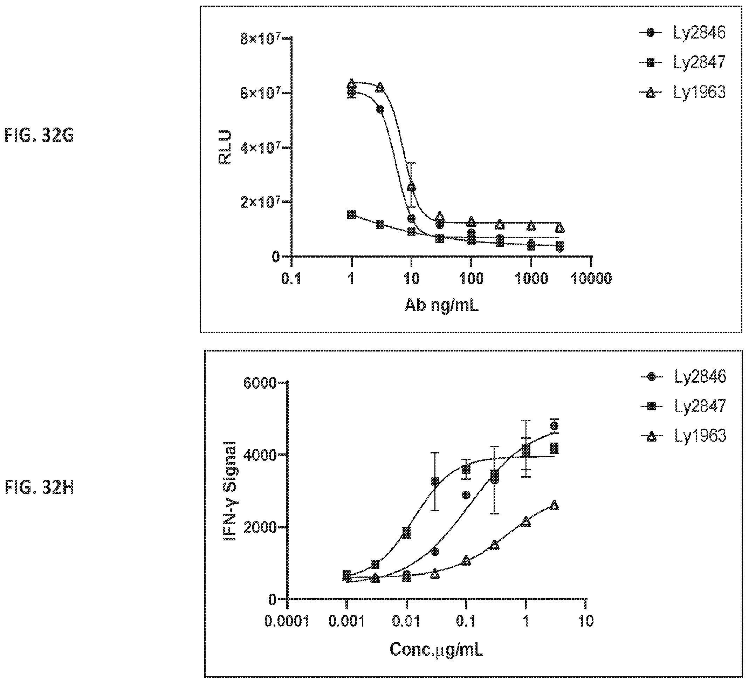

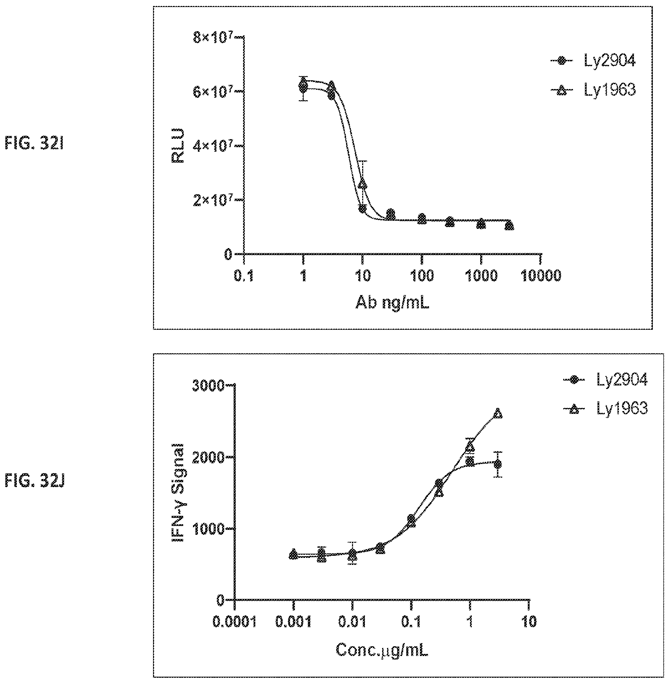

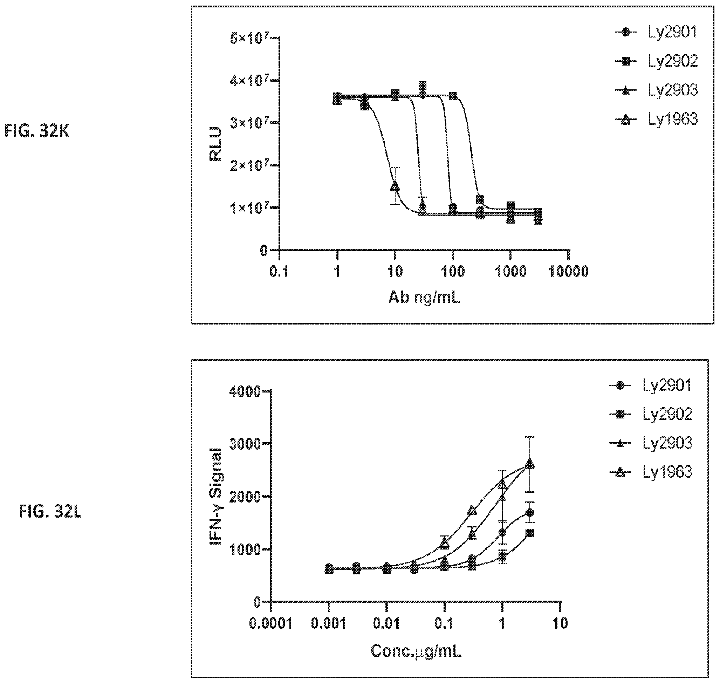

**FIGs. 32A-32L** include diagrams showing cytotoxicity activity and IFN-γ secretion of exemplary multi-specific antibodies. **FIG. 32A:** killing of B7H3-expressing A375 cells by human PBMCs when treated with clones Ly1963, Ly1965 and Ly2128. **FIG. 32B**: IFN-γ secretion by PBMCs when co-cultured B7H3-expressing A375 cells and treated with clones Ly1963, Ly1965 and Ly2128. **FIG. 32C****:** killing of A375-Luc cells by human PBMCs when treated with clones Ly2600, Ly2936, Ly2937, Ly2939 and Ly1963. **FIG. 32D**: IFN-γ secretion by PBMCs when co-cultured A375-Luc cells and treated with clones Ly2600, Ly2936, Ly2937, Ly2939 and Ly1963. **FIG. 32E****:** killing of A375-Luc cells by human PBMCs when treated with clones Ly2938 and Ly1963. **FIG. 32F**: IFN-γ secretion by PBMCs when co-cultured A375-Luc cells and treated with clones Ly2938 and Ly1963. **FIG. 32G****:** killing of A375-Luc cells by human PBMCs when treated with clones Ly2846, Ly2847 and Ly1963. **FIG. 32H**: IFN-γ secretion by PBMCs when co-cultured A375-Luc cells and treated with clones Ly2846, Ly2847 and Ly1963. **FIG. 32I****:** killing of A375-Luc cells by human PBMCs when treated with clones Ly2904 and Ly1963. **FIG. 32J**: IFN-γ secretion by PBMCs when co-cultured A375-Luc cells and treated with clones Ly2904 and Ly1963. **FIG. 32K****:** killing of A375-Luc cells by human PBMCs when treated with clones Ly2901, Ly2902, Ly2903 and Ly1963. **FIG. 32L**: IFN-γ secretion by PBMCs when co-cultured A375-Luc cells and treated with clones Ly2901, Ly2902, Ly2903 and Ly1963.

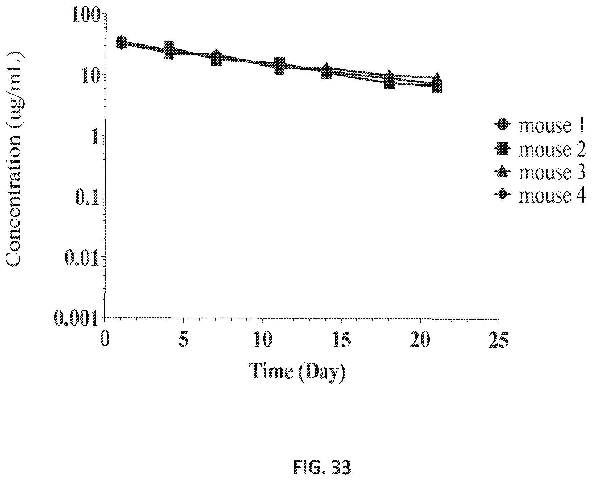

**FIG. 33** is a chart showing serum concentrations of multi-specific antibody Ly1963 comprising anti-B7H3 binding moiety in mice administered intraperitoneally, a single dose of 5 mg/kg.

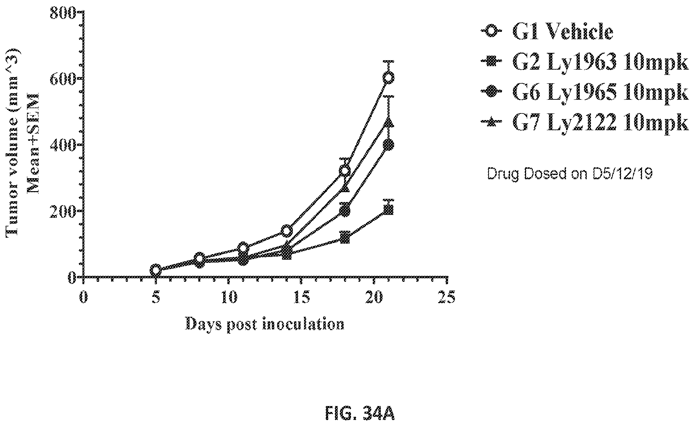

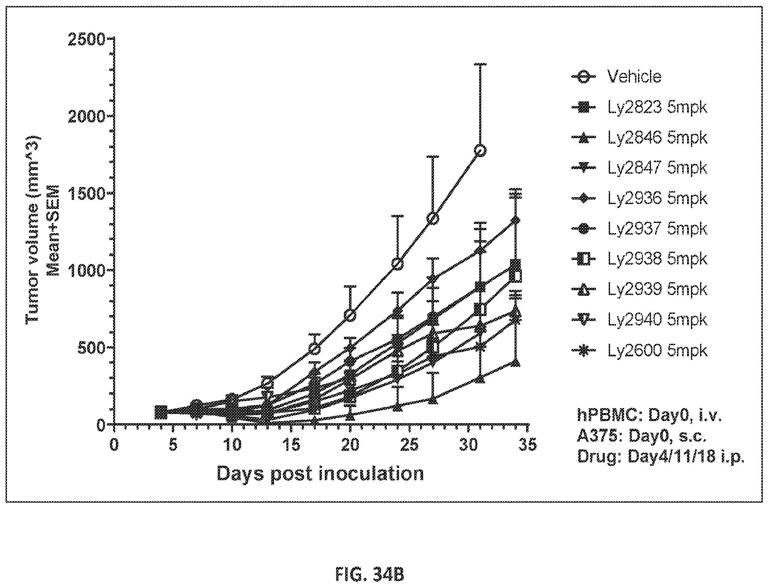

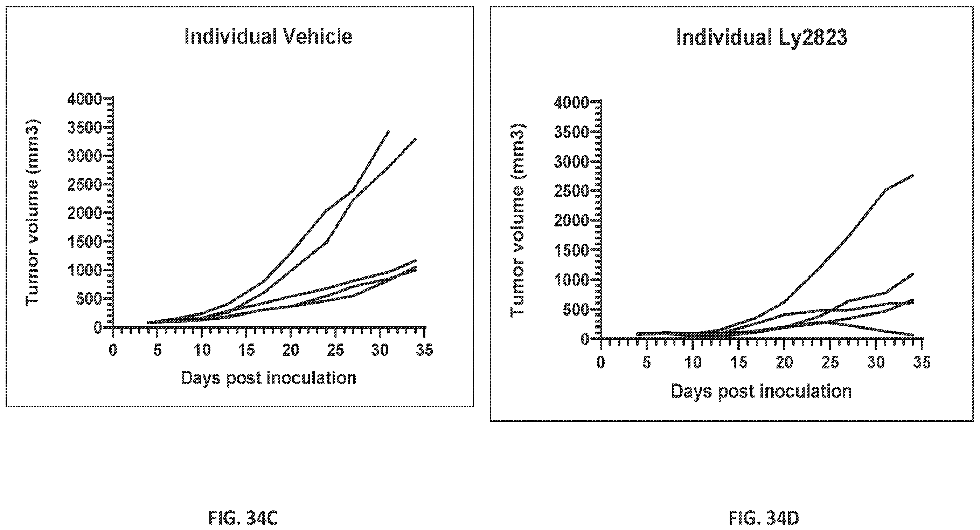

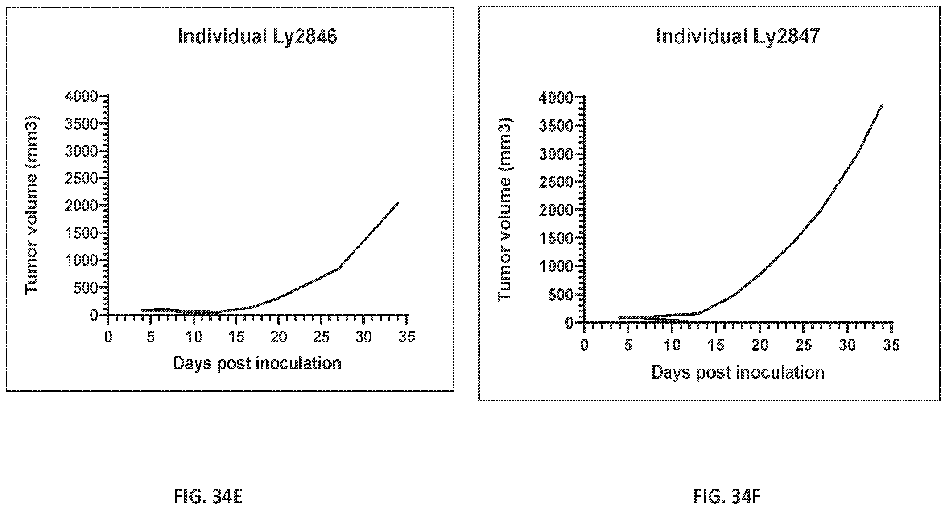

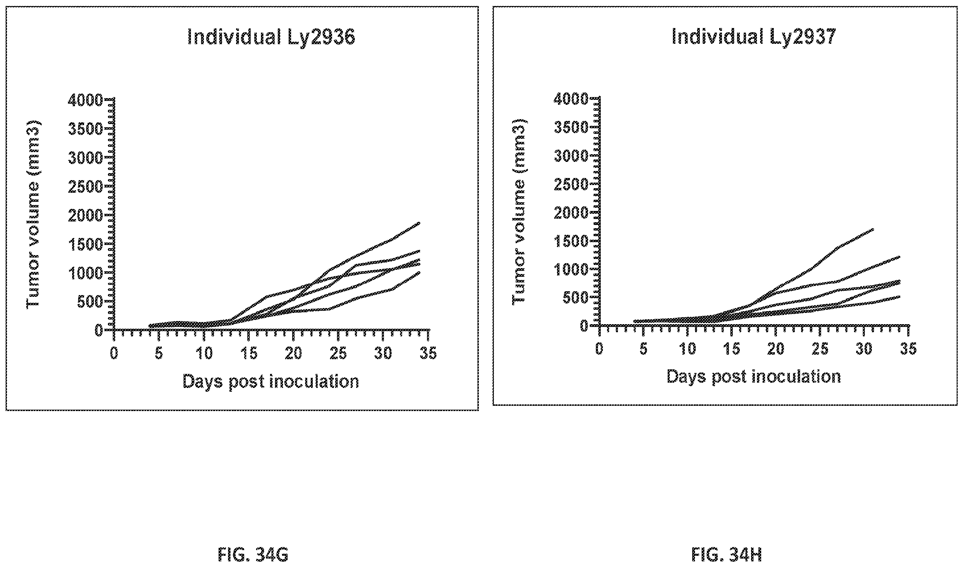

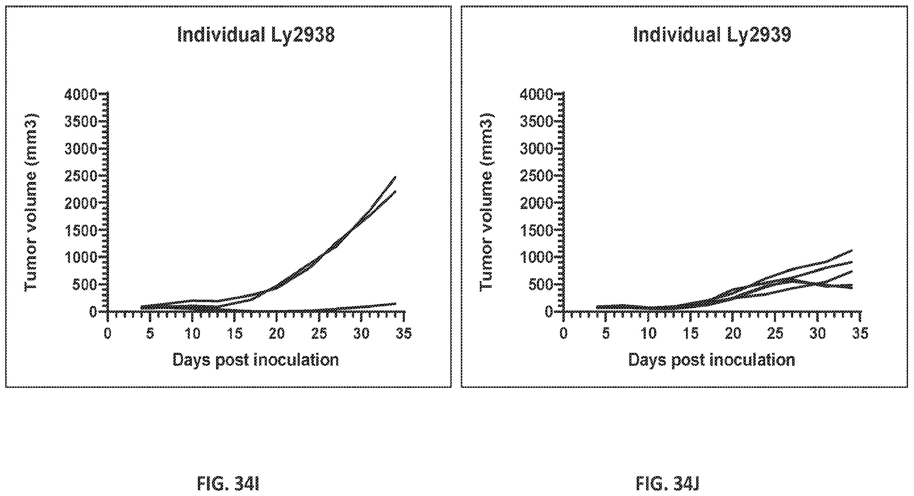

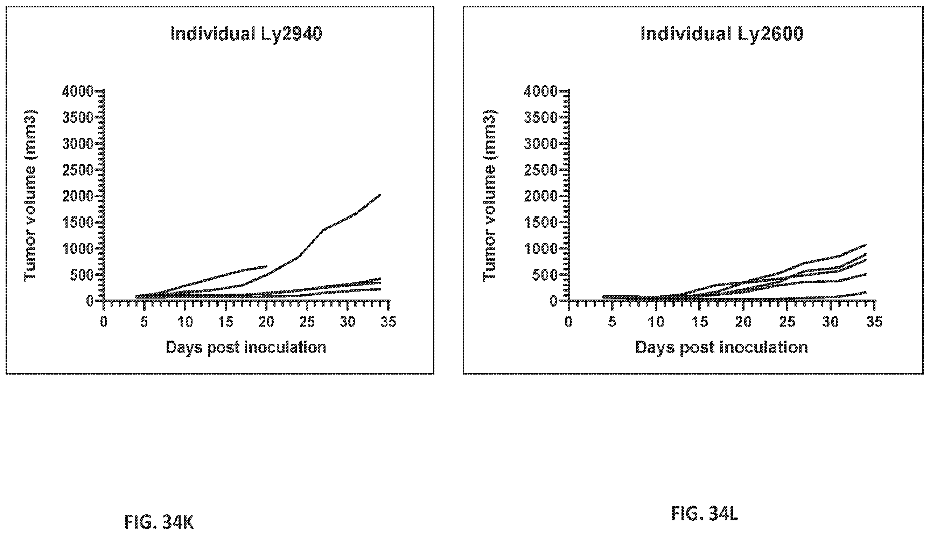

**FIGs. 34A-34L** include diagrams showing anti-tumor activity of exemplary multi-specific antibodies. **FIG. 34A**: anti-tumor activity for Ly1963, Ly1965 and Ly2122 in LL2-huB7H3 bearing mouse models of bone marrow transplanted from CD3 and CD137 knock-in mice. **FIG. 34B-34L**: anti-tumor activities for Ly2823, Ly2936, Ly2937, Ly2600, Ly2846, Ly2847, Ly2938, Ly2939 and Ly2940 in human PBMC engrafted mouse model bearing A375 tumor cells, mean and individual tumor volume of each group were shown in **FIG. 34B** and **FIGs. 34C****-34L** respectively.

### DETAILED DESCRIPTION OF THE INVENTION

The present disclosure provides multi-specific antibodies, optionally in multi-valent form, that utilize a format comprising one or more monovalent Fv fragments. Such monovalent Fv fragment would have relatively low binding affinity to the target antigens, *e.g.*, immune cell receptors. In some instances, the multi-specific antibodies disclosed herein may further comprise one or more high affinity binding moieties specific to a tumor-associated antigen and/or an immune cell receptor such as a T cell activation receptor or a inhibitory receptor (*e.g.*, a checkpoint receptor). As such, the multi-specific antibodies disclosed herein can bind to a target immune receptor at a minimum level so as not to stimulate the target immune receptor in the absence of the target antigens to which the high affinity binding moieties bind, thereby minimizing undesired immune responses and potential side effects.

It was reported herein that multi-specific antibodies comprising a Fv binding moiety would activate the signaling pathway mediated by the immune cell receptor to which the Fv fragment binds only in the presence of additional target antigens, *e.g.*, in tumor microenvironment. In addition, the two or more Fv fragments contained in the multi-specific antibody permit simultaneously binding to two or more immune cell receptors, offering a potential synergistic effect specific to those targets. Alternatively or in addition, utilization of the peptide linkers disclosed herein (including flexible peptide linkers and/or rigid peptide linkers), either taken alone or in combination with engineered Fc fragments, allows for reduction or elimination of undesired Fc effector functions, reduction of undesired aggregation or homodimerization of polypeptides, and/or enhancement of stability of the multi-specific antibodies comprising such.

Without being bound by theory, such multi-specific antibodies may modulate desired immune responses and therapeutic activity via cross-linking the multiple target antigens. The advantageous features of multi-specific antibodies having the specific Fv-containing format as disclosed herein have been demonstrated in multiple exemplary antibodies targeting different antigens. See, e.g., Examples below. As such, such advantageous features are attributable to the specific design of the multi-specific antibody format disclosed herein, i.e., utilizing one or more antigen-binding moieties in Fv fragment format, and optionally the desired peptide linker, as well as engineered Fc fragments. In other words, such advantageous features are not antigen-specific and/or antibody sequence-specific.

As used herein, multi-specific antibodies refer to antibodies capable of binding to two or more target antigens. In some examples, the multi-specific antibody disclosed herein may be a bi-specific antibody, *i.e.*, binding to two different target antigens or binding to two different epitopes of a target antigen. In some examples, the multi-specific antibody disclosed herein may be a tri-specific antibody, *e.g.*, binding to three different target antigens or epitopes. Alternatively, the multi-specific antibody disclosed herein may be a tetra-specific antibody, *e.g.*, binding to four different target antigens or epitopes.

As used herein, multi-valent antibodies refer to antibodies having two or more antigen binding sites. In some examples, the multi-specific antibody disclosed herein may have two antigen binding sites. In some examples, the multi-specific antibody disclosed herein may have three antigen binding sites. In some examples, the multi-specific antibody disclosed herein may have four antigen binding sites.

Accordingly, provided herein are multi-specific antibodies having the specific design as disclosed herein, nucleic acids encoding such, host cells carrying the encoding nucleic acids, methods of producing the multi-specific antibodies, and methods of using such for immune response modulation and treatment of target diseases such as cancer.

### I. Multi-Specific Antibodies

The multi-specific antibodies disclosed herein comprise two or more antigen-binding moieties, which can be linked via peptide linkers (*e.g.*, the flexible peptide linkers or the rigid peptide linkers provided herein) or heavy chain constant region fragments such as Fc fragments as also disclosed herein. At least one antigen-binding moiety in the multi-specific antibody disclosed is in Fv format.

### A. Antigen-Binding Moieties

Each antigen binding moiety in any of the multi-specific antibodies disclosed herein can be an antigen binding moiety in any form, including, but not limited to, intact (*i.e.*, full-length) antibodies, antigen-binding fragments thereof (such as Fab, Fab', F(ab').sub.2, Fv, tribody, triFabs, tandem linked Fabs, a Fab-Fv, tandem linked V domains, tandem linked scFvs, and among other formats), single chain antibodies (scFv antibodies), single domain antibody such as VHH, cross Fab, and tetravalent antibodies.

At least one antigen-binding moiety in the multi-specific antibody disclosed herein is in Fv format. The other antigen-binding moieties in the multi-specific antibodies may be in scFv format, in single domain antibody format (*e.g.*, VHH), in Fab format, in cross Fab format, or a combination thereof. In some examples, at least one antigen binding moiety in the multi-specific antibodies disclosed here is in Fab format. In other examples, at least one antigen binding moiety in the multi-specific antibodies disclosed here is in cross Fab format. Alternatively or in addition, at least one antigen binding moiety in the multi-specific antibodies disclosed here is in scFv format.

Any scFv fragment in a bi-specific or multi-specific antibody may be in V_{H}→V_{L} orientation. Alternatively, it can be in the V_{L}→V_{H} orientation. An antigen-binding moiety in Fab format contains two separate chains, one including a VH-CH1 fragment and the other including a VL-CL fragment. The CL domain may be a Ck domain or a Cl domain.

A single-domain antibody (sdAb), also known as a nanobody, is an antibody fragment consisting of a single monomeric variable antibody domain. In some embodiments, the single-domain antibody can be a heavy chain only (VHH) fragment, which may be derived from a camelid antibody.

A Fab fragment typically contains two separate chains, one chain containing a heavy chain variable region (VH) in connection with a heavy chain constant region fragment such as CH1 and the other chain containing a light chain variable region (VL) in connection with a light chain constant region (*e.g.*, Cκ or Cλ).

A cross Fab fragment has a similar two-chain structure as a Fab fragment but has the connection between VH/VL and the heavy/light chain constant region fragment swapped. A cross Fab fragment includes a first chain containing a VH connected to a light chain constant region (*e.g.*, Cκ or Cλ) and a second chain containing a VL connected to a heavy chain constant region fragment such as CH1.

The antigen-binding moieties in the multi-specific antibodies disclosed herein may specifically bind to multiple immune cell receptors (*e.g.*, immune cell activation receptors such as T cell activation receptors), or specifically bind to at least one immune cell receptor and at least one TAA. Exemplary immune cell receptor antigens include, but are not limited to, CD3, CD28, PD-1, PD-L1, CTLA4, CD47, and a member of the tumor necrosis factor receptor superfamily (TNFRSF). Examples of TNFRSF family members include, but are not limited to, FAS, TNFRSF12A, 4-1BB/CD137, TNFRSF13B, TNFRSF13C, CD27/TNFRSF7, CD30/TNFRSF8, CD40/TNFRSF5, DR3/TNFRSF25, DR4/TNFRSF10A, DR5/TNFRSF10B, DR6/TNFRSF21, GITR/TNFRSF18, HVEM/TNFRSF14, LTBR, OX40/TNFRSF4, TROY/TNFRSF19, RELT/TNFRSF19L, TNFRSF12A, TNFRSF13B, TL1A/TNFSF15, TNFRSF17, TNFRSF1A, TNFRSF11B, RANK/TNFRSF11A, TNFRSF11B, NGFR, EDA2R, and TNFRSF1B.

In some instances, the antigen-binding moieties in the multi-specific antibodies disclosed herein may specifically bind to multiple immune cell receptors such as immune cell checkpoint receptors include, but are not limited to PD-1, PD-L1, and CD47.

A tumor associated antigen (TAA) refers to an antigen produced by tumor cells. TAAs are tumor markers for identifying tumor cells and therapeutic targets for use in cancer therapy. Exemplary TAAs include, but are not limited to, B7H3, CD19, CD20, PSMA, HER2, CEA, BCMA, p53, p53mut, DLL3, MET, EGFR, B7H4, CD20, FGF, HER2, HER3, BCMA, P53mut, MSLN, EPCAM, ROR1, MAGE (*e.g.*, MAGE-A1, MAGE-A3, MAGE-A4, MAGE-A10, MAGE-A12, MAGE-B, or MAGE-C), SSX2, CAGE, GAGE, NY-ESO-1, SPANX-A, SPANX-C, SPANX-D, PRAME, PECAM, ICAM-3 and HLA-DR, PI3K, RAS, RAF, MEK, and ERK.

The Fv fragment (*e.g.*, the central or hidden Fv fragment) in the multi-specific antibody may specifically bind to an immune cell receptor such as a T cell receptor (e.g., a T cell activation receptor or a T cell checkpoint receptor). For example, a central (hidden) Fv fragment in the multi-specific antibodies disclosed herein may specific to CD3, CD137, CD40, CD28. In some instances, the multi-specific antibody may contain a central Fv fragment and a terminal Fv fragment. In some examples, the two Fv fragments may be specific to a pair of immune cell receptors, for example, CD3 and CD28, CD3 and CD137, CD3 and PD-1, CD3 and PD-L1, CD3 and CTLA4, CD137 and PD-1, CD40 and PD-1, CD40 and PD-L1, CD137 and GITR, CD137 and PD-L1, CD137 and CD40, CD137 and OX40. For example, the central Fv fragment may be specific to CD3 and the terminal Fv fragment may be specific to a different immune cell receptor, for example, CD40, CD137, CD28, PD-1, PD-L1, CTLA4, FAS, TNFRSF12A, 4-1BB/CD137, TNFRSF13B, TNFRSF13C, CD27/TNFRSF7, CD30/TNFRSF8, CD40/TNFRSF5, DR3/TNFRSF25, DR4/TNFRSF10A, DR5/TNFRSF10B, DR6/TNFRSF21, GITR/TNFRSF18, HVEM/TNFRSF14, LTBR, OX40/TNFRSF4, TROY/TNFRSF19, RELT/TNFRSF19L, TNFRSF12A, TNFRSF13B, TL1A/TNFSF15, TNFRSF17, TNFRSF1A, TNFRSF11B, RANK/TNFRSF11A, TNFRSF11B, NGFR, EDA2R, or TNFRSF1B. In other examples, the central Fv fragment may be specific to an immune cell receptor such as CD3, CD137, CD40, CD47, or CD28, and the terminal Fv fragment may be specific to a TAA, for example, any one of B7H3, CD19, CD20, MAGE-A4, PRAME, PSMA, HER2, CEA, BCMA, P53mut, DLL3, MET, and EGFR.

In addition to the Fv fragment(s), the multi-specific antibody disclosed herein may further comprise additional antigen-binding moieties, *e.g.*, in scFv format, in a single-domain antibody such as heavy chain only (VHH) format, in Fab format, or in cross Fab format, which can be specific to a different immune cell receptor or a different TAA. Collectively, the multi-specific antibody may bind to two or more immune cell receptors or bind to at least one immune cell receptor and at least one TAA.

In some embodiments, the multi-specific antibody disclosed herein can be a bi-specific antibody that binds two different immune cell receptors or binds to one immune cell receptor (e.g., CD3, CD40, CD137, PD-1, CD47, or PD-L1) and one TAA (*e.g.*, CD19, CD20, MAGE-A4, PRAME, B7H3, CEA, P53mut, PSMA, or HER2). Examples include, but are not limited to, CD3 and B7H3, CD137 and B7H3, CD3 and CD19, CD137 and CD19, CD137 and PD-1, CD40 and PD-1, CD40 and B7H3, CD40 and PD-L1, CD3 and PD-L1, CD3 and CEA, CD3 and P53mut, CD137 and P53mut, CD137 and PD-L1, CD3 and BCMA, CD137 and BCMA, CD3 and PSMA, CD137 and PSMA, CD3 and HER2, CD137 and HER2, CD40 and HER2, B7H3 and CD137, B7H3 and CD3, B7H3 and CD28, CD19 and CD3, CEA and CD137, PD-1 and CD137, PD-1 and CD40, or PMSA and CD3.

In some embodiments, the multi-specific antibody disclosed herein can be a tri-specific antibody that binds a mixture of three different antigens, which can be immune cell receptors and TAAs, *e.g.*, those disclosed herein. In some instances, the tri-specific antibody binds two different immune cell receptors and one TAA. In other instances, the tri-specific antibody binds two different TAAs and one immune cell receptor. Examples of antigen combinations include, but are not limited to: B7H3, CD3 and CD137; CD19, CD3 and CD137; B7H3, CD3 and CD28; CD19, CD3 and CD28; B7H3, CD137 and PD-1; B7H3, CD40 and PD-1; PMSA, CD3 and CD137; CD20, CD3 and CD137; HER2, CD3 and CD137; CEA, CD3 and CD137; BCMA, CD3 and CD137; P53mutant, CD3 and CD137; PD-L1, CD3 and CD137; PD-L1, CD3 and CD28; PD-L1, CD137 and B7H3; PD-L1, CD40 and B7H3; PD-1, CD40 and CD137; PD-1, CD137 and GITR; CD19, CD20, CD3 and CD137; PD-L1, CD40 and CD137; MET, EGFR and CD47; CD19, CD3 and CD137; B7H3, CD3 and PD-1; B7H3, CD3 and PD-L; B7H3, CD3 and CTLA4.

An antibody that "specifically binds" to an antigen or an epitope is a term well understood in the art. A molecule is said to exhibit "specific binding" if it reacts more frequently, more rapidly, with greater duration and/or with greater affinity with a particular target antigen than it does with alternative targets. An antibody "specifically binds" to a target antigen or epitope if it binds with greater affinity, avidity, more readily, and/or with greater duration than it binds to other substances. For example, an antibody that specifically (or preferentially) binds to an antigen (e.g., those listed above) or an antigenic epitope therein is an antibody that binds this target antigen with greater affinity, avidity, more readily, and/or with greater duration than it binds to other antigens or other epitopes in the same antigen. It is also understood with this definition that, for example, an antibody that specifically binds to a first target antigen may or may not specifically or preferentially bind to a second or third target antigen. As such, "specific binding" or "preferential binding" does not necessarily require (although it can include) exclusive binding. In some examples, an antibody that "specifically binds" to a target antigen or an epitope thereof may not bind to other antigens or other epitopes in the same antigen *(i.e.*, only baseline binding activity can be detected in a conventional method). Alternatively, or in addition, the antibodies described herein may specifically binds the human antigen or a fragment thereof as relative to the monkey counterpart, or *vice versa* (*e.g.*, having a binding affinity at least 10-fold higher to one antigen than the other as determined in the same assay under the same assay conditions). In other instances, the antibodies described herein may cross-react to human and a non-human antigen (*e.g.*, monkey), *e.g.*, the difference in binding affinity to the human and the non-human antigen is less than 5-fold, *e.g.*, less than 2-fold, or substantially similar.

In some embodiments, an antigen binding moiety in any of the bi-specific or multi-specific antibodies as described herein has a suitable binding affinity for the target antigen(s) (*e.g.,* an immune cell receptor or a TAA as disclosed herein) or antigenic epitopes thereof. As used herein, "binding affinity" refers to the apparent association constant or K_{A}. The K_{A} is the reciprocal of the dissociation constant (K_{D}). The antibody described herein may have a binding affinity (K_{D}) of at least 10⁻⁵, 10⁻⁶, 10⁻⁷, 10⁻⁸, 10⁻⁹, 10⁻¹⁰ M, or lower for the target antigen or antigenic epitope. An increased binding affinity corresponds to a decreased K_{D}. Higher affinity binding of an antibody for a first antigen relative to a second antigen can be indicated by a higher K_{A} (or a smaller numerical value K_{D}) for binding the first antigen than the K_{A} (or numerical value K_{D}) for binding the second antigen. In such cases, the antibody has specificity for the first antigen (*e.g.*, a first protein in a first conformation or mimic thereof) relative to the second antigen (*e.g.*, the same first protein in a second conformation or mimic thereof; or a second protein). Differences in binding affinity (*e.g.*, for specificity or other comparisons) can be at least 1.5, 2, 3, 4, 5, 10, 15, 20, 37.5, 50, 70, 80, 91, 100, 500, 1000, 10,000 or 10⁵-fold. In some embodiments, any of the antibodies may be further affinity matured to increase the binding affinity of the antibody to the target antigen or antigenic epitope thereof.

Binding affinity (or binding specificity) can be determined by a variety of methods including equilibrium dialysis, equilibrium binding, gel filtration, ELISA, surface plasmon resonance, or spectroscopy (*e.g.*, using a fluorescence assay). Exemplary conditions for evaluating binding affinity are in HBS-P buffer (10 mM HEPES pH7.4, 150 mM NaCl, 0.005% (v/v) Surfactant P20). These techniques can be used to measure the concentration of bound binding protein as a function of target protein concentration. The concentration of bound binding protein ([Bound]) is generally related to the concentration of free target protein ([Free]) by the following equation:

### [Bound] = [Free]/(Kd+[Free])

It is not always necessary to make an exact determination of K_{A}, though, since sometimes it is sufficient to obtain a quantitative measurement of affinity, *e.g.*, determined using a method such as ELISA or FACS analysis, is proportional to K_{A}, and thus can be used for comparisons, such as determining whether a higher affinity is, *e.g.*, 2-fold higher, to obtain a qualitative measurement of affinity, or to obtain an inference of affinity, *e.g.*, by activity in a functional assay, *e.g.*, an *in vitro* or *in vivo* assay.

### Exemplary Parent Antibodies

The antigen binding moieties of a multi-specific antibody as disclosed herein may be derived from the parent antibody specific to any of the immune cell receptor or TAA target antigens as disclosed herein. Exemplary parent antibodies, from which any of the antigen binding moieties are derived, are provided in **Table 1** below (heavy chain and light chain CDRs based on the Kabat scheme are identified in boldface).

As used herein, an antigen binding moiety in a multi-specific antibody "derived from" a parent antibody means that the parent antibody is used as a starting material for making one antigen binding moiety in the multi-specific antibody. The antigen binding moiety may comprise the same heavy chain and/or light chain CDRs as those of the parent antibody. Two antibodies having the same V_{H} and/or V_{L} CDRs means that their CDRs are identical when determined by the same approach (*e.g.*, the Kabat definition, the Chothia definition, the AbM definition, and/or the contact definition as known in the art).

In some instances, an antigen binding moiety derived from a parent antibody may be a functional variant of the parent antibody. Such functional variants are substantially similar to the reference antibody, both structurally and functionally. A functional variant comprises substantially the same V_{H} and V_{L} CDRs as the reference antibody. For example, it may comprise only up to 5 (*e.g.*, 4, 3, 2, or 1) amino acid residue variations in the total heavy chain CDR regions of the reference antibody and/or comprise only up to 5 (*e.g.*, 4, 3, 2, or 1) amino acid residue variations in the total light chain CDR regions of the reference antibody. In some examples, the functional variant may comprise up to 8 (*e.g.*, 7, 6, 5, 4, 3, 2, or 1) amino acid residue variations in the total heavy and light chain CDRs relative to those of the reference antibody. Such functional variants may bind the same epitope of B7H3 with substantially similar affinity (*e.g.*, having a K_{D} value in the same order). Alternatively, or in addition, the amino acid residue variations are conservative amino acid residue substitutions as disclosed herein.

In some embodiments, an antigen binding moiety in a multi-specific antibody as disclosed herein may comprise heavy chain CDRs that are at least 80% (*e.g.*, 85%, 90%, 95%, or 98%) sequence identity, individually or collectively, as compared with the V_{H} CDRs of the corresponding parent antibody. Alternatively, or in addition, the antigen binding moiety may comprise light chain CDRs that are at least 80% (*e.g.*, 85%, 90%, 95%, or 98%) sequence identity, individually or collectively, as compared with the V_{L} CDRs as the parent antibody.

In other embodiments, the antigen binding moiety may comprise heavy chain CDRs that are at least 80% (*e.g.*, 85%, 90%, 95%, or 98%) sequence identity, individually or collectively, as compared with the V_{H} CDRs of the corresponding parent antibody. Alternatively, or in addition, the antigen binding moiety may comprise light chain CDRs that are at least 80% (*e.g.*, 85%, 90%, 95%, or 98%) sequence identity, individually or collectively, as compared with the V_{L} CDRs as the parent antibody.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the invention. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (*e.g.*, XBLAST and NBLAST) can be used.

Alternatively, or in addition, the amino acid residue variations can be conservative amino acid residue substitutions. As used herein, a "conservative amino acid substitution" refers to an amino acid substitution that does not alter the relative charge or size characteristics of the protein in which the amino acid substitution is made. Variants can be prepared according to methods for altering polypeptide sequence known to one of ordinary skill in the art such as are found in references which compile such methods, *e.g.* Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

### B. Format of Multi-Specific Antibodies

The multi-specific antibodies disclosed herein are multi-chain molecules comprising two or more antigen binding moieties, at least one of which is in Fv format. The other antigen binding moieties in the multi-specific antibodies, if any, may be in a suitable format, for example, scFv format, a single-domain antibody format (e.g., a heavy chain antibody or VHH), Fab, or cross Fab), or a combination thereof. In other examples, one or more antigen-binding moiety used for constructing the multi-specific antibodies disclosed herein may be in BCR or TCR format.

In some embodiments, the multi-specific antibody may comprise one Fv antigen binding moiety, and one or more additional antigen binding moieties in scFv and/or Fab format. For example, the multi-specific antibody may comprise one Fv fragment and two Fab fragments. The two Fab fragments may have different structures for binding to different antigens or different epitopes of a target antigen. Alternatively, the two Fab fragments may be identical. In other examples, the multi-specific antibody may comprise one Fv fragment and one Fab fragment.

In other embodiments, the multi-specific antibody may comprise two Fv antigen binding moieties specific to different target antigens and optionally one or more additional antigen binding moieties in scFv and/or Fab format. In some instances, the multi-specific antibody may comprise two Fv fragment and two Fab fragments. The two Fab fragments may have different structures for binding to different antigens or different epitopes of a target antigen. Alternatively, the two Fab fragments may be identical. In other examples, the multi-specific antibody may comprise two Fv fragment and one Fab fragment.

The multiple antigen binding moieties in the multi-specific antibodies disclosed herein may be linked via peptide linkers, such as the flexible peptide linkers and rigid peptide linkers disclosed herein.

In some instances, two antigen-binding moieties may be connected via a heavy chain constant region fragment, for example, a fragment comprising the hinge domain, CH2, and CH3, taking advantage of the disulfide bond formation feature of such fragments (*e.g.*, at the hinge domain) to form dimers between two polypeptides of the multi-specific antibody. In some instances, the multi-specific antibodies disclosed herein may further comprise a heavy chain constant region fragment such as a Fc fragment (typically comprises the CH2 and CH3 of a heavy chain constant chain) to facilitate formation of a whole antibody molecule via disulfide bond formation and/or to further modulate immune responses via its Fc receptor binding activity.

### (a) Fv binding moiety

As used herein, an Fv fragment refers to an antigen-binding fragment containing a heavy chain variable domain (V_{H}) and a light chain variable domain (V_{L}), which are on separate chains. See, e.g., **FIG. 1A**. In some embodiments, an Fv fragment in a multi-specific antibody disclosed herein may be located in the middle of the antibody molecule, *i.e.*, each of V_{H} and V_{L} fragment is in the middle of a polypeptide with its N-terminus and C-terminus linked to other components of the antibody (*e.g.*, other antigen binding moieties). Such a Fv fragment is also called a central or hidden Fv as disclosed herein. See, e.g., **FIG. 1B**. In other embodiments, an Fv fragment in a multi-specific antibody disclosed herein may be located at one terminal of the antibody molecule. Each of V_{H} and V_{L} fragment is in the middle of a polypeptide with one of the N-terminus and C-terminus linked to other components of the antibody such as other antigen binding moieties. The other terminus may be the terminus of the whole polypeptide or may be linked to a peptide linker but not to any other antigen binding moieties or heavy chain constant region fragments such as Fc fragments. Such a Fv fragment is also called a terminal Fv as disclosed herein. See, *e.g.*, **FIG. 1C**.

In some instances, the multi-specific antibody disclosed herein contain one central Fv fragment, which may be specific to an immune cell receptor of interest as those disclosed herein (*e.g.*, CD3, CD137, CD40, GITR, OX40, CD47, PD-1, or CD28). In some instances, the multi-specific antibody disclosed herein may contain one terminal Fv fragment, which may be specific to an immune cell receptor of interest as those disclosed herein (*e.g.*, CD3, CD137, CD40, GITR, OX40, CD47, CD28, PD-1, PD-L1, CTLA4). In some instances, the multi-specific antibody disclosed herein may comprise at least two Fv fragments (*e.g.*, 2 Fv fragments), one being a central Fv and the other one being a terminal Fv. See, *e.g.*, **FIG. 1D**. The two Fv fragments may be specific to two different immune cell receptors, *e.g.*, two immune cell receptors that provide complementary signaling for optimal immune modulation. Alternatively, one of the two Fv fragments (e.g., the central Fv) may be specific to an immune cell receptor of interest as those disclosed herein (*e.g.*, CD3, CD137, CD40, GITR, OX40, CD47, or CD28) and the other one (e.g., the terminal Fv) may be specific to a TAA such as those provided herein. Alternatively, the other one (e.g., the terminal Fv) may be specific to a second immune cell receptor such as those disclosed herein.

It is expected that a Fv fragment in the multi-specific antibody, specifically a central Fv, would have relatively low binding affinity to a target antigen (*e.g.*, an immune cell receptor such as those disclosed herein, *e.g.*, CD3, CD137, CD40, GITR, OX40, CD47, PD-1, PD-L1, CTLA4 or CD28) in the absence of other antigens to which the other antigen-binding moieties in the same multi-specific antibody binds. Without being bound by theory, the presence of the other antigens may rescue or enhance Fv fragment binding affinity and activation potency, in some cases, to a similar or even higher level as compared with that of their parent mAb clones. As demonstrated in the following examples, the terminal Fv shows at least 2-10 fond decreased binding affinity or activation potency alone compared with their parent mAb clones. As for the central Fv, it presents even lower 10-100 fond decreased or null binding affinity or activation potency alone compared with that of their parent mAb clones. However, when co-cultured with other target antigens expressing cells, the binding affinity and activation potency of these central or terminal Fv is enhanced, in some cases, to a similar or higher level than that of their parent mAb clones.

Adopting this approach in designing the multi-specific antibodies disclosed herein would provide one or more binding arm(s) to one or more target immune cell receptor(s) or TA(s) of interest but avoid eliciting an immune response in the absence of high affinity target antigen, which may be accompanied with undesired clinical side effects. The multi-specific antibodies would create synthetic biology to activate the signaling pathway mediated by the immune cell receptor to which the Fv fragment binds only in the presence of additional target antigens in tumor microenvironment via avidity driven cross-linking of the multiple target antigens, to achieve therapeutic efficacy.

### (b) Peptide Linkers

A peptide linker may be located between two fragments in a polypeptide of a multi-specific antibody as disclosed herein, for example, between the V_{H} and V_{L} portions in a scFv fragment, between the V_{H} or V_{L} of a Fv fragment and a chain of another antigen binding moiety, or between the the VH or VL of a Fv fragment and a heavy chain constant region fragment such as a Fc fragment.

Any of the peptide linkers described herein can comprise naturally occurring amino acids and/or non-naturally occurring amino acids. Naturally occurring amino acids include alanine (Ala), arginine (Arg), asparagine (Asn), aspartic acid (Asp), cysteine (Cys), glutamic acid (Glu), glutamine (Gin), glycine (Gly), histidine (His), isoleucine (He), leucine (Leu), lysine (Lys) methionine (Met), ornithine (Orn), phenylalanine (Phe), proline (Pro), serine (Ser), threonine (Thr), tryptophan (Trp), tyrosine (Tyr), and valine (Val). Non-naturally occurring amino acids can include protected amino acids such as naturally occurring amino acids protected with groups such as acetyl, formyl, tosyl, nitro and the like. Non-limiting examples of non-naturally occurring amino acids include azidohomoalanine, homopropargylglycine, homoallylglycine, p-bromophenylalanine, p-iodophenylalanine, azidophenylalanine, acetylphenylalanine or ethynylephenylalanine, amino acids containing an internal alkene such as trans-crotylalkene, serine allyl ether, allyl glycine, propargyl glycine, vinyl glycine, pyrrolysine, N-sigma-o-azidobenzyloxycarbonyl-L-Lysine (AzZLys), N-sigma-propargyloxycarbonyl-L-Lysine, N-sigma-2-azidoethoxycarbonyl-L-Lysine, N-sigma-tert-butyloxycarbonyl-L-Lysine (BocLys), N-sigma-allyloxycarbonyl-L-Lysine (AlocLys), N-sigma-acetyl-L-Lysine (AcLys), N-sigma-benzyloxycarbonyl-L-Lysine (ZLys), N-sigma-cyclopentyloxycarbonyl-L-Lysine (CycLys), N-sigma-D-prolyl-L-Lysine, N-sigma-nicotinoyl-L-Lysine (NicLys), N-sigma-N-Me-anthraniloyl-L-Lysine (NmaLys), N-sigma-biotinyl-L-Lysine, N- sigma-9-fluorenylmethoxycarbonyl-L-Lysine, N-sigma-methyl-L-Lysine, N-sigma-dimethyl-L- Lysine, N-sigma-multimethyl-L-Lysine, N-sigma-isopropyl-L-Lysine, N-sigma-dansyl-L-Lysine, N-sigma-o,p-dinitrophenyl-L-Lysine, N-sigma-p-toluenesulfonyl-L-Lysine, N-sigma-DL-2-amino- 2carboxyethyl-L-Lysine, N-sigma-phenylpyruvamide-L-Lysine, N-sigma-pyruvamide-L-Lysine, azidohomoalanine, homopropargylglycine, homoallylglycine, p-bromophenylalanine, p-iodophenylalanine, azidophenylalanine, acetylphenylalanine or ethynylephenylalanine, amino acids containing and an internal alkene such as trans-crotylalkene, serine allyl ether, allyl glycine, propargyl glycine, and vinyl glycine.

The peptide linkers provided herein may contain about 5-160 amino acid residues, for example, about 10-120 amino acid residues, about 10-100 amino acid residues, about 10-80 amino acid residues, about 10-60 amino acid residues, about 10-50 amino acid residues, about 10-40 amino acid residues, about 10-30 amino acid residues, or about 10-20 amino acid residues.

In some embodiments, the peptide linker can be a flexible peptide linker, which typically contains small, flexible amino acid residues so as to connect various domains in the multi-specific antibody without affecting their binding activity. In some examples, the flexible peptide linker is a Gly-rich linker, for example, comprising the motif of (GxS)n, in which X is an integer of 1, 2, 3, 4, 5, or 6 and n is an integer of 1, 2, 3, 4, 5, 6, 7, 8, 9, or 10. Exemplary flexible peptide linkers are provided in **Table 1** below, any of which can be used for constructing the multi-specific antibodies disclosed herein.

In some embodiments, the peptide linker may be a so-called rigid peptide linker, which contains at least one cysteine residues (*e.g.*, 1 or 2 cysteine residues) such as it can form disulfide bonds with another rigid peptide linker. Use of one or more pairs of rigid peptide linkers in the multi-specific antibodies disclosed herein can facilitate dimer formation across the multiple polypeptides of the antibody via disulfide formation, thereby forming an intact multi-chain antibody molecule.

In some instances, the rigid peptide linker may be derived from a hinge domain of an IgG molecule (positions 216-230 or a fragment thereof, following the EU numbering system), for example, an IgG1 molecule, taking advantage of the disulfide bond formation capacity of such fragments. In some examples, such a peptide linker is a fragment of a wild-type IgG molecule (*e.g.*, a human IgG1 molecule). Alternatively, the rigid peptide linker may contain one or more mutations relative to the wild-type counterpart.

In some examples, the rigid peptide linker may contain only the hinge domain of an IgG molecule or a fragment thereof. In other examples, the rigid peptide linker may contain the hinge domain or a fragment thereof and a Gly-rich fragment (*e.g.*, those disclosed herein), which can be linked to either the N-terminus and/or the C-terminus of the hinge domain or the fragment thereof. Examples of the rigid peptide linkers for use in any of the multi-specific antibodies disclosed herein are provided in **Table 1** below.

### (c) Heavy chain constant region fragment and Fc fragments

In some embodiments, the multi-specific antibodies disclosed herein may contain a pair of heavy chain constant region fragment such as Fc fragments, each located on a separate polypeptide.

In some examples, the heavy chain constant region fragment contains a hinge domain, CH2 domain, and CH3 domain of a suitable immunoglobin (Ig) molecule, for example, an IgG molecule. In some instances, the heavy chain constant region fragment is derived from an IgG1 molecule. In some examples, the heavy chain constant region fragment may be an Fc fragment containing a CH2 domain and a CH3 domain of the suitable Ig molecule, for example, IgG such as IgG1. In some examples, the heavy chain constant region fragment is a fragment of a wild-type Ig molecule (e.g., IgG such as IgG1). Alternatively, the heavy chain constant region fragment may contain more or more mutations relative to the wildtype counterpart.

In some instances, the heavy chain constant region fragments such as Fc fragments may contain one or more mutations that enhance heterodimer formation. Examples include "knobs-into-holes" (Ridgway et al., Protein Engineering, 9 (7) , pp. 617-21 (1996) ; Merchant et al., Nature Biotechnology, 16 (7) , pp. 677-681 (1998) ), electrostatics (Gunasekaran et al., Journal of Biological Chemistry, 285 (25) , pp. 19637-19646 (2010) ) or negative state designs (Kreudenstein et al., mAbs, 5 (5) , pp. 646-654 (2013) ; Leaver-Fay et al., Structure, 24 (4) , pp. 641-651 (2016) ) (charged mutations). Other examples can be found in, *e.g.*, Brinkmann et al., MABS (2017), 9(2):182-212, the relevant disclosures are incorporated by reference for the subject matter and purpose referenced herein.

In some examples, the mutation may be at positions 366 (*e.g.*, T366W or T366S), 368 (*e.g.*, L368A), and/or 407 (e.g., G407V). In specific examples, one heavy chain constant region fragment in a multi-specific antibody may contain mutations at position 366 (e.g., T366W) and 407 (e.g., G407V) and a second heavy chain constant region fragment in the same multi-specific antibody may contain mutations at positions 366 (e.g., T366S), 368 (e.g., L368A), and 407 (G407V). Unless explicitly called out, all numbers referring to positions in an Ig molecule follow the EU numbering system.

In some instances, mutations that reduce binding affinity to Protein A may be introduced into one or both of the heavy chain Fc regions in a multi-specific antibody to facilitate purification of the multi-specific antibodies. Such mutations are known in the art. See, *e.g.*, Tustian et al., mAbs 8:828-838 (2016), the relevant disclosures of which are incorporated by reference for the purpose and subject matter referenced herein.

Alternatively, or in addition, mutations at positions involved in Fc receptor binding may be introduced into the heavy chain constant region fragments. Such mutations may modulate binding affinity and selectivity to Fc receptors. Unless otherwise explicitly pointed out, numberings of positions in an antibody heavy or light chain are according to the EU index numbering. In some instances, a heavy chain constant region fragment such as a Fc fragment may contain an amino acid substitution at one or more of positions 267, 273, 328, and 329. In some instances, the one or more mutations may be amino acid substitutions at one or more of positions 239, 265, 297, 329, 330, and 332. In one example, a heavy chain constant region fragment such as a Fc fragment may contain (i) a deletion at the position 237, and (ii) two amino acid substitutions at position 234 (*e.g.*, L234A) and 235 (*e.g.*, L235A). In another example, the heavy chain constant region fragments may comprise one or more of the following: (i) a deletion at the position 237, (ii) two amino acid substitutions selected from L234A, L235A and P329G, (iii) a deletion at position 237 and amino acid substitutions of D265A and N297A, (iv) amino acid substitutions S239D, A330L and I332E, and (v) a deletion at the position 237 and the amino acid substitution P329G. In some examples, an Fc fragment as disclosed herein may comprise (i) an amino acid substitution at position 329, which optionally is P329G, (ii) amino acid substitutions at positions 265 and 297, which optionally are D265A and N297A, (iii) amino acid substitutions at positions 239, 330, and 332, which optionally are S239D, A330L and I332E, or a combination of any one of (i)-(iii).

Additional mutations in the Fc fragment for modulating Fc receptor binding activities can be find in, *e.g.*, US-2020-0392227, the relevant disclosures of which are incorporated by reference for the subject matter and purpose referenced herein.

### C. Exemplary Multi-Specific Antibodies

Provided below are exemplary designs of the multi-specific antibodies disclosed herein.

### (a) Multi-Specific Antibodies with a Central Fv

In some embodiments, a multi-specific antibody disclosed herein is a multi-chain molecule comprising a first Fv fragment, which contains a first V_{H} and a first V_{L}. Each of the V_{H} and V_{L} chains is connected to a flexible peptide linker (those disclosed herein, see **Table 1** for examples) at one end and a rigid peptide linker (those disclosed herein, see **Table 1** for examples) at the other end. See **FIG. 1A**. Such a multi-specific antibody comprises a first polypeptide comprising a first flexible peptide linker, the V_{H}, and a first rigid peptide linker, and a second polypeptide comprising a second flexible peptide linker, the V_{L}, and a second rigid peptide linker. In some instances, the first flexible peptide and the second flexible peptide linker are identical. Alternatively, the first flexible peptide and the second flexible peptide linker are different. The first rigid peptide linker and the second rigid peptide linker form one or more disulfide bonds such as the first polypeptide and the second polypeptide can form a heterodimer.

The multi-specific antibody may further comprise a second antigen-binding moiety, optionally a third antibody binding moiety, which may be connected to the V_{H} and/or V_{L} via the first flexible peptide linker and/or the second flexible peptide linker. The second antigen-binding moiety, and optionally the third antigen-binding moiety may be in scFv format or in Fab format.

In some examples, the multi-specific antibody contains the second antigen-binding moiety, which is in Fab format - containing a VH-CH1 fragment and a VL-CL fragment. Either the VH-CH1 fragment or the VL-CL fragment is linked to the first flexible peptide linker and form the N-terminal fragment of the first polypeptide, and the other fragment exists as a separate polypeptide (the third polypeptide of the multi-specific antibody). Alternatively, either the VH-CH1 fragment or the VL-CL fragment is linked to the second flexible peptide linker and form the N-terminal fragment of the second polypeptide, and the other fragment exists as a separate polypeptide (the third polypeptide of the multi-specific antibody).

In some examples, the multi-specific antibody contains the second antigen-binding moiety, which is in Fab format (first Fab), and the third antigen-binding moiety, which may also be in Fab format (second Fab). The first Fab contains a first VH-CH1 fragment and a first VL-CL fragment and the second Fab contains a second VH-CH1 fragment and a second VL-CL fragment. Either the first VH-CH1 fragment or the first VL-CL fragment is linked to the first flexible peptide linker and form the N-terminal fragment of the first polypeptide, and the other fragment exists as a separate polypeptide (the third polypeptide of the multi-specific antibody). Further, either the second VH-CH1 fragment or the second VL-CL fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

In some examples, the multi-specific antibody contains the second antigen-binding moiety, which is in cross Fab format, and the third antigen-binding moiety, which may be in Fab format. The first cross Fab contains a VH-CL (Cλ or Cκ) fragment and a first VL-CH1 fragment and the Fab contains a VH-CH1 fragment and a VL-CL (Cλ or Cκ) fragment. Either the VH-CL fragment or the VL-CH1 fragment is linked to the first flexible peptide linker and form the N-terminal fragment of the first polypeptide, and the other fragment exists as a separate polypeptide (the third polypeptide of the multi-specific antibody). Further, either the VH-CH1 fragment or the VL-CL fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

In some examples, the multi-specific antibody contains the second antigen-binding moiety, which is in a cross Fab format (first cross Fab), and the third antigen-binding moiety, which may also be in a cross Fab format (second cross Fab). The first cross Fab contains a first VH-CL (Cλ or Cκ) fragment and a first VL-CH1 fragment and the second cross Fab contains a second VH-CL (Cλ or Cκ) fragment and a second VL-CH1 fragment. Either the first VH-CL fragment or the first VL-CH1 fragment is linked to the first flexible peptide linker and form the N-terminal fragment of the first polypeptide, and the other fragment exists as a separate polypeptide (the third polypeptide of the multi-specific antibody). Further, either the second VH-CL fragment or the second VL-CH1 fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

The multi-specific antibody disclosed herein may further comprise a first Fc fragment linked to the first V_{H} via the first rigid peptide linker and a second Fc fragment linked to the first V_{L} via the second rigid peptide linker. The Fc fragments may contain mutations disclosed herein, for example, knob-in-hole mutations to facilitate formation of heterodimers between the first polypeptide and the second polypeptide. See, *e.g.*, **FIG. 1B**.

### (b) Multi-Specific Antibodies with Terminal Fv

In some embodiments, a multi-specific antibody disclosed herein is a multi-chain molecule comprising a first Fv fragment, which contains a first V_{H} and a first V_{L}. Each of the V_{H} and V_{L} chains is connected to a first peptide linker such as a first flexible peptide linker (those disclosed herein, see **Table 1** for examples) at one end and optionally another peptide linker such as a rigid peptide linker (those disclosed herein, see **Table 1** for examples) at the other end. See, *e.g.*, **FIG. 1A**. Such a multi-specific antibody comprises a first polypeptide comprising a first peptide linker (e.g., a first flexible peptide linker), the V_{H}, and a second peptide linker (e.g., a first rigid peptide linker), and a second polypeptide comprising a third peptide linker (e.g., a second flexible peptide linker), the V_{L}, and a fourth peptide linker (e.g., a second rigid peptide linker). In some instances, the first peptide and the third peptide linker are identical. Alternatively, the first peptide and the third peptide linker are different. The second peptide linker (e.g., the first rigid peptide linker) and the fourth peptide linker (e.g., the second rigid peptide linker) may form one or more disulfide bonds such as the first polypeptide and the second polypeptide can form a heterodimer.

The multi-specific antibody disclosed herein may further comprise a first heavy chain constant region fragment linked to the first V_{H} via the first peptide linker and a second heavy chain constant region fragment linked to the first V_{L} via the second peptide linker. The heavy chain constant region fragments may contain mutations disclosed herein, for example, knob-in-hole mutations to facilitate formation of heterodimers between the first polypeptide and the second polypeptide.

The multi-specific antibody may further comprise a second antigen-binding moiety, optionally a third antibody binding moiety, which may be connected to the V_{H} and/or V_{L} via the first heavy chain constant region fragment and/or the second heavy chain constant region fragment. The second antigen-binding moiety, and optionally the third antigen-binding moiety may be in scFv format or in Fab or cross Fab format. Alternatively, the second antigen-binding moiety and/or the third antigen-binding moiety may be in BCR or TCR format.

In some examples, the multi-specific antibody contains the second antigen-binding moiety, which is in Fab format - containing a VH-CH1 fragment and a VL-CL fragment. Either the VH-CH1 fragment or the VL-CL fragment is linked to the first heavy chain constant region fragment and form the N-terminal fragment of the first polypeptide, and the other fragment exists as a separate polypeptide (the third polypeptide of the multi-specific antibody). Alternatively, either the VH-CH1 fragment or the VL-CL fragment is linked to the second heavy chain constant region fragment and form the N-terminal fragment of the second polypeptide, and the other fragment exists as a separate polypeptide (the third polypeptide of the multi-specific antibody).

In other examples, the multi-specific antibody contains the second antigen-binding moiety, which is in cross Fab format - containing a VH-Cκ or VH-Cλ fragment and a VL-CH1 fragment. Either the VH-Cκ or VH-Cλ fragment or the VL-CH1 fragment is linked to the first heavy chain constant region fragment and form the N-terminal fragment of the first polypeptide, and the other fragment exists as a separate polypeptide (the third polypeptide of the multi-specific antibody). Alternatively, either the VH-Cκ or VH-Cλ fragment or the VL-CH1 fragment is linked to the second heavy chain constant region fragment and form the N-terminal fragment of the second polypeptide, and the other fragment exists as a separate polypeptide (the third polypeptide of the multi-specific antibody).

In some examples, the multi-specific antibody contains the second antigen-binding moiety, which is in Fab format (first Fab), and the third antigen-binding moiety, which may also be in Fab format (second Fab). The first Fab contains a first VH-CH1 fragment and a first VL-CL fragment and the second Fab contains a second VH-CH1 fragment and a second VL-CL fragment. Either the first VH-CH1 fragment or the first VL-CL fragment is linked to the first heavy chain constant region fragment and form the N-terminal fragment of the first polypeptide, and the other fragment exists as a separate polypeptide (the third polypeptide of the multi-specific antibody). Further, either the second VH-CH1 fragment or the second VL-CL fragment is linked to the second heavy chain constant region fragment and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical). See, *e.g.*, **FIG. 1C**.

In other examples, the multi-specific antibody contains the second antigen-binding moiety, which is in cross Fab format, and the third antigen-binding moiety, which may be in Fab format. The cross Fab contains a VH-Cκ or VH-Cλ fragment and a VL-CH1 fragment and the Fab contains a VH-CH1 fragment and a VL-CL fragment. Either the VH-Cκ or VH-Cλ fragment or the VL-CH1 fragment is linked to the first heavy chain constant region fragment and form the N-terminal fragment of the first polypeptide, and the other fragment exists as a separate polypeptide (the third polypeptide of the multi-specific antibody). Further, either the VH-CH1 fragment or the VL-CL fragment is linked to the second heavy chain constant region fragment and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

In other examples, the multi-specific antibody contains the second antigen-binding moiety, which is in cross Fab format (first cross Fab), and the third antigen-binding moiety, which may also be in a cross Fab format (second cross Fab). The first cross Fab contains a first VH-Cκ or first VH-Cλ fragment and a first VL-CH1 fragment and the second cross Fab contains a second VH-Cκ or second VH-Cλ fragment and a second VL-CH1 fragment. Either the first VH-Cκ or first VH-Cλ fragment or the first VL-CH1 fragment is linked to the first heavy chain constant region fragment and form the N-terminal fragment of the first polypeptide, and the other fragment exists as a separate polypeptide (the third polypeptide of the multi-specific antibody). Further, either the second VH-Cκ or second VH-Cλ fragment and the second VL-CH1 fragment is linked to the second heavy chain constant region fragment and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

Alternatively, the first Fv fragment contains a first V_{H} and a first V_{L}. Each of the V_{H} and V_{L} chains is connected to a flexible peptide linker (those disclosed herein, see **Table 1** for examples) at one end and contains no peptide linker at the other end. See, **FIG. 1C**.

### (c) Multi-Specific Antibodies with Both a Central Fv and a Terminal Fv

In some embodiments, a multi-specific antibody disclosed herein is a multi-chain molecule comprising a first Fv fragment (a central Fv), which contains a first V_{H} and a first V_{L}. Each of the V_{H} and V_{L} chains is connected to a flexible peptide linker (those disclosed herein, see **Table 1** for examples) at one end and a rigid peptide linker (those disclosed herein, see **Table 1** for examples) at the other end. See **FIG. 1A**. Such a multi-specific antibody comprises a first polypeptide comprising a first flexible peptide linker, the V_{H}, and a first rigid peptide linker, and a second polypeptide comprising a second flexible peptide linker, the V_{L}, and a second rigid peptide linker. In some instances, the first flexible peptide and the second flexible peptide linker are identical. Alternatively, the first flexible peptide and the second flexible peptide linker are different. The first rigid peptide linker and the second rigid peptide linker form one or more disulfide bonds such as the first polypeptide and the second polypeptide can form a heterodimer.

The multi-specific antibody may further comprise a second Fv fragment (a terminal Fv), which comprises a second V_{H} and a second V_{L}. Each of the second V_{H} and second V_{L} is linked to a peptide linker at its N-terminus. The peptide linker may be a flexible peptide linker as disclosed herein (see, *e.g.*, **Table 1** for examples). In some instances, the peptide linker connected to the second V_{H} and that connected to the second VL may be identical. Alternatively, the second V_{H} and second V_{L} may be in connection with different peptide linkers. In some instances, the second V_{H} and second V_{L} may be linked to peptide linkers at their C-termini. Such C-terminal peptide linkers may be rigid peptide linkers to form disulfide bonds. Examples are provided in **Table 1** below. Alternatively, the second V_{H} and second V_{L} are not linked to any peptide linkers at their C-termini.

The first Fv fragment and the second Fv fragment in the multi-specific antibody may be connected through Fc fragments, *e.g.*, in the format of first V_{H} - first rigid peptide linker - first Fc fragment - peptide linker - second V_{H} and first V_{L} - second rigid peptide linker - second Fc fragment - peptide linker - second V_{L.} In some instances, the first V_{H} and second VL may be located on one polypeptide and the first V_{L} and second V_{H} may be located on one polypeptide, e.g., in the format of first V_{H} - first rigid peptide linker - first Fc fragment - peptide linker - second V_{L} and first V_{L} - second rigid peptide linker - second Fc fragment - peptide linker - second V_{H}.

The multi-specific antibody may further comprise a second antigen-binding moiety, optionally a third antibody binding moiety, which may be connected to the first V_{H} and/or first V_{L} of the first Fv (central Fv) via the first flexible peptide linker and/or the second flexible peptide linker. The second antigen-binding moiety, and optionally the third antigen-binding moiety may be in scFv format, in single domain antibody such as VHH format, in Fab format, or in cross Fab format. Alternatively, the second antigen-binding moiety, and optionally the third antigen-binding moiety may be in BCR or TCR format.

In some examples, the multi-specific antibody contains the second antigen-binding moiety, which is in Fab format - containing a VH-CH1 fragment and a VL-CL fragment. Either the VH-CH1 fragment or the VL-CL fragment is linked to the first flexible peptide linker and form the N-terminal fragment of the first polypeptide, and the other fragment exists as a separate polypeptide (the third polypeptide of the multi-specific antibody). Alternatively, either the VH-CH1 fragment or the VL-CL fragment is linked to the second flexible peptide linker and form the N-terminal fragment of the second polypeptide, and the other fragment exists as a separate polypeptide (the third polypeptide of the multi-specific antibody).

In other examples, the multi-specific antibody contains the second antigen-binding moiety, which is in cross Fab format - containing a VH-Cκ or second VH-Cλ fragment and a VL-CH1 fragment. Either the VH-Cκ or second VH-Cλ fragment or the VL-CH1 fragment is linked to the first flexible peptide linker and form the N-terminal fragment of the first polypeptide, and the other fragment exists as a separate polypeptide (the third polypeptide of the multi-specific antibody). Alternatively, either the VH-Cκ or second VH-Cλ fragment or the VL-CH1 fragment is linked to the second flexible peptide linker and form the N-terminal fragment of the second polypeptide, and the other fragment exists as a separate polypeptide (the third polypeptide of the multi-specific antibody).

In some examples, the multi-specific antibody contains the second antigen-binding moiety, which is in Fab format (first Fab), and the third antigen-binding moiety, which may also be in Fab format (second Fab). The first Fab contains a first VH-CH1 fragment and a first VL-CL fragment and the second Fab contains a second VH-CH1 fragment and a second VL-CL fragment. Either the first VH-CH1 fragment or the first VL-CL fragment is linked to the first flexible peptide linker and form the N-terminal fragment of the first polypeptide, and the other fragment exists as a separate polypeptide (the third polypeptide of the multi-specific antibody). Further, either the second VH-CH1 fragment or the second VL-CL fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical). See, *e.g.*, **FIG. 1D**.

In some examples, the multi-specific antibody contains the second antigen-binding moiety, which is in cross Fab format, and the third antigen-binding moiety, which may be in Fab format. The cross Fab contains a VH-Cκ or VH-Cλ fragment and a VL-CH1 fragment and the Fab contains a VH-CH1 fragment and a VL-CL fragment. Either the VH-Cκ or the VH-Cλ fragment or the VL-CH1 fragment is linked to the first flexible peptide linker and form the N-terminal fragment of the first polypeptide, and the other fragment exists as a separate polypeptide (the third polypeptide of the multi-specific antibody). Further, either the VH-CH1 fragment or the VL-CL fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

In yet other examples, the multi-specific antibody contains the second antigen-binding moiety, which is in cross Fab format (first cross Fab), and the third antigen-binding moiety, which may also be in a cross Fab format (second cross Fab). The first cross Fab contains a first VH-Cκ or VH-Cλ fragment and a first VL-CH1 fragment and the second cross Fab contains a second VH-Cκ or VH-Cλ fragment and a second VL-CH1 fragment. Either the first VH-Cκ /VH-Cλ fragment or the first VL-CH1 fragment is linked to the first flexible peptide linker and form the N-terminal fragment of the first polypeptide, and the other fragment exists as a separate polypeptide (the third polypeptide of the multi-specific antibody). Further, either the second VH-Cκ /VH-Cλ fragment or the second VL-CH1 fragment is linked to the second flexible peptide linker and form the N-terminal fragment of the second polypeptide, and the other fragment exists as a separate polypeptide (the fourth polypeptide of the multi-specific antibody). In some instances, the second antigen-binding moiety and the third antigen-binding moiety are identical (the third polypeptide and the fourth polypeptide are identical).

Exemplary multi-specific antibodies as disclosed herein are provided in **Table 2** below.

### III. Methods for Antibody Preparation

Any of the multi-specific antibodies, including bi-specific antibodies and tri-specific antibodies as described herein can be made by any method known in the art. See, for example, Harlow and Lane, (1998) Antibodies: A Laboratory Manual, Cold Spring Harbor Laboratory, New York.

In some embodiments, the multi-specific antibody may be produced via, *e.g.*, conventional recombinant technology as exemplified below.

Nucleic acids encoding the multiple chains of a multi-specific antibody as described herein can be cloned into one expression vector, each nucleotide sequence being in operable linkage to a suitable promoter. In one example, each of the nucleotide sequences encoding the heavy chain and light chain is in operable linkage to a distinct prompter. Alternatively, the nucleotide sequences encoding the multiple chains can be in operable linkage with a single promoter, such that both heavy and light chains are expressed from the same promoter. When necessary, an internal ribosomal entry site (IRES) can be inserted between the heavy chain and light chain encoding sequences.

In some examples, the nucleotide sequences encoding the multiple chains of the antibody are cloned into two or more vectors, which can be introduced into the same or different cells. When the multiple chains are expressed in different cells, each of them can be isolated from the host cells expressing such and the isolated multiple chains can be mixed and incubated under suitable conditions allowing for the formation of the multi-chain antibody.

Generally, a nucleic acid sequence encoding one or all chains of an antibody can be cloned into a suitable expression vector in operable linkage with a suitable promoter using methods known in the art. For example, the nucleotide sequence and vector can be contacted, under suitable conditions, with a restriction enzyme to create complementary ends on each molecule that can pair with each other and be joined together with a ligase. Alternatively, synthetic nucleic acid linkers can be ligated to the termini of a gene. These synthetic linkers contain nucleic acid sequences that correspond to a particular restriction site in the vector. The selection of expression vectors/promoter would depend on the type of host cells for use in producing the antibodies.

A variety of promoters can be used for expression of the antibodies described herein, including, but not limited to, cytomegalovirus (CMV) intermediate early promoter, a viral LTR such as the Rous sarcoma virus LTR, HIV-LTR, HTLV-1 LTR, the simian virus 40 (SV40) early promoter, E. coli lac UV5 promoter, and the herpes simplex tk virus promoter.

Regulatable promoters can also be used. Such regulatable promoters include those using the lac repressor from E. coli as a transcription modulator to regulate transcription from lac operator-bearing mammalian cell promoters (Brown, M. et al., Cell, 49:603-612 (1987)), those using the tetracycline repressor (tetR) (Gossen, M., and Bujard, H., Proc. Natl. Acad. Sci. USA 89:5547-5551 (1992); Yao, F. et al., Human Gene Therapy, 9:1939-1950 (1998); Shockelt, P., et al., Proc. Natl. Acad. Sci. USA, 92:6522-6526 (1995)). Other systems include FK506 dimer, VP16 or p65 using astradiol, RU486, diphenol murislerone, or rapamycin. Inducible systems are available from Invitrogen, Clontech and Ariad.

Regulatable promoters that include a repressor with the operon can be used. In one embodiment, the lac repressor from E. coli can function as a transcriptional modulator to regulate transcription from lac operator-bearing mammalian cell promoters (M. Brown et al., Cell, 49:603-612 (1987); Gossen and Bujard (1992); M. Gossen et al., Natl. Acad. Sci. USA, 89:5547-5551 (1992)) combined the tetracycline repressor (tetR) with the transcription activator (VP 16) to create a tetR-mammalian cell transcription activator fusion protein, tTa (tetR-VP 16), with the tetO-bearing minimal promoter derived from the human cytomegalovirus (hCMV) major immediate-early promoter to create a tetR-tet operator system to control gene expression in mammalian cells. In one embodiment, a tetracycline inducible switch is used. The tetracycline repressor (tetR) alone, rather than the tetR-mammalian cell transcription factor fusion derivatives can function as potent trans-modulator to regulate gene expression in mammalian cells when the tetracycline operator is properly positioned downstream for the TATA element of the CMVIE promoter (Yao et al., Human Gene Therapy, 10(16):1392-1399 (2003)). One particular advantage of this tetracycline inducible switch is that it does not require the use of a tetracycline repressor-mammalian cells transactivator or repressor fusion protein, which in some instances can be toxic to cells (Gossen et al., Natl. Acad. Sci. USA, 89:5547-5551 (1992); Shockett et al., Proc. Natl. Acad. Sci. USA, 92:6522-6526 (1995)), to achieve its regulatable effects.

Additionally, the vector can contain, for example, some or all of the following: a selectable marker gene, such as the neomycin gene for selection of stable or transient transfectants in mammalian cells; enhancer/promoter sequences from the immediate early gene of human CMV for high levels of transcription; transcription termination and RNA processing signals from SV40 for mRNA stability; SV40 polyoma origins of replication and ColE1 for proper episomal replication; internal ribosome binding sites (IRESes), versatile multiple cloning sites; and T7 and SP6 RNA promoters for in vitro transcription of sense and antisense RNA. Suitable vectors and methods for producing vectors containing transgenes are well known and available in the art.

Examples of polyadenylation signals useful to practice the methods described herein include, but are not limited to, human collagen I polyadenylation signal, human collagen II polyadenylation signal, and SV40 polyadenylation signal.

One or more vectors (e.g., expression vectors) comprising nucleic acids encoding any of the antibodies may be introduced into suitable host cells for producing the antibodies. The host cells can be cultured under suitable conditions for expression of the antibody or any polypeptide chain thereof. Such antibodies or polypeptide chains thereof can be recovered by the cultured cells (*e.g.*, from the cells or the culture supernatant) via a conventional method, *e.g.*, affinity purification. If necessary, polypeptide chains of the antibody can be incubated under suitable conditions for a suitable period of time allowing for production of the antibody.

In some embodiments, methods for preparing an antibody described herein involve a recombinant expression vector that encodes all of the multiple chains of a multi-specific antibody as also described herein. The recombinant expression vector can be introduced into a suitable host cell (*e.g.*, a dhfr- CHO cell) by a conventional method, *e.g.*, calcium phosphate-mediated transfection. Positive transformant host cells can be selected and cultured under suitable conditions allowing for the expression of the multiple polypeptide chains (*e.g.*, three or four) that form the antibody, which can be recovered from the cells or from the culture medium. When necessary, the multiple chains recovered from the host cells can be incubated under suitable conditions allowing for the formation of the multi-chain antibody.

In one example, two or more recombinant expression vectors are provided, each encoding one or more of the multiple chains of the antibody. The two or more recombinant expression vectors can be introduced into a suitable host cell (*e.g.*, dhfr- CHO cell) by a conventional method, *e.g.*, calcium phosphate-mediated transfection. Alternatively, each of the expression vectors can be introduced into a suitable host cell. Positive transformants can be selected and cultured under suitable conditions allowing for the expression of the polypeptide chains of the antibody. When the two or more expression vectors are introduced into the same host cells, the antibody produced therein can be recovered from the host cells or from the culture medium. If necessary, the polypeptide chains can be recovered from the host cells or from the culture medium and then incubated under suitable conditions allowing for formation of the antibody. When the two or more expression vectors are introduced into different host cells, each of them can be recovered from the corresponding host cells or from the corresponding culture media. The multiple polypeptide chains can then be incubated under suitable conditions for formation of the antibody.

Standard molecular biology techniques are used to prepare the recombinant expression vector, transfect the host cells, select for transformants, culture the host cells and recovery of the antibodies from the culture medium. For example, some antibodies can be isolated by affinity chromatography with a Protein A or Protein G coupled matrix.

Any of the nucleic acids encoding the multiple chains of a multi-specific antibody as disclosed herein, vectors (*e.g.*, expression vectors) containing such; and host cells comprising the vectors are within the scope of the present disclosure.

### IV. Pharmaceutical Compositions

Any of the multi-specific antibodies, including bi-specific antibodies and tri-specific disclosed herein, as well as the encoding nucleic acids or nucleic acid sets, vectors comprising such, or host cells comprising the vectors, as described herein can be mixed with a pharmaceutically acceptable carrier (excipient) to form a pharmaceutical composition for use in treating a target disease. "Acceptable" means that the carrier must be compatible with the active ingredient of the composition (and preferably, capable of stabilizing the active ingredient) and not deleterious to the subject to be treated. Pharmaceutically acceptable excipients (carriers) including buffers, which are well known in the art. See, *e.g.*, Remington: The Science and Practice of Pharmacy 20th Ed. (2000) Lippincott Williams and Wilkins, Ed. K. E. Hoover.

The pharmaceutical compositions to be used in the present methods can comprise pharmaceutically acceptable carriers, excipients, or stabilizers in the form of lyophilized formulations or aqueous solutions. (Remington: The Science and Practice of Pharmacy 20th Ed. (2000) Lippincott Williams and Wilkins, Ed. K. E. Hoover). Acceptable carriers, excipients, or stabilizers are nontoxic to recipients at the dosages and concentrations used, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextran; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN, PLURONICS or polyethylene glycol (PEG).

In some examples, the pharmaceutical composition described herein comprises liposomes containing the antibodies (or the encoding nucleic acids) which can be prepared by methods known in the art, such as described in Epstein, et al., Proc. Natl. Acad. Sci. USA 82:3688 (1985); Hwang, et al., Proc. Natl. Acad. Sci. USA 77:4030 (1980); and U.S. Pat. Nos. 4,485,045 and 4,544,545. Liposomes with enhanced circulation time are disclosed in U.S. Pat. No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The antibodies, or the encoding nucleic acid(s), may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are known in the art, see, *e.g.*, Remington, The Science and Practice of Pharmacy 20th Ed. Mack Publishing (2000).

In other examples, the pharmaceutical composition described herein can be formulated in sustained-release format. Suitable examples of sustained-release preparations include semipermeable matrices of solid hydrophobic polymers containing the antibody, matrices are in the form of shaped articles, *e.g.*, films, or microcapsules. Examples of sustained-release matrix include polyesters, hydrogels (for example, poly(2-hydroxyethyl-methacrylate), or poly(vinyl alcohol)), polylactides (U.S. Pat. No. 3,773,919), copolymers of L-glutamic acid and 7 ethyl-L-glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), sucrose acetate isobutyrate, and poly-D-(-)-3-hydroxybutyric acid.

The pharmaceutical compositions to be used for *in vivo* administration must be sterile. This is readily accomplished by, for example, filtration through sterile filtration membranes. Therapeutic antibody compositions are generally placed into a container having a sterile access port, for example, an intravenous solution bag or vial having a stopper pierceable by a hypodermic injection needle.

The pharmaceutical compositions described herein can be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient can be mixed with a pharmaceutical carrier, *e.g.*, conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable surface-active agents include, in particular, non-ionic agents, such as polyoxyethylenesorbitans (*e.g.*, TWEEN 20, 40, 60, 80 or 85) and other sorbitans (*e.g.*, SPAN 20, 40, 60, 80 or 85). Compositions with a surface-active agent will conveniently comprise between 0.05 and 5% surface-active agent and can be between 0.1 and 2.5%. It will be appreciated that other ingredients may be added, for example mannitol or other pharmaceutically acceptable vehicles, if necessary.

Suitable emulsions may be prepared using commercially available fat emulsions, such as INTRALIPID, LIPOSYN, INFONUTROl, LIPOFUNDIN and LIPIPHYSAN. The active ingredient may be either dissolved in a pre-mixed emulsion composition or alternatively it may be dissolved in an oil (*e.g.*, soybean oil, safflower oil, cottonseed oil, sesame oil, corn oil or almond oil) and an emulsion formed upon mixing with a phospholipid (*e.g.* egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets between 0.1 and 1.0 µm, particularly 0.1 and 0.5 µm, and have a pH in the range of 5.5 to 8.0.

The emulsion compositions can be those prepared by mixing an antibody with INTRALIPID or the components thereof (soybean oil, egg phospholipids, glycerol and water).

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulized by use of gases. Nebulized solutions may be breathed directly from the nebulizing device or the nebulizing device may be attached to a face mask, tent or intermittent positive pressure breathing machine. Solution, suspension or powder compositions may be administered, preferably orally or nasally, from devices which deliver the formulation in an appropriate manner.

### V. Therapeutic Applications

Any of the multi-specific antibodies disclosed herein, may be used in clinical settings (*e.g.*, therapeutic) or in non-clinical settings (*e.g.*, for research purposes).

In some aspects, provided herein are methods of using any of the antibodies disclosed herein for modulating immune responses or for treating a targeting disease in a subject in need of the treatment. To practice the method disclosed herein, an effective amount of the pharmaceutical composition described herein can be administered to a subject (*e.g.*, a human) in need of the treatment *via* a suitable route, such as intravenous administration, *e.g.*, as a bolus or by continuous infusion over a period of time, by intramuscular, intraperitoneal, intracerebrospinal, subcutaneous, intra-articular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution. Alternatively, the antibodies as described herein can be aerosolized using a fluorocarbon formulation and a metered dose inhaler or inhaled as a lyophilized and milled powder.

The subject to be treated by the methods described herein can be a mammal, more preferably a human. Mammals include, but are not limited to, farm animals, sport animals, pets, primates, horses, dogs, cats, mice, and rats. A human subject who needs the treatment may be a human patient having, at risk for, or suspected of having a target disease/disorder, such as a cancer or an immune disorder such as an autoimmune disease.

Examples of cancers include, but are not limited to, breast cancer; biliary tract cancer; bladder cancer; brain cancer including glioblastomas and medulloblastomas; cervical cancer; choriocarcinoma; colon cancer; endomemultial cancer; esophageal cancer; gasmultic cancer; hematological neoplasms including acute lymphocytic and myelogenous leukemia, *e.g.*, B Cell CLL; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor.

In some instances, the multi-specific antibody for use in the treatment methods disclosed herein contain one binding arm specific to a TAA and the patient for treatment carries cancer cells expressing the target TAA. For example, the multi-specific antibody is specific to CD19 and the patient carries a CD19+ cancer. In other examples, the multi-specific antibody is specific to CD20 and the patient carries a CD20+ cancer. In other examples, the multi-specific antibody is specific to BCMA and the patient carries a BCMA+ cancer. In other examples, the multi-specific antibody is specific to B7H3 and the patient carries a B7H3+ cancer. In some examples, the multi-specific antibody is specific to HER2 and the patient carries a HER2+ cancer. In some examples, the multi-specific antibody is specific to p53mut and the patient carries a P53mut+ cancer. In some examples, the multi-specific antibody is specific to MET and the patient carries a MET+ cancer. In some examples, the multi-specific antibody is specific to PSMA and the patient carries a PSMA+ cancer. In some examples, the multi-specific antibody is specific to CEA and the patient carries a CEA+ cancer. In some examples, the multi-specific antibody is specific to EGFR and the patient carries an EGFR+ cancer. In some examples, the multi-specific antibody is specific to DLL3 and the patient carries a DLL3+ cancer. In some examples, the multi-specific antibody is specific to MAGE-A4 and the patient carries a MAGE-A4+ cancer. In some examples, the multi-specific antibody is specific to PRAME and the patient carries a PRAME+ cancer.

In some instances, the multi-specific antibody for use in the treatment methods disclosed herein contain one binding arm specific to an immune cell receptor and the patient for treatment carries the immune cells expressing such immune cell receptor. For example, the multi-specific antibody is specific to PD-(L)1 and the patient carries a PD-(L)1+ immune cells. In some examples, the multi-specific antibody is specific to CD137 and the patient carries a CD137+ immune cells. In some examples, the multi-specific antibody is specific to CD3 and the patient carries CD3+ immune cells. In some examples, the multi-specific antibody is specific to CTLA4 and the patient carries CTLA4+ immune cells. In some examples, the multi-specific antibody is specific to CD40 and the patient carries a CD40+ immune cells. In some examples, the multi-specific antibody is specific to GITR and the patient carries a GITR+ immune cells. In some examples, the multi-specific antibody is specific to at least two of immune cell receptors, such as PD-1, CD137, CD3, CTLA4, CD40, OX40, GITR and the patient carries at two such immune cells receptors expressing immune cells.

A subject having a target cancer can be identified by routine medical examination, *e.g.*, laboratory tests, organ functional tests, CT scans, ultrasounds, and/or genetic testing. In some embodiments, the subject to be treated by the method described herein may be a human cancer patient who has undergone or is subjecting to an anti-cancer therapy, for example, chemotherapy, radiotherapy, immunotherapy, or surgery.

Immune disorders refer to a dysfunction of the immune system. Examples include autoimmune diseases, immunodeficiencies, or allergies. In some embodiments, the target disease for treatment is an autoimmune disease. Examples include, but are not limited to, rheumatoid arthritis (RA), systemic lupus erythematosus (SLE), Myasthenia Gravis (MG), Graves' Disease, Idiopathic Thrombocytopenia Purpura (ITP), Guillain-Barre Syndrome, autoimmune myocarditis, Membrane Glomerulonephritis, Hyper IgM syndrome, diabetes mellitus, Type I or Type II diabetes, multiple sclerosis, Reynaud's syndrome, autoimmune thyroiditis, gasmultitis, Celiac Disease, Vitiligo, Hepatitis, primary biliary cirrhosis, inflammatory bowel disease, spondyloarthropathies, experimental autoimmune encephalomyelitis, immune neutropenia, juvenile onset diabetes, and immune responses associated with delayed hypersensitivity mediated by cytokines, T-lymphocytes typically found in tuberculosis, sarcoidosis, and polymyositis, polyarteritis, cutaneous vasculitis, pemphigus, pemphigold, Goodpasture's syndrome, Kawasaki's disease, systemic sclerosis, anti-phospholipid syndrome, Sjogren's syndrome, graft-versus-host (GVH) disease, and immune thrombocytopenia.

A subject having a target autoimmune disease can be identified by routine medical examination, *e.g.*, presence of antinuclear antibodies, anti-mitochondrial autoantibodies, anti-neutrophil cytoplasmic antibody, anti-phospholipid antibodies, anti-citrullinated peptide (anti-CCP), anti-rheumatoid factor, immunoglobulin A, C-reactive protein test, complement test, erythrocyte sedimentation rate (ESR) test, blood clotting profile, and protein electrophoresis/immunofixation electrophoresis, and/or genetic testings. In some embodiments, the subject to be treated by the method described herein may be a human subject with an autoimmune disease who has undergone or is subjecting to an autoimmune disease treatment, for example, immunosuppressive mediation, hormone replacement therapy, blood transfusions, anti-inflammatory medication, and/or pain medication.

A subject suspected of having any of such target disease/disorder might show one or more symptoms of the disease/disorder. A subject at risk for the disease/disorder can be a subject having one or more of the risk factors for that disease/disorder.

As used herein, "an effective amount" refers to the amount of each active agent required to confer therapeutic effect on the subject, either alone or in combination with one or more other active agents. Determination of whether an amount of the antibody achieved the therapeutic effect would be evident to one of skill in the art. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment.

Empirical considerations, such as the half-life, generally will contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of a target disease/disorder. Alternatively, sustained continuous release formulations of an antibody may be appropriate. Various formulations and devices for achieving sustained release are known in the art.

In one example, dosages for an antibody as described herein may be determined empirically in individuals who have been given one or more administration(s) of the antibody. Individuals are given incremental dosages of the agonist. To assess efficacy of the agonist, an indicator of the disease/disorder can be followed.

Generally, for administration of any of the antibodies described herein, an initial candidate dosage can be about 2 mg/kg. For the purpose of the present disclosure, a typical daily dosage might range from about any of 0.1 µg/kg to 3 µg/kg to 30 µg/kg to 300 µg/kg to 3 mg/kg, to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to alleviate a target disease or disorder, or a symptom thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner wishes to achieve. For example, dosing from one-four times a week is contemplated. In some embodiments, dosing ranging from about 3 µg/mg to about 2 mg/kg (such as about 3 µg/mg, about 10 µg/mg, about 30 µg/mg, about 100 µg/mg, about 300 µg/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy is easily monitored by conventional techniques and assays. The dosing regimen (including the antibody used) can vary over time.

In some embodiments, for an adult patient of normal weight, doses ranging from about 0.003 to 5.00 mg/kg may be administered. In some examples, the dosage of the antibody described herein can be 10 mg/kg. The particular dosage regimen, *i*.*e*.., dose, timing and repetition, will depend on the particular individual and that individual's medical history, as well as the properties of the individual agents (such as the half-life of the agent, and other considerations well known in the art).

For the purpose of the present disclosure, the appropriate dosage of an antibody as described herein will depend on the specific antibody, antibodies, and/or non-antibody peptide (or compositions thereof) employed, the type and severity of the disease/disorder, whether the antibody is administered for preventive or therapeutic purposes, previous therapy, the patient's clinical history and response to the agonist, and the discretion of the attending physician. Typically, the clinician will administer an antibody, until a dosage is reached that achieves the desired result. In some embodiments, the desired result is an increase in anti-tumor immune response in the tumor microenvironment. Methods of determining whether a dosage resulted in the desired result would be evident to one of skill in the art. Administration of one or more antibodies can be continuous or intermittent, depending, for example, upon the recipient's physiological condition, whether the purpose of the administration is therapeutic or prophylactic, and other factors known to skilled practitioners. The administration of an antibody may be essentially continuous over a preselected period of time or may be in a series of spaced dose, *e.g.*, either before, during, or after developing a target disease or disorder.

As used herein, the term "treating" refers to the application or administration of a composition including one or more active agents to a subject, who has a target disease or disorder, a symptom of the disease/disorder, or a predisposition toward the disease/disorder, with the purpose to cure, heal, alleviate, relieve, alter, remedy, ameliorate, improve, or affect the disorder, the symptom of the disease, or the predisposition toward the disease or disorder.

Alleviating a target disease/disorder includes delaying the development or progression of the disease or reducing disease severity or prolonging survival. Alleviating the disease or prolonging survival does not necessarily require curative results. As used therein, "delaying" the development of a target disease or disorder means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of a target disease or disorder includes initial onset and/or recurrence.

Conventional methods, known to those of ordinary skill in the art of medicine, can be used to administer the pharmaceutical composition to the subject, depending upon the type of disease to be treated or the site of the disease. This composition can also be administered via other conventional routes, *e.g.*, administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, it can be administered to the subject via injectable depot routes of administration such as using 1-, 3-, or 6-month depot injectable or biodegradable materials and methods. In some examples, the pharmaceutical composition is administered intraocularly or intravitreally.

Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically acceptable excipient is infused. Physiologically acceptable excipients may include, for example, 5% dextrose, 0.9% saline, Ringer's solution or other suitable excipients. Intramuscular preparations, *e.g.*, a sterile formulation of a suitable soluble salt form of the antibody, can be dissolved and administered in a pharmaceutical excipient such as Water-for-Injection, 0.9% saline, or 5% glucose solution.

In one embodiment, an antibody is administered *via* site-specific or targeted local delivery techniques. Examples of site-specific or targeted local delivery techniques include various implantable depot sources of the antibody or local delivery catheters, such as infusion catheters, an indwelling catheter, or a needle catheter, synthetic grafts, adventitial wraps, shunts and stents or other implantable devices, site specific carriers, direct injection, or direct application. See, *e.g.*, PCT Publication No. WO 00/53211 and U.S. Pat. No. 5,981,568.

Targeted delivery of therapeutic compositions containing an antisense polynucleotide, expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods and Applications Of Direct Gene Transfer (J. A. Wolff, ed.) (1994); Wu et al., J. Biol. Chem. (1988) 263:621; Wu et al., J. Biol. Chem. (1994) 269:542; Zenke et al., Proc. Natl. Acad. Sci. USA (1990) 87:3655; Wu et al., J. Biol. Chem. (1991) 266:338.

Therapeutic compositions containing a polynucleotide (*e*.*g*., those encoding the antibodies described herein) are administered in a range of about 100 ng to about 200 mg of DNA for local administration in a gene therapy protocol. In some embodiments, concentration ranges of about 500 ng to about 50 mg, about 1 µg to about 2 mg, about 5 µg to about 500 µg, and about 20 µg to about 100 µg of DNA or more can also be used during a gene therapy protocol.

The therapeutic polynucleotides and polypeptides described herein can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters and/or enhancers. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, *e*.*g*., PCT Publication Nos. WO 90/07936; WO 94/03622; WO 93/25698; WO 93/25234; WO 93/11230; WO 93/10218; WO 91/02805; U.S. Pat. Nos. 5,219,740 and 4,777,127; GB Patent No. 2,200,651; and EP Patent No. 0 345 242), alphavirus-based vectors (*e*.*g*., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, *e*.*g*., PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, *e*.*g*., Curiel, Hum. Gene Ther. (1992) 3:147); ligand-linked DNA (see, *e*.*g*., Wu, J. Biol. Chem. (1989) 264:16985); eukaryotic cell delivery vehicles cells (see, *e*.*g*., U.S. Pat. No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Pat. No. 5,580,859. Liposomes that can act as gene delivery vehicles are described in U.S. Pat. No. 5,422,120; PCT Publication Nos. WO 95/13796; WO 94/23697; WO 91/14445; and EP Patent No. 0524968. Additional approaches are described in Philip, Mol. Cell. Biol. (1994) 14:2411, and in Woffendin, Proc. Natl. Acad. Sci. (1994) 91:1581.

The particular dosage regimen, *i*.*e*.., dose, timing and repetition, used in the method described herein will depend on the particular subject and that subject's medical history.

In some embodiments, more than one antibody, or a combination of an antibody and another suitable therapeutic agent, may be administered to a subject in need of the treatment. The antibody can also be used in conjunction with other agents that serve to enhance and/or complement the effectiveness of the agents. Treatment efficacy for a target disease/disorder can be assessed by methods well-known in the art.

When any of the antibodies described herein is used for treating a cancer, it can be combined with an anti-cancer therapy, for example, those known in the art. Additional anti-cancer therapy includes chemotherapy, surgery, radiation, immunotherapy, gene therapy, and so forth.

Alternatively, the treatment of the present disclosure can be combined with a chemotherapeutic agent, for example, pyrimidine analogs (5-fluorouracil, floxuridine, capecitabine, gemcitabine and cytarabine), purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethyhnelamineoxaliplatin, iphosphamide, melphalan, merchlorehtamine, mitomycin, mitoxantrone, nitrosourea, plicamycin, procarbazine, taxol, taxotere, teniposide, multiethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (*e*.*g*., TNP-470, genistein, bevacizumab) and growth factor inhibitors (*e*.*g*., fibroblast growth factor (FGF) inhibitors); angiotensin receptor blocker; nimultic oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; and chromatin disruptors.

When any of the antibodies described herein is for use in treating an immune disorder, it can be co-used with other immunomodulatory treatments such as, *e*.*g*., therapeutic vaccines (including but not limited to GVAX, DC-based vaccines, *etc*.), or checkpoint inhibitors (including but not limited to agents that block CTLA4, PD1, LAG3, TIM3, *etc*.). In some instances, the antibody can be combined with another therapy for autoimmune diseases. Examples include, but are not limited to, intravenous Ig therapy; nonsteroidal anti-inflammatory drugs (NSAID); corticosteroids; cyclosporins, rapamycins, ascomycins; cyclophosphamide; azathioprene; methotrexate; brequinar; FTY 720; leflunomide; mizoribine; mycophenolic acid; mycophenolate mofetil; 15-deoxyspergualine; an immunosuppressive agent, or an adhesion molecule inhibitor.

For examples of additional useful agents see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

When a second therapeutic agent is used, such an agent can be administered simultaneously or sequentially (in any order) with the therapeutic agent described herein. When co-administered with an additional therapeutic agent, suitable therapeutically effective dosages for each agent may be lowered due to the additive action or synergy.

### VI. Kits Comprising Multi-Specific Antibodies Disclosed Herein

The present disclosure also provides kits for use in treating or alleviating a target disease, such as cancer or immune disorders as described herein. Such kits can include one or more containers comprising any of the multi-specific antibodies disclosed herein, and optionally a second therapeutic agent to be co-used with the antibody, which is also described herein.

In some embodiments, the kit can comprise instructions for use in accordance with any of the methods described herein. The included instructions can comprise a description of administration of the antibody, and optionally the second therapeutic agent, to treat, delay the onset, or alleviate a target disease as those described herein. The kit may further comprise a description of selecting an individual suitable for treatment based on identifying whether that individual has the target disease, *e*.*g*.*,* applying the diagnostic method as described herein. In still other embodiments, the instructions comprise a description of administering an antibody to an individual at risk of the target disease.

The instructions relating to the use of an antibody generally include information as to dosage, dosing schedule, and route of administration for the intended treatment. The containers may be unit doses, bulk packages (*e*.*g*., multi-dose packages) or sub-unit doses. Instructions supplied in the kits of the invention are typically written instructions on a label or package insert (*e*.*g*., a paper sheet included in the kit), but machine-readable instructions (*e*.*g*., instructions carried on a magnetic or optical storage disk) are also acceptable.

The label or package insert indicates that the composition is used for treating, delaying the onset and/or alleviating the disease, such as cancer or immune disorders (*e*.*g*., an autoimmune disease). Instructions may be provided for practicing any of the methods described herein.

The kits of this invention are in suitable packaging. Suitable packaging includes, but is not limited to, vials, bottles, jars, flexible packaging (*e*.*g*., sealed Mylar or plastic bags), and the like. Also contemplated are packages for use in combination with a specific device, such as an inhaler, nasal administration device (*e*.*g*., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an antibody as those described herein.

Kits may optionally provide additional components such as buffers and interpretive information. Normally, the kit comprises a container and a label or package insert(s) on or associated with the container. In some embodiments, the invention provides articles of manufacture comprising contents of the kits described above.

### General techniques

The practice of the present disclosure will employ, unless otherwise indicated, conventional techniques of molecular biology (including recombinant techniques), microbiology, cell biology, biochemistry, and immunology, which are within the skill of the art. Such techniques are explained fully in the literature, such as Molecular Cloning: A Laboratory Manual, second edition (Sambrook, et al., 1989) Cold Spring Harbor Press; Oligonucleotide Synthesis (M. J. Gait, ed. 1984); Methods in Molecular Biology, Humana Press; Cell Biology: A Laboratory Notebook (J. E. Cellis, ed., 1989) Academic Press; Animal Cell Culture (R. I. Freshney, ed. 1987); Introuction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds. 1993-8) J. Wiley and Sons; Methods in Enzymology (Academic Press, Inc.); Handbook of Experimental Immunology (D. M. Weir and C. C. Blackwell, eds.): Gene Transfer Vectors for Mammalian Cells (J. M. Miller and M. P. Calos, eds., 1987); Current Protocols in Molecular Biology (F. M. Ausubel, et al. eds. 1987); PCR: The Polymerase Chain Reaction, (Mullis, et al., eds. 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practice approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds. Harwood Academic Publishers, 1995); DNA Cloning: A practical Approach, Volumes I and II (D.N. Glover ed. 1985); Nucleic Acid Hybridization (B.D. Hames & S.J. Higgins eds.(1985»; Transcription and Translation (B.D. Hames & S.J. Higgins, eds. (1984»; Animal Cell Culture (R.I. Freshney, ed. (1986»; Immobilized Cells and Enzymes (lRL Press, (1986»; and B. Perbal, A practical Guide To Molecular Cloning (1984); F.M. Ausubel *et al.* (eds.).

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### EXAMPLES

### Example 1: Construction and Production of Multi-Specific and Multivalent Antibodies

Exemplary multi-specific antibodies shown in **Table 2** were produced by recombinant technology and characterized for their antigen binding activities and bioactivities as disclosed herein.

Briefly, cDNAs encoding the heavy chain variable region (V_{H}) and the light chain variable region (V_{L}) of the parent clones (see **Table 1**) were used as the starting materials for making the multi-specific antibodies. The coding sequences of multiple chains of each exemplary multi-specific antibodies were cloned into expression vectors, which were transfected into CHO cells for transient expression. The multi-specific antibodies produced in CHO cells were purified from the culture supernatant by Protein A affinity chromatography. Antibody properties were examined using standard protocols or as described herein.

### Example 2: Effect of Flexible Peptide Linkers on Multi-specific Antibody Properties.

This example explores impact of flexible peptide linkers, which are G/S-rich peptide linkers, on features of multi-specific antibodies comprising such. Multi-specific antibodies compromising various length of flexible peptide linkers were tested in this example.

Exemplary multi-specific antibodies were produced in CHO cells as described in Example 1 above. Binding of the multi-specific antibodies to the corresponding target antigen were evaluated by ELISA or FCM following standard procedure.

As shown in **FIGs. 2A-2F** **and Table 3,** length of flexible peptide linkers has neither significant impact on the binding of the binding moiety connected by the flexible peptide linker, nor the overall quality of these multi-specific antibodies compromising such (data not shown). Two sets of multi-specific antibodies were tested, and similar results were obtained.

**Table 3. Effect of flexible peptide linkers on the binding of the multi-specific antibodies**

| Antibodies | Flexible peptide linker | B7H3 binding | CD3 binding |
|---|---|---|---|
| Ly1612 | N/A | +++++ | - |
| Ly305 | N/A | - | +++++ |
| Ly2364 | (G₄S)₅ (SEQ ID NO: 7) | +++++ | +++ |
| Ly1963 | (G₄S)₄ (SEQ ID NO: 6) | +++++ | +++ |
| Ly2365 | (G₄S)₃ (SEQ ID NO: 5) | +++++ | +++ |
| Ly2366 | (G₄S)₂ (SEQ ID NO: 4) | +++++ | +++ |
| Ly2367 | G₄S (SEQ ID NO: 3) | +++++ | +++ |
| Ly2368 | N/A | +++++ | +++ |
| Ly2384 | G₃S (SEQ ID NO: 2) | +++++ | +++ |
| Ly2385 | G₂S | +++++ | +++ |
| Ly2136 | (G₄S)₅ (SEQ ID NO: 7) | +++++ | +++ |
| Ly2128 | (G₄S)₄ (SEQ ID NO: 6) | +++++ | +++ |
| Ly2137 | (G₄S)₃ (SEQ ID NO: 5) | +++++ | +++ |
| Ly2138 | (G₄S)₂ (SEQ ID NO: 4) | +++++ | +++ |
| Ly2139 | G₄S (SEQ ID NO: 3) | +++++ | +++ |

### Example 3: Effect of Rigid Peptide Linkers on Multi-specific Antibody Properties.

This example explores impact of rigid peptide linkers, which are capable of forming disulfide fond between each other, on features of multi-specific antibodies comprising such.

### (i) Disulfide Formation Property of Rigid Peptide Linker to Antibody Stability and Binding

Multi-specific antibodies compromising an exemplary rigid peptide linker, GGGGSDKTHTCPPCPAPEAAGP (SEQ ID NO: 13), which includes Cys residues for disulfide bond formation, or a control peptide linker PAPEAAGP (SEQ ID NO: 28), which does not include Cys residues and thus unable to form disulfide bonds, were tested in this example.

Exemplary multi-specific antibodies including the peptide linkers noted above were produced in CHO cells as described in Example 1 above. Proteins were prepared with reduced or non-reduced LDS sample buffer and loaded onto the Bis-Tris precast gels. The gel was run at 140 volts for 40 min with MOPS SDS buffer. After electrophoresis, gels were stained with Coomassie blue. **FIG. 3A** shows mobility of antibodies Ly1891, Ly1899 and Ly1963 having the rigid peptide linker (SEQ ID NO: 13) and antibodies Ly1800, Ly1803 and Ly1961 having the control peptide linker (SEQ ID NO: 28). Antibodies Ly1891, Ly1899 and Ly1963 showed the expected mobility on SDS-PAGE under both reducing and nonreducing conditions. In contrast, while the antibodies Ly1800, Ly1803 and Ly1961 showed the expected mobility under reducing conditions, only half-mers of the intact molecules were visible under nonreducing condition, suggesting poor stability of the later antibodies due to absence of the disulfide forming segment.

Next, size-exclusion chromatography (SEC) was performed using a High-performance liquid chromatography system. Samples were injected into Zenix-SEC-300 column or equivalent, using DPBS, pH 7.4 as the mobile phase. SEC-HPLC analysis revealed that antibody constructs without disulfides (Ly1800, Ly1803 and Ly1961) formed more aggregates compared to their disulfides containing counterparts (Ly1891, Ly1899 and Ly1963), as demonstrated by the percentage of high molecular weight (HMW) peak as summarized in **Table 4** below.

**Table 4. SEC analysis of constructs without or with Rigid peptide linker**

| **Protein** | **Main Peak (%)** | **HMW Peak (%)** |
|---|---|---|
| Ly1800 | 85.1 | 14.0 |
| Ly1891 | 93.4 | 6.45 |
| Ly1803 | 72.1 | 21.9 |
| Ly1899 | 98.8 | 1.2 |
| Ly1961 | 81.6 | 18.4 |
| Ly1963 | 96.2 | 3.8 |

Binding of these antibody constructs to CD3-expressing Jurkat cells was assessed by flow cytometry (FCM) as Example 2. FCM analysis revealed that presence of the disulfide forming segment of Ly1899 and Ly1891 increased target dependent binding to cells compared to antibodies (Ly1803 and Ly1800) that lacked the disulfide forming segment (**FIG. 3B**), consistent with above observation of the less stability and more aggregates of the constructs without linker disulfides.

### (ii) Contribution of the G4S Segment in the Rigid Peptide Linker to Antibody Stability and Aggregation

To investigate how the G₄S motif would affect properties of multi-specific antibodies having a rigid peptide linker that includes the G₄S motif, clones Ly2128, Ly1963 and Ly1967, comprising the linker of GGGGSDKTHTCPPCPAPEAAGP (SEQ ID NO: 13) and clones Ly2125, Ly2157 and Ly2167, having the rigid peptide linker DKTHTCPPCPAPEAAGP (SEQ ID NO:21) were tested.

SEC-HPLC analysis performed as described in the previous section indicated that antibodies without the G₄S motif in the rigid peptide linker had relatively shorter retention times (RT), suggesting their ability to exists as oligomers (**Table 5**). Native mass spectrometry analysis for Ly2125 revealed a mass of 398 kDa, which indicated a dimer structure of the desired intact molecule for this antibody.

**Table 5. SEC analysis of Exemplary Multi-Specific Antibodies**

| **Antibodies** | **Main peak (%)** | **Main peak RT/min** |
|---|---|---|
| Ly2125 | 76.4 | 6.9 |
| Ly2128 | 89.6 | 7.7 |
| Ly2157 | 78.0 | 7.2 |
| Ly1963 | 87.5 | 7.9 |
| Ly2167 | 83.2 | 6.2 |
| Ly1967 | 83.0 | 7.0 |

Binding of these antibody constructs to CD3 target antigens was evaluated by ELISA as Example 2. As shown in **FIGs. 4A-4C**, absence of the G₄S motif in the rigid peptide linker (Ly2125, Ly2157 and Ly2167) increased CD3 binding when compared to rigid peptide linkers having the G₄S motif (Ly2128, Ly1963 and Ly1967). The higher binding potency of Ly2125, Ly2157 and Ly2167 is likely a result of oligomer formation.

### Example 4: Activities of Central Fv Binding Moiety in Multi-Specific Antibodies

This example evaluates binding activity of the central Fv fragment in a multi-specific antibody.

### CD3 modulators

Several bispecific or tri-specific antibodies were constructed as CD3 modulators to target B7H3 and/or CD19. The tri-specific antibodies have either a CD137 or a CD28 binding domain and a central (hidden) CD3 binding domain. Binding of these antibodies to CD3 was determined as described in Example 2, and they showed 10-50-fold weaker binding to CD3 as compared to the parent anti-CD3 mAb Ly305 (**Table 6** below and **FIGs. 4A-4C**).

A CD3 reporter assay was performed to determine the potency of these antibodies. A CD3 reporter assay system comprising Jurkat/NFAT-Luc2P cells (Jurkat cells expressing a luciferase reporter driven by an NFAT response element) was used. Briefly, Jurkat/NFAT-Luc2P cells were harvested and aliquoted at 50000 cells/well in a 96-well plate and co-cultured with or without additional target expressing cells. Test antibodies were added, and the plate were incubated for additional 6 hours at 37°C, followed by Bright-Glo^{™} Luciferase Assay (Promega Cat #E2620). NFAT-mediated luminescence in this assay corresponds to activation of CD3 by the multi-specific antibody in the absence or presence of high affinity target antigen.

The CD3 reporter assay showed a much-reduced potency (no activity at 10 µg/mL) for these antibodies in the absence of additional targets, compared to the parent anti-CD3 mAb (**Table 6**). However, activity of CD3 was greatly enhanced in the presence of additional targets for crosslinking, such as tumor antigen or immune receptor (**Table 6** below and **FIGs. 5A-5I**).

**Table 6. CD3 binding and activation**

| **Protein** | **CD3 binding EC50 (ng/mL)** | **CD3 activation** | **CD3 activation in the presence binding to additional target(s)** |
|---|---|---|---|
| Ly305 | 30 | +++ | +++ |
| Ly1965 | 419 | - | ++++ |
| Ly1963 | 740 | - | ++++ |
| Ly2128 | 1081 | - | ++++ |
| Ly2278 | 1457 | - | ++++ |
| Ly1966 | 348 | - | ++++ |
| Ly1967 | 1557 | - | ++++ |

Cellular killing by exemplary multi-specific antibodies was determined by measuring lactate dehydrogenase (LDH) release. Target cells and PBMC effector cells were seeded into 96-well plates and incubated with the test antibodies. The plates were incubated at 37°C for 48 h. LDH released from damaged cells was measured using a LDH assay kit (Promega) following manufacturer instructions. Additionally, activation of immune cells were evaluated by measuring IFN-γ levels using a HTRF assay kit from Cisbio following manufacturer instructions. **FIGs. 6A** and **6B** show that CD3 activation induced T cell killing activity towards tumor cells expressing specific TA for the multi-specific antibodies respectively.

These examples demonstrated that the central CD3 Fv binding module has significantly reduced CD3 binding and minimal ability to induce CD3 activation by itself. In multi-specific format however, the CD3 Fv binding module has high clustering and activation potential due to presence of second and/or third binding partners, much higher CD3 activation capacity compared to the parent mAb Ly305.

### CD137 modulators

Several bispecific or tri-specific antibodies such as Ly2118 and Ly2281 were constructed as CD137 modulators and targeting B7H3 and/or PD-1. These bispecific or tri-specific antibodies showed 20-30-fold weaker CD137 binding as compared to the parent anti-CD137 mAb (**Table 7** below or **FIGs. 7A-7B**).

A CD137 reporter assay was performed to determine the potency of these antibodies using GS-H2-huCD137 reporter cells expressing human CD137, and a downstream signaling assay for IL-8 expression. GS-H2-huCD137 reporter cells with or without additional target expressing cells were seeded in the assay plate at 3000 cells/well and 25000 cells/well respectively. Exemplary multi-specific antibodies were added to the assay plate. The plate was incubated in 37°C, in a CO2 (5%) incubator for 18-20 hours. 8 µL of supernatant from each well of the assay plate was subjected for Homogeneous Time Resolved Fluorescence (HTRF) assay (Cisbio). A Human Interleukin 8 (reporter of CD137 activation) detection assay was performed in a 16µL assay volume using a Human IL-8 Assay Kit (Cisbio, Cat#62IL8PEB). The results were read using Tecan F200pro.

The results from the CD137 reporter assay showed minimal activity for the antibodies tested. However, activity was greatly enhanced when additional targets are available for crosslinking, such as tumor antigen or immune receptor (**Table 7** below and **FIGs. 8A-8E**).

**Table 7. CD137 Binding and Activation**

| **Protein** | **CD137 binding EC₅₀ (ng/mL)** | **CD137 activation** | **CD137 activation in the presence additional target antigens** |
|---|---|---|---|
| TM173 | N/D | +++ | +++ |
| Ly1630 | 80 | - | - |
| Ly2118 | 1780 | - | +++ |
| Ly2281 | 2555 | - | +++ |

As shown in **Table 7** and **FIGs. 7-8**, the central Fv CD137-binding moiety showed much lower binding affinity to CD137 as compared with the parent anti-CD137 antibody (Ly1630) and none of the parent clone and the multi-specific antibodies showed CD137 activation activity in the presence of GS-H2-huCD137 reporter cells. However, in the presence of cells expressing other target antigens (B7H3 or PD-1), the multi-specific antibodies showed high capacity in activating CD137, while the parent anti-CD137 did not show such effect.

These examples demonstrated that the central CD137 Fv binding module has significantly reduced CD137 binding and minimal ability to induce CD137 activation. In multi-specific format, however, the CD137 Fv binding module has high clustering and activation potential due to presence of second and/or third binding partners.

### CD40 modulators

Several bispecific or tri-specific antibodies including Ly2121, Ly2279 and Ly2280 were constructed as CD40 modulators and targeting B7H3 and/or PD-1. These bispecific or tri-specific antibodies showed 3-6-fold weaker CD40 binding as compared to the parent anti-CD40 mAb (**Table 8** below and **FIGs. 9A-9B**).

A CD40 reporter assay was performed to determine the agonist activity of exemplary multi-specific antibodies using reporter cells expressing human CD40, and a downstream signaling assay for IL-8 expression. GS-H2-huCD40 reporter cells with or without additional target expressing cells were seeded in the assay plate at 1000 cells/well and 25000 cells/well respectively. Exemplary multi-specific antibodies were added to the assay plate. The assay plate was incubated in 37°C, in a CO2 (5%) incubator for 18-20 hours. 8 µL of the supernatant from each well of the assay plate was subjected for HTRF detection assay (Cisbio). A human Interleukin 8 (reporter of CD40 activation) detection assay in a 16µL assay volume was performed (Cisbio, Cat#62IL8PEB). The results were read using Tecan F200pro.

Results from the CD40 reporter assay showed reduced activity in CD40 activation reporter assay. However, activity was greatly enhanced in the presence of additional targets for crosslinking, such as tumor antigen or immune receptor (**Table 8** below and **FIGs. 10A-10F**).

**Table 8. CD40 Binding and Activation**

| **Protein** | **CD40 binding EC₅₀ (ng/mL)** | **CD40 activation** | **CD40 activation in the presence additional target antigens** |
|---|---|---|---|
| TM383 | 480 | +++ | +++ |
| Ly2121 | 1681 | - | ++ |
| Ly2279 | 1494 | - | ++ |
| Ly2280 | 3165 | - | ++ |

These examples demonstrated that the central CD40 Fv binding module has significantly reduced CD40 binding and low ability to induce CD40 activation. In multi-specific format however, the CD40 Fv binding module has high clustering and activation potential due to presence of second and/or third binding partners, including TAA B7H3 or immune checkpoint inhibitor PD-1, suggesting a synthetic biology of avidity driven xLink (cross-linking) activity via high affinity target antigen, and to modulate immune responses optimally where multi-valent bindings can readily occur, e.g., in tumor microenvironment.

### Example 5: Contribution of Peptide Linker Sequence to Binding in Tri-Specific Antibodies

This example explores the impact of peptide linker in connection with a Fv, on features of multi-specific antibodies comprising such.

Tri-specific B7H3/CD3/CD28 antibodies Ly2132, Ly2133, Ly2134, Ly2135 and Ly2128, all including a terminal Fv CD28 binding module, were examined in this study. The V_{H} and V_{L} fragment of the anti-CD28 terminal Fv in these antibodies were each connected to an Fc fragment via a peptide linker (flexible or rigid) as indicated below:
Ly2134: GGGGS (SEQ ID NO: 3);
Ly2133: GGGGSGGGGS (SEQ ID NO: 4);
Ly2132: GGGGSGGGGSGGGGS (SEQ ID NO: 5);
Ly2128: GGGGSGGGGSGGGGSGGGGS (SEQ ID NO: 6);
Ly2135: DKTHTCPPCPGGGGSGGGGSGGGGS (SEQ ID NO: 17).

**FIG. 11** shows the effect of these peptide linkers on binding to target antigen. A longer (G₄S)n sequence correlated with higher binding activity (Ly2128>Ly2132> Ly2133>Ly2134), with the shorter G₄S in Ly2134 of minimal binding. Presence of the DKTHTCPPCP (SEQ ID NO: 8) peptide sequence in the linker negatively impacted binding activities, since Ly2135 showed lower binding than Ly2132. The binding affinity of the Fv fragment in the multi-specific antibodies were found to be around 100 times lower than that of the parent mAb (in IgG format).

**FIGs. 12A-12B** shows the agonistic properties of the tri-specific antibody. A longer (G₄S)n sequence correlated with agonistic activity (Ly2128>Ly2132> Ly2133>Ly2134). Presence of the DKTHTCPPCP (SEQ ID NO: 8) peptide sequence in the peptide linker negatively impacted agonistic activities, since Ly2135 showed lower agonistic activity than Ly2132. A similar trend was observed when reporter cells were treated with antibodies listed above along with B7H3 expressing CHO cells.

Taken together, the data demonstrated that the length and structure of the linker connecting the Fv moiety could nuancedly modulate Fv binding potency, therefore, further to obtain desired functionality.

### Example 6: Characterization of Multi-specific Antibodies Containing a Terminal Fv Binding Moiety

Several multi-specific antibodies were constructed using Fv CD137, CD40, CD28 or CD3 binding modules described in this invention at the C-terminus, including Ly1963, Ly1966, Ly1967, Ly1800, Ly1891, Ly2122, Ly2128, Ly2326, Ly2396, Ly2405, Ly2415, Ly2424, Ly2434, Ly2444, Ly2454, Ly2580, Ly2579, Ly2638, Ly2639, Ly2640, Ly2641, Ly2642, Ly2643 and Ly2644. Binding of these multi-specific antibodies to corresponding targets were evaluated by FCM or ELISA performed as described above. Activation of CD137, CD40, CD28 or CD3 signaling by these multi-specific antibodies were also evaluated using the appropriate reporter systems described above.

### CD137 modulators

Several B7H3, CD19 or PD-1 targeting bispecific or tri-specific antibodies were constructed with CD137 modulators via their terminal Fv fragment. These bispecific or tri-specific antibodies showed weaker CD137 binding as compared to the parent anti-CD137 mAb (**Table 9** below and **FIGs. 17A-17B** and **25A-25C**).

A CD137 reporter assay was performed to determine the potency of these antibodies as described in the above examples. The results from the CD137 reporter assay showed minimal activity for the antibodies tested in the absence of additional targets. However, activity was greatly enhanced when additional targets are available for crosslinking, such as tumor antigen or immune receptor (**Table 9** below and **FIGs. 31A-31C**).

**Table 9. CD137 Binding and Activation**

| **Protein** | **CD137 binding** | **CD137 activation without co-culture** | **CD137 activation with B7H3** | **CD137 activation with CD19** | **CD137 activation with CD3** |
|---|---|---|---|---|---|
| TM173 | +++++ | +++ | +++ | +++ | +++ |
| Ly1630 | +++++ | - | - | - | - |
| Ly1963 | ++ | - | ++ | N/A | ++ |
| Ly2122 | +/- | - | ++ | N/A | N/A |
| Ly1966 | ++ | +/- | N/A | ++ | + |
| Ly1967 | ++ | +/- | N/A | ++ | + |
| Ly2326 | ++ | - | N/A | - | N/A |

| | | | | | |
|---|---|---|---|---|---|
| Note: N/A=Not Applicable | | | | | |

As shown in **FIGs. 17A-17B** and **FIGs. 25A-25C**, the terminal Fv CD137-binding moiety showed 20-100 fold lower binding affinity to CD137 as compared with the reference and parent anti-CD137 antibody (TM173 and Ly1630) and none of the multi-specific antibodies showed CD137 activation activity in the absence of additional targets. However, in the presence of cells expressing other target antigens (B7H3, CD19, PD-1, or CD3), the multi-specific antibodies showed high capacity in activating CD137, while the parent anti-CD137 mAb did not show such crosslinking effect. See also **FIGs. 31A-31C**.

These examples demonstrated that the terminal CD137 Fv binding module at C-terminus has significantly reduced CD137 binding and minimal ability to induce CD137 activation. In multi-specific format however, the CD137 Fv binding module has high clustering and activation potential due to presence of second and/or third binding partners.

### CD28 modulators

Several B7H3, BCMA, CEA or HER2 targeting multi-specific antibodies were constructed with CD28 modulators via their terminal Fv fragment.

As shown in **FIGs. 13A-13D****,** the terminal Fv CD28-binding moiety showed 20-100 fold lower binding affinity to CD28 as compared with the parent anti-CD28 antibody Ly224.

A Jurkat reporter assay was performed to determine the potency of these antibodies as described in the above examples. The results from the reporter assay showed weak activity for the antibodies tested in the absence of additional targets. However, activity was greatly enhanced when additional targets are available for crosslinking, such as tumor antigens (**FIG. 13E-13N**).

These examples demonstrated that the terminal CD28 Fv binding module at C-terminus has significantly reduced CD28 binding. In multi-specific format however, the CD28 Fv binding module has high clustering and activation potential due to presence of second and/or third binding partners, as summarized in **Table 10** below.

**Table 10. CD28 Binding and T cell Activation**

| **Protein** | **CD28 binding** | **CD28 activation without co-culture** | **CD28 activation with additional target** |
|---|---|---|---|
| Ly224 | +++++ | +++ | +++ |
| Ly2128 | +++ | + | +++++ |
| Ly2956 | +++ | ++ | +++++ |
| Ly3103 | +++ | ++ | +++++ |
| Ly3169 | +++ | ++ | +++++ |
| Ly3190 | +++ | ++ | +++++ |
| Ly3196 | +++ | ++ | +++++ |

### PD-L1 modulators

Several B7H3 targeting multi-specific antibodies were constructed with PD-L1 modulators via their terminal Fv fragment.

As shown in **FIG. 14A**, the terminal Fv PD-L1-binding moiety showed 20-100 fold lower binding affinity to PD-L1 as compared with the parent anti-PD-L1 antibody Ly076 and Ly2530.

A Jurkat reporter assay was performed to determine the blocking potency of the terminal Fv PD-L1-binding moiety. The results from the reporter assay showed weak blocking activity for the antibodies tested in the absence of additional targets. However, activity was slightly enhanced when additional targets are available for crosslinking, such as tumor antigen (**FIGs. 14B and 14C**).

These examples demonstrated that the terminal PD-L1 Fv binding module at C-terminus has significantly reduced PD-L1 binding and weak blocking activity. In multi-specific format however, the PD-L1 Fv binding module has clustering and activation potential due to presence of second and/or third binding partners, as summarized in **Table 11** below.

**Table 11. PD-L1 Binding and blocking activity**

| **Protein** | **PD-L1 binding** | **blocking activity without co-culture** | **blocking activity with additional target** |
|---|---|---|---|
| Ly076 | +++++ | +++ | +++ |
| Ly2530 | +++++ | +++++ | +++++ |
| Ly2846 | ++ | + | + |
| Ly2847 | +++ | ++ | ++ |

### Example 7: Assessment of Multi-Specific Antibodies with Hidden Fv Module for Cancer Immunotherapy

This example tests the anti-tumor efficacy of exemplary multi-specific antibodies with hidden Fv module(s) and additional binding domains targeting tumor microenvironment.

### A. Multi-Specific Antibodies Targeting CD3 and CD19 and/or CD20

### (i) Binding to Target Antigens

Bispecific antibodies targeting CD3 and CD19 or targeting CD3 and CD20 have been shown to be efficacious against B cell lymphomas. However, such bispecific antibodies showed some disadvantages, including short half-life and high side effects. To address these issues, several multi-specific antibodies targeting CD19 and/or CD20, with either or both anti-CD3 and anti-CD137 Fv, such as Ly1966, Ly1967, Ly2278, Ly2326, Ly2579, Ly2580, Ly2800, Ly2802, Ly2943, Ly2944, Ly2945, Ly2946, Ly2947 and Ly2948, were evaluated in this example. Binding of these multi-specific antibodies to corresponding targets were assessed by FCM or ELISA as described earlier, and their binding to tumor antigen expressing tumor cells are also examined, *e*.*g*., Raji cells (CD20+CD19+). A summary of these data is presented in **Table 12**, and in **FIG. 4C**, and **FIGs. 15****-17**. These data demonstrate that the binding activity of the Fv binding modules have been significantly reduced.

**Table 12. Target binding of anti-CD19/20 multi-specific antibodies**

| **Protein** | **CD19 binding** | **CD20 binding** | **Raji binding** | **CD3 binding** | **CD137 binding** |
|---|---|---|---|---|---|
| Ly238 | ++++ | N/A | ++++ | N/A | N/A |
| Ly239 | N/A | ++++ | +++++ | N/A | N/A |
| Ly305 | N/A | N/A | N/A | ++++ | N/A |
| Ly1630 | N/A | N/A | N/A | N/A | +++++ |
| Ly1966 | ++++ | N/A | ++++ | + | + |
| Ly1967 | ++++ | N/A | ++++ | + | + |
| Ly2278 | ++++ | N/A | ++++ | + | - |
| Ly2326 | ++++ | N/A | ++++ | N/A | + |
| Ly2800 | +++ | ++ | ++ | + | + |
| Ly2802 | ++ | +++ | +++ | + | + |
| Ly2943 | +++ | ++ | ++ | + | + |
| Ly2944 | ++ | +++ | +++ | + | + |

### (ii) Reporter Assays

Reporter assays described earlier were performed to determine activation of CD3 signaling by multi-specific antibodies comprising an anti-CD19/CD20 and/or CD137 binding moiety. These data are summarized in **Table 13**, and in **FIGs.5C-5I****,** and **FIGs. 18A-18L**. The presence of CD19, CD20, including tumor cells (e.g., Raji cell) expressing those antigens, drastically increased CD3 activation of the multi-specific antibodies including Ly1966, Ly1967, Ly2278, Ly2800, Ly2802, Ly2943 and Ly2944. Binding to CD137 enhanced CD3 activation of the multi-specific antibody Ly1966, Ly1967, Ly2800, Ly2802, Ly2943 and Ly2944, albeit at higher concentrations as compared to CD19 binding, reflecting lower affinity of the CD137 binding Fv module.

**Table 13. Activation of CD3 signaling by multi-specific antibodies targeting CD19 or CD20**

| **Protein** | **CD3 activation without co-culture** | **CD3 activation with CD19** | **CD3 activation with CD20** | **CD3 activation with Raji cells** | **CD3 activation with CD137** |
|---|---|---|---|---|---|
| Ly305 | ++ | ++ | ++ | ++ | ++ |
| Ly1966 | - | ++++ | N/A | | +++ |
| Ly1967 | - | ++++ | N/A | ++++ | +++ |
| Ly2278 | - | ++++ | N/A | ++++ | N/A |
| Ly2326 | - | - | N/A | ++++ | - |
| Ly2800 | - | ++++ | +++ | ++++ | +++ |
| Ly2802 | - | ++++ | +++ | ++++ | +++ |
| Ly2943 | - | ++++ | +++ | ++++ | +++ |
| Ly2944 | - | ++++ | +++ | ++++ | +++ |

Reporter assays were also used to determine activation of CD137 signaling by multi-specific antibodies targeting CD19 or CD20. The results are summarized in **Table 14** and in **FIGs.19A-19G****.**

**Table 14. Activation of CD137 signaling by anti-CD19/20 multi-specific antibodies**

| **Protein** | **CD137 activation without co-culture** | **CD137 activation with CD19** | **CD137 activation with CD20** | **CD137 activation with Raji cells** | **CD137 activation with CD3** |
|---|---|---|---|---|---|
| TM173 | +++ | +++ | +++ | +++ | +++ |
| Ly1966 | +/- | + | N/A | N/A | N/A |
| Ly1967 | +/- | + | N/A | N/A | N/A |
| Ly2278 | N/A | N/A | N/A | N/A | N/A |
| Ly2326 | +/- | + | N/A | N/A | N/A |
| Ly2800 | +/- | + | ++ | ++ | + |
| Ly2802 | +/- | + | ++ | ++ | + |
| Ly2943 | +/- | + | ++ | ++ | + |
| Ly2944 | +/- | + | ++ | ++ | + |

Activation of the immune receptor CD3 or CD137 by multi-specific antibodies was minimal but significantly increased under cross-linking conditions, supporting the avidity mediated effect of the design.

### (iii) Tumor Cell Killing, Pharmacokinetics and Antitumor Efficacy

Tumor cell killing by immune cells induced by the anti-CD19/20 multi-specific antibodies were examined *in vitro*.

Raji cells and human PBMCs were mixed and incubated with various test articles for 48 hours incubation, and release of LDH from damaged cells was measured using kit from Promega following the instructions. Cytotoxicity of test articles were calculated based on the LDH level in the culture supernatant. Ly1966 and Ly1967 showed highly potent activity with EC50 values of 10.8 pM and 8.9 pM, respectively (**FIG. 20A**). The B7H3-targeting control Ly1963 showed no significant activity in this assay, supporting CD19 target specific cytotoxicity activated by Ly1966 and Ly1967 (**FIG. 20A**).

Nalm-6-luc cells and human PBMCs were mixed and incubated with various test articles for 48 hours, and luminescence from tumor cells was measured using kit from Promega following the instructions. Cytotoxicity of test articles were reversely correlated to luminescence intensity. The anti-CD19(/CD20) antibodies integrated with anti-CD3 and/or anti-CD137 Fv showed significant tumor cells killing activity comparable or superior to an anti-CD20/CD3 bsAb reference Ly2309 in this assay (**FIG. 20B**).

### (iv) Pharmacokinetics

Ly1967 was dosed in mice for pharmacokinetics analysis. Briefly C57BL/6 mice (6-7 weeks old, 19-20 g, female, purchased from Vital River) were used for this study. Antibodies were diluted in DPBS and administered by *i.p.* injection at 5 mg/kg in a group of 4 mice. Blood sampling was done at pre-dose, 1d, 4d, 7d, 10d, 14d, 17d and 21d by serial bleeding. 10 µL blood per time point was added to 40 µL of PBS-BSA solution. The sample was then mixed well and centrifuged at 2000xg for 5 min at 4°C. The supernatant was frozen on dry ice and stored at -70°C until analysis. Blood antibody concentrations were determined from target recombinant human protein binding by ELISA. After a single intraperitoneal injection of 5 mg/kg, serum concentrations of the active molecule were determined by dual antigen binding ELISA. As shown in **FIG. 21**, Ly1967 remained in the circulation over 3 weeks after dosing, with a PK profile similar to that of a regular antibody.

### (v) Antitumor Efficacy

Antitumor efficacy was examined in bone marrow transplanted syngeneic mouse models using human CD19 overexpressing murine colon cancer cell line MC38, murine melanoma cell line B16F10, or human PBMC engrafted NCG mouse model using human Burkitt's lymphoma cell line Raji. Test articles were injected *i.p.*. Mice were weighed and tumor growth was measured twice weekly using calipers. Tumor volume was estimated using the formula 0.5x (length × width²). Strong antitumor activity was achieved by both Ly1967 and Ly2278 as shown in **FIG. 22A**. In addition, the fact that Ly1967 shows stronger antitumor activities than Ly531 (anti-CD19/CD3 bispecific antibody) (in **FIG. 22B**), Ly1967 stronger than Ly2278 (in **FIG.22C**)**,** and Ly2800/2802 stronger than Ly2307 or Ly531 (in **FIG. 22D**), and Ly2943/2944 stronger than Ly2307 or Ly531 (in **FIG. 22E**)**,** collectively suggests the advantage of anti-CD19/20 multi-specific antibodies over reference anti-CD3 bsAb (Ly2278, Ly2307 and Ly531) in terms of antitumor activity in vivo.

### B. Multi-Specific Antibodies Targeting CD3 and B7H3

B7H3 targeted CD3 bispecific T cell engagers have the potential to mediate immune killing of a variety of solid tumors, where high level of B7H3 expression is common. We made several anti-B7H3 multi-specific antibodies using Fv CD3 and CD137, CD28, PD-1, PD-L1 or CTLA4 binding modules described in this invention, including Ly1965, Ly1963, Ly2128, Ly2122, Ly2936, Ly2937, Ly2939 and Ly2940.

### (i) Binding to Target Antigens

Binding of these multi-specific antibodies to corresponding targets were evaluated by FCM or ELISA and summarized in **Table 15** below and in **FIGs. 4A-4C** and **FIGs. 23-29**. The multi-specific antibodies exhibited affinity binding to TA target B7H3 and significantly weakened affinity for immune target CD3, CD137, CD28, PD-1, PD-L1 or CTLA4.

**Table 15. Target binding of anti-B7H3 multi-specific antibodies**

| **Protein** | **B7H3 binding** | **CD3 binding** | **CD137 binding** | **CD28 binding** | **PD-1 binding** | **PD-L1 binding** | **CTLA4 binding** |
|---|---|---|---|---|---|---|---|
| Ly305 | N/A | ++++ | N/A | N/A | N/A | N/A | N/A |
| Ly1630 | N/A | N/A | +++++ | N/A | N/A | N/A | N/A |
| Ly224 | N/A | N/A | N/A | +++++ | N/A | N/A | N/A |
| Ly711 | N/A | N/A | N/A | N/A | +++++ | N/A | N/A |
| Ly2530 | N/A | N/A | N/A | N/A | N/A | +++++ | N/A |
| Ly2896 | N/A | N/A | N/A | N/A | N/A | N/A | +++++ |
| Ly1965 | ++++ | +/++* | N/A | N/A | N/A | N/A | N/A |
| Ly1963 | ++++ | +/- | ++ | N/A | N/A | N/A | N/A |
| Ly2128 | ++++ | ++* | N/A | N/A | N/A | N/A | N/A |
| Ly2122 | ++++ | N/A | ++ | N/A | N/A | N/A | N/A |
| Ly2600 | ++++ | ++ | ++ | N/A | N/A | N/A | N/A |
| Ly2936 | ++++ | ++ | ++ | N/A | N/A | N/A | N/A |
| Ly2937 | ++++ | - | ++ | N/A | N/A | N/A | N/A |
| Ly2939 | ++++ | ++ | N/A | N/A | N/A | N/A | N/A |
| Ly2940 | ++++ | N/A | ++ | N/A | N/A | N/A | N/A |
| Ly2823 | +++++ | ++ | ++ | N/A | N/A | N/A | N/A |
| Ly2846 | ++++ | ++ | N/A | N/A | N/A | ++ | N/A |
| Ly2847 | ++++ | ++ | N/A | N/A | N/A | +++ | N/A |
| Ly2904 | ++++ | ++ | N/A | N/A | + | N/A | N/A |
| Ly2901 | ++++ | ++ | N/A | N/A | N/A | N/A | ++ |
| Ly2902 | ++++ | ++ | N/A | N/A | N/A | N/A | + |
| Ly2903 | ++++ | ++ | N/A | N/A | N/A | N/A | +++ |
| Ly2938 | ++++ | +/- | N/A | +++ | N/A | N/A | N/A |

| | | | | | | | |
|---|---|---|---|---|---|---|---|
| Note: N/A=Not Applicable; +/- = weak binding; *weak cellular binding evaluated by FCM, moderate protein binding evaluated by ELISA | | | | | | | |

### (ii) Reporter Assays

Activation of CD3 signaling by anti-B7H3 multi-specific antibodies was evaluated using reporter system and summarized in **Table 16** below and in **FIGs.5A-5I,** and **30A-30Q.** The presence of B7H3 target antigen drastically increased CD3 activation of the multi-specific antibodies including Ly1963 and Ly1965. Binding to terminal Fv targets such as CD137, CD28, PD-1 or PD-L1 enhanced CD3 activation of the corresponding multi-specific antibodies.

**Table 16. Activation of CD3 signaling by anti-B7H3 multi-specific antibodies**

| **Protein** | **CD3 activation without co-culture** | **CD3 activation with B7H3** | **CD3 activation with CD137** | **CD3 activation with PD-1** | **CD3 activation with PD-L1** |
|---|---|---|---|---|---|
| Ly305 | ++ | ++ | ++ | ++ | ++ |
| Ly1965 | - | ++++ | - | N/A | N/A |
| Ly1963 | - | ++++ | +++ | N/A | N/A |
| Ly2128 | N/A | N/A | N/A | N/A | N/A |
| Ly2122 | N/A | N/A | N/A | N/A | N/A |
| Ly2600 | - | ++++ | +++ | N/A | N/A |
| Ly2936 | - | ++++ | +++ | N/A | N/A |
| Ly2937 | - | ++++ | +++ | N/A | N/A |
| Ly2939 | - | ++++ | - | N/A | N/A |
| Ly2940 | - | - | - | N/A | N/A |
| Ly2823 | - | +++++ | ++++ | N/A | N/A |
| Ly2846 | - | ++++ | N/A | N/A | +++ |
| Ly2847 | - | ++++ | N/A | N/A | +++ |
| Ly2904 | - | ++++ | N/A | +++ | N/A |
| Ly2901 | - | ++++ | N/A | N/A | N/A |
| Ly2902 | - | ++++ | N/A | N/A | N/A |
| Ly2903 | - | ++++ | N/A | N/A | N/A |

Activation of CD137 signaling by anti-B7H3 multi-specific antibodies was evaluated using reporter system and summarized in **Table 17** below and in **FIGs. 31A-31F****.**

**Table 17. Activation of CD137 signaling by anti-B7H3 multi-specific antibodies**

| **Protein** | **CD137 activation without co-culture** | **CD137 activation with B7H3** | **CD137 activation with CD3** |
|---|---|---|---|
| TM173 | +++ | +++ | +++ |
| Ly1965 | N/A | N/A | N/A |
| Ly1963 | - | ++ | ++ |
| Ly2122 | - | ++ | N/A |
| Ly2600 | +/- | ++ | ++++ |
| Ly2936 | +/- | ++ | ++++ |
| Ly2937 | +/- | ++ | ++++ |
| Ly2823 | +/- | ++ | ++++ |

| | | | |
|---|---|---|---|
| Note: N/A=Not Applicable | | | |

Blockade of PD-(L)1 signaling by anti-B7H3 multi-specific antibodies was evaluated using reporter system and summarized in **Table 18** below and in **FIGs. 14B-14C****.**

**Table 18. Blockade of PD-(L)1 signaling by anti-B7H3 multi-specific antibodies**

| **Protein** | **PD-(L)1 blockade without co-culture** | **PD-(L)1 blockade with B7H3** |
|---|---|---|
| Ly2530 | +++++ | +++++ |
| Ly076 | ++++ | ++++ |
| Ly711 | ++++ | ++++ |
| Ly2846 | +/- | + |
| Ly2847 | + | ++ |

| | | |
|---|---|---|
| Note: N/A=Not Applicable | | |

Activation of the immune receptor CD3 or CD137 by multi-specific antibodies is minimal but significantly increased under cross-linking conditions, supporting the avidity mediated effect of the design. The impact of multi-specific antibodies cross-linking effect on PD-L1 signaling was also observed in exemplary anti-B7H3/CD3/PD-L1 multi-specific antibodies.

### (iii) Tumor Cell Killing, Pharmacokinetics and Antitumor Efficacy

Tumor cell killing by immune cells induced by the anti-B7H3 multi-specific antibodies were examined *in vitro*. A375 cells or A375-Luc cells and human PBMCs were mixed and incubated with various test antibodies for 48 hours and release of LDH or luminescence intensity measured as described earlier.

**FIGs. 32A-32B** provides a summary of these results in an *in vitro* assay for killing of A375 cancer cells. Ly2128 and Ly1963 showed a trend of stronger cytotoxicity than Ly1965, suggesting a contribution of the additional CD28 or CD137 signaling induced by the corresponding terminal Fv. Ly2128 induced higher cytokine production than Ly1963 but without apparent difference in tumor cell killing.

**FIGs. 32C-32D** provides a summary of these results in an *in vitro* assay for killing of A375-luc cancer cells. Ly2600, Ly2936 and Ly1963 showed a trend of stronger cytotoxicity than Ly2939, suggesting a contribution of the additional CD137 terminal Fv.

**FIGs. 32E-32F** provides the result in an *in vitro* assay for killing of A375-luc cancer cells by Ly2938 with CD28 terminal Fv.

**FIGs. 32G-32H** provides the result of B7H3/CD3/PD-L1 exemplary multispecific antibodies in an *in vitro* assay for killing of A375-luc cancer cells. Ly2846 and Ly2847 showed comparable or stronger cytotoxicity than Ly1963 and higher IFN-γ secretion than Ly1963, suggesting a contribution of the additional PD-L1 terminal Fv.

**FIGs. 32I-32J** provides the result of B7H3/CD3/PD-1 exemplary multispecific antibodies in an *in vitro* assay for killing of A375-luc cancer cells. Ly2904 showed as strong cytotoxicity as Ly1963 in terms of tumor cells killing and IFN-γ secretion, suggesting a contribution of the additional PD-1 terminal Fv.

**FIGs. 32K-32L** provides the result of B7H3/CD3/CTLA4 exemplary multispecific antibodies in an *in vitro* assay for killing of A375-luc cancer cells. Ly2901, Ly2902 and Ly2903 showed a varied and slightly lower cytotoxicity and IFN-γ secretion level than Ly1963, suggesting the contribution of the additional CTLA4 terminal Fv.

Pharmacokinetic analysis on these multi-specific antibodies were performed using mouse models as described above. **FIG. 33** shows presence of circulating Ly1963 antibody over a 21-day period, with a PK profile similar to that of a regular antibody.

Antitumor efficacy was examined in bone marrow transplanted mouse models. Bone marrow from homozygous human CD3 and CD137 knock in mice were transplanted into wild type C57 mice subjected to total body irradiation 12-24h earlier. Three weeks after bone marrow transfer, human B7H3 expressing LL2 tumors were inoculated *s.c.* Five days after tumor inoculation, mice were grouped, and test articles were injected *i.p.* weekly. Mice were weighed and tumor growth was measured twice weekly using calipers. Tumor volume was estimated as 1/2(length x width²). Strong antitumor activity was achieved by Ly1963 as shown in **FIG. 34A**. Trispecific antibody Ly1963 showed more robust antitumor efficacy than either bispecific antibodies Ly1965 or Ly2122 suggests potential benefits in designing synthetic CD3 and CD137 activity for stronger antitumor immunity.

Antitumor efficacy was examined in human PBMC engrafted mouse models. Human B7H3 expressing A375 tumors were inoculated *s.c.* and human PBMC were inoculated i.v. on day0. Four days after tumor inoculation, mice were grouped, and test articles were injected *i.p.* weekly. Mice were weighed and tumor growth was measured twice weekly using calipers. Tumor volume was estimated as 1/2(length x width²). Antitumor activity were achieved by exemplary multispecfic antibodies Ly2823, Ly2936, Ly2937, Ly2600, Ly2846, Ly2847, Ly2938, Ly2939 and Ly2940 as shown in **FIGs. 34B-34L**. It is worth noting that bispecfic antibody Ly2940 showing robust antitumor efficacy suggests potential benefits of anti-CD137 Fv on C-terminal.

### C. Multi-Specific Antibodies Targeting other immune modulators and B7H3

Bispecific antibodies targeting B7H3 and other immune modulators have been evaluated for their anti-tumor efficacy in clinical trials. We made several anti-B7H3 multi-specific antibodies using Fv CD137, and/or CD40 binding modules described in this invention, including Ly2279, Ly2424, Ly2428, Ly2434, Ly2641 Ly2642 Ly2512, Ly2513, Ly2514 and Ly2515.

### (i) Binding to Target Antigens

Binding of these multi-specific antibodies to corresponding targets were evaluated by FCM or ELISA and summarized in **Table 19** below. The multi-specific antibodies exhibited affinity binding to TAA target B7H3 and significantly weakened affinity for immune target CD40 or CD137.

**Table 19. Target binding of anti-B7H3 multi-specific antibodies**

| **Protein** | **B7H3 binding** | **CD40 binding** | **CD137 binding** |
|---|---|---|---|
| TM383 | N/A | ++++ | N/A |
| TM559 | N/A | ++++ | N/A |
| Ly1630 | N/A | N/A | +++++ |
| Ly2279 | ++++ | +++ | N/A |
| Ly2424 | ++++ | +++ | N/A |
| Ly2428 | ++++ | +++ | N/A |
| Ly2434 | ++++ | +++ | N/A |

| | | | |
|---|---|---|---|
| Note: N/A=Not Applicable | | | |

Activation of CD137 or CD40 signaling by these multi-specific antibodies are evaluated using reporter system. Activation of immune cells and tumor cells killing induced by these multi-specific antibodies are examined in vitro. These multi-specific antibodies are dosed in mice for pharmacokinetics analysis. Antitumor efficacy of these multi-specific antibodies are examined in mouse models.

### D. Tri-specific and Bispecific Anti-PD-1 Antibodies

Several anti-PD-1 multi-specific antibodies are made using Fv CD137, CD40 or GITR binding modules described in this invention, including Ly2281, Ly2396, Ly2638, Ly2280, Ly2405, Ly2409, Ly2415, Ly2639, Ly2640, Ly2505, Ly2506, Ly2509, Ly2510, Ly2511, Ly2578, Ly2507, Ly2508, Ly2576 and Ly2577.

Binding of these multi-specific antibodies to corresponding targets are evaluated by FCM or ELISA and summarized in **Table 20** below. The multi-specific antibodies exhibited affinity binding to target PD-1 and significantly weakened affinity for immune target CD40, CD137 or GITR.

**Table 20. Target binding of anti-PD-1 multi-specific antibodies**

| **Protein** | **PD-1 binding** | **CD137 binding** | **CD40 binding** | **GITR binding** |
|---|---|---|---|---|
| Ly2281 | ++++ | ++ | N/A | N/A |
| Ly2396 | ++++ | ++ | N/A | N/A |
| Ly2638 | | | N/A | N/A |
| Ly2280 | ++++ | N/A | +++ | N/A |
| Ly2405 | +++ | N/A | +++ | N/A |
| Ly2409 | ++++ | N/A | ++++ | N/A |
| Ly2415 | ++++ | N/A | ++++ | N/A |

| | | | | |
|---|---|---|---|---|
| Note: N/A=Not Applicable | | | | |

Activation of CD137 or CD40 or GITR, or inhibition of PD-1 signaling by these multi-specific antibodies were evaluated using the reporter systems described earlier or below.

A PD-1 reporter assay was performed to determine the potency of these antibodies. The assay consists of two genetically engineered cell lines, Jurkat T cells expressing human PD-1 and a luciferase reporter driven by an NFκB-response element, Raji cells expressing human PD-L1. Jurkat and Raji cells with or without additional target expressing cells were seeded in the assay plate. Exemplary multi-specific antibodies were added to the assay plate. The plate was incubated in 37°C, in a CO2 (5%) incubator for 6 hours. The luminescence signal was examined with Bright-glo kit from Promega.

Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### E. Tri-specific and Bispecific Anti-PD-L1 Antibodies

Several anti-PD-L1 multi-specific antibodies are made using Fv CD137, CD40, CD28 or CD3 binding modules described in this invention, including Ly2438, Ly2444, Ly2453, Ly2454, Ly2643, Ly2644, Ly2517, Ly2518, Ly2519, Ly2520, Ly2521, Ly2522, Ly2626, Ly2627, Ly2628, Ly2629, Ly2630, Ly2631, Ly2632, Ly2633, Ly2634, Ly2635, Ly2636 and Ly2637.

Binding of these multi-specific antibodies to corresponding targets are evaluated by FCM or ELISA and summarized in **Table 21** below. The multi-specific antibodies exhibited affinity binding to target PD-L1 and significantly weakened affinity for immune target CD40, CD137, CD3 or CD28.

**Table 21. Target binding of anti-PD-L1 multi-specific antibodies**

| **Protein** | **PD-L1 binding** | **CD40 binding** | **CD3 binding** | **CD137 binding** | **CD28 binding** |
|---|---|---|---|---|---|
| Ly2438 | ++++ | ++++ | N/A | N/A | N/A |
| Ly2444 | ++++ | ++++ | N/A | N/A | N/A |
| Ly2453 | ++++ | +++ | N/A | N/A | N/A |
| Ly2454 | ++++ | +++ | N/A | N/A | N/A |

| | | | | | |
|---|---|---|---|---|---|
| Note: N/A=Not Applicable | | | | | |

Activation of CD137, CD40, CD28 or CD3 signaling and blocking of PD-1/PD-L1 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### Example 8: Additional Characterization of Multi-Specific Antibodies

Multi-specific antibodies may be further characterized as exemplified below:

### (i) Tri-specific and Bispecific Anti-PSMA Antibodies

Several anti-PSMA multi-specific antibodies are constructed using Fv CD3 binding modules described in this invention, including Ly2083, Ly2084, Ly2086, Ly2606, Ly2607, Ly2608 and Ly2609. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (ii) Tri-specific and Bispecific Anti-BCMA Antibodies

Several anti-BCMA multi-specific antibodies are made using Fv CD3 binding modules described in this invention, including Ly2312, Ly2315, Ly2602, Ly2603, Ly2604 and Ly2605. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (iii) Tri-specific and Bispecific Anti-HER2 Antibodies

Several anti-HER2 multi-specific antibodies are made using Fv CD3 binding modules described in this invention, including Ly2316, Ly2317, Ly2318, Ly2319, Ly2610, Ly2611, Ly2612, Ly2613, Ly2614, Ly2615, Ly2616 and Ly2617. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (iv) Tri-specific and Bispecific Anti-CEA Antibodies

Several anti-CEA multi-specific antibodies are made using Fv CD3 binding modules described in this invention, including Ly2320, Ly2321, Ly2322, Ly2618, Ly2619, Ly2620, Ly2621, Ly2622 and Ly2623. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (v) Tri-specific and Bispecific Anti-P53 R175H Antibodies

Several anti-p53 R175H multi-specific antibodies are made using Fv CD3 binding modules described in this invention, including Ly2288, Ly2624 and Ly2625. Binding of these multi-specific antibodies to corresponding targets are evaluated by FCM or ELISA. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (vi) Tri-specific and Bispecific Anti-MAGE-A4 Antibodies

Several anti-MAGE-A4 multi-specific antibodies are made using Fv CD3 binding modules described in this invention. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (vii) Tri-specific and Bispecific Anti-PRAME Antibodies

Several anti-CEA multi-specific antibodies are made using Fv CD3 binding modules described in this invention. Binding of these multi-specific antibodies to corresponding targets are evaluated by FCM or ELISA. Activation of CD3 or CD137 signaling by these multi-specific antibodies are assessed using the appropriate reporter systems described earlier. Tumor cell killing by immune cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### (viii) Tri-specific and Bispecific Anti-CD47 Antibodies

Several anti-CD47 multi-specific antibodies are made using Fv CD47 binding modules described in this invention, including Ly2147 and Ly2148. Binding of these multi-specific antibodies to corresponding targets are evaluated by FCM or ELISA. Phagocytosis of tumor cells induced by these multi-specific antibodies are examined *in vitro*. Pharmacokinetics are evaluated in mice by injecting the multi-specific antibodies and measuring the circulating levels of these antibodies for at least 21 days. Anti-tumor efficacy of these multi-specific antibodies are examined using mouse tumor models.

### SEQUENCE TABLES

**Table 1: Components in Bi/Multi-specific Antibodies**

| **Description** | | **Sequence** | **SEQ ID NO** |
|---|---|---|---|
| Flexible Linker Formula | | (GxS)n which X is an integer between 1-6 inclusive, and n is an integer between 1-10, inclusive | 29, 512-516 |
| Flexible Linker 1 (G₂S) | | GGS | 1 |
| Flexible Linker 2 (G₃S) | | GGGS | 2 |
| Flexible Linker 3 (G₄S) | | GGGGS | 3 |
| Flexible Linker 4 (G₄S)₂ | | GGGGSGGGGS | 4 |
| Flexible Linker 5 (G₄S)₃ | | GGGGSGGGGSGGGGS | 5 |
| Flexible Linker 6 (G₄S)₄ | | GGGGSGGGGSGGGGSGGGGS | 6 |
| Flexible Linker 7 (G₄S)₅ | | GGGGSGGGGSGGGGSGGGGSGGGGS | 7 |
| Rigid Linker Motif 1* | | DKTHTCPPCP | 8 |
| Rigid Linker Motif 2* | | DKTHTCPPCPAPELLGP | 9 |
| Rigid Linker Motif 3* | | GGGGSDKTHTCPPC | 10 |
| Rigid Linker A | | GGGSDKTHTCPPCPAPEAAGP | 11 |
| Rigid Linker B | | GGGSGGGSDKTHTCPPCPAPEAAGP | 12 |
| Rigid Linker C | | GGGGSDKTHTCPPCPAPEAAGP | 13 |
| Rigid Linker D | | GGSDKTHTCPPCPAPEAAGP | 14 |
| Rigid Linker E | | GGGGSGGGGSDKTHTCPPCPAPEAAGP | 15 |
| Rigid Linker F | | GGGGSGGGGSGGGGSDKTHTCPPCPAPEAAGP | 16 |
| Rigid Linker G | | DKTHTCPPCPGGGGSGGGGSGGGGS | 17 |
| Rigid Linker H | | DKTHTCPPCGGGGSGGGGSGGGGS | 18 |
| Rigid Linker I | | DKTHTCPPCGGGGSGGGGSGGGGSGGGGS | 19 |
| Rigid Linker J | | DKTHTCPPCGGGGSGGGGS | 20 |
| Rigid Linker K | | DKTHTCPPCPAPEAAGP | 21 |
| Rigid Linker L | | DKTHTCPPCGGGGS | 22 |
| Rigid Linker M | | GGGGSDKTHTCPPCPAPEAAGGP | 23 |
| Rigid Linker N | | GGGGSDKTHTCPPCPAPELLGGP | 24 |
| Rigid Linker O | | GGGGSDKTHTCPPCPAPELLGP | 25 |
| Rigid Linker P | | DKTHTCPPC | 26 |
| Rigid Linker Q | | DKTHTCPPCPAPELLGGP | 27 |
| Peptide linker | | PAPEAAGP | 28 |
| CH2 ADCC mutation S239D/A330L/I332E | | DVFLFPPKPKDTLMISRTPEVTCVVVDVSHEDPEVKFNWYVDGVEVHNAKTKPREEQ YNSTYRVVSVLTVLHQDWLNGKEYKCKVSNKALPLPEEKTISKAK | 30 |
| CH2 DANA mutation | | SVFLFPPKPKDTLMISRTPEVTCVVVAVSHEDPEVKFNWYVDGVEVHNAKTKPREEQ YASTYRVVSVLTVLHQDWLNGKEYKCKVSNKALPAPIEKTISKAK | 31 |
| CH2 P329G | | SVFLFPPKPKDTLMISRTPEVTCVVVDVSHEDPEVKFNWYVDGVEVHNAKTKPREEQ YNSTYRVVSVLTVLHQDWLNGKEYKCKVSNKALGAPIEKTISKAK | 32 |
| CH2, wild-type | | SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIE KTISKAK | 33 |
| CH3, Knob mutation | | GQPREPQVYTLPPSREEMTKNQVSLWCLVKGFYPSDIAVEWESNGQPE NNYKTTPPVLDSDGSFFLYSKLTVDKSRWQQGNVFSCSVMHEALHNH YTQKSLSLSPG | 34 |
| CH3, Hole mutation Protein A binding mutation | | GQPREPQVYTLPPSREEMTKNQVSLSCAVKGFYPSDIAVEWESNGQPE NNYKTTPPVLDSDGSFFLVSKLTVDKSRWQQGNVFSCSVMHEALHNR FTQKSLSLSPG | 35 |
| CH3, Hole mutation | | GQPREPQVYTLPPSREEMTKNQVSLSCAVKGFYPSDIAVEWESNGQPE NNYKTTPPVLDSDGSFFLVSKLTVDKSRWQQGNVFSCSVMHEALHNH YTQKSLSLSPG | 36 |
| CH3, wild-type | | GQPREPQVYTLPPSREEMTKNQVSLTCLVKGFYPSDIAVEWESNGQPE NNYKTTPPVLDSDGSFFLYSKLTVDKSRWQQGNVFSCSVMHEALHNH YTQKSLSLSPG | 37 |
| Anti-CD3 Ly305 | VH | EVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFA YWGQGTLVTVSS | 38 |
| | VL | QAVVTQEPSLTVSPGGTVTLTCRSSTGAVTTSNYANWVQQKPGQAPRGLIGGTNKR APWTPARFSGSLLGGKAALTITGAQAEDEADYYCALWYSNLWVFGGGTKLTVL | 39 |
| Anti-CD28 Ly224 | VH | QVQLVQSGAEVKKPGASVKVSCKASGYTFTSYYIHWVRQAPGQGLEWIGCIYPGNVN TNYNEKFKDRATLTVDTSISTAYMELSRLRSDDTAVYFCTRSHYGLDWNFDVWGQGT TVTVSS | 40 |
| | VL | DIQMTQSPSSLSASVGDRVTITCHASQNIYVWLNWYQQKPGKAPKLLIYKASNLHTGV PSRFSGSGSGTDFTLTISSLQPEDFATYYCQQGQTYPYTFGGGTKVEIK | 41 |
| Anti-CD28 Ly2153 | VH | QVQLVQSGAEVVKPGASVKVSCKASGYTFTSYYIHWVRQAPGQGLEWIGSIYPGNVN TNYAQKFQGRATLTVDTSISTAYMELSRLRSDDTAVYYCTRSHYGLDWNFDVWGKG TTVTVSS | 42 |
| | VL | DIQMTQSPSSLSASVGDRVTITCQASQNIYVWLNWYQQKPGKAPKLLIYKASNLHTGV PSRFSGSGSGTDFTLTISSLQPEDIATYYCQQGQTYPYTFGQGTKLEIK | 43 |
| Anti-CD28 Ly2154 | VH | QVQLQESGPGLVKPSETLSLTCTVSGGSISSYYWSWIRQPPGKGLEWIGYIYYSGITH YNPSLKSRVTISVDTSKIQFSLKLSSVTAADTAVYYCARWGVRRDYYYYGMDVWGQG TTVTVSS | 44 |
| | VL | EIVLTQSPGTLSLSPGERATLSCRASQSVSSSYLAWYQQKPGQAPRLLIYGASSRAT GIPDRFSGSGSGTDFTLTISRLEPEDFAVYYCQQYGSSPWTFGQGTKVEIK | 45 |
| Anti-B7H3 Ly1612 | VH | QVQLVQSGAEVKKPGASVKVSCKASGYTFTSYVMHWVRQKPGQGLEWIGYINPYNE GTESTDKFKGRATMTSDKSTSTVYMELSSLRSEDTAVYYCASIYYGYEGTYFGVWGQ GTLVTVSS | 46 |
| | VL | EIVLTQSPATLSLSPGERATLSCRASSSVSYMHWYQQKPGQAPRPWIYATSNLASGIP ARFSGSRSGTDYTLTISSLEPEDFAVYYCQQWSSNTLTFGGGTKVEIK | 47 |
| Anti-CD19 Ly238 | VH | QVQLQQSGAELVRPGSSVKISCKASGYAFSSYWMNWVKQRPGQGLEWIGQIWPGD GDTNYNGKFKGKATLTADESSSTAYMQLSSLASEDSAVYFCARRETTTVGRYYYAMD YWGQGTTVTVSS | 48 |
| | VL | DIQLTQSPASLAVSLGQRATISCKASQSVDYDGDSYLNWYQQIPGQPPKLLIYDASNL VSGIPPRFSGSGSGTDFTLNIHPVEKVDAATYHCQQSTEDPWTFGGGTKLEIK | 49 |
| Anti-PSMA Ly2082 | VH | EVQLVQSGPEVKKPGATVKISCKTSGYTFTEYTIHWVKQAPGKGLEWIGNINPNNGGT TYNQKFEDKATLTVDKSTDTAYMELSSLRSEDTAVYYCAAGWNFDYWGQGTLLTVSS | 50 |
| | VL | DIQMTQSPSSLSTSVGDRVTLTCKASQDVGTAVDWYQQKPGPSPKLLIYWASTRHTG IPSRFSGSGSGTDFTLTISSLQPEDFADYYCQQYNSYPLTFGPGTKVDIK | 51 |
| Anti-PSMA Ly2085 | VH | QVQLVESGGGLVKPGESLRLSCAASGFTFSDYYMYWVRQAPGKGLEWVAIISDGGY YTYYSDIIKGRFTISRDNAKNSLYLQMNSLKAEDTAVYYCARGFPLLRHGAMDYWGQ GTLVTVSS | 52 |
| | VL | DIQMTQSPSSLSASVGDRVTITCKASQNVDTNVAWYQQKPGQAPKSLIYSASYVYWD VPSRFSGSASGTDFTLTISSVQSEDFATYYCQQYDQQLITFGGGTKLEIK | 53 |
| Anti-CD137 Ly1630 | VH | QVQLVQSGAEVKKPGASVKVSCKASGYTFAGFEMHWVRQAPGQGLEWMGAIDPKT GGTDYNQKFKDRVTMTRDTSISTAYMELSRLRSDDTAVYYCARDLGYFDVWGQGTL VTVSS | 54 |
| | VL | DIQMTQSPSSLSASVGDRVTITCRASQDIRSNLNWYQQKPGGAVKLLIYYTSRLHSGV PSRFSGSGSGTDYTLTISSLQPEDFATYFCQQSEKLPRTFGGGTKVEIR | 55 |
| Anti-CD137 TM173 | VH | | 56 |
| | VL | | 57 |
| Anti-PD-1 Ly516 | VH | QVTLKESGPALVKPTQTLTLTCTFSGFSLSTSGTCVSWIRQPPGKALEWLATICWEDS KGYNPSLKSRLTISKDTSKNQAVLTMTNMDPVDTATYYCARREDSGYFWFPYWGQG TLVTVSS | 58 |
| | VL | NIQMTQSPSSLSASVGDRVTITCKAGQNVNNYLAWYQQKPGKAPKVLIFNANSLQTG VPSRFSGSGSGTDFTLTISSLQPEDFATYYCQQYNSWPTFGGGTKVEIK | 59 |
| Anti-PD-L1 Ly076 | VH | QVQLVQSGAEVKKPGASVKVSCKASGYTFTDFWMSWVRQAPGQGLEWMGQIYPNT GTTHSNEKFKGRVTMTRDTSTSTVYMELSSLRSEDTAVYYCARSYHISTTPNWFAYW GQGTLVTVSS | 60 |
| | VL | DIQMTQSPSSLSASVGDRVTITCKASQNVYKKLEWYQQKPGKVPKVLIYHTNILQTGV PSRFSGSGSGTDYTLTISSLQPEDVATYYCYQWNSGPTFGGGTKVEIK | 61 |
| Anti-CD40 TM383 | VH | QVQLVQSGAEVKKPGASVKVSCKASGYTFTGYYMHWVRQAPGQGLEWMGWINPDS GGTNYAQKFQGRVTMTRDTSISTAYMELNRLRSDDTAVYYCARDQPLGYCTNGVCS YFDYWGQGTLVTVSS | 62 |
| | VL | DIQMTQSPSSVSASVGDRVTITCRASQGIYSWLAWYQQKPGKAPNLLIYTASTLQSG VPSRFSGSGSGTDFTLTISSLQPEDFATYYCQQANIFPLTFGGGTKVEIK | 63 |
| Anti-CD40 TM559 | VH | EVQLVESGGGLVQPGGSLRLSCAASGFTFTNYGLHWVRQAPGKGLEWVSSISPSGG VTYYRDSVKGRFTISRDNSKNTLYLQMNSLRAEDTAVYYCAKPFLGWGGANWIAHW GQGTLVTVSS | 64 |
| | VL | DIQMTQSPSSLSASVGDRVTITCLASEDISNDLAWYQQKPGKAPKLLIYFVDRLLDGV PSRFSGSGSGTDFTLTISSLQPEDFATYYCQQSYKYPPTFGQGTKLEIK | 65 |
| Anti-CD20 Ly239 | VH | | 66 |
| | VL | | 67 |
| Anti-GITR TM685 | VH | QVQLQESGPGLVKPSETLSLTCTVSGFSLTSYNVHWIRQPPGKGLEWIGVIWSGVRT DYNSVLKPRVTISVDTSKNQFSLKLSSVTAADTAVYYCARGTYDDNYHDVMDAWGQ GTLVTVSS | 68 |
| | VL | EIVLTQSPATLSLSPGERATLSCRASKSVRTGMHWYQQKPGQAPRLLIYGASNLESGI PARFSGSGSGTDFTLTISSLEPEDFAVYYCQQSWNHFTFGQGTKLEIK | 69 |
| Anti-HER2 Ly591 | VH | | 70 |
| | VL | | 71 |
| Anti-HER2 TM737 | VH | | 72 |
| | VL | | 73 |
| Anti-P53 mutant Ly2289 | VH | EVQLVESGGGLVQPGGSLRLSCAASGFNVYASGMHWVRQAPGKGLEWVAKIYPDS DYTYYADSVKGRFTISADTSKNTAYLQMNSLRAEDTAVYYCSRDSSFYYVYAMDYWG QGTLVTVSS | 74 |
| | VL | DIQMTQSPSSLSASVGDRVTITCRASQDVNTAVAWYQQKPGKAPKLLIYSAYFLYSGV PSRFSGSRSGTDFTLTISSLQPEDFATYYCQQYSRYSPVTFGQGTKVEIK | 75 |
| Anti-CEA Ly3242 | VH | QVQLVQSGAEVKKPGASVKVSCKASGYTFTEFGMNWVRQAPGQGLEWMGWINTKT GEATYVEEFKGRVTFTTDTSTSTAYMELRSLRSDDTAVYYCARWDFAYYVEAMDYW GQGTTVTVSS | 76 |
| | VL | DIQMTQSPSSLSASVGDRVTITCKASAAVGTYVAWYQQKPGKAPKLLIYSASYRKRG VPSRFSGSGSGTDFTLTISSLQPEDFATYYCHQYYTYPLFTFGQGTKLEIK | 77 |
| Anti-CEA B Ly311 | VH | EVQLVESGGGVVQPGRSLRLSCSASGFDFTTYWMSWVRQAPGKGLEWIGEIHPDSS TINYAPSLKDRFTISRDNAKNTLFLQMDSLRPEDTGVYFCASLYFGFPWFAYWGQGT PVTVSS | 78 |
| | VL | DIQLTQSPSSLSASVGDRVTITCKASQDVGTSVAWYQQKPGKAPKLLIYWTSTRHTGV PSRFSGSGSGTDFTFTISSLQPEDIATYYCQQYSLYRSFGQGTKVEIK | 79 |
| Anti-CEA C Ly312 | VH | EVQLVESGGGLVQPGGSLRLSCAASGFNIKDTYMHWVRQAPGKGLEWVARIDPANG NSKYADSVKGRFTISADTSKNTAYLQMNSLRAEDTAVYYCAPFGYYVSDYAMAYWG QGTLVTVSS | 80 |
| | VL | DIQLTQSPSSLSASVGDRVTITCRAGESVDIFGVGFLHWYQQKPGKAPKLLIYRASNL ESGVPSRFSGSGSRTDFTLTISSLQPEDFATYYCQQTNEDPYTFGQGTKVEIK | 81 |
| Anti-BCMA Ly3019 | VH | QLQLQESGPGLVKPSETLSLTCTVSGGSISSGSYFWGWIRQPPGKGLEWIGSIYYSGI TYYNPSLKSRVTISVDTSKNQFSLKLSSVTAADTAVYYCARHDGAVAGLFDYWGQGT LVTVSS | 82 |
| | VL | SYVLTQPPSVSVAPGQTARITCGGNNIGSKSVHWYQQPPGQAPVVVVYDDSDRPSGI PERFSGSNSGNTATLTISRVEAGDEAVYYCQVWDSSSDHVVFGGGTKLTVL | 83 |
| Anti-BCMA Ly560 | VH | QVQLVQSGAEVKKPGASVKVSCKASGYTFTNHIIHWVRQAPGQGLEWMGYINPYPG YHAYNEKFQGRATMTSDTSTSTVYMELSSLRSEDTAVYYCARDGYYRDTDVLDYWG QGTLVTVSS | 84 |
| | VL | DIQMTQSPSSLSASVGDRVTITCQASQDISNYLNWYQQKPGKAPKLLIYYTSRLHTGV PSRFSGSGSGTDFTFTISSLEPEDIATYYCQQGNTLPWTFGQGTKVEIK | 85 |
| Anti-CD47 Ly1854 | VH | EVQLLESGGGLVQPGGSLRLSCAASGFTFSNFAMSWVRQAPGKGLEWVSTISASGG RTFYADSVKGRITISRDNSKNTLFLQMNGLRAEDTAVYYCAKEGSFGEGVDPWGQGT LVTVSS | 86 |
| | VL | SYEMTQPPSVSVSPGQTARITCSGDALPKKYAYWYQQKSGQAPVLVIYEDNKRPSGI PERFSGSSSGTMATLTISGAQVEDEADYYCYSTDISGNHWVFGGGTELTVL | 87 |
| Anti-MET Ly2149 | VH | | 88 |
| | VL | | 89 |
| Anti-EGFR Ly1369 | VH | | 90 |
| | VL | | 91 |
| Anti-CD3 Ly1761 | VH | | 92 |
| | VL | | 93 |
| Anti-B7H3 Ly2471 | VH | EVQLVESGGGLVKPGGSLKLSCEASRFTFSSYAMSWVRQTPEKRLEWVAAISGGGR YTYYPDSMKGRFTISRDNAKNFLYLQMSSLRSEDTAMYYCARHYDGYLDYWGQGTT LTVSS | 94 |
| | VL | DIVMTQSHKFMSTSIGARVSITCKASQDVRTAVAWYQQKPGQSPKLLIYSASYRYTGV PDRFTGSGSGTDFTFTISSVQAEDLAVYYCQQHYGTPPWTFGGGTKLEIK | 95 |
| Anti-PD-1 Ly711 | VH | | 96 |
| | VL | | 97 |
| Anti-PD-L1 Ly2530 | VH | | 98 |
| | VL | | 99 |
| Anti-CTLA-4 Ly2896 | VH | QVQLVESGGGVVQPGRSLRLSCAASGFTFSSYTMHWVRQAPGKGLEWVTFISYDGN NKYYADSVKGRFTISRDNSKNTLYLQMNSLRAEDTAIYYCARTGWLGPFDYWGQGTL VTVSS | 100 |
| | VL | EIVLTQSPGTLSLSPGERATLSCRASQSVGSSYLAWYQQKPGQAPRLLIYGAFSRAT GIPDRFSGSGSGTDFTLTISRLEPEDFAVYYCQQYGSSPWTFGQGTKVEIK | 101 |
| Anti-CTLA-4 Ly2897 | VH | QVQLVESGGGVVQPGRSLRLSCAASGFTFSSYGMHWVRQAPGKGLEWVAVIWYDG SNKYYADSVKGRFTISRDNSKNTLYLQMNSLRAEDTAVYYCARDPRGATLYYYYYGM DVWGQGTTVTVSS | 102 |
| | VL | DIQMTQSPSSLSASVGDRVTITCRASQSINSYLDWYQQKPGKAPKLLIYAASSLQSGV PSRFSGSGSGTDFTLTISSLQPEDFATYYCQQYYSTPFTFGPGTKVEIK | 103 |
| Anti-CTLA-4 Ly2898 | VH | QVQLVESGAEVKKPGASVKVSCKASGYSFTGYTMNWVRQAPGQCLEWIGLINPYNNI TNYAQKFQGRVTFTVDTSISTAYMELSRLRSDDTGVYFCARLDYRSYWGQGTLVTVS A | 104 |
| | VL | QAVVTQEPSLTVSPGGTVTLTCGSSTGAVTTSNFPNWVQQKPGQAPRSLIGGTNNK ASWTPARFSGSLLGGKAALTISGAQPEDEAEYYCALWYSNHWVFGCGTKLTVL | 105 |

| | | | |
|---|---|---|---|
| *: May have a (GxS)n fragment located at the N-terminus and/or the C-terminus. X is an integer between 1-6 inclusive, and n is an integer between 1-10, inclusive | | | |

**Table 2: Sequences for Exemplary Bi-Multi-Specific Antibodies**

| **Molecule name** | **Chains** | **Sequence** | **SEQ ID NO** |
|---|---|---|---|
| Ly1800 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLWCLVKGFYPSDIAVEWESNGQPENNYKTTPPVLDSDGSFFLYSKLTVDKS RWQQGNVFSCSVMHEALHNHYTQKSLSLSPGGGGGSGGGGSGGGGSGGGGSQVQLVQS GAEVKKPGASVKVSCKASGYTFAGFEMHWVRQAPGQGLEWMGAIDPKTGGTDYNQKFKDR VTMTRDTSISTAYMELSRLRSDDTAVYYCARDLGYFDVWGQGTLVTVSSGGGGSGGGGSG GGGSGGGGSDIQMTQSPSSLSASVGDRVTITCRASQDIRSNLNWYQQKPGGAVKLLIYYTSR LHSGVPSRFSGSGSGTDYTLTISSLQPEDFATYFCQQSEKLPRTFGGGTKVEIR | 359 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSQVQLVQSGAEVKKPGASVKVSCK ASGYTFAGFEMHWVRQAPGQGLEWMGAIDPKTGGTDYNQKFKDRVTMTRDTSISTAYMELS RLRSDDTAVYYCARDLGYFDVWGQGTLVTVSSGGGGSGGGGSGGGGSGGGGSDIQMTQS PSSLSASVGDRVTITCRASQDIRSNLNWYQQKPGGAVKLLIYYTSRLHSGVPSRFSGSGSGT DYTLTISSLQPEDFATYFCQQSEKLPRTFGGGTKVEIR | 413 |
| | 3rd | EIVLTQSPATLSLSPGERATLSCRASSSVSYMHWYQQKPGQAPRPWIYATSNLASGIPARFS GSRSGTDYTLTISSLEPEDFAVYYCQQWSSNTLTFGGGTKVEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 131 |
| Ly1803 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLWCLVKGFYPSDIAVEWESNGQPENNYKTTPPVLDSDGSFFLYSKLTVDKS RWQQGNVFSCSVMHEALHNHYTQKSLSLSPGGGGGSGGGGSGGGGSGGGGSQVQLVQS GAEVKKPGASVKVSCKASGYTFAGFEMHWVRQAPGQGLEWMGAIDPKTGGTDYNQKFKDR VTMTRDTSISTAYMELSRLRSDDTAVYYCARDLGYFDVWGQGTLVTVSS | 361 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 415 |
| | 3rd | *see above* | 131 |
| Ly1891 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSSGGGGSGGGGSGGGGSGGGGSDIQMTQSPSSLSASVGDRVTITCRASQDIRSNLNWY QQKPGGAVKLLIYYTSRLHSGVPSRFSGSGSGTDYTLTISSLQPEDFATYFCQQSEKLPRTFG GGTKVEIR | 360 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSQVQLVQSGA EVKKPGASVKVSCKASGYTFAGFEMHWVRQAPGQGLEWMGAIDPKTGGTDYNQKFKDRVT MTRDTSISTAYMELSRLRSDDTAVYYCARDLGYFDVWGQGTLVTVSSGGGGSGGGGSGGG GSGGGGSDIQMTQSPSSLSASVGDRVTITCRASQDIRSNLNWYQQKPGGAVKLLIYYTSRLH SGVPSRFSGSGSGTDYTLTISSLQPEDFATYFCQQSEKLPRTFGGGTKVEIR | 414 |
| | 3rd | *see above* | 131 |
| Ly1899 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 362 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 416 |
| | 3rd | *see above* | 131 |
| Ly1961 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLWCLVKGFYPSDIAVEWESNGQPENNYKTTPPVLDSDGSFFLYSKLTVDKS RWQQGNVFSCSVMHEALHNHYTQKSLSLSPGGGGGSGGGGSGGGGSGGGGSQVQLVQS GAEVKKPGASVKVSCKASGYTFAGFEMHWVRQAPGQGLEWMGAIDPKTGGTDYNQKFKDR VTMTRDTSISTAYMELSRLRSDDTAVYYCARDLGYFDVWGQGTLVTVSS | 363 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNHYTQKSLSLSPGGGGGSGGGGSGGGGSGGGGSDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 417 |
| | 3rd | *see above* | 131 |
| Ly1963 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 364 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNHYTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 418 |
| | 3rd | *see above* | 131 |
| Ly1965 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPG | 369 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPG | 423 |
| | 3rd | *see above* | 131 |
| Ly1966 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFAGFEMHWVRQAPGQGLE WMGAIDPKTGGTDYNQKFKDRVTMTRDTSISTAYMELSRLRSDDTAVYYCARDLGYFDVWG QGTLVTVSS | 235 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPGGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 247 |
| | 3rd | DIQLTQSPASLAVSLGQRATISCKASQSVDYDGDSYLNWYQQIPGQPPKLLIYDASNLVSGIPP RFSGSGSGTDFTLNIHPVEKVDAATYHCQQSTEDPWTFGGGTKLEIKRTVAAPSVFIFPPSDE QLKSGTASVVCLLNNFYPREAKVQWKVDNALQSGNSQESVTEQDSKDSTYSLSSTLTLSKAD YEKHKVYACEVTHQGLSSPVTKSFNRGEC | 107 |
| Ly1967 | 1st | *see above* | 235 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNHYTQKSLSLSPGGGGGSGGGGSGGGGSGGGGSDIQM TQSPSSLSASVGDRVTITCRASQDIRSNLNWYQQKPGGAVKLLIYYTSRLHSGVPSRFSGSG SGTDYTLTISSLQPEDFATYFCQQSEKLPRTFGGGTKVEIR | 248 |
| | 3rd | *see above* | 107 |
| Ly2083 | 1st | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCGGGGSGGGGSGGGGSGGGGSEVQLVESG GGLVQPGGSLRLSCAASGFTFSTYAMNWVRQAPGKGLEWVGRIRSKYNNYATYYADSVKG RFTISRDDSKNSLYLQMNSLKTEDTAVYYCVRHGNFGNSYVSWFAYWGQGTLVTVSSGGGG SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPGGGGGSGGGGSGGGGSGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSS | 202 |
| | 2nd | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCGGGGSGGGGSGGGGSGGGGSQAVVTQEP SLTVSPGGTVTLTCRSSTGAVTTSNYANWVQQKPGQAPRGLIGGTNKRAPWTPARFSGSLL GGKAALTITGAQAEDEADYYCALWYSNLWVFGGGTKLTVLGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 204 |
| | 3rd | DIQMTQSPSSLSTSVGDRVTLTCKASQDVGTAVDWYQQKPGPSPKLLIYWASTRHTGIPSRF SGSGSGTDFTLTISSLQPEDFADYYCQQYNSYPLTFGPGTKVDIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 127 |
| Ly2084 | 1st | *see above* | 202 |
| | 2nd | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCGGGGSGGGGSGGGGSGGGGSQAVVTQEP SLTVSPGGTVTLTCRSSTGAVTTSNYANWVQQKPGQAPRGLIGGTNKRAPWTPARFSGSLL GGKAALTITGAQAEDEADYYCALWYSNLWVFGGGTKLTVLGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNHYTQKSLSLSPGGGGGSGGGGSGGGGSGGGGSDIQMTQSPSSLSASV GDRVTITCRASQDIRSNLNWYQQKPGGAVKLLIYYTSRLHSGVPSRFSGSGSGTDYTLTISSL QPEDFATYFCQQSEKLPRTFGGGTKVEIR | 205 |
| | 3rd | *see above* | 127 |
| Ly2086 | 1st | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSEV QLVESGGGLVQPGGSLRLSCAASGFTFSTYAMNWVRQAPGKGLEWVGRIRSKYNNYATYYA DSVKGRFTISRDDSKNSLYLQMNSLKTEDTAVYYCVRHGNFGNSYVSWFAYWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 269 |
| | 2nd | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 271 |
| | 3rd | DIQMTQSPSSLSASVGDRVTITCKASQNVDTNVAWYQQKPGQAPKSLIYSASYVYWDVPSRF SGSASGTDFTLTISSVQSEDFATYYCQQYDQQLITFGGGTKLEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 114 |
| Ly2118 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFAGFEMHWVRQAPGQGLEWMGAIDPKTGGTDYN QKFKDRVTMTRDTSISTAYMELSRLRSDDTAVYYCARDLGYFDVWGQGTLVTVSSGGGGSD KTHTCPPCPAPEAAGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREP QVYTLPPSREEMTKNQVSLWCLVKGFYPSDIAVEWESNGQPENNYKTTPPVLDSDGSFFLYS KLTVDKSRWQQGNVFSCSVMHEALHNHYTQKSLSLSPGGGGGSGGGGSGGGGSGGGGS QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSS | 451 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIRGGGGSDKTHTCPPCPAPEA AGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSREEMTK NQVSLSCAVKGFYPSDIAVEWESNGQPENNYKTTPPVLDSDGSFFLVSKLTVDKSRWQQGN VFSCSVMHEALHNRFTQKSLSLSPGGGGGSGGGGSGGGGSGGGGSNIQMTQSPSSLSASV GDRVTITCKAGQNVNNYLAWYQQKPGKAPKVLIFNANSLQTGVPSRFSGSGSGTDFTLTISSL QPEDFATYYCQQYNSWPTFGGGTKVEIK | 339 |
| | 3rd | *see above* | 131 |
| Ly2121 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFTGYYMHWVRQAPGQGLEWMGWINPDSGGTNY AQKFQGRVTMTRDTSISTAYMELNRLRSDDTAVYYCARDQPLGYCTNGVCSYFDYWGQGTL VTVSSGGGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVTLKESGPALVKPTQTLTLTCTFSGFSLSTSGTCVSWIRQPPGKALEWLATI CWEDSKGYNPSLKSRLTISKDTSKNQAVLTMTNMDPVDTATYYCARREDSGYFWFPYWGQ GTLVTVSS | 452 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSVSASVGDRVTITCRASQGIYSWLAWYQQKPGKAPNLLIYTASTLQSGVPSRFSG SGSGTDFTLTISSLQPEDFATYYCQQANIFPLTFGGGTKVEIKGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSNIQMTQSPSSLSASVG DRVTITCKAGQNVNNYLAWYQQKPGKAPKVLIFNANSLQTGVPSRFSGSGSGTDFTLTISSLQ PEDFATYYCQQYNSWPTFGGGTKVEIK | 344 |
| | 3rd | *see above* | 131 |
| Ly2122 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSQVQLVQSGAEVKKPGASVKVSCK ASGYTFAGFEMHWVRQAPGQGLEWMGAIDPKTGGTDYNQKFKDRVTMTRDTSISTAYMELS RLRSDDTAVYYCARDLGYFDVWGQGTLVTVSS | 319 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 320 |
| | 3rd | *see above* | 131 |
| Ly2125 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGSGGGGSGGGGSGG GGSQVQLVQSGAEVKKPGASVKVSCKASGYTFTSYYIHWVRQAPGQGLEWIGCIYPGNVNT NYNEKFKDRATLTVDTSISTAYMELSRLRSDDTAVYFCTRSHYGLDWNFDVWGQGTTVTVSS | 349 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCHASQNIYVWLNWYQQKPGKAPKLLIYKASNLHTGVPSRFSGSGSGTDFTLTISSLQ PEDFATYYCQQGQTYPYTFGGGTKVEIK | 403 |
| | 3rd | *see above* | 131 |
| Ly2126 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFTSYYIHWVRQAPGQGLEWIGCIYPGN VNTNYNEKFKDRATLTVDTSISTAYMELSRLRSDDTAVYFCTRSHYGLDWNFDVWGQGTTVT VSS | 350 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGSDKTHTCPPCPAP EAAGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEM TKNQVSLSCAVKGFYPSDIAVEWESNGQPENNYKTTPPVLDSDGSFFLVSKLTVDKSRWQQ GNVFSCSVMHEALHNRFTQKSLSLSPGGGGGSGGGGSGGGGSGGGGSDIQMTQSPSSLS ASVGDRVTITCHASQNIYVWLNWYQQKPGKAPKLLIYKASNLHTGVPSRFSGSGSGTDFTLTI SSLQPEDFATYYCQQGQTYPYTFGGGTKVEIK | 404 |
| | 3rd | *see above* | 131 |
| Ly2127 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFTSYYIHWVRQAPGQGLEWIGCIYPGN VNTNYNEKFKDRATLTVDTSISTAYMELSRLRSDDTAVYFCTRSHYGLDWNFDVWGQGTTVT VSS | 351 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCHASQNIYVWLNWYQQKPGKAPKLLIYKASNLHTGVPSRFSGSGSGTDFTL TISSLQPEDFATYYCQQGQTYPYTFGGGTKVEIK | 405 |
| | 3rd | *see above* | 131 |
| Ly2128 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFTSYYIHWVRQAPGQGLEWIGCIYP GNVNTNYNEKFKDRATLTVDTSISTAYMELSRLRSDDTAVYFCTRSHYGLDWNFDVWGQGT TVTVSS | 346 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCHASQNIYVWLNWYQQKPGKAPKLLIYKASNLHTGVPSRFSGSGSGTDFTL TISSLQPEDFATYYCQQGQTYPYTFGGGTKVEIK | 400 |
| | 3rd | *see above* | 131 |
| Ly2129 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGSGGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFTSYYIHWVRQAPGQGLEWIGCIY PGNVNTNYNEKFKDRATLTVDTSISTAYMELSRLRSDDTAVYFCTRSHYGLDWNFDVWGQG TTVTVSS | 352 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGSGGGSDKTHTC PPCPAPEAAGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNRFTQKSLSLSPGGGGGSGGGGSGGGGSGGGGSDIQMTQ SPSSLSASVGDRVTITCHASQNIYVWLNWYQQKPGKAPKLLIYKASNLHTGVPSRFSGSGSG TDFTLTISSLQPEDFATYYCQQGQTYPYTFGGGTKVEIK | 406 |
| | 3rd | *see above* | 131 |
| Ly2130 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFTSYYIHWVRQAPGQGLEWI GCIYPGNVNTNYNEKFKDRATLTVDTSISTAYMELSRLRSDDTAVYFCTRSHYGLDWNFDVW GQGTTVTVSS | 353 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPGGGGGSGGGGSGGGGSGGGGSDIQMT QSPSSLSASVGDRVTITCHASQNIYVWLNWYQQKPGKAPKLLIYKASNLHTGVPSRFSGSGS GTDFTLTISSLQPEDFATYYCQQGQTYPYTFGGGTKVEIK | 407 |
| | 3rd | *see above* | 131 |
| Ly2131 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSGGGGSGGGGSDKTHTCPPCPAPEAAGPSVFLFPPKPKDTLMISRTPEVTCVVVD VSHEDPEVKFNWYVDGVEVHNAKTKPREEQYNSTYRVVSVLTVLHQDWLNGKEYKCKVSNK ALPAPIEKTISKAKGQPREPQVYTLPPSREEMTKNQVSLWCLVKGFYPSDIAVEWESNGQPE NNYKTTPPVLDSDGSFFLYSKLTVDKSRWQQGNVFSCSVMHEALHNHYTQKSLSLSPGGGG GSGGGGSGGGGSGGGGSQVQLVQSGAEVKKPGASVKVSCKASGYTFTSYYIHWVRQAPG QGLEWIGCIYPGNVNTNYNEKFKDRATLTVDTSISTAYMELSRLRSDDTAVYFCTRSHYGLDW NFDVWGQGTTVTVSS | 354 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSGGGGSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLSCAVKGFYPSDIAVEWESNGQPENNYKTTPPVLDSDGSFF LVSKLTVDKSRWQQGNVFSCSVMHEALHNRFTQKSLSLSPGGGGGSGGGGSGGGGSGGG GSDIQMTQSPSSLSASVGDRVTITCHASQNIYVWLNWYQQKPGKAPKLLIYKASNLHTGVPSR FSGSGSGTDFTLTISSLQPEDFATYYCQQGQTYPYTFGGGTKVEIK | 408 |
| | 3rd | *see above* | 131 |
| Ly2132 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSQVQLVQSGAEVKKPGASVKVSCKASGYTFTSYYIHWVRQAPGQGLEWIGCIYPGNVNT NYNEKFKDRATLTVDTSISTAYMELSRLRSDDTAVYFCTRSHYGLDWNFDVWGQGTTVTVSS | 355 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSDIQMTQSPSSLSASVG DRVTITCHASQNIYVWLNWYQQKPGKAPKLLIYKASNLHTGVPSRFSGSGSGTDFTLTISSLQ PEDFATYYCQQGQTYPYTFGGGTKVEIK | 409 |
| | 3rd | *see above* | 131 |
| Ly2133 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSQVQ LVQSGAEVKKPGASVKVSCKASGYTFTSYYIHWVRQAPGQGLEWIGCIYPGNVNTNYNEKFK DRATLTVDTSISTAYMELSRLRSDDTAVYFCTRSHYGLDWNFDVWGQGTTVTVSS | 356 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSDIQMTQSPSSLSASVGDRVTIT CHASQNIYVWLNWYQQKPGKAPKLLIYKASNLHTGVPSRFSGSGSGTDFTLTISSLQPEDFAT YYCQQGQTYPYTFGGGTKVEIK | 410 |
| | 3rd | *see above* | 131 |
| Ly2134 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSQVQLVQSG AEVKKPGASVKVSCKASGYTFTSYYIHWVRQAPGQGLEWIGCIYPGNVNTNYNEKFKDRATL TVDTSISTAYMELSRLRSDDTAVYFCTRSHYGLDWNFDVWGQGTTVTVSS | 357 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSDIQMTQSPSSLSASVGDRVTITCHASQ NIYVWLNWYQQKPGKAPKLLIYKASNLHTGVPSRFSGSGSGTDFTLTISSLQPEDFATYYCQQ GQTYPYTFGGGTKVEIK | 411 |
| | 3rd | *see above* | 131 |
| Ly2135 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCPGGG GSGGGGSGGGGSQVQLVQSGAEVKKPGASVKVSCKASGYTFTSYYIHWVRQAPGQGLEWI GCIYPGNVNTNYNEKFKDRATLTVDTSISTAYMELSRLRSDDTAVYFCTRSHYGLDWNFDVW GQGTTVTVSS | 358 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCPGGGGSGGGGSGGGGSDIQMT QSPSSLSASVGDRVTITCHASQNIYVWLNWYQQKPGKAPKLLIYKASNLHTGVPSRFSGSGS GTDFTLTISSLQPEDFATYYCQQGQTYPYTFGGGTKVEIK | 412 |
| | 3rd | *see above* | 131 |
| Ly2136 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFTSYYIHWVRQAPGQGLEWI GCIYPGNVNTNYNEKFKDRATLTVDTSISTAYMELSRLRSDDTAVYFCTRSHYGLDWNFDVW GQGTTVTVSS | 396 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPGGGGGSGGGGSGGGGSGGGGSDIQMT QSPSSLSASVGDRVTITCHASQNIYVWLNWYQQKPGKAPKLLIYKASNLHTGVPSRFSGSGS GTDFTLTISSLQPEDFATYYCQQGQTYPYTFGGGTKVEIK | 398 |
| | 3rd | *see above* | 131 |
| Ly2137 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSEVQLVES GGGLVQPGGSLRLSCAASGFTFSTYAMNWVRQAPGKGLEWVGRIRSKYNNYATYYADSVK GRFTISRDDSKNSLYLQMNSLKTEDTAVYYCVRHGNFGNSYVSWFAYWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGGSGGGGSGGGGSGG GGSQVQLVQSGAEVKKPGASVKVSCKASGYTFTSYYIHWVRQAPGQGLEWIGCIYPGNVNT NYNEKFKDRATLTVDTSISTAYMELSRLRSDDTAVYFCTRSHYGLDWNFDVWGQGTTVTVSS | 336 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSQAVVTQE PSLTVSPGGTVTLTCRSSTGAVTTSNYANWVQQKPGQAPRGLIGGTNKRAPWTPARFSGSL LGGKAALTITGAQAEDEADYYCALWYSNLWVFGGGTKLTVLGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCHASQNIYVWLNWYQQKPGKAPKLLIYKASNLHTGVPSRFSGSGSGTDFTLTISSLQ PEDFATYYCQQGQTYPYTFGGGTKVEIK | 456 |
| | 3rd | *see above* | 131 |
| Ly2138 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSEVQLVESGGGLV QPGGSLRLSCAASGFTFSTYAMNWVRQAPGKGLEWVGRIRSKYNNYATYYADSVKGRFTIS RDDSKNSLYLQMNSLKTEDTAVYYCVRHGNFGNSYVSWFAYWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLVQSGAEVKKPGASVKVSCKASGYTFTSYYIHWVRQAPGQGLEWIGCIYPGNVNTNYNEKF KDRATLTVDTSISTAYMELSRLRSDDTAVYFCTRSHYGLDWNFDVWGQGTTVTVSS | 334 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSQAVVTQEPSLTV SPGGTVTLTCRSSTGAVTTSNYANWVQQKPGQAPRGLIGGTNKRAPWTPARFSGSLLGGKA ALTITGAQAEDEADYYCALWYSNLWVFGGGTKLTVLGGGGSDKTHTCPPCPAPEAAGPSVFL FPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSREEMTKNQVSLSC AVKGFYPSDIAVEWESNGQPENNYKTTPPVLDSDGSFFLVSKLTVDKSRWQQGNVFSCSVM HEALHNRFTQKSLSLSPGGGGGSGGGGSGGGGSGGGGSDIQMTQSPSSLSASVGDRVTIT CHASQNIYVWLNWYQQKPGKAPKLLIYKASNLHTGVPSRFSGSGSGTDFTLTISSLQPEDFAT YYCQQGQTYPYTFGGGTKVEIK | 458 |
| | 3rd | *see above* | 131 |
| Ly2139 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSEVQLVESGGGLVQPGGS LRLSCAASGFTFSTYAMNWVRQAPGKGLEWVGRIRSKYNNYATYYADSVKGRFTISRDDSK NSLYLQMNSLKTEDTAVYYCVRHGNFGNSYVSWFAYWGQGTLVTVSSGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLWCLVKGFYPSDIAVEWESNGQPENNYKTTPPVLDSDGSFFLYSKLTVDKSR WQQGNVFSCSVMHEALHNHYTQKSLSLSPGGGGGSGGGGSGGGGSGGGGSQVQLVQSG AEVKKPGASVKVSCKASGYTFTSYYIHWVRQAPGQGLEWIGCIYPGNVNTNYNEKFKDRATL TVDTSISTAYMELSRLRSDDTAVYFCTRSHYGLDWNFDVWGQGTTVTVSS | 332 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSQAVVTQEPSLTVSPGGTV TLTCRSSTGAVTTSNYANWVQQKPGQAPRGLIGGTNKRAPWTPARFSGSLLGGKAALTITGA QAEDEADYYCALWYSNLWVFGGGTKLTVLGGGGSDKTHTCPPCPAPEAAGPSVFLFPPKPK DTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLSCAVKGF YPSDIAVEWESNGQPENNYKTTPPVLDSDGSFFLVSKLTVDKSRWQQGNVFSCSVMHEALH NRFTQKSLSLSPGGGGGSGGGGSGGGGSGGGGSDIQMTQSPSSLSASVGDRVTITCHASQ NIYVWLNWYQQKPGKAPKLLIYKASNLHTGVPSRFSGSGSGTDFTLTISSLQPEDFATYYCQQ GQTYPYTFGGGTKVEIK | 460 |
| | 3rd | *see above* | 131 |
| Ly2145 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVVKPGASVKVSCKASGYTFTSYYIHWVRQAPGQGLEWIGSIYP GNVNTNYAQKFQGRATLTVDTSISTAYMELSRLRSDDTAVYYCTRSHYGLDWNFDVWGKGT TVTVSS | 347 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCQASQNIYVWLNWYQQKPGKAPKLLIYKASNLHTGVPSRFSGSGSGTDFTL TISSLQPEDIATYYCQQGQTYPYTFGQGTKLEIK | 401 |
| | 3rd | *see above* | 131 |
| Ly2146 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLQESGPGLVKPSETLSLTCTVSGGSISSYYWSWIRQPPGKGLEWIGYIYYS GITHYNPSLKSRVTISVDTSKIQFSLKLSSVTAADTAVYYCARWGVRRDYYYYGMDVWGQGT TVTVSS | 348 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSEIVLTQSPGTL SLSPGERATLSCRASQSVSSSYLAWYQQKPGQAPRLLIYGASSRATGIPDRFSGSGSGTDFT LTISRLEPEDFAVYYCQQYGSSPWTFGQGTKVEIK | 402 |
| | 3rd | *see above* | 131 |
| Ly2147 | 1st | EVQLVESGGGLVQPGGSLRLSCAASGYTFTSYWLHWVRQAPGKGLEWVGMIDPSNSDTRF NPNFKDRFTISADTSKNTAYLQMNSLRAEDTAVYYCATYRSYVTPLDYWGQGTLVTVSSASV AAPSVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGSGGG GSEVQLLESGGGLVQPGGSLRLSCAASGFTFSNFAMSWVRQAPGKGLEWVSTISASGGRTF YADSVKGRITISRDNSKNTLFLQMNGLRAEDTAVYYCAKEGSFGEGVDPWGQGTLVTVSSG GGGSDKTHTCPPCPAPELLGGPDVFLFPPKPKDTLMISRTPEVTCVVVDVSHEDPEVKFNWY VDGVEVHNAKTKPREEQYNSTYRVVSVLTVLHQDWLNGKEYKCKVSNKALPLPEEKTISKAK GQPREPQVYTLPPSRDELTKNQVSLWCLVKGFYPSDIAVEWESNGQPENNYKTTPPVLDSD GSFFLYSKLTVDKSRWQQGNVFSCSVMHEALHNHYTQKSLSLSPGK | 192 |
| | 2nd | QVQLQESGPGLVKPSETLSLTCTVSGGSVSSGDYYWTWIRQSPGKGLEWIGHIYYSGNTNY NPSLKSRLTISIDTSKTQFSLKLSSVTAADTAIYYCVRDRVTGAFDIWGQGTMVTVSSASTKGP SVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCGGGGSGGGGSGGGGSGGGGSSYEMT QPPSVSVSPGQTARITCSGDALPKKYAYWYQQKSGQAPVLVIYEDNKRPSGIPERFSGSSSG TMATLTISGAQVEDEADYYCYSTDISGNHWVFGGGTELTVLGGGGSDKTHTCPPCPAPELLG GPDVFLFPPKPKDTLMISRTPEVTCVVVDVSHEDPEVKFNWYVDGVEVHNAKTKPREEQYNS TYRVVSVLTVLHQDWLNGKEYKCKVSNKALPLPEEKTISKAKGQPREPQVYTLPPSRDELTKN QVSLSCAVKGFYPSDIAVEWESNGQPENNYKTTPPVLDSDGSFFLVSKLTVDKSRWQQGNV FSCSVMHEALHNRFTQKSLSLSPGK | 221 |
| | 3rd | DIQMTQSPSSLSASVGDRVTITCKSSQSLLYTSSQKNYLAWYQQKPGKAPKLLIYWASTRESG VPSRFSGSGSGTDFTLTISSLQPEDFATYYCQQYYAYPWTFGQGTKVEIKSSASTKGPSVFPL APSSKSTSGGTAALGCLVKDYFPEPVTVSWNSGALTSGVHTFPAVLQSSGLYSLSSVVTVPS SSLGTQTYICNVNHKPSNTKVDKKVEPKSC | 116 |
| | 4th | DIQMTQSPSSLSASVGDRVTITCQASQDISNYLNWYQQKPGKAPKLLIYDASNLETGVPSRFS GSGSGTDFTFTISSLQPEDIATYFCQHFDHLPLAFGGGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 117 |
| Ly2148 | 1st | EVQLVESGGGLVQPGGSLRLSCAASGYTFTSYWLHWVRQAPGKGLEWVGMIDPSNSDTRF NPNFKDRFTISADTSKNTAYLQMNSLRAEDTAVYYCATYRSYVTPLDYWGQGTLVTVSSASV AAPSVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECDKTHTCPPCPAPELLGGPDV FLFPPKPKDTLMISRTPEVTCVVVDVSHEDPEVKFNWYVDGVEVHNAKTKPREEQYNSTYRV VSVLTVLHQDWLNGKEYKCKVSNKALPLPEEKTISKAKGQPREPQVYTLPPSRDELTKNQVSL WCLVKGFYPSDIAVEWESNGQPENNYKTTPPVLDSDGSFFLYSKLTVDKSRWQQGNVFSCS VMHEALHNHYTQKSLSLSPGKGGGGSGGGGSGGGGSGGGGSEVQLLESGGGLVQPGGSL RLSCAASGFTFSNFAMSWVRQAPGKGLEWVSTISASGGRTFYADSVKGRITISRDNSKNTLFL QMNGLRAEDTAVYYCAKEGSFGEGVDPWGQGTLVTVSS | 191 |
| | 2nd | QVQLQESGPGLVKPSETLSLTCTVSGGSVSSGDYYWTWIRQSPGKGLEWIGHIYYSGNTNY NPSLKSRLTISIDTSKTQFSLKLSSVTAADTAIYYCVRDRVTGAFDIWGQGTMVTVSSASTKGP SVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPDVFLFPPKPK DTLMISRTPEVTCVVVDVSHEDPEVKFNWYVDGVEVHNAKTKPREEQYNSTYRVVSVLTVLH QDWLNGKEYKCKVSNKALPLPEEKTISKAKGQPREPQVYTLPPSRDELTKNQVSLSCAVKGF YPSDIAVEWESNGQPENNYKTTPPVLDSDGSFFLVSKLTVDKSRWQQGNVFSCSVMHEALH NRFTQKSLSLSPGKGGGGSGGGGSGGGGSGGGGSSYEMTQPPSVSVSPGQTARITCSGD ALPKKYAYWYQQKSGQAPVLVIYEDNKRPSGIPERFSGSSSGTMATLTISGAQVEDEADYYC YSTDISGNHWVFGGGTELTVL | 220 |
| | 3rd | *see above* | 116 |
| | 4th | *see above* | 117 |
| Ly2157 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGSGGGGSGGGGSGG GGSQVQLVQSGAEVKKPGASVKVSCKASGYTFAGFEMHWVRQAPGQGLEWMGAIDPKTG GTDYNQKFKDRVTMTRDTSISTAYMELSRLRSDDTAVYYCARDLGYFDVWGQGTLVTVSS | 370 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 424 |
| | 3rd | *see above* | 131 |
| Ly2158 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 371 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGSDKTHTCPPCPAP EAAGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEM TKNQVSLSCAVKGFYPSDIAVEWESNGQPENNYKTTPPVLDSDGSFFLVSKLTVDKSRWQQ GNVFSCSVMHEALHNRFTQKSLSLSPGGGGGSGGGGSGGGGSGGGGSDIQMTQSPSSLS ASVGDRVTITCRASQDIRSNLNWYQQKPGGAVKLLIYYTSRLHSGVPSRFSGSGSGTDYTLTI SSLQPEDFATYFCQQSEKLPRTFGGGTKVEIR | 425 |
| | 3rd | *see above* | 131 |
| Ly2159 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 372 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 426 |
| | 3rd | *see above* | 131 |
| Ly2160 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGSGGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 373 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGSGGGSDKTHTC PPCPAPEAAGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNRFTQKSLSLSPGGGGGSGGGGSGGGGSGGGGSDIQMTQ SPSSLSASVGDRVTITCRASQDIRSNLNWYQQKPGGAVKLLIYYTSRLHSGVPSRFSGSGSG TDYTLTISSLQPEDFATYFCQQSEKLPRTFGGGTKVEIR | 427 |
| | 3rd | *see above* | 131 |
| Ly2161 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFAGFEMHWVRQAPGQGLE WMGAIDPKTGGTDYNQKFKDRVTMTRDTSISTAYMELSRLRSDDTAVYYCARDLGYFDVWG QGTLVTVSS | 374 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPGGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 428 |
| | 3rd | *see above* | 131 |
| Ly2162 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSGGGGSGGGGSDKTHTCPPCPAPEAAGPSVFLFPPKPKDTLMISRTPEVTCVVVD VSHEDPEVKFNWYVDGVEVHNAKTKPREEQYNSTYRVVSVLTVLHQDWLNGKEYKCKVSNK ALPAPIEKTISKAKGQPREPQVYTLPPSREEMTKNQVSLWCLVKGFYPSDIAVEWESNGQPE NNYKTTPPVLDSDGSFFLYSKLTVDKSRWQQGNVFSCSVMHEALHNHYTQKSLSLSPGGGG GSGGGGSGGGGSGGGGSQVQLVQSGAEVKKPGASVKVSCKASGYTFAGFEMHWVRQAP GQGLEWMGAIDPKTGGTDYNQKFKDRVTMTRDTSISTAYMELSRLRSDDTAVYYCARDLGY FDVWGQGTLVTVSS | 375 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSGGGGSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLSCAVKGFYPSDIAVEWESNGQPENNYKTTPPVLDSDGSFF LVSKLTVDKSRWQQGNVFSCSVMHEALHNRFTQKSLSLSPGGGGGSGGGGSGGGGSGGG GSDIQMTQSPSSLSASVGDRVTITCRASQDIRSNLNWYQQKPGGAVKLLIYYTSRLHSGVPSR FSGSGSGTDYTLTISSLQPEDFATYFCQQSEKLPRTFGGGTKVEIR | 429 |
| | 3rd | *see above* | 131 |
| Ly2163 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSQVQLVQSGAEVKKPGASVKVSCKASGYTFAGFEMHWVRQAPGQGLEWMGAIDPKTG GTDYNQKFKDRVTMTRDTSISTAYMELSRLRSDDTAVYYCARDLGYFDVWGQGTLVTVSS | 376 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 430 |
| | 3rd | *see above* | 131 |
| Ly2164 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSS | 377 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSDIQMTQSPSSLSASVGDRVTIT CRASQDIRSNLNWYQQKPGGAVKLLIYYTSRLHSGVPSRFSGSGSGTDYTLTISSLQPEDFAT YFCQQSEKLPRTFGGGTKVEIR | 431 |
| | 3rd | *see above* | 131 |
| Ly2165 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSQVQLVQSG AEVKKPGASVKVSCKASGYTFAGFEMHWVRQAPGQGLEWMGAIDPKTGGTDYNQKFKDRV TMTRDTSISTAYMELSRLRSDDTAVYYCARDLGYFDVWGQGTLVTVSS | 378 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSDIQMTQSPSSLSASVGDRVTITCRASQ DIRSNLNWYQQKPGGAVKLLIYYTSRLHSGVPSRFSGSGSGTDYTLTISSLQPEDFATYFCQQ SEKLPRTFGGGTKVEIR | 432 |
| | 3rd | *see above* | 131 |
| Ly2166 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCGGGG SGGGGSGGGGSQVQLVQSGAEVKKPGASVKVSCKASGYTFAGFEMHWVRQAPGQGLEW MGAIDPKTGGTDYNQKFKDRVTMTRDTSISTAYMELSRLRSDDTAVYYCARDLGYFDVWGQ GTLVTVSS | 379 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCGGGGSGGGGSGGGGSDIQMTQ SPSSLSASVGDRVTITCRASQDIRSNLNWYQQKPGGAVKLLIYYTSRLHSGVPSRFSGSGSG TDYTLTISSLQPEDFATYFCQQSEKLPRTFGGGTKVEIR | 433 |
| | 3rd | *see above* | 131 |
| Ly2167 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 240 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 253 |
| | 3rd | *see above* | 107 |
| Ly2168 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 241 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGSDKTHTC PPCPAPEAAGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNRFTQKSLSLSPGGGGGSGGGGSGGGGSGGGGSDIQMTQ SPSSLSASVGDRVTITCRASQDIRSNLNWYQQKPGGAVKLLIYYTSRLHSGVPSRFSGSGSG TDYTLTISSLQPEDFATYFCQQSEKLPRTFGGGTKVEIR | 254 |
| | 3rd | *see above* | 107 |
| Ly2169 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPGGGGGSGGGG SGGGGSGGGGSQVQLVQSGAEVKKPGASVKVSCKASGYTFAGFEMHWVRQAPGQGLEW MGAIDPKTGGTDYNQKFKDRVTMTRDTSISTAYMELSRLRSDDTAVYYCARDLGYFDVWGQ GTLVTVSS | 242 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGSDKTHT CPPCPAPEAAGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYTL PPSREEMTKNQVSLSCAVKGFYPSDIAVEWESNGQPENNYKTTPPVLDSDGSFFLVSKLTVD KSRWQQGNVFSCSVMHEALHNRFTQKSLSLSPGGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 255 |
| | 3rd | *see above* | 107 |
| Ly2170 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGSGGGSDKTHTCPPCPAPEAAGPSVFLFPPKPKDTLMISRTPEVTCVVVDVS HEDPEVKFNWYVDGVEVHNAKTKPREEQYNSTYRVVSVLTVLHQDWLNGKEYKCKVSNKAL PAPIEKTISKAKGQPREPQVYTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGGGGGS GGGGSGGGGSGGGGSQVQLVQSGAEVKKPGASVKVSCKASGYTFAGFEMHWVRQAPGQ GLEWMGAIDPKTGGTDYNQKFKDRVTMTRDTSISTAYMELSRLRSDDTAVYYCARDLGYFDV WGQGTLVTVSS | 243 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGSGGGSD KTHTCPPCPAPEAAGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREP QVYTLPPSREEMTKNQVSLSCAVKGFYPSDIAVEWESNGQPENNYKTTPPVLDSDGSFFLVS KLTVDKSRWQQGNVFSCSVMHEALHNRFTQKSLSLSPGGGGGSGGGGSGGGGSGGGGSD IQMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIR | 256 |
| | 3rd | *see above* | 107 |
| Ly2171 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSGGGGSDKTHTCPPCPAPEAAGPSVFLFPPKPKDTLMISRTPEVTCVVVD VSHEDPEVKFNWYVDGVEVHNAKTKPREEQYNSTYRVVSVLTVLHQDWLNGKEYKCKVSNK ALPAPIEKTISKAKGQPREPQVYTLPPSREEMTKNQVSLWCLVKGFYPSDIAVEWESNGQPE NNYKTTPPVLDSDGSFFLYSKLTVDKSRWQQGNVFSCSVMHEALHNHYTQKSLSLSPGGGG GSGGGGSGGGGSGGGGSQVQLVQSGAEVKKPGASVKVSCKASGYTFAGFEMHWVRQAP GQGLEWMGAIDPKTGGTDYNQKFKDRVTMTRDTSISTAYMELSRLRSDDTAVYYCARDLGY FDVWGQGTLVTVSS | 244 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLSCAVKGFYPSDIAVEWESNGQPENNYKTTPPVLDSDGSFF LVSKLTVDKSRWQQGNVFSCSVMHEALHNRFTQKSLSLSPGGGGGSGGGGSGGGGSGGG GSDIQMTQSPSSLSASVGDRVTITCRASQDIRSNLNWYQQKPGGAVKLLIYYTSRLHSGVPSR FSGSGSGTDYTLTISSLQPEDFATYFCQQSEKLPRTFGGGTKVEIR | 257 |
| | 3rd | *see above* | 107 |
| Ly2172 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSGGGGSGGGGSDKTHTCPPCPAPEAAGPSVFLFPPKPKDTLMISRTPEV TCVVVDVSHEDPEVKFNWYVDGVEVHNAKTKPREEQYNSTYRVVSVLTVLHQDWLNGKEYK CKVSNKALPAPIEKTISKAKGQPREPQVYTLPPSREEMTKNQVSLWCLVKGFYPSDIAVEWES NGQPENNYKTTPPVLDSDGSFFLYSKLTVDKSRWQQGNVFSCSVMHEALHNHYTQKSLSLS PGGGGGSGGGGSGGGGSGGGGSQVQLVQSGAEVKKPGASVKVSCKASGYTFAGFEMHW VRQAPGQGLEWMGAIDPKTGGTDYNQKFKDRVTMTRDTSISTAYMELSRLRSDDTAVYYCA RDLGYFDVWGQGTLVTVSS | 245 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSGGG GSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLSCAVKGFYPSDIAVEWESNGQPENNYKTTPPVLD SDGSFFLVSKLTVDKSRWQQGNVFSCSVMHEALHNRFTQKSLSLSPGGGGGSGGGGSGGG GSGGGGSDIQMTQSPSSLSASVGDRVTITCRASQDIRSNLNWYQQKPGGAVKLLIYYTSRLH SGVPSRFSGSGSGTDYTLTISSLQPEDFATYFCQQSEKLPRTFGGGTKVEIR | 258 |
| | 3rd | *see above* | 107 |
| Ly2278 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPG | 234 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPG | 246 |
| | 3rd | *see above* | 107 |
| Ly2279 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFTGYYMHWVRQAPGQGLEWMGWINPDSGGTNY AQKFQGRVTMTRDTSISTAYMELNRLRSDDTAVYYCARDQPLGYCTNGVCSYFDYWGQGTL VTVSSGGGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPG | 450 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSVSASVGDRVTITCRASQGIYSWLAWYQQKPGKAPNLLIYTASTLQSGVPSRFSG SGSGTDFTLTISSLQPEDFATYYCQQANIFPLTFGGGTKVEIKGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 343 |
| | 3rd | *see above* | 131 |
| Ly2280 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFTGYYMHWVRQAPGQGLEWMGWINPDSGGTNYAQ KFQGRVTMTRDTSISTAYMELNRLRSDDTAVYYCARDQPLGYCTNGVCSYFDYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPG | 503 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSVSASVGDRVTITCRASQGIYSWLAWYQQKPGKAPNLLIYTASTLQSGVPSRFSGS GSGTDFTLTISSLQPEDFATYYCQQANIFPLTFGGGTKVEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 489 |
| | 3rd | NIQMTQSPSSLSASVGDRVTITCKAGQNVNNYLAWYQQKPGKAPKVLIFNANSLQTGVPSRF SGSGSGTDFTLTISSLQPEDFATYYCQQYNSWPTFGGGTKVEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 207 |
| Ly2281 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPG | 502 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 481 |
| | 3rd | *see above* | 207 |
| Ly2288 | 1st | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 156 |
| | 2nd | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGGGGGSGGGGSGGGGSGGGGSDIQMTQSPS SLSASVGDRVTITCRASQDIRSNLNWYQQKPGGAVKLLIYYTSRLHSGVPSRFSGSGSGTDY TLTISSLQPEDFATYFCQQSEKLPRTFGGGTKVEIR | 158 |
| | 3rd | DIQMTQSPSSLSASVGDRVTITCRASQDVNTAVAWYQQKPGKAPKLLIYSAYFLYSGVPSRFS GSRSGTDFTLTISSLQPEDFATYYCQQYSRYSPVTFGQGTKVEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 124 |
| Ly2295 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLVQSGAEVKKPGASVKVSCKASGYTFTSYVMHWVRQKPGQGLEWIGYINPYNEGTESTDK FKGRATMTSDKSTSTVYMELSSLRSEDTAVYYCASIYYGYEGTYFGVWGQGTLVTVSS | 504 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSEIVLTQSPATLSLSPG ERATLSCRASSSVSYMHWYQQKPGQAPRPWIYATSNLASGIPARFSGSRSGTDYTLTISSLE PEDFAVYYCQQWSSNTLTFGGGTKVEIK | 483 |
| | 3rd | *see above* | 207 |
| Ly2298 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFTGYYMHWVRQAPGQGLEWMGWINPDSGGTNYAQ KFQGRVTMTRDTSISTAYMELNRLRSDDTAVYYCARDQPLGYCTNGVCSYFDYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFTSYVMHWVRQKPGQGLEWIGYIN PYNEGTESTDKFKGRATMTSDKSTSTVYMELSSLRSEDTAVYYCASIYYGYEGTYFGVWGQ GTLVTVSS | 505 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSVSASVGDRVTITCRASQGIYSWLAWYQQKPGKAPNLLIYTASTLQSGVPSRFSGS GSGTDFTLTISSLQPEDFATYYCQQANIFPLTFGGGTKVEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSEIVLTQSPATLSLSPG ERATLSCRASSSVSYMHWYQQKPGQAPRPWIYATSNLASGIPARFSGSRSGTDYTLTISSLE PEDFAVYYCQQWSSNTLTFGGGTKVEIK | 490 |
| | 3rd | *see above* | 207 |
| Ly2307 | 1st | EVQLVQSGAEVKKPGASVKVSCKASGYTFTNYYIHWVRQAPGQGLEWIGWIYPGDGNTKYN EKFKGRATLTADTSTSTAYLELSSLRSEDTAVYYCARDSYSNYYFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPELLGGPSVFLFPPK PKDTLMISRTPEVTCVVVDVSHEDPEVKFNWYVDGVEVHNAKTKPREEQYGSTYRVVSVLTV LHQDWLNGKEYKCKVSNKALPAPIEKTISKAKGQPREPQVYTLPPSREEMTKNQVSLSCAVK GFYPSDIAVEWESNGQPENNYKTTPPVLDSDGSFFLVSKLTVDKSRWQQGNVFSCSVMHEA LHNHYTQKSLSLSPGK | 199 |
| | 2nd | EVQLVESGGGLVQPGGSLRLSCAASGYTFTSYNMHWVRQAPGKGLEWVGAIYPGNGDTSY NQKFKGRFTISVDKSKNTLYLQMNSLRAEDTAVYYCARVVYYSNSYWYFDVWGQGTLVTVS SASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYGSTYRVV SVLTVLHQDWLNGKEYKCKVSNKALPAPIEKTISKAKGQPREPQVYTLPPSREEMTKNQVSL WCLVKGFYPSDIAVEWESNGQPENNYKTTPPVLDSDGSFFLYSKLTVDKSRWQQGNVFSCS VMHEALHNHYTQKSLSLSPGK | 190 |
| | 3rd | DIVMTQSPDSLAVSLGERATINCKSSQSLLNSRTRKNYLAWYQQKPGQPPKLLIYWASTRES GVPDRFSGSGSGTDFTLTISSLQAEDVAVYYCTQSFILRTFGQGTKVEIKRTVAAPSVFIFPPS DEQLKSGTASVVCLLNNFYPREAKVQWKVDNALQSGNSQESVTEQDSKDSTYSLSSTLTLSK ADYEKHKVYACEVTHQGLSSPVTKSFNRGEC | 129 |
| | 4th | DIQMTQSPSSLSASVGDRVTITCRASSSVSYMHWYQQKPGKAPKPLIYAPSNLASGVPSRFS GSGSGTDFTLTISSLQPEDFATYYCQQWSFNPPTFGQGTKVEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 126 |
| Ly2309 | 1st | QVQLVQSGAEVKKPGSSVKVSCKASGYAFSYSWINWVRQAPGQGLEWMGRIFPGDGDTDY NGKFKGRVTITADKSTSTAYMELSSLRSEDTAVYYCARNVFDGYWLVYWGQGTLVTVSSAST KGPSVFPLAPSSKSTSGGTAALGCLVEDYFPEPVTVSWNSGALTSGVHTFPAVLQSSGLYSL SSVVTVPSSSLGTQTYICNVNHKPSNTKVDEKVEPKSCDKTHTCPPCPAPEAAGGPSVFLFP PKPKDTLMISRTPEVTCVVVDVSHEDPEVKFNWYVDGVEVHNAKTKPREEQYNSTYRVVSVL TVLHQDWLNGKEYKCKVSNKALGAPIEKTISKAKGQPREPQVCTLPPSRDELTKNQVSLSCA VKGFYPSDIAVEWESNGQPENNYKTTPPVLDSDGSFFLVSKLTVDKSRWQQGNVFSCSVMH EALHNHYTQKSLSLSPGK | 468 |
| | 2nd | QVQLVQSGAEVKKPGSSVKVSCKASGYAFSYSWINWVRQAPGQGLEWMGRIFPGDGDTDY NGKFKGRVTITADKSTSTAYMELSSLRSEDTAVYYCARNVFDGYWLVYWGQGTLVTVSSAST KGPSVFPLAPSSKSTSGGTAALGCLVEDYFPEPVTVSWNSGALTSGVHTFPAVLQSSGLYSL SSVVTVPSSSLGTQTYICNVNHKPSNTKVDEKVEPKSCDGGGGSGGGGSQAVVTQEPSLTV SPGGTVTLTCGSSTGAVTTSNYANWVQEKPGQAFRGLIGGTNKRAPGTPARFSGSLLGGKA ALTLSGAQPEDEAEYYCALWYSNLWVFGGGTKLTVLSSASTKGPSVFPLAPSSKSTSGGTAA LGCLVKDYFPEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPEVTCVVVDVS HEDPEVKFNWYVDGVEVHNAKTKPREEQYNSTYRVVSVLTVLHQDWLNGKEYKCKVSNKAL GAPIEKTISKAKGQPREPQVYTLPPCRDELTKNQVSLWCLVKGFYPSDIAVEWESNGQPENN YKTTPPVLDSDGSFFLYSKLTVDKSRWQQGNVFSCSVMHEALHNHYTQKSLSLSPGK | 467 |
| | 3rd | EVQLLESGGGLVQPGGSLRLSCAASGFTFSTYAMNWVRQAPGKGLEWVSRIRSKYNNYATY YADSVKGRFTISRDDSKNTLYLQMNSLRAEDTAVYYCVRHGNFGNSYVSWFAYWGQGTLVT VSSASVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | 132 |
| | 4th | DIVMTQTPLSLPVTPGEPASISCRSSKSLLHSNGITYLYWYLQKPGQSPQLLIYQMSNLVSGVP DRFSGSGSGTDFTLKISRVEAEDVGVYYCAQNLELPYTFGGGTKVEIKRTVAAPSVFIFPPSD RKLKSGTASVVCLLNNFYPREAKVQWKVDNALQSGNSQESVTEQDSKDSTYSLSSTLTLSKA DYEKHKVYACEVTHQGLSSPVTKSFNRGEC | 130 |
| Ly2312 | 1st | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 311 |
| | 2nd | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGGGGGSGGGGSGGGGSGGGGSDIQMTQSPS SLSASVGDRVTITCRASQDIRSNLNWYQQKPGGAVKLLIYYTSRLHSGVPSRFSGSGSGTDY TLTISSLQPEDFATYFCQQSEKLPRTFGGGTKVEIR | 315 |
| | 3rd | DIQMTQSPSSLSASVGDRVTITCQASQDISNYLNWYQQKPGKAPKLLIYYTSRLHTGVPSRFS GSGSGTDFTFTISSLEPEDIATYYCQQGNTLPWTFGQGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 118 |
| Ly2315 | 1st | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 212 |
| | 2nd | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPGGGGGSGGGGSGGGGSGGGGSDIQMTQSPSSL SASVGDRVTITCRASQDIRSNLNWYQQKPGGAVKLLIYYTSRLHSGVPSRFSGSGSGTDYTLT ISSLQPEDFATYFCQQSEKLPRTFGGGTKVEIR | 216 |
| | 3rd | SYVLTQPPSVSVAPGQTARITCGGNNIGSKSVHWYQQPPGQAPVVVVYDDSDRPSGIPERFS GSNSGNTATLTISRVEAGDEAVYYCQVWDSSSDHVVFGGGTKLTVLGQPKAAPSVTLFPPSS EELQANKATLVCLISDFYPGAVTVAWKGDSSPVKAGVETTTPSKQSNNKYAASSYLSLTPEQ WKSHRSYSCQVTHEGSTVEKTVAPTECS | 511 |
| Ly2316 | 1st | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSEV QLVESGGGLVQPGGSLRLSCAASGFTFSTYAMNWVRQAPGKGLEWVGRIRSKYNNYATYYA DSVKGRFTISRDDSKNSLYLQMNSLKTEDTAVYYCVRHGNFGNSYVSWFAYWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 137 |
| | 2nd | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 139 |
| | 3rd | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGQGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 122 |
| Ly2317 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 180 |
| | 2nd | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 182 |
| | 3rd | DIQMTQSPSSLSASVGDRVTITCKASQDVSIGVAWYQQKPGKAPKLLIYSASYRYTGVPSRFS GSGSGTDFTLTISSLQPEDFATYYCQQYYIYPYTFGQGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 112 |
| Ly2318 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS VAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGGSGG GGSEVQLVESGGGLVQPGGSLRLSCAASGFTFSTYAMNWVRQAPGKGLEWVGRIRSKYNN YATYYADSVKGRFTISRDDSKNSLYLQMNSLKTEDTAVYYCVRHGNFGNSYVSWFAYWGQG TLVTVSSGGGGSDKTHTCPPCPAPEAAGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPGGGGGSGGGG SGGGGSGGGGSQVQLVQSGAEVKKPGASVKVSCKASGYTFAGFEMHWVRQAPGQGLEW MGAIDPKTGGTDYNQKFKDRVTMTRDTSISTAYMELSRLRSDDTAVYYCARDLGYFDVWGQ GTLVTVSS | 186 |
| | 2nd | *see above* | 139 |
| | 3rd | DIQMTQSPSSLSASVGDRVTITCKASQDVSIGVAWYQQKPGKAPKLLIYSASYRYTGVPSRFS GSGSGTDFTLTISSLQPEDFATYYCQQYYIYPYTFGQGTKVEIKSSASTKGPSVFPLAPSSKST SGGTAALGCLVKDYFPEPVTVSWNSGALTSGVHTFPAVLQSSGLYSLSSVVTVPSSSLGTQT YICNVNHKPSNTKVDKKVEPKSC | 113 |
| | 4th | *see above* | 122 |
| Ly2319 | 1st | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGQGTKVEIKGGGGSGGGGSGGGGSGG GGSEVQLVESGGGLVQPGGSLRLSCAASGFNIKDTYIHWVRQAPGKGLEWVARIYPTNGYT RYADSVKGRFTISADTSKNTAYLQMNSLRAEDTAVYYCSRWGGDGFYAMDYWGQGTLVTVS SGGGGSGGGGSGGGGSGGGGSEVQLVESGGGLVQPGGSLRLSCAASGFTFSTYAMNWV RQAPGKGLEWVGRIRSKYNNYATYYADSVKGRFTISRDDSKNSLYLQMNSLKTEDTAVYYCV RHGNFGNSYVSWFAYWGQGTLVTVSSGGGGSDKTHTCPPCPAPEAAGPSVFLFPPKPKDT LMISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLWCLVKGFYP SDIAVEWESNGQPENNYKTTPPVLDSDGSFFLYSKLTVDKSRWQQGNVFSCSVMHEALHNH YTQKSLSLSPGGGGGSGGGGSGGGGSGGGGSQVQLVQSGAEVKKPGASVKVSCKASGYT FAGFEMHWVRQAPGQGLEWMGAIDPKTGGTDYNQKFKDRVTMTRDTSISTAYMELSRLRSD DTAVYYCARDLGYFDVWGQGTLVTVSS | 120 |
| | 2nd | *see above* | 182 |
| | 3rd | *see above* | 112 |
| Ly2320 | 1st | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 195 |
| | 2nd | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPGGGGGSGGGGSGGGGSGGGGSDIQMTQSPSSL SASVGDRVTITCRASQDIRSNLNWYQQKPGGAVKLLIYYTSRLHSGVPSRFSGSGSGTDYTLT ISSLQPEDFATYFCQQSEKLPRTFGGGTKVEIR | 197 |
| | 3rd | DIQLTQSPSSLSASVGDRVTITCKASQDVGTSVAWYQQKPGKAPKLLIYWTSTRHTGVPSRFS GSGSGTDFTFTISSLQPEDIATYYCQQYSLYRSFGQGTKVEIKRTVAAPSVFIFPPSDEQLKSG TASVVCLLNNFYPREAKVQWKVDNALQSGNSQESVTEQDSKDSTYSLSSTLTLSKADYEKHK VYACEVTHQGLSSPVTKSFNRGEC | 109 |
| Ly2321 | 1st | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 146 |
| | 2nd | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 150 |
| | 3rd | DIQLTQSPSSLSASVGDRVTITCRAGESVDIFGVGFLHWYQQKPGKAPKLLIYRASNLESGVP SRFSGSGSRTDFTLTISSLQPEDFATYYCQQTNEDPYTFGQGTKVEIKRTVAAPSVFIFPPSDE QLKSGTASVVCLLNNFYPREAKVQWKVDNALQSGNSQESVTEQDSKDSTYSLSSTLTLSKAD YEKHKVYACEVTHQGLSSPVTKSFNRGEC | 110 |
| Ly2322 | 1st | QVQLVQSGAEVKKPGASVKVSCKASGYTFTEFGMNWVRQAPGQGLEWMGWINTKTGEATY VEEFKGRVTFTTDTSTSTAYMELRSLRSDDTAVYYCARWDFAYYVEAMDYWGQGTTVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFSTYAMNWVRQAPGKGLEWVGRIRSKYNNYAT YYADSVKGRFTISRDDSKNSLYLQMNSLKTEDTAVYYCVRHGNFGNSYVSWFAYWGQGTLV TVSSGGGGSDKTHTCPPCPAPEAAGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKTI SKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 305 |
| | 2nd | QVQLVQSGAEVKKPGASVKVSCKASGYTFTEFGMNWVRQAPGQGLEWMGWINTKTGEATY VEEFKGRVTFTTDTSTSTAYMELRSLRSDDTAVYYCARWDFAYYVEAMDYWGQGTTVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPGGGGGSGGGGSGGGGSGGGGSDIQMTQS PSSLSASVGDRVTITCRASQDIRSNLNWYQQKPGGAVKLLIYYTSRLHSGVPSRFSGSGSGT DYTLTISSLQPEDFATYFCQQSEKLPRTFGGGTKVEIR | 307 |
| | 3rd | DIQMTQSPSSLSASVGDRVTITCKASAAVGTYVAWYQQKPGKAPKLLIYSASYRKRGVPSRFS GSGSGTDFTLTISSLQPEDFATYYCHQYYTYPLFTFGQGTKLEIKRTVAAPSVFIFPPSDEQLK SGTASVVCLLNNFYPREAKVQWKVDNALQSGNSQESVTEQDSKDSTYSLSSTLTLSKADYEK HKVYACEVTHQGLSSPVTKSFNRGEC | 111 |
| Ly2326 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPEAAGPS VFLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSREEMTKNQV SLWCLVKGFYPSDIAVEWESNGQPENNYKTTPPVLDSDGSFFLYSKLTVDKSRWQQGNVFS CSVMHEALHNHYTQKSLSLSPGGGGGSGGGGSGGGGSGGGGSQVQLVQSGAEVKKPGAS VKVSCKASGYTFAGFEMHWVRQAPGQGLEWMGAIDPKTGGTDYNQKFKDRVTMTRDTSIST AYMELSRLRSDDTAVYYCARDLGYFDVWGQGTLVTVSS | 231 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPEAAGPS VFLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSREEMTKNQV SLSCAVKGFYPSDIAVEWESNGQPENNYKTTPPVLDSDGSFFLVSKLTVDKSRWQQGNVFS CSVMHEALHNRFTQKSLSLSPGGGGGSGGGGSGGGGSGGGGSDIQMTQSPSSLSASVGD RVTITCRASQDIRSNLNWYQQKPGGAVKLLIYYTSRLHSGVPSRFSGSGSGTDYTLTISSLQP EDFATYFCQQSEKLPRTFGGGTKVEIR | 232 |
| | 3rd | *see above* | 107 |
| Ly2364 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFAGFEMHWVRQAPGQGLE WMGAIDPKTGGTDYNQKFKDRVTMTRDTSISTAYMELSRLRSDDTAVYYCARDLGYFDVWG QGTLVTVSS | 397 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNRFTQKSLSLSPGGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 399 |
| | 3rd | *see above* | 131 |
| Ly2365 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSEVQLVES GGGLVQPGGSLRLSCAASGFTFSTYAMNWVRQAPGKGLEWVGRIRSKYNNYATYYADSVK GRFTISRDDSKNSLYLQMNSLKTEDTAVYYCVRHGNFGNSYVSWFAYWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGSGGGGSGGGGSGG GGSQVQLVQSGAEVKKPGASVKVSCKASGYTFAGFEMHWVRQAPGQGLEWMGAIDPKTG GTDYNQKFKDRVTMTRDTSISTAYMELSRLRSDDTAVYYCARDLGYFDVWGQGTLVTVSS | 337 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSQAVVTQE PSLTVSPGGTVTLTCRSSTGAVTTSNYANWVQQKPGQAPRGLIGGTNKRAPWTPARFSGSL LGGKAALTITGAQAEDEADYYCALWYSNLWVFGGGTKLTVLGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 457 |
| | 3rd | *see above* | 131 |
| Ly2366 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSEVQLVESGGGLV QPGGSLRLSCAASGFTFSTYAMNWVRQAPGKGLEWVGRIRSKYNNYATYYADSVKGRFTIS RDDSKNSLYLQMNSLKTEDTAVYYCVRHGNFGNSYVSWFAYWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLVQSGAEVKKPGASVKVSCKASGYTFAGFEMHWVRQAPGQGLEWMGAIDPKTGGTDYNQ KFKDRVTMTRDTSISTAYMELSRLRSDDTAVYYCARDLGYFDVWGQGTLVTVSS | 335 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSQAVVTQEPSLTV SPGGTVTLTCRSSTGAVTTSNYANWVQQKPGQAPRGLIGGTNKRAPWTPARFSGSLLGGKA ALTITGAQAEDEADYYCALWYSNLWVFGGGTKLTVLGGGGSDKTHTCPPCPAPEAAGPSVFL FPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSREEMTKNQVSLSC AVKGFYPSDIAVEWESNGQPENNYKTTPPVLDSDGSFFLVSKLTVDKSRWQQGNVFSCSVM HEALHNRFTQKSLSLSPGGGGGSGGGGSGGGGSGGGGSDIQMTQSPSSLSASVGDRVTIT CRASQDIRSNLNWYQQKPGGAVKLLIYYTSRLHSGVPSRFSGSGSGTDYTLTISSLQPEDFAT YFCQQSEKLPRTFGGGTKVEIR | 459 |
| | 3rd | *see above* | 131 |
| Ly2367 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSEVQLVESGGGLVQPGGS LRLSCAASGFTFSTYAMNWVRQAPGKGLEWVGRIRSKYNNYATYYADSVKGRFTISRDDSK NSLYLQMNSLKTEDTAVYYCVRHGNFGNSYVSWFAYWGQGTLVTVSSGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLWCLVKGFYPSDIAVEWESNGQPENNYKTTPPVLDSDGSFFLYSKLTVDKSR WQQGNVFSCSVMHEALHNHYTQKSLSLSPGGGGGSGGGGSGGGGSGGGGSQVQLVQSG AEVKKPGASVKVSCKASGYTFAGFEMHWVRQAPGQGLEWMGAIDPKTGGTDYNQKFKDRV TMTRDTSISTAYMELSRLRSDDTAVYYCARDLGYFDVWGQGTLVTVSS | 333 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSQAVVTQEPSLTVSPGGTV TLTCRSSTGAVTTSNYANWVQQKPGQAPRGLIGGTNKRAPWTPARFSGSLLGGKAALTITGA QAEDEADYYCALWYSNLWVFGGGTKLTVLGGGGSDKTHTCPPCPAPEAAGPSVFLFPPKPK DTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLSCAVKGF YPSDIAVEWESNGQPENNYKTTPPVLDSDGSFFLVSKLTVDKSRWQQGNVFSCSVMHEALH NRFTQKSLSLSPGGGGGSGGGGSGGGGSGGGGSDIQMTQSPSSLSASVGDRVTITCRASQ DIRSNLNWYQQKPGGAVKLLIYYTSRLHSGVPSRFSGSGSGTDYTLTISSLQPEDFATYFCQQ SEKLPRTFGGGTKVEIR | 461 |
| | 3rd | *see above* | 131 |
| Ly2368 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCEVQLVESGGGLVQPGGSLRLSCA ASGFTFSTYAMNWVRQAPGKGLEWVGRIRSKYNNYATYYADSVKGRFTISRDDSKNSLYLQ MNSLKTEDTAVYYCVRHGNFGNSYVSWFAYWGQGTLVTVSSGGGGSDKTHTCPPCPAPEA AGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSREEMTK NQVSLWCLVKGFYPSDIAVEWESNGQPENNYKTTPPVLDSDGSFFLYSKLTVDKSRWQQGN VFSCSVMHEALHNHYTQKSLSLSPGGGGGSGGGGSGGGGSGGGGSQVQLVQSGAEVKKP GASVKVSCKASGYTFAGFEMHWVRQAPGQGLEWMGAIDPKTGGTDYNQKFKDRVTMTRDT SISTAYMELSRLRSDDTAVYYCARDLGYFDVWGQGTLVTVSS | 331 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCQAVVTQEPSLTVSPGGTVTLTCRS STGAVTTSNYANWVQQKPGQAPRGLIGGTNKRAPWTPARFSGSLLGGKAALTITGAQAEDE ADYYCALWYSNLWVFGGGTKLTVLGGGGSDKTHTCPPCPAPEAAGPSVFLFPPKPKDTLMIS RTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLSCAVKGFYPSDIA VEWESNGQPENNYKTTPPVLDSDGSFFLVSKLTVDKSRWQQGNVFSCSVMHEALHNRFTQ KSLSLSPGGGGGGSGGGGSGGGGSGGGGSDIQMTQSPSSLSASVGDRVTITCRASQDIRSNL NWYQQKPGGAVKLLIYYTSRLHSGVPSRFSGSGSGTDYTLTISSLQPEDFATYFCQQSEKLP RTFGGGTKVEIR | 466 |
| | 3rd | *see above* | 131 |
| Ly2384 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGSEVQLVESGGGLVQPGGSLR LSCAASGFTFSTYAMNWVRQAPGKGLEWVGRIRSKYNNYATYYADSVKGRFTISRDDSKNSL YLQMNSLKTEDTAVYYCVRHGNFGNSYVSWFAYWGQGTLVTVSSGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLWCLVKGFYPSDIAVEWESNGQPENNYKTTPPVLDSDGSFFLYSKLTVDKSRWQ QGNVFSCSVMHEALHNHYTQKSLSLSPGGGGGSGGGGSGGGGSGGGGSQVQLVQSGAEV KKPGASVKVSCKASGYTFAGFEMHWVRQAPGQGLEWMGAIDPKTGGTDYNQKFKDRVTMT RDTSISTAYMELSRLRSDDTAVYYCARDLGYFDVWGQGTLVTVSS | 462 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGSQAVVTQEPSLTVSPGGTVT LTCRSSTGAVTTSNYANWVQQKPGQAPRGLIGGTNKRAPWTPARFSGSLLGGKAALTITGAQ AEDEADYYCALWYSNLWVFGGGTKLTVLGGGGSDKTHTCPPCPAPEAAGPSVFLFPPKPKD TLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLSCAVKGFY PSDIAVEWESNGQPENNYKTTPPVLDSDGSFFLVSKLTVDKSRWQQGNVFSCSVMHEALHN RFTQKSLSLSPGGGGGSGGGGSGGGGSGGGGSDIQMTQSPSSLSASVGDRVTITCRASQDI RSNLNWYQQKPGGAVKLLIYYTSRLHSGVPSRFSGSGSGTDYTLTISSLQPEDFATYFCQQS EKLPRTFGGGTKVEIR | 463 |
| | 3rd | *see above* | 131 |
| Ly2385 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGSEVQLVESGGGLVQPGGSLRL SCAASGFTFSTYAMNWVRQAPGKGLEWVGRIRSKYNNYATYYADSVKGRFTISRDDSKNSL YLQMNSLKTEDTAVYYCVRHGNFGNSYVSWFAYWGQGTLVTVSSGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLWCLVKGFYPSDIAVEWESNGQPENNYKTTPPVLDSDGSFFLYSKLTVDKSRWQ QGNVFSCSVMHEALHNHYTQKSLSLSPGGGGGSGGGGSGGGGSGGGGSQVQLVQSGAEV KKPGASVKVSCKASGYTFAGFEMHWVRQAPGQGLEWMGAIDPKTGGTDYNQKFKDRVTMT RDTSISTAYMELSRLRSDDTAVYYCARDLGYFDVWGQGTLVTVSS | 464 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGSQAVVTQEPSLTVSPGGTVTLT CRSSTGAVTTSNYANWVQQKPGQAPRGLIGGTNKRAPWTPARFSGSLLGGKAALTITGAQA EDEADYYCALWYSNLWVFGGGTKLTVLGGGGSDKTHTCPPCPAPEAAGPSVFLFPPKPKDT LMISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLSCAVKGFYP SDIAVEWESNGQPENNYKTTPPVLDSDGSFFLVSKLTVDKSRWQQGNVFSCSVMHEALHNR FTQKSLSLSPGGGGGSGGGGSGGGGSGGGGSDIQMTQSPSSLSASVGDRVTITCRASQDIR SNLNWYQQKPGGAVKLLIYYTSRLHSGVPSRFSGSGSGTDYTLTISSLQPEDFATYFCQQSE KLPRTFGGGTKVEIR | 465 |
| | 3rd | *see above* | 131 |
| Ly2386 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCGGGG SGGGGSGGGGSGGGGSQVQLVQSGAEVKKPGASVKVSCKASGYTFAGFEMHWVRQAPG QGLEWMGAIDPKTGGTDYNQKFKDRVTMTRDTSISTAYMELSRLRSDDTAVYYCARDLGYF DVWGQGTLVTVSS | 380 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCGGGGSGGGGSGGGGSGGGGSD IQMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIR | 434 |
| | 3rd | *see above* | 131 |
| Ly2387 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 381 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCGGGGSGGGGSDIQMTQSPSSLS ASVGDRVTITCRASQDIRSNLNWYQQKPGGAVKLLIYYTSRLHSGVPSRFSGSGSGTDYTLTI SSLQPEDFATYFCQQSEKLPRTFGGGTKVEIR | 435 |
| | 3rd | *see above* | 131 |
| Ly2388 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSS | 382 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCGGGGSDIQMTQSPSSLSASVGDR VTITCRASQDIRSNLNWYQQKPGGAVKLLIYYTSRLHSGVPSRFSGSGSGTDYTLTISSLQPE DFATYFCQQSEKLPRTFGGGTKVEIR | 436 |
| | 3rd | *see above* | 131 |
| Ly2389 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGDKTHTCPPCQVQLV QSGAEVKKPGASVKVSCKASGYTFAGFEMHWVRQAPGQGLEWMGAIDPKTGGTDYNQKFK DRVTMTRDTSISTAYMELSRLRSDDTAVYYCARDLGYFDVWGQGTLVTVSS | 383 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDKTHTCPPCDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 437 |
| | 3rd | *see above* | 131 |
| Ly2390 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGQVQLVQSGAEVKKP GASVKVSCKASGYTFAGFEMHWVRQAPGQGLEWMGAIDPKTGGTDYNQKFKDRVTMTRDT SISTAYMELSRLRSDDTAVYYCARDLGYFDVWGQGTLVTVSS | 384 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGDIQMTQSPSSLSASVGDRVTITCRASQDIRSNL NWYQQKPGGAVKLLIYYTSRLHSGVPSRFSGSGSGTDYTLTISSLQPEDFATYFCQQSEKLP RTFGGGTKVEIR | 438 |
| | 3rd | *see above* | 131 |
| Ly2396 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFAGFEMHWVRQAPGQGLEWMGAIDPKTGGTDYNQKFKDRVTMTRDTSISTAYMELSRL RSDDTAVYYCARDLGYFDVWGQGTLVTVSS | 469 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCRAS QDIRSNLNWYQQKPGGAVKLLIYYTSRLHSGVPSRFSGSGSGTDYTLTISSLQPEDFATYFCQ QSEKLPRTFGGGTKVEIR | 470 |
| | 3rd | *see above* | 207 |
| Ly2405 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFTGYYMHWVRQAPGQGLEWMGWINPDSGGTNYAQKFQGRVTMTRDTSISTAYMELNR LRSDDTAVYYCARDQPLGYCTNGVCSYFDYWGQGTLVTVSS | 473 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSVSASVGDRVTITCRAS QGIYSWLAWYQQKPGKAPNLLIYTASTLQSGVPSRFSGSGSGTDFTLTISSLQPEDFATYYCQ QANIFPLTFGGGTKVEIK | 474 |
| | 3rd | *see above* | 207 |
| Ly2409 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFTNYGLHWVRQAPGKGLEWVSSISPSGGVTYYRDSV KGRFTISRDNSKNTLYLQMNSLRAEDTAVYYCAKPFLGWGGANWIAHWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPG | 496 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCLASEDISNDLAWYQQKPGKAPKLLIYFVDRLLDGVPSRFSGSG SGTDFTLTISSLQPEDFATYYCQQSYKYPPTFGQGTKLEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 482 |
| | 3rd | *see above* | 207 |
| Ly2415 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSEVQLVESGGGLVQPGGSLRLSCAAS GFTFTNYGLHWVRQAPGKGLEWVSSISPSGGVTYYRDSVKGRFTISRDNSKNTLYLQMNSLR AEDTAVYYCAKPFLGWGGANWIAHWGQGTLVTVSS | 475 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCLAS EDISNDLAWYQQKPGKAPKLLIYFVDRLLDGVPSRFSGSGSGTDFTLTISSLQPEDFATYYCQ QSYKYPPTFGQGTKLEIK | 476 |
| | 3rd | *see above* | 207 |
| Ly2424 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSQVQLVQSGAEVKKPGASVKVSCK ASGYTFTGYYMHWVRQAPGQGLEWMGWINPDSGGTNYAQKFQGRVTMTRDTSISTAYMEL NRLRSDDTAVYYCARDQPLGYCTNGVCSYFDYWGQGTLVTVSS | 321 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSVSASVGDRVTITCR ASQGIYSWLAWYQQKPGKAPNLLIYTASTLQSGVPSRFSGSGSGTDFTLTISSLQPEDFATYY CQQANIFPLTFGGGTKVEIK | 322 |
| | 3rd | *see above* | 131 |
| Ly2428 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFTNYGLHWVRQAPGKGLEWVSSISPSGGVTYYRD SVKGRFTISRDNSKNTLYLQMNSLRAEDTAVYYCAKPFLGWGGANWIAHWGQGTLVTVSSG GGGSDKTHTCPPCPAPEAAGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKG QPREPQVYTLPPSREEMTKNQVSLWCLVKGFYPSDIAVEWESNGQPENNYKTTPPVLDSDG SFFLYSKLTVDKSRWQQGNVFSCSVMHEALHNHYTQKSLSLSPG | 385 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSLSASVGDRVTITCLASEDISNDLAWYQQKPGKAPKLLIYFVDRLLDGVPSRFSGS GSGTDFTLTISSLQPEDFATYYCQQSYKYPPTFGQGTKLEIKGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 338 |
| | 3rd | *see above* | 131 |
| Ly2434 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSEVQLVESGGGLVQPGGSLRLSCA ASGFTFTNYGLHWVRQAPGKGLEWVSSISPSGGVTYYRDSVKGRFTISRDNSKNTLYLQMN SLRAEDTAVYYCAKPFLGWGGANWIAHWGQGTLVTVSS | 323 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCLA SEDISNDLAWYQQKPGKAPKLLIYFVDRLLDGVPSRFSGSGSGTDFTLTISSLQPEDFATYYC QQSYKYPPTFGQGTKLEIK | 324 |
| | 3rd | *see above* | 131 |
| Ly2438 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFTNYGLHWVRQAPGKGLEWVSSISPSGGVTYY RDSVKGRFTISRDNSKNTLYLQMNSLRAEDTAVYYCAKPFLGWGGANWIAHWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 290 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSLSASVGDRVTITCLASEDISNDLAWYQQKPGKAPKLLIYFVDRLLDGVPSRFS GSGSGTDFTLTISSLQPEDFATYYCQQSYKYPPTFGQGTKLEIKGGGGSDKTHTCPPCPAPE AAGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSREEMT KNQVSLSCAVKGFYPSDIAVEWESNGQPENNYKTTPPVLDSDGSFFLVSKLTVDKSRWQQG NVFSCSVMHEALHNRFTQKSLSLSPG | 284 |
| | 3rd | DIQMTQSPSSLSASVGDRVTITCKASQNVYKKLEWYQQKPGKVPKVLIYHTNILQTGVPSRFS GSGSGTDYTLTISSLQPEDVATYYCYQWNSGPTFGGGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 115 |
| Ly2444 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVM HEALHNHYTQKSLSLSPGGGGGSGGGGSGGGGSGGGGSEVQLVESGGGLVQPGGSLRLS CAASGFTFTNYGLHWVRQAPGKGLEWVSSISPSGGVTYYRDSVKGRFTISRDNSKNTLYLQ MNSLRAEDTAVYYCAKPFLGWGGANWIAHWGQGTLVTVSS | 275 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSLSASVGDRVTITCL ASEDISNDLAWYQQKPGKAPKLLIYFVDRLLDGVPSRFSGSGSGTDFTLTISSLQPEDFATYY CQQSYKYPPTFGQGTKLEIK | 276 |
| | 3rd | *see above* | 115 |
| Ly2453 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQVQLVQSGAEVKKPGASVKVSCKASGYTFTGYYMHWVRQAPGQGLEWMGWINPDSGGT NYAQKFQGRVTMTRDTSISTAYMELNRLRSDDTAVYYCARDQPLGYCTNGVCSYFDYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPG | 298 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSVSASVGDRVTITCRASQGIYSWLAWYQQKPGKAPNLLIYTASTLQSGVPSRF SGSGSGTDFTLTISSLQPEDFATYYCQQANIFPLTFGGGTKVEIKGGGGSDKTHTCPPCPAPE AAGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSREEMT KNQVSLSCAVKGFYPSDIAVEWESNGQPENNYKTTPPVLDSDGSFFLVSKLTVDKSRWQQG NVFSCSVMHEALHNRFTQKSLSLSPG | 283 |
| | 3rd | *see above* | 115 |
| Ly2454 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVM HEALHNHYTQKSLSLSPGGGGGSGGGGSGGGGSGGGGSQVQLVQSGAEVKKPGASVKVS CKASGYTFTGYYMHWVRQAPGQGLEWMGWINPDSGGTNYAQKFQGRVTMTRDTSISTAYM ELNRLRSDDTAVYYCARDQPLGYCTNGVCSYFDYWGQGTLVTVSS | 273 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSVSASVGDRVTITC RASQGIYSWLAWYQQKPGKAPNLLIYTASTLQSGVPSRFSGSGSGTDFTLTISSLQPEDFATY YCQQANIFPLTFGGGTKVEIK | 274 |
| | 3rd | *see above* | 115 |
| Ly2505 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFTGYYMHWVRQAPGQGLEWMGWINPDSGGTNYAQ KFQGRVTMTRDTSISTAYMELNRLRSDDTAVYYCARDQPLGYCTNGVCSYFDYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 506 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSVSASVGDRVTITCRASQGIYSWLAWYQQKPGKAPNLLIYTASTLQSGVPSRFSGS GSGTDFTLTISSLQPEDFATYYCQQANIFPLTFGGGTKVEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 491 |
| | 3rd | *see above* | 207 |
| Ly2506 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFTNYGLHWVRQAPGKGLEWVSSISPSGGVTYYRDSV KGRFTISRDNSKNTLYLQMNSLRAEDTAVYYCAKPFLGWGGANWIAHWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGGSGGGGSGGGGSGG GGSQVQLVQSGAEVKKPGASVKVSCKASGYTFAGFEMHWVRQAPGQGLEWMGAIDPKTG GTDYNQKFKDRVTMTRDTSISTAYMELSRLRSDDTAVYYCARDLGYFDVWGQGTLVTVSS | 497 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCLASEDISNDLAWYQQKPGKAPKLLIYFVDRLLDGVPSRFSGSG SGTDFTLTISSLQPEDFATYYCQQSYKYPPTFGQGTKLEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 484 |
| | 3rd | *see above* | 207 |
| Ly2507 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFTGYYMHWVRQAPGQGLEWMGWINPDSGGTNYAQ KFQGRVTMTRDTSISTAYMELNRLRSDDTAVYYCARDQPLGYCTNGVCSYFDYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLQESGPGLVKPSETLSLTCTVSGFSLTSYNVHWIRQPPGKGLEWIGVIWS GVRTDYNSVLKPRVTISVDTSKNQFSLKLSSVTAADTAVYYCARGTYDDNYHDVMDAWGQG TLVTVSS | 510 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSVSASVGDRVTITCRASQGIYSWLAWYQQKPGKAPNLLIYTASTLQSGVPSRFSGS GSGTDFTLTISSLQPEDFATYYCQQANIFPLTFGGGTKVEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSEIVLTQSPATLSLSPG ERATLSCRASKSVRTGMHWYQQKPGQAPRLLIYGASNLESGIPARFSGSGSGTDFTLTISSLE PEDFAVYYCQQSWNHFTFGQGTKLEIK | 492 |
| | 3rd | *see above* | 207 |
| Ly2508 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFTNYGLHWVRQAPGKGLEWVSSISPSGGVTYYRDSV KGRFTISRDNSKNTLYLQMNSLRAEDTAVYYCAKPFLGWGGANWIAHWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGSGGGGSGGGGSGG GGSQVQLQESGPGLVKPSETLSLTCTVSGFSLTSYNVHWIRQPPGKGLEWIGVIWSGVRTDY NSVLKPRVTISVDTSKNQFSLKLSSVTAADTAVYYCARGTYDDNYHDVMDAWGQGTLVTVSS | 498 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCLASEDISNDLAWYQQKPGKAPKLLIYFVDRLLDGVPSRFSGSG SGTDFTLTISSLQPEDFATYYCQQSYKYPPTFGQGTKLEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSEIVLTQSPATLSLSPG ERATLSCRASKSVRTGMHWYQQKPGQAPRLLIYGASNLESGIPARFSGSGSGTDFTLTISSLE PEDFAVYYCQQSWNHFTFGQGTKLEIK | 488 |
| | 3rd | *see above* | 207 |
| Ly2509 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLVQSGAEVKKPGASVKVSCKASGYTFTGYYMHWVRQAPGQGLEWMGWINPDSGGTNYA QKFQGRVTMTRDTSISTAYMELNRLRSDDTAVYYCARDQPLGYCTNGVCSYFDYWGQGTLV TVSS | 507 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSVSASV GDRVTITCRASQGIYSWLAWYQQKPGKAPNLLIYTASTLQSGVPSRFSGSGSGTDFTLTISSL QPEDFATYYCQQANIFPLTFGGGTKVEIK | 485 |
| | 3rd | *see above* | 207 |
| Ly2510 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSEV QLVESGGGLVQPGGSLRLSCAASGFTFTNYGLHWVRQAPGKGLEWVSSISPSGGVTYYRDS VKGRFTISRDNSKNTLYLQMNSLRAEDTAVYYCAKPFLGWGGANWIAHWGQGTLVTVSS | 508 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCLASEDISNDLAWYQQKPGKAPKLLIYFVDRLLDGVPSRFSGSGSGTDFTLTISSLQP EDFATYYCQQSYKYPPTFGQGTKLEIK | 486 |
| | 3rd | *see above* | 207 |
| Ly2511 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLQESGPGLVKPSETLSLTCTVSGFSLTSYNVHWIRQPPGKGLEWIGVIWSGVRTDYNSVLK PRVTISVDTSKNQFSLKLSSVTAADTAVYYCARGTYDDNYHDVMDAWGQGTLVTVSS | 509 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGGSGGGGSGGGGSGGGGSEIVLTQSPATLSLSPG ERATLSCRASKSVRTGMHWYQQKPGQAPRLLIYGASNLESGIPARFSGSGSGTDFTLTISSLE PEDFAVYYCQQSWNHFTFGQGTKLEIK | 487 |
| | 3rd | *see above* | 207 |
| Ly2512 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFTGYYMHWVRQAPGQGLEWMGWINPDSGGTNY AQKFQGRVTMTRDTSISTAYMELNRLRSDDTAVYYCARDQPLGYCTNGVCSYFDYWGQGTL VTVSSGGGGSDKTHTCPPCPAPEAAGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKT ISKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 453 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSVSASVGDRVTITCRASQGIYSWLAWYQQKPGKAPNLLIYTASTLQSGVPSRFSG SGSGTDFTLTISSLQPEDFATYYCQQANIFPLTFGGGTKVEIKGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 345 |
| | 3rd | *see above* | 131 |
| Ly2513 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFTNYGLHWVRQAPGKGLEWVSSISPSGGVTYYRD SVKGRFTISRDNSKNTLYLQMNSLRAEDTAVYYCAKPFLGWGGANWIAHWGQGTLVTVSSG GGGSDKTHTCPPCPAPEAAGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKG QPREPQVYTLPPSREEMTKNQVSLWCLVKGFYPSDIAVEWESNGQPENNYKTTPPVLDSDG SFFLYSKLTVDKSRWQQGNVFSCSVMHEALHNHYTQKSLSLSPGGGGGSGGGGSGGGGS GGGGSQVQLVQSGAEVKKPGASVKVSCKASGYTFAGFEMHWVRQAPGQGLEWMGAIDPK TGGTDYNQKFKDRVTMTRDTSISTAYMELSRLRSDDTAVYYCARDLGYFDVWGQGTLVTVS S | 386 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSLSASVGDRVTITCLASEDISNDLAWYQQKPGKAPKLLIYFVDRLLDGVPSRFSGS GSGTDFTLTISSLQPEDFATYYCQQSYKYPPTFGQGTKLEIKGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 340 |
| | 3rd | *see above* | 131 |
| Ly2514 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFAGFEMHWVRQAPGQGLEWMGAIDPKTGGTDYN QKFKDRVTMTRDTSISTAYMELSRLRSDDTAVYYCARDLGYFDVWGQGTLVTVSSGGGGSD KTHTCPPCPAPEAAGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREP QVYTLPPSREEMTKNQVSLWCLVKGFYPSDIAVEWESNGQPENNYKTTPPVLDSDGSFFLYS KLTVDKSRWQQGNVFSCSVMHEALHNHYTQKSLSLSPGGGGGSGGGGSGGGGSGGGGS QVQLVQSGAEVKKPGASVKVSCKASGYTFTGYYMHWVRQAPGQGLEWMGWINPDSGGTN YAQKFQGRVTMTRDTSISTAYMELNRLRSDDTAVYYCARDQPLGYCTNGVCSYFDYWGQGT LVTVSS | 454 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIRGGGGSDKTHTCPPCPAPEA AGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSREEMTK NQVSLSCAVKGFYPSDIAVEWESNGQPENNYKTTPPVLDSDGSFFLVSKLTVDKSRWQQGN VFSCSVMHEALHNRFTQKSLSLSPGGGGGSGGGGSGGGGSGGGGSDIQMTQSPSSVSASV GDRVTITCRASQGIYSWLAWYQQKPGKAPNLLIYTASTLQSGVPSRFSGSGSGTDFTLTISSL QPEDFATYYCQQANIFPLTFGGGTKVEIK | 341 |
| | 3rd | *see above* | 131 |
| Ly2515 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLVQSGAEVKKPGASVKVSCKASGYTFAGFEMHWVRQAPGQGLEWMGAIDPKTGGTDYN QKFKDRVTMTRDTSISTAYMELSRLRSDDTAVYYCARDLGYFDVWGQGTLVTVSSGGGGSD KTHTCPPCPAPEAAGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREP QVYTLPPSREEMTKNQVSLWCLVKGFYPSDIAVEWESNGQPENNYKTTPPVLDSDGSFFLYS KLTVDKSRWQQGNVFSCSVMHEALHNHYTQKSLSLSPGGGGGSGGGGSGGGGSGGGGSE VQLVESGGGLVQPGGSLRLSCAASGFTFTNYGLHWVRQAPGKGLEWVSSISPSGGVTYYRD SVKGRFTISRDNSKNTLYLQMNSLRAEDTAVYYCAKPFLGWGGANWIAHWGQGTLVTVSS | 455 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSDI QMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIRGGGGSDKTHTCPPCPAPEA AGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSREEMTK NQVSLSCAVKGFYPSDIAVEWESNGQPENNYKTTPPVLDSDGSFFLVSKLTVDKSRWQQGN VFSCSVMHEALHNRFTQKSLSLSPGGGGGSGGGGSGGGGSGGGGSDIQMTQSPSSLSASV GDRVTITCLASEDISNDLAWYQQKPGKAPKLLIYFVDRLLDGVPSRFSGSGSGTDFTLTISSLQ PEDFATYYCQQSYKYPPTFGQGTKLEIK | 342 |
| | 3rd | *see above* | 131 |
| Ly2517 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFSTYAMNWVRQAPGKGLEWVGRIRSKYNNYAT YYADSVKGRFTISRDDSKNSLYLQMNSLKTEDTAVYYCVRHGNFGNSYVSWFAYWGQGTLV TVSSGGGGSDKTHTCPPCPAPEAAGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKTI SKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPG | 291 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPG | 295 |
| | 3rd | *see above* | 115 |
| Ly2518 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 167 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPG | 171 |
| | 3rd | DIQMTQSPSSLSASVGDRVTITCRASQDVSTAVAWYQQKPGKAPKLLIYSASFLYSGVPSRFS GSGSGTDFTLTISSLQPEDFATYYCQQYLYHPATFGQGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 125 |
| Ly2519 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFSTYAMNWVRQAPGKGLEWVGRIRSKYNNYAT YYADSVKGRFTISRDDSKNSLYLQMNSLKTEDTAVYYCVRHGNFGNSYVSWFAYWGQGTLV TVSSGGGGSDKTHTCPPCPAPEAAGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKTI SKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 292 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPGGGGGSGGGGSGGGGSGGGGSDIQMTQS PSSLSASVGDRVTITCRASQDIRSNLNWYQQKPGGAVKLLIYYTSRLHSGVPSRFSGSGSGT DYTLTISSLQPEDFATYFCQQSEKLPRTFGGGTKVEIR | 296 |
| | 3rd | *see above* | 115 |
| Ly2520 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 168 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPGGGGGSGGGGSGGGGSGGGGSDIQMTQSPSSL SASVGDRVTITCRASQDIRSNLNWYQQKPGGAVKLLIYYTSRLHSGVPSRFSGSGSGTDYTLT ISSLQPEDFATYFCQQSEKLPRTFGGGTKVEIR | 172 |
| | 3rd | *see above* | 125 |
| Ly2521 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFSTYAMNWVRQAPGKGLEWVGRIRSKYNNYAT YYADSVKGRFTISRDDSKNSLYLQMNSLKTEDTAVYYCVRHGNFGNSYVSWFAYWGQGTLV TVSSGGGGSDKTHTCPPCPAPEAAGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKTI SKAKGQPREPQVYTLPPSREEMTKNQVSLWCLVKGFYPSDIAVEWESNGQPENNYKTTPPV LDSDGSFFLYSKLTVDKSRWQQGNVFSCSVMHEALHNHYTQKSLSLSPGGGGGSGGGGSG GGGSGGGGSQVQLVQSGAEVKKPGASVKVSCKASGYTFTSYYIHWVRQAPGQGLEWIGCIY PGNVNTNYNEKFKDRATLTVDTSISTAYMELSRLRSDDTAVYFCTRSHYGLDWNFDVWGQG TTVTVSS | 293 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPGGGGGSGGGGSGGGGSGGGGSDIQMTQS PSSLSASVGDRVTITCHASQNIYVWLNWYQQKPGKAPKLLIYKASNLHTGVPSRFSGSGSGT DFTLTISSLQPEDFATYYCQQGQTYPYTFGGGTKVEIK | 297 |
| | 3rd | *see above* | 115 |
| Ly2522 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFTSYYIHWVRQAPGQGLEWIGCIYPGN VNTNYNEKFKDRATLTVDTSISTAYMELSRLRSDDTAVYFCTRSHYGLDWNFDVWGQGTTVT VSS | 169 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPGGGGGSGGGGSGGGGSGGGGSDIQMTQSPSSL SASVGDRVTITCHASQNIYVWLNWYQQKPGKAPKLLIYKASNLHTGVPSRFSGSGSGTDFTLT ISSLQPEDFATYYCQQGQTYPYTFGGGTKVEIK | 173 |
| | 3rd | *see above* | 125 |
| Ly2524 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVAVSHEDPEVKFN WYVDGVEVHNAKTKPREEQYASTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 365 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVAVSHEDPEVKFNWYVDGVEVHNAKTKPR EEQYASTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNHYTQKSLSLSPGGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 419 |
| | 3rd | *see above* | 131 |
| Ly2525 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALGAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGKGGGGSGGGGSG GGGSGGGGSQVQLVQSGAEVKKPGASVKVSCKASGYTFAGFEMHWVRQAPGQGLEWMG AIDPKTGGTDYNQKFKDRVTMTRDTSISTAYMELSRLRSDDTAVYYCARDLGYFDVWGQGTL VTVSS | 366 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALGAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNHYTQKSLSLSPGKGGGGSGGGGSGGGGSGGGGSDIQMTQ SPSSLSASVGDRVTITCRASQDIRSNLNWYQQKPGGAVKLLIYYTSRLHSGVPSRFSGSGSG TDYTLTISSLQPEDFATYFCQQSEKLPRTFGGGTKVEIR | 420 |
| | 3rd | *see above* | 131 |
| Ly2526 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 367 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNHYTQKSLSLSPGKGGGGSGGGGSGGGGSGGGGSDIQMTQS PSSLSASVGDRVTITCRASQDIRSNLNWYQQKPGGAVKLLIYYTSRLHSGVPSRFSGSGSGT DYTLTISSLQPEDFATYFCQQSEKLPRTFGGGTKVEIR | 421 |
| | 3rd | *see above* | 131 |
| Ly2527 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPELLGPSVFLFPPKPKDTLMISRTPEVTCVVVAVSHEDP EVKFNWYVDGVEVHNAKTKPREEQYASTYRVVSVLTVLHQDWLNGKEYKCKVSNKALPAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPGGGGGSGGGG SGGGGSGGGGSQVQLVQSGAEVKKPGASVKVSCKASGYTFAGFEMHWVRQAPGQGLEW MGAIDPKTGGTDYNQKFKDRVTMTRDTSISTAYMELSRLRSDDTAVYYCARDLGYFDVWGQ GTLVTVSS | 236 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPELLGPSVFLFPPKPKDTLMISRTPEVTCVVVAVSHEDPEVKFNWYVDGVEVHNA KTKPREEQYASTYRVVSVLTVLHQDWLNGKEYKCKVSNKALPAPIEKTISKAKGQPREPQVYT LPPSREEMTKNQVSLSCAVKGFYPSDIAVEWESNGQPENNYKTTPPVLDSDGSFFLVSKLTV DKSRWQQGNVFSCSVMHEALHNHYTQKSLSLSPGGGGGSGGGGSGGGGSGGGGSDIQM TQSPSSLSASVGDRVTITCRASQDIRSNLNWYQQKPGGAVKLLIYYTSRLHSGVPSRFSGSG SGTDYTLTISSLQPEDFATYFCQQSEKLPRTFGGGTKVEIR | 249 |
| | 3rd | *see above* | 107 |
| Ly2528 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYKCKVSNKALGA PIEKTISKAKGQPREPQVYTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGSG GGGSGGGGSGGGGSQVQLVQSGAEVKKPGASVKVSCKASGYTFAGFEMHWVRQAPGQG LEWMGAIDPKTGGTDYNQKFKDRVTMTRDTSISTAYMELSRLRSDDTAVYYCARDLGYFDV WGQGTLVTVSS | 237 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKAKGQPREPQV YTLPPSREEMTKNQVSLSCAVKGFYPSDIAVEWESNGQPENNYKTTPPVLDSDGSFFLVSKL TVDKSRWQQGNVFSCSVMHEALHNHYTQKSLSLSPGKGGGGSGGGGSGGGGSGGGGSDI QMTQSPSSLSASVGDRVTITCRASQDIRSNLNWYQQKPGGAVKLLIYYTSRLHSGVPSRFSG SGSGTDYTLTISSLQPEDFATYFCQQSEKLPRTFGGGTKVEIR | 250 |
| | 3rd | *see above* | 107 |
| Ly2529 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALGAP IEKTISKAKGQPREPQVYTLPPSREEMTKNQVSLWCLVKGFYPSDIAVEWESNGQPENNYKT TPPVLDSDGSFFLYSKLTVDKSRWQQGNVFSCSVMHEALHNHYTQKSLSLSPGKGGGGSG GGGSGGGGSGGGGSQVQLVQSGAEVKKPGASVKVSCKASGYTFAGFEMHWVRQAPGQG LEWMGAIDPKTGGTDYNQKFKDRVTMTRDTSISTAYMELSRLRSDDTAVYYCARDLGYFDV WGQGTLVTVSS | 238 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNHYTQKSLSLSPGKGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIR | 251 |
| | 3rd | *see above* | 107 |
| Ly2576 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LQESGPGLVKPSETLSLTCTVSGFSLTSYNVHWIRQPPGKGLEWIGVIWSGVRTDYNSVLKP RVTISVDTSKNQFSLKLSSVTAADTAVYYCARGTYDDNYHDVMDAWGQGTLVTVSSGGGGS DKTHTCPPCPAPEAAGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPRE PQVYTLPPSREEMTKNQVSLWCLVKGFYPSDIAVEWESNGQPENNYKTTPPVLDSDGSFFLY SKLTVDKSRWQQGNVFSCSVMHEALHNHYTQKSLSLSPGGGGGSGGGGSGGGGSGGGGS QVQLVQSGAEVKKPGASVKVSCKASGYTFTGYYMHWVRQAPGQGLEWMGWINPDSGGTN YAQKFQGRVTMTRDTSISTAYMELNRLRSDDTAVYYCARDQPLGYCTNGVCSYFDYWGQGT LVTVSS | 500 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEIVL TQSPATLSLSPGERATLSCRASKSVRTGMHWYQQKPGQAPRLLIYGASNLESGIPARFSGSG SGTDFTLTISSLEPEDFAVYYCQQSWNHFTFGQGTKLEIKGGGGSDKTHTCPPCPAPEAAGP SVFLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSREEMTKNQ VSLSCAVKGFYPSDIAVEWESNGQPENNYKTTPPVLDSDGSFFLVSKLTVDKSRWQQGNVF SCSVMHEALHNRFTQKSLSLSPGGGGGSGGGGSGGGGSGGGGSDIQMTQSPSSVSASVG DRVTITCRASQGIYSWLAWYQQKPGKAPNLLIYTASTLQSGVPSRFSGSGSGTDFTLTISSLQ PEDFATYYCQQANIFPLTFGGGTKVEIK | 494 |
| | 3rd | *see above* | 207 |
| Ly2577 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LQESGPGLVKPSETLSLTCTVSGFSLTSYNVHWIRQPPGKGLEWIGVIWSGVRTDYNSVLKP RVTISVDTSKNQFSLKLSSVTAADTAVYYCARGTYDDNYHDVMDAWGQGTLVTVSSGGGGS DKTHTCPPCPAPEAAGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPRE PQVYTLPPSREEMTKNQVSLWCLVKGFYPSDIAVEWESNGQPENNYKTTPPVLDSDGSFFLY SKLTVDKSRWQQGNVFSCSVMHEALHNHYTQKSLSLSPGGGGGSGGGGSGGGGSGGGGS EVQLVESGGGLVQPGGSLRLSCAASGFTFTNYGLHWVRQAPGKGLEWVSSISPSGGVTYYR DSVKGRFTISRDNSKNTLYLQMNSLRAEDTAVYYCAKPFLGWGGANWIAHWGQGTLVTVSS | 501 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEIVL TQSPATLSLSPGERATLSCRASKSVRTGMHWYQQKPGQAPRLLIYGASNLESGIPARFSGSG SGTDFTLTISSLEPEDFAVYYCQQSWNHFTFGQGTKLEIKGGGGSDKTHTCPPCPAPEAAGP SVFLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSREEMTKNQ VSLSCAVKGFYPSDIAVEWESNGQPENNYKTTPPVLDSDGSFFLVSKLTVDKSRWQQGNVF SCSVMHEALHNRFTQKSLSLSPGGGGGSGGGGSGGGGSGGGGSDIQMTQSPSSLSASVG DRVTITCLASEDISNDLAWYQQKPGKAPKLLIYFVDRLLDGVPSRFSGSGSGTDFTLTISSLQP EDFATYYCQQSYKYPPTFGQGTKLEIK | 495 |
| | 3rd | *see above* | 207 |
| Ly2578 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LQESGPGLVKPSETLSLTCTVSGFSLTSYNVHWIRQPPGKGLEWIGVIWSGVRTDYNSVLKP RVTISVDTSKNQFSLKLSSVTAADTAVYYCARGTYDDNYHDVMDAWGQGTLVTVSSGGGGS DKTHTCPPCPAPEAAGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPRE PQVYTLPPSREEMTKNQVSLWCLVKGFYPSDIAVEWESNGQPENNYKTTPPVLDSDGSFFLY SKLTVDKSRWQQGNVFSCSVMHEALHNHYTQKSLSLSPGGGGGSGGGGSGGGGSGGGGS QVQLVQSGAEVKKPGASVKVSCKASGYTFAGFEMHWVRQAPGQGLEWMGAIDPKTGGTDY NQKFKDRVTMTRDTSISTAYMELSRLRSDDTAVYYCARDLGYFDVWGQGTLVTVSS | 499 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEIVL TQSPATLSLSPGERATLSCRASKSVRTGMHWYQQKPGQAPRLLIYGASNLESGIPARFSGSG SGTDFTLTISSLEPEDFAVYYCQQSWNHFTFGQGTKLEIKGGGGSDKTHTCPPCPAPEAAGP SVFLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSREEMTKNQ VSLSCAVKGFYPSDIAVEWESNGQPENNYKTTPPVLDSDGSFFLVSKLTVDKSRWQQGNVF SCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 493 |
| | 3rd | *see above* | 207 |
| Ly2579 | 1st | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASVAAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGECGGGGSGGGGSGGGGS GGGGSEVQLVESGGGLVQPGGSLRLSCAASGFTFSTYAMNWVRQAPGKGLEWVGRIRSKY NNYATYYADSVKGRFTISRDDSKNSLYLQMNSLKTEDTAVYYCVRHGNFGNSYVSWFAYWG QGTLVTVSSGGGGSDKTHTCPPCPAPEAAGPSVFLFPPKPKDTLMISRTPEVTCVVVDVSHE DPEVKFNWYVDGVEVHNAKTKPREEQYNSTYRVVSVLTVLHQDWLNGKEYKCKVSNKALGA PIEKTISKAKGQPREPQVYTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGSG GGGSGGGGSGGGGSQVQLVQSGAEVKKPGASVKVSCKASGYTFAGFEMHWVRQAPGQG LEWMGAIDPKTGGTDYNQKFKDRVTMTRDTSISTAYMELSRLRSDDTAVYYCARDLGYFDV WGQGTLVTVSS | 228 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPEAAGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNHYTQKSLSLSPGKGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIR | 259 |
| | 3rd | QIVLSQSPAILSASPGEKVTMTCRASSSVSYIHWFQQKPGSSPKPWIYATSNLASGVPVRFSG SGSGTSYSLTISRVEAEDAATYYCQQWTSNPPTFGGGTKLEIKSSASTKGPSVFPLAPSSKST SGGTAALGCLVKDYFPEPVTVSWNSGALTSGVHTFPAVLQSSGLYSLSSVVTVPSSSLGTQT YICNVNHKPSNTKVDKKVEPKSC | 209 |
| | 4th | *see above* | 107 |
| Ly2580 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECGGGGSGGGGSGGG GSGGGGSEVQLVESGGGLVQPGGSLRLSCAASGFTFSTYAMNWVRQAPGKGLEWVGRIRS KYNNYATYYADSVKGRFTISRDDSKNSLYLQMNSLKTEDTAVYYCVRHGNFGNSYVSWFAY WGQGTLVTVSSGGGGSDKTHTCPPCPAPEAAGPSVFLFPPKPKDTLMISRTPEVTCVVVDVS HEDPEVKFNWYVDGVEVHNAKTKPREEQYNSTYRVVSVLTVLHQDWLNGKEYKCKVSNKAL GAPIEKTISKAKGQPREPQVYTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGKGGGG SGGGGSGGGGSGGGGSQVQLVQSGAEVKKPGASVKVSCKASGYTFAGFEMHWVRQAPG QGLEWMGAIDPKTGGTDYNQKFKDRVTMTRDTSISTAYMELSRLRSDDTAVYYCARDLGYF DVWGQGTLVTVSS | 264 |
| | 2nd | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPEAAGPSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNHYTQKSLSLSPGKGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 223 |
| | 3rd | DIQLTQSPASLAVSLGQRATISCKASQSVDYDGDSYLNWYQQIPGQPPKLLIYDASNLVSGIPP RFSGSGSGTDFTLNIHPVEKVDAATYHCQQSTEDPWTFGGGTKLEIKSSASTKGPSVFPLAP SSKSTSGGTAALGCLVKDYFPEPVTVSWNSGALTSGVHTFPAVLQSSGLYSLSSVVTVPSSS LGTQTYICNVNHKPSNTKVDKKVEPKSC | 108 |
| | 4th | QIVLSQSPAILSASPGEKVTMTCRASSSVSYIHWFQQKPGSSPKPWIYATSNLASGVPVRFSG SGSGTSYSLTISRVEAEDAATYYCQQWTSNPPTFGGGTKLEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGEC | 208 |
| Ly2600 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 368 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNHYTQKSLSLSPGGGGGSGGGGSGGGGSGGGGSDIQMTQSP SSLSASVGDRVTITCRASQDIRSNLNWYQQKPGGAVKLLIYYTSRLHSGVPSRFSGSGSGTD YTLTISSLQPEDFATYFCQQSEKLPRTFGGGTKVEIR | 422 |
| | 3rd | *see above* | 131 |
| Ly2601 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSEVQLVESGGGLVQPGGSLRLSCAASGFTFSTYAMNWVRQAPGKGLEWVGRIRSKYN NYATYYADSVKGRFTISRDDSKNSLYLQMNSLKTEDTAVYYCVRHGNFGNSYVSWFAYWGQ GTLVTVSSGGGGSDKTHTCPPCPAPELLGPSVFLFPPKPKDTLMISRTPEVTCVVVDVSHEDP EVKFNWYVDGVEVHNAKTKPREEQYNSTYRVVSVLTVLHQDWLNGKEYKCKVSNKALGAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFAGFEMHWVRQAPGQGLE WMGAIDPKTGGTDYNQKFKDRVTMTRDTSISTAYMELSRLRSDDTAVYYCARDLGYFDVWG QGTLVTVSS | 239 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPELLGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNHYTQKSLSLSPGGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIR | 252 |
| | 3rd | *see above* | 107 |
| Ly2602 | 1st | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPG | 312 |
| | 2nd | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPG | 316 |
| | 3rd | *see above* | 118 |
| Ly2603 | 1st | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 213 |
| | 2nd | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPG | 217 |
| | 3rd | *see above* | 511 |
| Ly2604 | 1st | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGPSVFLFP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLWCL VKGFYPSDIAVEWESNGQPENNYKTTPPVLDSDGSFFLYSKLTVDKSRWQQGNVFSCSVMH EALHNHYTQKSLSLSPGGGGGSGGGGSGGGGSGGGGSQVQLVQSGAEVKKPGASVKVSC KASGYTFAGFEMHWVRQAPGQGLEWMGAIDPKTGGTDYNQKFKDRVTMTRDTSISTAYMEL SRLRSDDTAVYYCARDLGYFDVWGQGTLVTVSS | 309 |
| | 2nd | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGPSVFLFP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSLSASVGDRVTITC RASQDIRSNLNWYQQKPGGAVKLLIYYTSRLHSGVPSRFSGSGSGTDYTLTISSLQPEDFATY FCQQSEKLPRTFGGGTKVEIR | 310 |
| | 3rd | *see above* | 118 |
| Ly2605 | 1st | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFAGFEMHWVRQAPGQGLEWMGAIDPKTGGTDYNQKFKDRVTMTRDTSISTAYMELSRL RSDDTAVYYCARDLGYFDVWGQGTLVTVSS | 210 |
| | 2nd | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCRAS QDIRSNLNWYQQKPGGAVKLLIYYTSRLHSGVPSRFSGSGSGTDYTLTISSLQPEDFATYFCQ QSEKLPRTFGGGTKVEIR | 211 |
| | 3rd | *see above* | 511 |
| Ly2606 | 1st | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCGGGGSGGGGSGGGGSGGGGSEVQLVESG GGLVQPGGSLRLSCAASGFTFSTYAMNWVRQAPGKGLEWVGRIRSKYNNYATYYADSVKG RFTISRDDSKNSLYLQMNSLKTEDTAVYYCVRHGNFGNSYVSWFAYWGQGTLVTVSSGGGG SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPG | 203 |
| | 2nd | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCGGGGSGGGGSGGGGSGGGGSQAVVTQEP SLTVSPGGTVTLTCRSSTGAVTTSNYANWVQQKPGQAPRGLIGGTNKRAPWTPARFSGSLL GGKAALTITGAQAEDEADYYCALWYSNLWVFGGGTKLTVLGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 206 |
| | 3rd | *see above* | 127 |
| Ly2607 | 1st | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSEV QLVESGGGLVQPGGSLRLSCAASGFTFSTYAMNWVRQAPGKGLEWVGRIRSKYNNYATYYA DSVKGRFTISRDDSKNSLYLQMNSLKTEDTAVYYCVRHGNFGNSYVSWFAYWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 270 |
| | 2nd | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPG | 272 |
| | 3rd | *see above* | 114 |
| Ly2608 | 1st | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPEAAGPSVFLFPPKPKDTL MISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLWCLVKGFYP SDIAVEWESNGQPENNYKTTPPVLDSDGSFFLYSKLTVDKSRWQQGNVFSCSVMHEALHNH YTQKSLSLSPGGGGGSGGGGSGGGGSGGGGSQVQLVQSGAEVKKPGASVKVSCKASGYT FAGFEMHWVRQAPGQGLEWMGAIDPKTGGTDYNQKFKDRVTMTRDTSISTAYMELSRLRSD DTAVYYCARDLGYFDVWGQGTLVTVSS | 200 |
| | 2nd | EVQLVQSGPEVKKPGATVKISCKTSGYTFTEYTIHWVKQAPGKGLEWIGNINPNNGGTTYNQ KFEDKATLTVDKSTDTAYMELSSLRSEDTAVYYCAAGWNFDYWGQGTLLTVSSASTKGPSVF PLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPEAAGPSVFLFPPKPKDTL MISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLSCAVKGFYP SDIAVEWESNGQPENNYKTTPPVLDSDGSFFLVSKLTVDKSRWQQGNVFSCSVMHEALHNR FTQKSLSLSPGGGGGSGGGGSGGGGSGGGGSDIQMTQSPSSLSASVGDRVTITCRASQDIR SNLNWYQQKPGGAVKLLIYYTSRLHSGVPSRFSGSGSGTDYTLTISSLQPEDFATYFCQQSE KLPRTFGGGTKVEIR | 201 |
| | 3rd | *see above* | 127 |
| Ly2609 | 1st | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPEAAGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTKNQVSLWCLVK GFYPSDIAVEWESNGQPENNYKTTPPVLDSDGSFFLYSKLTVDKSRWQQGNVFSCSVMHEA LHNHYTQKSLSLSPGGGGGSGGGGSGGGGSGGGGSQVQLVQSGAEVKKPGASVKVSCKA SGYTFAGFEMHWVRQAPGQGLEWMGAIDPKTGGTDYNQKFKDRVTMTRDTSISTAYMELS RLRSDDTAVYYCARDLGYFDVWGQGTLVTVSS | 267 |
| | 2nd | QVQLVESGGGLVKPGESLRLSCAASGFTFSDYYMYWVRQAPGKGLEWVAIISDGGYYTYYS DIIKGRFTISRDNAKNSLYLQMNSLKAEDTAVYYCARGFPLLRHGAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPEAAGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTKNQVSLSCAVK GFYPSDIAVEWESNGQPENNYKTTPPVLDSDGSFFLVSKLTVDKSRWQQGNVFSCSVMHEA LHNRFTQKSLSLSPGGGGGSGGGGSGGGGSGGGGSDIQMTQSPSSLSASVGDRVTITCRA SQDIRSNLNWYQQKPGGAVKLLIYYTSRLHSGVPSRFSGSGSGTDYTLTISSLQPEDFATYFC QQSEKLPRTFGGGTKVEIR | 268 |
| | 3rd | *see above* | 114 |
| Ly2610 | 1st | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSEV QLVESGGGLVQPGGSLRLSCAASGFTFSTYAMNWVRQAPGKGLEWVGRIRSKYNNYATYYA DSVKGRFTISRDDSKNSLYLQMNSLKTEDTAVYYCVRHGNFGNSYVSWFAYWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 138 |
| | 2nd | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APEAAGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPG | 140 |
| | 3rd | *see above* | 122 |
| Ly2611 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPG | 181 |
| | 2nd | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPG | 183 |
| | 3rd | *see above* | 112 |
| Ly2612 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS VAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGGSGG GGSEVQLVESGGGLVQPGGSLRLSCAASGFTFSTYAMNWVRQAPGKGLEWVGRIRSKYNN YATYYADSVKGRFTISRDDSKNSLYLQMNSLKTEDTAVYYCVRHGNFGNSYVSWFAYWGQG TLVTVSSGGGGSDKTHTCPPCPAPEAAGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPG | 187 |
| | 2nd | *see above* | 140 |
| | 3rd | *see above* | 113 |
| | 4th | *see above* | 122 |
| Ly2613 | 1st | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGQGTKVEIKGGGGSGGGGSGGGGSGG GGSEVQLVESGGGLVQPGGSLRLSCAASGFNIKDTYIHWVRQAPGKGLEWVARIYPTNGYT RYADSVKGRFTISADTSKNTAYLQMNSLRAEDTAVYYCSRWGGDGFYAMDYWGQGTLVTVS SGGGGSGGGGSGGGGSGGGGSEVQLVESGGGLVQPGGSLRLSCAASGFTFSTYAMNWV RQAPGKGLEWVGRIRSKYNNYATYYADSVKGRFTISRDDSKNSLYLQMNSLKTEDTAVYYCV RHGNFGNSYVSWFAYWGQGTLVTVSSGGGGSDKTHTCPPCPAPEAAGPSVFLFPPKPKDT LMISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLWCLVKGFYP SDIAVEWESNGQPENNYKTTPPVLDSDGSFFLYSKLTVDKSRWQQGNVFSCSVMHEALHNH YTQKSLSLSPG | 121 |
| | 2nd | *see above* | 183 |
| | 3rd | *see above* | 112 |
| Ly2614 | 1st | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPEAAGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTKNQVSLWCLVK GFYPSDIAVEWESNGQPENNYKTTPPVLDSDGSFFLYSKLTVDKSRWQQGNVFSCSVMHEA LHNHYTQKSLSLSPGGGGGSGGGGSGGGGSGGGGSQVQLVQSGAEVKKPGASVKVSCKA SGYTFAGFEMHWVRQAPGQGLEWMGAIDPKTGGTDYNQKFKDRVTMTRDTSISTAYMELS RLRSDDTAVYYCARDLGYFDVWGQGTLVTVSS | 135 |
| | 2nd | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPEAAGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTKNQVSLSCAVK GFYPSDIAVEWESNGQPENNYKTTPPVLDSDGSFFLVSKLTVDKSRWQQGNVFSCSVMHEA LHNRFTQKSLSLSPGGGGGSGGGGSGGGGSGGGGSDIQMTQSPSSLSASVGDRVTITCRA SQDIRSNLNWYQQKPGGAVKLLIYYTSRLHSGVPSRFSGSGSGTDYTLTISSLQPEDFATYFC QQSEKLPRTFGGGTKVEIR | 136 |
| | 3rd | *see above* | 122 |
| Ly2615 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSQVQLVQSGAEVKKPGASVKVSCK ASGYTFAGFEMHWVRQAPGQGLEWMGAIDPKTGGTDYNQKFKDRVTMTRDTSISTAYMELS RLRSDDTAVYYCARDLGYFDVWGQGTLVTVSS | 178 |
| | 2nd | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 179 |
| | 3rd | *see above* | 112 |
| Ly2616 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS VAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECDKTHTCPPCPAPEAAGPSV FLFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQVSL WCLVKGFYPSDIAVEWESNGQPENNYKTTPPVLDSDGSFFLYSKLTVDKSRWQQGNVFSCS VMHEALHNHYTQKSLSLSPGGGGGSGGGGSGGGGSGGGGSQVQLVQSGAEVKKPGASVK VSCKASGYTFAGFEMHWVRQAPGQGLEWMGAIDPKTGGTDYNQKFKDRVTMTRDTSISTAY MELSRLRSDDTAVYYCARDLGYFDVWGQGTLVTVSS | 185 |
| | 2nd | *see above* | 136 |
| | 3rd | *see above* | 113 |
| | 4th | *see above* | 122 |
| Ly2617 | 1st | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGQGTKVEIKGGGGSGGGGSGGGGSGG GGSEVQLVESGGGLVQPGGSLRLSCAASGFNIKDTYIHWVRQAPGKGLEWVARIYPTNGYT RYADSVKGRFTISADTSKNTAYLQMNSLRAEDTAVYYCSRWGGDGFYAMDYWGQGTLVTVS SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPGGGGGSGGGGSGGGGSGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSS | 119 |
| | 2nd | *see above* | 179 |
| | 3rd | *see above* | 112 |
| Ly2618 | 1st | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPEAAGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPG | 196 |
| | 2nd | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PEAAGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNRFTQKSLSLSPG | 198 |
| | 3rd | *see above* | 109 |
| Ly2619 | 1st | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPEAAGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPG | 147 |
| | 2nd | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPEAAGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPG | 151 |
| | 3rd | *see above* | 110 |
| Ly2620 | 1st | qvqlvqsgaevkkpgasvkvsckasgytftefgmnwvrqapgqglewmgwintktgeatyveefkgrvtfttdtststaymelrslr sddtavyycarwdfayyveamdywgqgttvtvssASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVE PKSCGGGGSGGGGSGGGGSGGGGSEVQLVESGGGLVQPGGSLRLSCAASGFTFSTYAMN WVRQAPGKGLEWVGRIRSKYNNYATYYADSVKGRFTISRDDSKNSLYLQMNSLKTEDTAVYY CVRHGNFGNSYVSWFAYWGQGTLVTVSSGGGGSDKTHTCPPCPAPEAAGPSVFLFPPKPK DTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLWCLVKGF YPSDIAVEWESNGQPENNYKTTPPVLDSDGSFFLYSKLTVDKSRWQQGNVFSCSVMHEALH NHYTQKSLSLSPG | 306 |
| | 2nd | qvqlvqsgaevkkpgasvkvsckasgytftefgmnwvrqapgqglewmgwintktgeatyveefkgrvtfttdtststaymelrslr sddtavyycarwdfayyveamdywgqgttvtvssASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVE PKSCGGGGSGGGGSGGGGSGGGGSQAVVTQEPSLTVSPGGTVTLTCRSSTGAVTTSNYAN WVQQKPGQAPRGLIGGTNKRAPWTPARFSGSLLGGKAALTITGAQAEDEADYYCALWYSNL WVFGGGTKLTVLGGGGSDKTHTCPPCPAPEAAGPSVFLFPPKPKDTLMISRTPEVTCVVVDV SHEDPEVKFNWYVDGVEVHNAKTKPREEQYNSTYRVVSVLTVLHQDWLNGKEYKCKVSNKA LPAPIEKTISKAKGQPREPQVYTLPPSREEMTKNQVSLSCAVKGFYPSDIAVEWESNGQPENN YKTTPPVLDSDGSFFLVSKLTVDKSRWQQGNVFSCSVMHEALHNRFTQKSLSLSPG | 308 |
| | 3rd | *see above* | 111 |
| Ly2621 | 1st | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFAGFEMHWVRQAPGQGLEWMGAIDPKTGGTDYNQKFKDRVTMTRDTSISTAYMELSRL RSDDTAVYYCARDLGYFDVWGQGTLVTVSS | 193 |
| | 2nd | EVQLVESGGGVVQPGRSLRLSCSASGFDFTTYWMSWVRQAPGKGLEWIGEIHPDSSTINYA PSLKDRFTISRDNAKNTLFLQMDSLRPEDTGVYFCASLYFGFPWFAYWGQGTPVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCRAS QDIRSNLNWYQQKPGGAVKLLIYYTSRLHSGVPSRFSGSGSGTDYTLTISSLQPEDFATYFCQ QSEKLPRTFGGGTKVEIR | 194 |
| | 3rd | *see above* | 109 |
| Ly2622 | 1st | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSQVQLVQSGAEVKKPGASVKVSCK ASGYTFAGFEMHWVRQAPGQGLEWMGAIDPKTGGTDYNQKFKDRVTMTRDTSISTAYMELS RLRSDDTAVYYCARDLGYFDVWGQGTLVTVSS | 144 |
| | 2nd | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCR ASQDIRSNLNWYQQKPGGAVKLLIYYTSRLHSGVPSRFSGSGSGTDYTLTISSLQPEDFATYF CQQSEKLPRTFGGGTKVEIR | 145 |
| | 3rd | *see above* | 110 |
| Ly2623 | 1st | qvqlvqsgaevkkpgasvkvsckasgytftefgmnwvrqapgqglewmgwintktgeatyveefkgrvtfttdtststaymelrslr sddtavyycarwdfayyveamdywgqgttvtvssASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVE PKSCDKTHTCPPCPAPEAAGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKG QPREPQVYTLPPSREEMTKNQVSLWCLVKGFYPSDIAVEWESNGQPENNYKTTPPVLDSDG SFFLYSKLTVDKSRWQQGNVFSCSVMHEALHNHYTQKSLSLSPGGGGGSGGGGSGGGGS GGGGSQVQLVQSGAEVKKPGASVKVSCKASGYTFAGFEMHWVRQAPGQGLEWMGAIDPK TGGTDYNQKFKDRVTMTRDTSISTAYMELSRLRSDDTAVYYCARDLGYFDVWGQGTLVTVS S | 303 |
| | 2nd | qvqlvqsgaevkkpgasvkvsckasgytftefgmnwvrqapgqglewmgwintkgeatyveefkgrvtfttdtststaymelrslr sddtavyycarwdfayyveamdywgqgttvtvssASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVE PKSCDKTHTCPPCPAPEAAGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKG QPREPQVYTLPPSREEMTKNQVSLSCAVKGFYPSDIAVEWESNGQPENNYKTTPPVLDSDG SFFLVSKLTVDKSRWQQGNVFSCSVMHEALHNRFTQKSLSLSPGGGGGSGGGGSGGGGS GGGGSDIQMTQSPSSLSASVGDRVTITCRASQDIRSNLNWYQQKPGGAVKLLIYYTSRLHSG VPSRFSGSGSGTDYTLTISSLQPEDFATYFCQQSEKLPRTFGGGTKVEIR | 304 |
| | 3rd | *see above* | 111 |
| Ly2624 | 1st | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPG | 157 |
| | 2nd | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPEAAGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPG | 159 |
| | 3rd | *see above* | 124 |
| Ly2625 | 1st | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGPSVFLFP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLWCL VKGFYPSDIAVEWESNGQPENNYKTTPPVLDSDGSFFLYSKLTVDKSRWQQGNVFSCSVMH EALHNHYTQKSLSLSPGGGGGSGGGGSGGGGSGGGGSQVQLVQSGAEVKKPGASVKVSC KASGYTFAGFEMHWVRQAPGQGLEWMGAIDPKTGGTDYNQKFKDRVTMTRDTSISTAYMEL SRLRSDDTAVYYCARDLGYFDVWGQGTLVTVSS | 154 |
| | 2nd | EVQLVESGGGLVQPGGSLRLSCAASGFNVYASGMHWVRQAPGKGLEWVAKIYPDSDYTYY ADSVKGRFTISADTSKNTAYLQMNSLRAEDTAVYYCSRDSSFYYVYAMDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGPSVFLFP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSLSASVGDRVTITC RASQDIRSNLNWYQQKPGGAVKLLIYYTSRLHSGVPSRFSGSGSGTDYTLTISSLQPEDFATY FCQQSEKLPRTFGGGTKVEIR | 155 |
| | 3rd | *see above* | 124 |
| Ly2626 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQVQLVQSGAEVKKPGASVKVSCKASGYTFTGYYMHWVRQAPGQGLEWMGWINPDSGGT NYAQKFQGRVTMTRDTSISTAYMELNRLRSDDTAVYYCARDQPLGYCTNGVCSYFDYWGQ GTLVTVSSGGGGSDKTHTCPPCPAPEAAGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPI EKTISKAKGQPREPQVYTLPPSREEMTKNQVSLWCLVKGFYPSDIAVEWESNGQPENNYKTT PPVLDSDGSFFLYSKLTVDKSRWQQGNVFSCSVMHEALHNHYTQKSLSLSPGGGGGSGGG GSGGGGSGGGGSQVQLVQSGAEVKKPGASVKVSCKASGYTFAGFEMHWVRQAPGQGLE WMGAIDPKTGGTDYNQKFKDRVTMTRDTSISTAYMELSRLRSDDTAVYYCARDLGYFDVWG QGTLVTVSS | 299 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSVSASVGDRVTITCRASQGIYSWLAWYQQKPGKAPNLLIYTASTLQSGVPSRF SGSGSGTDFTLTISSLQPEDFATYYCQQANIFPLTFGGGTKVEIKGGGGSDKTHTCPPCPAPE AAGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSREEMT KNQVSLSCAVKGFYPSDIAVEWESNGQPENNYKTTPPVLDSDGSFFLVSKLTVDKSRWQQG NVFSCSVMHEALHNRFTQKSLSLSPGGGGGSGGGGSGGGGSGGGGSDIQMTQSPSSLSAS VGDRVTITCRASQDIRSNLNWYQQKPGGAVKLLIYYTSRLHSGVPSRFSGSGSGTDYTLTISS LQPEDFATYFCQQSEKLPRTFGGGTKVEIR | 285 |
| | 3rd | *see above* | 115 |
| Ly2627 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SEVQLVESGGGLVQPGGSLRLSCAASGFTFTNYGLHWVRQAPGKGLEWVSSISPSGGVTYY RDSVKGRFTISRDNSKNTLYLQMNSLRAEDTAVYYCAKPFLGWGGANWIAHWGQGTLVTVS SGGGGSDKTHTCPPCPAPEAAGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGG SGGGGSQVQLVQSGAEVKKPGASVKVSCKASGYTFAGFEMHWVRQAPGQGLEWMGAIDP KTGGTDYNQKFKDRVTMTRDTSISTAYMELSRLRSDDTAVYYCARDLGYFDVWGQGTLVTV SS | 294 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSLSASVGDRVTITCLASEDISNDLAWYQQKPGKAPKLLIYFVDRLLDGVPSRFS GSGSGTDFTLTISSLQPEDFATYYCQQSYKYPPTFGQGTKLEIKGGGGSDKTHTCPPCPAPE AAGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSREEMT KNQVSLSCAVKGFYPSDIAVEWESNGQPENNYKTTPPVLDSDGSFFLVSKLTVDKSRWQQG NVFSCSVMHEALHNRFTQKSLSLSPGGGGGSGGGGSGGGGSGGGGSDIQMTQSPSSLSAS VGDRVTITCRASQDIRSNLNWYQQKPGGAVKLLIYYTSRLHSGVPSRFSGSGSGTDYTLTISS LQPEDFATYFCQQSEKLPRTFGGGTKVEIR | 286 |
| | 3rd | *see above* | 115 |
| Ly2628 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSSGGGG SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPGGGGGSGGGGSGGGGSGGGG SQVQLVQSGAEVKKPGASVKVSCKASGYTFTGYYMHWVRQAPGQGLEWMGWINPDSGGT NYAQKFQGRVTMTRDTSISTAYMELNRLRSDDTAVYYCARDQPLGYCTNGVCSYFDYWGQ GTLVTVSS | 300 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSLSASVGDRVTITCRASQDIRSNLNWYQQKPGGAVKLLIYYTSRLHSGVPSRF SGSGSGTDYTLTISSLQPEDFATYFCQQSEKLPRTFGGGTKVEIRGGGGSDKTHTCPPCPAP EAAGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEM TKNQVSLSCAVKGFYPSDIAVEWESNGQPENNYKTTPPVLDSDGSFFLVSKLTVDKSRWQQ GNVFSCSVMHEALHNRFTQKSLSLSPGGGGGSGGGGSGGGGSGGGGSDIQMTQSPSSVS ASVGDRVTITCRASQGIYSWLAWYQQKPGKAPNLLIYTASTLQSGVPSRFSGSGSGTDFTLTI SSLQPEDFATYYCQQANIFPLTFGGGTKVEIK | 287 |
| | 3rd | *see above* | 115 |
| Ly2629 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSSGGGG SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPGGGGGSGGGGSGGGGSGGGG SEVQLVESGGGLVQPGGSLRLSCAASGFTFTNYGLHWVRQAPGKGLEWVSSISPSGGVTYY RDSVKGRFTISRDNSKNTLYLQMNSLRAEDTAVYYCAKPFLGWGGANWIAHWGQGTLVTVS S | 301 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSLSASVGDRVTITCRASQDIRSNLNWYQQKPGGAVKLLIYYTSRLHSGVPSRF SGSGSGTDYTLTISSLQPEDFATYFCQQSEKLPRTFGGGTKVEIRGGGGSDKTHTCPPCPAP EAAGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEM TKNQVSLSCAVKGFYPSDIAVEWESNGQPENNYKTTPPVLDSDGSFFLVSKLTVDKSRWQQ GNVFSCSVMHEALHNRFTQKSLSLSPGGGGGSGGGGSGGGGSGGGGSDIQMTQSPSSLS ASVGDRVTITCLASEDISNDLAWYQQKPGKAPKLLIYFVDRLLDGVPSRFSGSGSGTDFTLTIS SLQPEDFATYYCQQSYKYPPTFGQGTKLEIK | 288 |
| | 3rd | *see above* | 115 |
| Ly2630 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFTGYYMHWVRQAPGQGLEWMGWINPDSGGTNYAQ KFQGRVTMTRDTSISTAYMELNRLRSDDTAVYYCARDQPLGYCTNGVCSYFDYWGQGTLVT VSSGGGGSDKTHTCPPCPAPEAAGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 174 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSVSASVGDRVTITCRASQGIYSWLAWYQQKPGKAPNLLIYTASTLQSGVPSRFSGS GSGTDFTLTISSLQPEDFATYYCQQANIFPLTFGGGTKVEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 166 |
| | 3rd | *see above* | 125 |
| Ly2631 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFTNYGLHWVRQAPGKGLEWVSSISPSGGVTYYRDSV KGRFTISRDNSKNTLYLQMNSLRAEDTAVYYCAKPFLGWGGANWIAHWGQGTLVTVSSGGG GSDKTHTCPPCPAPEAAGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGGGGGSGGGGSGGGGSGG GGSQVQLVQSGAEVKKPGASVKVSCKASGYTFAGFEMHWVRQAPGQGLEWMGAIDPKTG GTDYNQKFKDRVTMTRDTSISTAYMELSRLRSDDTAVYYCARDLGYFDVWGQGTLVTVSS | 170 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCLASEDISNDLAWYQQKPGKAPKLLIYFVDRLLDGVPSRFSGSG SGTDFTLTISSLQPEDFATYYCQQSYKYPPTFGQGTKLEIKGGGGSDKTHTCPPCPAPEAAG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCRASQDIRSNLNWYQQKPGGAVKLLIYYTSRLHSGVPSRFSGSGSGTDYTLTISSLQ PEDFATYFCQQSEKLPRTFGGGTKVEIR | 162 |
| | 3rd | *see above* | 125 |
| Ly2632 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSQV QLVQSGAEVKKPGASVKVSCKASGYTFTGYYMHWVRQAPGQGLEWMGWINPDSGGTNYA QKFQGRVTMTRDTSISTAYMELNRLRSDDTAVYYCARDQPLGYCTNGVCSYFDYWGQGTLV TVSS | 175 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSVSASV GDRVTITCRASQGIYSWLAWYQQKPGKAPNLLIYTASTLQSGVPSRFSGSGSGTDFTLTISSL QPEDFATYYCQQANIFPLTFGGGTKVEIK | 163 |
| | 3rd | *see above* | 125 |
| Ly2633 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPGGGGGSGGGGSGGGGSGGGGSEV QLVESGGGLVQPGGSLRLSCAASGFTFTNYGLHWVRQAPGKGLEWVSSISPSGGVTYYRDS VKGRFTISRDNSKNTLYLQMNSLRAEDTAVYYCAKPFLGWGGANWIAHWGQGTLVTVSS | 176 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPGGGGGSGGGGSGGGGSGGGGSDIQMTQSPSSLSASVG DRVTITCLASEDISNDLAWYQQKPGKAPKLLIYFVDRLLDGVPSRFSGSGSGTDFTLTISSLQP EDFATYYCQQSYKYPPTFGQGTKLEIK | 164 |
| | 3rd | *see above* | 125 |
| Ly2634 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQVQLVQSGAEVKKPGASVKVSCKASGYTFAGFEMHWVRQAPGQGLEWMGAIDPKTGGTD YNQKFKDRVTMTRDTSISTAYMELSRLRSDDTAVYYCARDLGYFDVWGQGTLVTVSSGGGG SDKTHTCPPCPAPEAAGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTPPVLDSDGSFFL YSKLTVDKSRWQQGNVFSCSVMHEALHNHYTQKSLSLSPG | 302 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SDIQMTQSPSSLSASVGDRVTITCRASQDIRSNLNWYQQKPGGAVKLLIYYTSRLHSGVPSRF SGSGSGTDYTLTISSLQPEDFATYFCQQSEKLPRTFGGGTKVEIRGGGGSDKTHTCPPCPAP EAAGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEM TKNQVSLSCAVKGFYPSDIAVEWESNGQPENNYKTTPPVLDSDGSFFLVSKLTVDKSRWQQ GNVFSCSVMHEALHNRFTQKSLSLSPG | 289 |
| | 3rd | *see above* | 115 |
| Ly2635 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVM HEALHNHYTQKSLSLSPGGGGGSGGGGSGGGGSGGGGSQVQLVQSGAEVKKPGASVKVS CKASGYTFAGFEMHWVRQAPGQGLEWMGAIDPKTGGTDYNQKFKDRVTMTRDTSISTAYM ELSRLRSDDTAVYYCARDLGYFDVWGQGTLVTVSS | 281 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSLSASVGDRVTITC RASQDIRSNLNWYQQKPGGAVKLLIYYTSRLHSGVPSRFSGSGSGTDYTLTISSLQPEDFATY FCQQSEKLPRTFGGGTKVEIR | 282 |
| | 3rd | *see above* | 115 |
| Ly2636 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQVQ LVQSGAEVKKPGASVKVSCKASGYTFAGFEMHWVRQAPGQGLEWMGAIDPKTGGTDYNQK FKDRVTMTRDTSISTAYMELSRLRSDDTAVYYCARDLGYFDVWGQGTLVTVSSGGGGSDKT HTCPPCPAPEAAGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQV YTLPPSREEMTKNQVSLWCLVKGFYPSDIAVEWESNGQPENNYKTTPPVLDSDGSFFLYSKL TVDKSRWQQGNVFSCSVMHEALHNHYTQKSLSLSPG | 177 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIRGGGGSDKTHTCPPCPAPEAA GPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKN QVSLSCAVKGFYPSDIAVEWESNGQPENNYKTTPPVLDSDGSFFLVSKLTVDKSRWQQGNV FSCSVMHEALHNRFTQKSLSLSPG | 165 |
| | 3rd | *see above* | 125 |
| Ly2637 | 1st | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFAGFEMHWVRQAPGQGLEWMGAIDPKTGGTDYNQKFKDRVTMTRDTSISTAYMELSRL RSDDTAVYYCARDLGYFDVWGQGTLVTVSS | 160 |
| | 2nd | EVQLVESGGGLVQPGGSLRLSCAASGFTFSDSWIHWVRQAPGKGLEWVAWISPYGGSTYYA DSVKGRFTISADTSKNTAYLQMNSLRAEDTAVYYCARRHWPGGFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCRAS QDIRSNLNWYQQKPGGAVKLLIYYTSRLHSGVPSRFSGSGSGTDYTLTISSLQPEDFATYFCQ QSEKLPRTFGGGTKVEIR | 161 |
| | 3rd | *see above* | 125 |
| Ly2638 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFAGFEMHWVRQAPGQGLEWMGAIDPKTGGTDYNQKFKDRVTMTRDTSISTAYMELSRL RSDDTAVYYCARDLGYFDVWGQGTLVTVSSGGGGSDKTHTCPPCPAPEAAGP | 471 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCRAS QDIRSNLNWYQQKPGGAVKLLIYYTSRLHSGVPSRFSGSGSGTDYTLTISSLQPEDFATYFCQ QSEKLPRTFGGGTKVEIRGGGGSDKTHTCPPCPAPEAAGP | 472 |
| | 3rd | *see above* | 207 |
| Ly2639 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSQVQLVQSGAEVKKPGASVKVSCKAS GYTFTGYYMHWVRQAPGQGLEWMGWINPDSGGTNYAQKFQGRVTMTRDTSISTAYMELNR LRSDDTAVYYCARDQPLGYCTNGVCSYFDYWGQGTLVTVSSGGGGSDKTHTCPPCPAPEA AGP | 477 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSVSASVGDRVTITCRAS QGIYSWLAWYQQKPGKAPNLLIYTASTLQSGVPSRFSGSGSGTDFTLTISSLQPEDFATYYCQ QANIFPLTFGGGTKVEIKGGGGSDKTHTCPPCPAPEAAGP | 478 |
| | 3rd | *see above* | 207 |
| Ly2640 | 1st | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLWCLVKG FYPSDIAVEWESNGQPENNYKTTPPVLDSDGSFFLYSKLTVDKSRWQQGNVFSCSVMHEAL HNHYTQKSLSLSPGGGGGSGGGGSGGGGSGGGGSEVQLVESGGGLVQPGGSLRLSCAAS GFTFTNYGLHWVRQAPGKGLEWVSSISPSGGVTYYRDSVKGRFTISRDNSKNTLYLQMNSLR AEDTAVYYCAKPFLGWGGANWIAHWGQGTLVTVSSGGGGSDKTHTCPPCPAPEAAGP | 479 |
| | 2nd | QVTLKESGPALVKPTQTLTLTCTFSGFSLSTSGTCVSWIRQPPGKALEWLATICWEDSKGYNP SLKSRLTISKDTSKNQAVLTMTNMDPVDTATYYCARREDSGYFWFPYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLVSKLTVDKSRWQQGNVFSCSVMHEAL HNRFTQKSLSLSPGGGGGSGGGGSGGGGSGGGGSDIQMTQSPSSLSASVGDRVTITCLAS EDISNDLAWYQQKPGKAPKLLIYFVDRLLDGVPSRFSGSGSGTDFTLTISSLQPEDFATYYCQ QSYKYPPTFGQGTKLEIKGGGGSDKTHTCPPCPAPEAAGP | 480 |
| | 3rd | *see above* | 207 |
| Ly2641 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSQVQLVQSGAEVKKPGASVKVSCK ASGYTFTGYYMHWVRQAPGQGLEWMGWINPDSGGTNYAQKFQGRVTMTRDTSISTAYMEL NRLRSDDTAVYYCARDQPLGYCTNGVCSYFDYWGQGTLVTVSSGGGGSDKTHTCPPCPAP EAAGP | 325 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSVSASVGDRVTITCR ASQGIYSWLAWYQQKPGKAPNLLIYTASTLQSGVPSRFSGSGSGTDFTLTISSLQPEDFATYY CQQANIFPLTFGGGTKVEIKGGGGSDKTHTCPPCPAPEAAGP | 326 |
| | 3rd | *see above* | 131 |
| Ly2642 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGGGGGSGGGGSGGGGSGGGGSEVQLVESGGGLVQPGGSLRLSCA ASGFTFTNYGLHWVRQAPGKGLEWVSSISPSGGVTYYRDSVKGRFTISRDNSKNTLYLQMN SLRAEDTAVYYCAKPFLGWGGANWIAHWGQGTLVTVSSGGGGSDKTHTCPPCPAPEAAGP | 327 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGGGGGSGGGGSGGGGSGGGGSDIQMTQSPSSLSASVGDRVTITCLA SEDISNDLAWYQQKPGKAPKLLIYFVDRLLDGVPSRFSGSGSGTDFTLTISSLQPEDFATYYC QQSYKYPPTFGQGTKLEIKGGGGSDKTHTCPPCPAPEAAGP | 328 |
| | 3rd | *see above* | 131 |
| Ly2643 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVM HEALHNHYTQKSLSLSPGGGGGSGGGGSGGGGSGGGGSQVQLVQSGAEVKKPGASVKVS CKASGYTFTGYYMHWVRQAPGQGLEWMGWINPDSGGTNYAQKFQGRVTMTRDTSISTAYM ELNRLRSDDTAVYYCARDQPLGYCTNGVCSYFDYWGQGTLVTVSSGGGGSDKTHTCPPCP APEAAGP | 277 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGSGGGGSGGGGSGGGGSDIQMTQSPSSVSASVGDRVTITC RASQGIYSWLAWYQQKPGKAPNLLIYTASTLQSGVPSRFSGSGSGTDFTLTISSLQPEDFATY YCQQANIFPLTFGGGTKVEIKGGGGSDKTHTCPPCPAPEAAGP | 278 |
| | 3rd | *see above* | 115 |
| Ly2644 | 1st | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVM HEALHNHYTQKSLSLSPGGGGGSGGGGSGGGGSGGGGSEVQLVESGGGLVQPGGSLRLS CAASGFTFTNYGLHWVRQAPGKGLEWVSSISPSGGVTYYRDSVKGRFTISRDNSKNTLYLQ MNSLRAEDTAVYYCAKPFLGWGGANWIAHWGQGTLVTVSSGGGGSDKTHTCPPCPAPEAA GP | 279 |
| | 2nd | QVQLVQSGAEVKKPGASVKVSCKASGYTFTDFWMSWVRQAPGQGLEWMGQIYPNTGTTHS NEKFKGRVTMTRDTSTSTVYMELSSLRSEDTAVYYCARSYHISTTPNWFAYWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGPSVFLF PPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSREEMTKNQVSLSCA VKGFYPSDIAVEWESNGQPENNYKTTPPVLDSDGSFFLVSKLTVDKSRWQQGNVFSCSVMH EALHNRFTQKSLSLSPGGGGGGSGGGGSGGGGSGGGGSDIQMTQSPSSLSASVGDRVTITCL ASEDISNDLAWYQQKPGKAPKLLIYFVDRLLDGVPSRFSGSGSGTDFTLTISSLQPEDFATYY CQQSYKYPPTFGQGTKLEIKGGGGSDKTHTCPPCPAPEAAGP | 280 |
| | 3rd | *see above* | 115 |
| Ly2800 | 1st | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASVAAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGECGGGGSGGGGSGGGGS GGGGSEVQLVESGGGLVQPGGSLRLSCAASGFTFSTYAMNWVRQAPGKGLEWVGRIRSKY NNYATYYADSVKGRFTISRDDSKNSLYLQMNSLKTEDTAVYYCVRHGNFGNSYVSWFAYWG QGTLVTVSSGGGGSDKTHTCPPCPAPELLGPSVFLFPPKPKDTLMISRTPEVTCVVVDVSHE DPEVKFNWYVDGVEVHNAKTKPREEQYNSTYRVVSVLTVLHQDWLNGKEYKCKVSNKALGA PIEKTISKAKGQPREPQVYTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGSG GGGSGGGGSGGGGSQVQLVQSGAEVKKPGASVKVSCKASGYTFAGFEMHWVRQAPGQG LEWMGAIDPKTGGTDYNQKFKDRVTMTRDTSISTAYMELSRLRSDDTAVYYCARDLGYFDV WGQGTLVTVSS | 229 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPELLGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNHYTQKSLSLSPGKGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIR | 261 |
| | 3rd | *see above* | 209 |
| | 4th | *see above* | 107 |
| Ly2802 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECGGGGSGGGGSGGG GSGGGGSEVQLVESGGGLVQPGGSLRLSCAASGFTFSTYAMNWVRQAPGKGLEWVGRIRS KYNNYATYYADSVKGRFTISRDDSKNSLYLQMNSLKTEDTAVYYCVRHGNFGNSYVSWFAY WGQGTLVTVSSGGGGSDKTHTCPPCPAPELLGPSVFLFPPKPKDTLMISRTPEVTCVVVDVS HEDPEVKFNWYVDGVEVHNAKTKPREEQYNSTYRVVSVLTVLHQDWLNGKEYKCKVSNKAL GAPIEKTISKAKGQPREPQVYTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGKGGGG SGGGGSGGGGSGGGGSQVQLVQSGAEVKKPGASVKVSCKASGYTFAGFEMHWVRQAPG QGLEWMGAIDPKTGGTDYNQKFKDRVTMTRDTSISTAYMELSRLRSDDTAVYYCARDLGYF DVWGQGTLVTVSS | 265 |
| | 2nd | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPELLGPSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNHYTQKSLSLSPGKGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 225 |
| | 3rd | *see above* | 108 |
| | 4th | *see above* | 208 |
| Ly2823 | 1st | EVQLVESGGGLVKPGGSLKLSCEASRFTFSSYAMSWVRQTPEKRLEWVAAISGGGRYTYYP DSMKGRFTISRDNAKNFLYLQMSSLRSEDTAMYYCARHYDGYLDYWGQGTTLTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCGGGGSGGGGSGGGGSGGGGSEVQL VESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPELLGPSVFLFPPKPKDTLMISRTPEVTCVVVDVSHEDPEVKFNWY VDGVEVHNAKTKPREEQYNSTYRVVSVLTVLHQDWLNGKEYKCKVSNKALGAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGKGGGGSGGGGSGGG GSGGGGSQVQLVQSGAEVKKPGASVKVSCKASGYTFAGFEMHWVRQAPGQGLEWMGAID PKTGGTDYNQKFKDRVTMTRDTSISTAYMELSRLRSDDTAVYYCARDLGYFDVWGQGTLVT VSS | 133 |
| | 2nd | EVQLVESGGGLVKPGGSLKLSCEASRFTFSSYAMSWVRQTPEKRLEWVAAISGGGRYTYYP DSMKGRFTISRDNAKNFLYLQMSSLRSEDTAMYYCARHYDGYLDYWGQGTTLTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCGGGGSGGGGSGGGGSGGGGSQAVV TQEPSLTVSPGGTVTLTCRSSTGAVTTSNYANWVQQKPGQAPRGLIGGTNKRAPWTPARFS GSLLGGKAALTITGAQAEDEADYYCALWYSNLWVFGGGTKLTVLGGGGSDKTHTCPPCPAP ELLGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALGAPIEKTISKAKGQPREPQVYTLPPSREE MTKNQVSLSCAVKGFYPSDIAVEWESNGQPENNYKTTPPVLDSDGSFFLVSKLTVDKSRWQ QGNVFSCSVMHEALHNHYTQKSLSLSPGKGGGGSGGGGSGGGGSGGGGSDIQMTQSPSS LSASVGDRVTITCRASQDIRSNLNWYQQKPGGAVKLLIYYTSRLHSGVPSRFSGSGSGTDYTL TISSLQPEDFATYFCQQSEKLPRTFGGGTKVEIR | 134 |
| | 3rd | DIVMTQSHKFMSTSIGARVSITCKASQDVRTAVAWYQQKPGQSPKLLIYSASYRYTGVPDRFT GSGSGTDFTFTISSVQAEDLAVYYCQQHYGTPPWTFGGGTKLEIKRTVAAPSVFIFPPSDEQL KSGTASVVCLLNNFYPREAKVQWKVDNALQSGNSQESVTEQDSKDSTYSLSSTLTLSKADYE KHKVYACEVTHQGLSSPVTKSFNRGEC | 128 |
| Ly2846 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVQSGAEVKKPGASVKVSCKASGYTFTDFWMSWVRQAPGQGLEWMGQI YPNTGTTHSNEKFKGRVTMTRDTSTSTVYMELSSLRSEDTAVYYCARSYHISTTPNWFAYWG QGTLVTVSS | 394 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCKASQNVYKKLEWYQQKPGKVPKVLIYHTNILQTGVPSRFSGSGSGTDY TLTISSLQPEDVATYYCYQWNSGPTFGGGTKVEIK | 446 |
| | 3rd | *see above* | 131 |
| Ly2847 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSEVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGGFDYWGQGTL VTVSS | 395 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCRASQDVSTAVAWYQQKPGKAPKLLIYSASFLYSGVPSRFSGSGSGTD FTLTISSLQPEDFATYYCQQYLYHPATFGQGTKVEIK | 447 |
| | 3rd | *see above* | 131 |
| Ly2901 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVESGGGVVQPGRSLRLSCAASGFTFSSYTMHWVRQAPGKGLEWVTFIS YDGNNKYYADSVKGRFTISRDNSKNTLYLQMNSLRAEDTAIYYCARTGWLGPFDYWGQGTL VTVSS | 390 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSEIVLTQSP GTLSLSPGERATLSCRASQSVGSSYLAWYQQKPGQAPRLLIYGAFSRATGIPDRFSGSGSGT DFTLTISRLEPEDFAVYYCQQYGSSPWTFGQGTKVEIK | 442 |
| | 3rd | *see above* | 131 |
| Ly2902 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVESGGGVVQPGRSLRLSCAASGFTFSSYGMHWVRQAPGKGLEWVAVI WYDGSNKYYADSVKGRFTISRDNSKNTLYLQMNSLRAEDTAVYYCARDPRGATLYYYYYGM DVWGQGTTVTVSS | 391 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCRASQSINSYLDWYQQKPGKAPKLLIYAASSLQSGVPSRFSGSGSGTDF TLTISSLQPEDFATYYCQQYYSTPFTFGPGTKVEIK | 443 |
| | 3rd | *see above* | 131 |
| Ly2903 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVESGAEVKKPGASVKVSCKASGYSFTGYTMNWVRQAPGQCLEWIGLIN PYNNITNYAQKFQGRVTFTVDTSISTAYMELSRLRSDDTGVYFCARLDYRSYWGQGTLVTVS A | 392 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSQAVVTQE PSLTVSPGGTVTLTCGSSTGAVTTSNFPNWVQQKPGQAPRSLIGGTNNKASWTPARFSGSLL GGKAALTISGAQPEDEAEYYCALWYSNHWVFGCGTKLTVL | 444 |
| | 3rd | *see above* | 131 |
| Ly2904 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVTLKESGPALVKPTQTLTLTCTFSGFSLSTSGTCVSWIRQPPGKALEWLATIC WEDSKGYNPSLKSRLTISKDTSKNQAVLTMTNMDPVDTATYYCARREDSGYFWFPYWGQGT LVTVSS | 393 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSNIQMTQSP SSLSASVGDRVTITCKAGQNVNNYLAWYQQKPGKAPKVLIFNANSLQTGVPSRFSGSGSGTD FTLTISSLQPEDFATYYCQQYNSWPTFGGGTKVEIK | 445 |
| | 3rd | *see above* | 131 |
| Ly2915 | 1st | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGK | 149 |
| | 2nd | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGK | 153 |
| | 3rd | *see above* | 110 |
| Ly2936 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVQSGAEVKKPGASVKVSCKASGYTFAGFEMHWVRQAPGQGLEWMGAI DPKTGGTDYNQKFKDRVTMTRDTSISTAYMELSRLRSDDTAVYYCARDLGYFDVWGQGTLV TVSS | 387 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCRASQDIRSNLNWYQQKPGGAVKLLIYYTSRLHSGVPSRFSGSGSGTD YTLTISSLQPEDFATYFCQQSEKLPRTFGGGTKVEIR | 439 |
| | 3rd | *see above* | 131 |
| Ly2937 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ VQLQQSGAELARPGASVKMSCKASGYTFTRYTMHWVKQRPGQGLEWIGYINPSRGYTNYN QKFKDKATLTTDKSSSTAYMQLSSLTSEDSAVYYCARYYDDHYCLDYWGQGTTLTVSSGGG GSDKTHTCPPCPAPELLGPSVFLFPPKPKDTLMISRTPEVTCVVVDVSHEDPEVKFNWYVDG VEVHNAKTKPREEQYNSTYRVVSVLTVLHQDWLNGKEYKCKVSNKALGAPIEKTISKAKGQP REPQVYTLPPSREEMTKNQVSLWCLVKGFYPSDIAVEWESNGQPENNYKTTPPVLDSDGSF FLYSKLTVDKSRWQQGNVFSCSVMHEALHNHYTQKSLSLSPGKGGGGSGGGGSGGGGSG GGGSQVQLVQSGAEVKKPGASVKVSCKASGYTFAGFEMHWVRQAPGQGLEWMGAIDPKT GGTDYNQKFKDRVTMTRDTSISTAYMELSRLRSDDTAVYYCARDLGYFDVWGQGTLVTVSS | 449 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQI VLTQSPAIMSASPGEKVTMTCSASSSVSYMNWYQQKSGTSPKRWIYDTSKLASGVPAHFRG SGSGTSYSLTISGMEAEDAATYYCQQWSSNPFTFGSGTKLEINGGGGSDKTHTCPPCPAPEL LGPSVFLFPPKPKDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQVYTLPPSREEMTK NQVSLSCAVKGFYPSDIAVEWESNGQPENNYKTTPPVLDSDGSFFLVSKLTVDKSRWQQGN VFSCSVMHEALHNRFTQKSLSLSPGKGGGGSGGGGSGGGGSGGGGSDIQMTQSPSSLSAS VGDRVTITCRASQDIRSNLNWYQQKPGGAVKLLIYYTSRLHSGVPSRFSGSGSGTDYTLTISS LQPEDFATYFCQQSEKLPRTFGGGTKVEIR | 448 |
| | 3rd | *see above* | 131 |
| Ly2938 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVQSGAEVKKPGASVKVSCKASGYTFTSYYIHWVRQAPGQGLEWIGCIYP GNVNTNYNEKFKDRATLTVDTSISTAYMELSRLRSDDTAVYFCTRSHYGLDWNFDVWGQGT TVTVSS | 388 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCHASQNIYVWLNWYQQKPGKAPKLLIYKASNLHTGVPSRFSGSGSGTD FTLTISSLQPEDFATYYCQQGQTYPYTFGGGTKVEIK | 440 |
| | 3rd | *see above* | 131 |
| Ly2939 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGK | 389 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGK | 441 |
| | 3rd | *see above* | 131 |
| Ly2940 | 1st | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGPSVFLFPP KPKDTLMISRTPEVTCVVVDVSHEDPEVKFNWYVDGVEVHNAKTKPREEQYNSTYRVVSVLT VLHQDWLNGKEYKCKVSNKALGAPIEKTISKAKGQPREPQVYTLPPSREEMTKNQVSLWCLV KGFYPSDIAVEWESNGQPENNYKTTPPVLDSDGSFFLYSKLTVDKSRWQQGNVFSCSVMHE ALHNHYTQKSLSLSPGKGGGGSGGGGSGGGGSGGGGSQVQLVQSGAEVKKPGASVKVSC KASGYTFAGFEMHWVRQAPGQGLEWMGAIDPKTGGTDYNQKFKDRVTMTRDTSISTAYMEL SRLRSDDTAVYYCARDLGYFDVWGQGTLVTVSS | 329 |
| | 2nd | QVQLVQSGAEVKKPGASVKVSCKASGYTFTSYVMHWVRQKPGQGLEWIGYINPYNEGTEST DKFKGRATMTSDKSTSTVYMELSSLRSEDTAVYYCASIYYGYEGTYFGVWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGPSVFLFPP KPKDTLMISRTPEVTCVVVDVSHEDPEVKFNWYVDGVEVHNAKTKPREEQYNSTYRVVSVLT VLHQDWLNGKEYKCKVSNKALGAPIEKTISKAKGQPREPQVYTLPPSREEMTKNQVSLSCAV KGFYPSDIAVEWESNGQPENNYKTTPPVLDSDGSFFLVSKLTVDKSRWQQGNVFSCSVMHE ALHNRFTQKSLSLSPGKGGGGSGGGGSGGGGSGGGGSDIQMTQSPSSLSASVGDRVTITC RASQDIRSNLNWYQQKPGGAVKLLIYYTSRLHSGVPSRFSGSGSGTDYTLTISSLQPEDFATY FCQQSEKLPRTFGGGTKVEIR | 330 |
| | 3rd | *see above* | 131 |
| Ly2943 | 1st | *see above* | 229 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPELLGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNRFTQKSLSLSPGKGGGGSGGGGSGGGGSGGGGSDIQ MTQSPSSLSASVGDRVTITCRASQDIRSNLNWYQQKPGGAVKLLIYYTSRLHSGVPSRFSGS GSGTDYTLTISSLQPEDFATYFCQQSEKLPRTFGGGTKVEIR | 260 |
| | 3rd | *see above* | 209 |
| | 4th | *see above* | 107 |
| Ly2944 | 1st | *see above* | 265 |
| | 2nd | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPELLGPSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNRFTQKSLSLSPGKGGGGSGGGGSGGGGSGGGGSDIQMT QSPSSLSASVGDRVTITCRASQDIRSNLNWYQQKPGGAVKLLIYYTSRLHSGVPSRFSGSGS GTDYTLTISSLQPEDFATYFCQQSEKLPRTFGGGTKVEIR | 224 |
| | 3rd | *see above* | 108 |
| | 4th | *see above* | 208 |
| Ly2945 | 1st | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASVAAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGECGGGGSGGGGSGGGGS GGGGSEVQLVESGGGLVQPGGSLRLSCAASGFTFSTYAMNWVRQAPGKGLEWVGRIRSKY NNYATYYADSVKGRFTISRDDSKNSLYLQMNSLKTEDTAVYYCVRHGNFGNSYVSWFAYWG QGTLVTVSSGGGGSDKTHTCPPCPAPELLGPSVFLFPPKPKDTLMISRTPEVTCVVVDVSHE DPEVKFNWYVDGVEVHNAKTKPREEQYNSTYRVVSVLTVLHQDWLNGKEYKCKVSNKALGA PIEKTISKAKGQPREPQVYTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGK | 230 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCGGGGSGGGGSGGGGSG GGGSQAVVTQEPSLTVSPGGTVTLTCRSSTGAVTTSNYANWVQQKPGQAPRGLIGGTNKRA PWTPARFSGSLLGGKAALTITGAQAEDEADYYCALWYSNLWVFGGGTKLTVLGGGGSDKTH TCPPCPAPELLGPSVFLFPPKPKDTLMISRTPEVTCVVVDVSHEDPEVKFNWYVDGVEVHNA KTKPREEQYNSTYRVVSVLTVLHQDWLNGKEYKCKVSNKALGAPIEKTISKAKGQPREPQVY TLPPSREEMTKNQVSLSCAVKGFYPSDIAVEWESNGQPENNYKTTPPVLDSDGSFFLVSKLT VDKSRWQQGNVFSCSVMHEALHNRFTQKSLSLSPGK | 262 |
| | 3rd | *see above* | 209 |
| | 4th | *see above* | 107 |
| Ly2946 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECGGGGSGGGGSGGG GSGGGGSEVQLVESGGGLVQPGGSLRLSCAASGFTFSTYAMNWVRQAPGKGLEWVGRIRS KYNNYATYYADSVKGRFTISRDDSKNSLYLQMNSLKTEDTAVYYCVRHGNFGNSYVSWFAY WGQGTLVTVSSGGGGSDKTHTCPPCPAPELLGPSVFLFPPKPKDTLMISRTPEVTCVVVDVS HEDPEVKFNWYVDGVEVHNAKTKPREEQYNSTYRVVSVLTVLHQDWLNGKEYKCKVSNKAL GAPIEKTISKAKGQPREPQVYTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGK | 266 |
| | 2nd | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCGGGGSGGGGSGGGGSGGGG SQAVVTQEPSLTVSPGGTVTLTCRSSTGAVTTSNYANWVQQKPGQAPRGLIGGTNKRAPWT PARFSGSLLGGKAALTITGAQAEDEADYYCALWYSNLWVFGGGTKLTVLGGGGSDKTHTCP PCPAPELLGPSVFLFPPKPKDTLMISRTPEVTCVVVDVSHEDPEVKFNWYVDGVEVHNAKTK PREEEQYNSTYRVVSVLTVLHQDWLNGKEYKCKVSNKALGAPIEKTISKAKGQPREPQVYTLP PSREEMTKNQVSLSCAVKGFYPSDIAVEWESNGQPENNYKTTPPVLDSDGSFFLVSKLTVDK SRWQQGNVFSCSVMHEALHNRFTQKSLSLSPGK | 226 |
| | 3rd | *see above* | 108 |
| | 4th | *see above* | 208 |
| Ly2947 | 1st | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASVAAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGECDKTHTCPPCPAPELLGP SVFLFPPKPKDTLMISRTPEVTCVVVDVSHEDPEVKFNWYVDGVEVHNAKTKPREEQYNSTY RVVSVLTVLHQDWLNGKEYKCKVSNKALGAPIEKTISKAKGQPREPQVYTLPPSREEMTKNQ VSLWCLVKGFYPSDIAVEWESNGQPENNYKTTPPVLDSDGSFFLYSKLTVDKSRWQQGNVF SCSVMHEALHNHYTQKSLSLSPGKGGGGSGGGGSGGGGSGGGGSQVQLVQSGAEVKKPG ASVKVSCKASGYTFAGFEMHWVRQAPGQGLEWMGAIDPKTGGTDYNQKFKDRVTMTRDTS ISTAYMELSRLRSDDTAVYYCARDLGYFDVWGQGTLVTVSS | 227 |
| | 2nd | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLGPSV FLFPPKPKDTLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSREEMTKNQVS LSCAVKGFYPSDIAVEWESNGQPENNYKTTPPVLDSDGSFFLVSKLTVDKSRWQQGNVFSC SVMHEALHNRFTQKSLSLSPGKGGGGSGGGGSGGGGSGGGGSDIQMTQSPSSLSASVGD RVTITCRASQDIRSNLNWYQQKPGGAVKLLIYYTSRLHSGVPSRFSGSGSGTDYTLTISSLQP EDFATYFCQQSEKLPRTFGGGTKVEIR | 233 |
| | 3rd | *see above* | 209 |
| | 4th | *see above* | 107 |
| Ly2948 | 1st | QVQLQQSGAELVRPGSSVKISCKASGYAFSSYWMNWVKQRPGQGLEWIGQIWPGDGDTNY NGKFKGKATLTADESSSTAYMQLSSLASEDSAVYFCARRETTTVGRYYYAMDYWGQGTTVT VSSASVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECDKTHTCPPCPAPELL GPSVFLFPPKPKDTLMISRTPEVTCVVVDVSHEDPEVKFNWYVDGVEVHNAKTKPREEQYNS TYRVVSVLTVLHQDWLNGKEYKCKVSNKALGAPIEKTISKAKGQPREPQVYTLPPSREEMTK NQVSLWCLVKGFYPSDIAVEWESNGQPENNYKTTPPVLDSDGSFFLYSKLTVDKSRWQQGN VFSCSVMHEALHNHYTQKSLSLSPGKGGGGSGGGGSGGGGSGGGGSQVQLVQSGAEVKK PGASVKVSCKASGYTFAGFEMHWVRQAPGQGLEWMGAIDPKTGGTDYNQKFKDRVTMTRD TSISTAYMELSRLRSDDTAVYYCARDLGYFDVWGQGTLVTVSS | 263 |
| | 2nd | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVSA ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGPSVFLF PPKPKDTLMISRTPEVTCVVVDVSHEDPEVKFNWYVDGVEVHNAKTKPREEQYNSTYRVVSV LTVLHQDWLNGKEYKCKVSNKALGAPIEKTISKAKGQPREPQVYTLPPSREEMTKNQVSLSC AVKGFYPSDIAVEWESNGQPENNYKTTPPVLDSDGSFFLVSKLTVDKSRWQQGNVFSCSVM HEALHNRFTQKSLSLSPGKGGGGSGGGGSGGGGSGGGGSDIQMTQSPSSLSASVGDRVTI TCRASQDIRSNLNWYQQKPGGAVKLLIYYTSRLHSGVPSRFSGSGSGTDYTLTISSLQPEDFA TYFCQQSEKLPRTFGGGTKVEIR | 222 |
| | 3rd | *see above* | 108 |
| | 4th | *see above* | 208 |
| Ly2951 | 1st | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPELLGPSVFLFPPKPKDTLMISRTPEVTCVVVDVSHEDPEVKF NWYVDGVEVHNAKTKPREEQYNSTYRVVSVLTVLHQDWLNGKEYKCKVSNKALGAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGK | 314 |
| | 2nd | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPELLGPSVFLFPPKPKDTLMISRTPEVTCVVVDVSHEDPEVKFNWYVDGVEVHNAKTKP REEQYNSTYRVVSVLTVLHQDWLNGKEYKCKVSNKALGAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPGK | 318 |
| | 3rd | *see above* | 118 |
| Ly2956 | 1st | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS EVQLVESGGGLVQPGGSLRLSCAASGFTFSTYAMNWVRQAPGKGLEWVGRIRSKYNNYATY YADSVKGRFTISRDDSKNSLYLQMNSLKTEDTAVYYCVRHGNFGNSYVSWFAYWGQGTLVT VSSGGGGSDKTHTCPPCPAPELLGPSVFLFPPKPKDTLMISRTPEVTCVVVDVSHEDPEVKF NWYVDGVEVHNAKTKPREEQYNSTYRVVSVLTVLHQDWLNGKEYKCKVSNKALGAPIEKTIS KAKGQPREPQVYTLPPSREEMTKNQVSLWCLVKGFYPSDIAVEWESNGQPENNYKTTPPVL DSDGSFFLYSKLTVDKSRWQQGNVFSCSVMHEALHNHYTQKSLSLSPGKGGGGSGGGGSG GGGSGGGGSQVQLVQSGAEVKKPGASVKVSCKASGYTFTSYYIHWVRQAPGQGLEWIGCIY PGNVNTNYNEKFKDRATLTVDTSISTAYMELSRLRSDDTAVYFCTRSHYGLDWNFDVWGQG TTVTVSS | 313 |
| | 2nd | QVQLVQSGAEVKKPGASVKVSCKASGYTFTNHIIHWVRQAPGQGLEWMGYINPYPGYHAYN EKFQGRATMTSDTSTSTVYMELSSLRSEDTAVYYCARDGYYRDTDVLDYWGQGTLVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCGGGGSGGGGSGGGGSGGGGS QAVVTQEPSLTVSPGGTVTLTCRSSTGAVTTSNYANWVQQKPGQAPRGLIGGTNKRAPWTP ARFSGSLLGGKAALTITGAQAEDEADYYCALWYSNLWVFGGGTKLTVLGGGGSDKTHTCPP CPAPELLGPSVFLFPPKPKDTLMISRTPEVTCVVVDVSHEDPEVKFNWYVDGVEVHNAKTKP REEQYNSTYRVVSVLTVLHQDWLNGKEYKCKVSNKALGAPIEKTISKAKGQPREPQVYTLPP SREEMTKNQVSLSCAVKGFYPSDIAVEWESNGQPENNYKTTPPVLDSDGSFFLVSKLTVDKS RWQQGNVFSCSVMHEALHNRFTQKSLSLSPGKGGGGSGGGGSGGGGSGGGGSDIQMTQ SPSSLSASVGDRVTITCHASQNIYVWLNWYQQKPGKAPKLLIYKASNLHTGVPSRFSGSGSG TDFTLTISSLQPEDFATYYCQQGQTYPYTFGGGTKVEIK | 317 |
| | 3rd | *see above* | 118 |
| Ly3098 | 1st | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPELLGPSVFLFPPKPKDTLMISRTPEVTCVVVDVSHEDPEVKFNWY VDGVEVHNAKTKPREEQYNSTYRVVSVLTVLHQDWLNGKEYKCKVSNKALGAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGK | 215 |
| | 2nd | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PELLGPSVFLFPPKPKDTLMISRTPEVTCVVVDVSHEDPEVKFNWYVDGVEVHNAKTKPREE QYNSTYRVVSVLTVLHQDWLNGKEYKCKVSNKALGAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGK | 219 |
| | 3rd | *see above* | 511 |
| Ly3103 | 1st | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSEVQ LVESGGGLVQPGGSLRLSCAASGFTFSTYAMNWVRQAPGKGLEWVGRIRSKYNNYATYYAD SVKGRFTISRDDSKNSLYLQMNSLKTEDTAVYYCVRHGNFGNSYVSWFAYWGQGTLVTVSS GGGGSDKTHTCPPCPAPELLGPSVFLFPPKPKDTLMISRTPEVTCVVVDVSHEDPEVKFNWY VDGVEVHNAKTKPREEQYNSTYRVVSVLTVLHQDWLNGKEYKCKVSNKALGAPIEKTISKAK GQPREPQVYTLPPSREEMTKNQVSLWCLVKGFYPSDIAVEWESNGQPENNYKTTPPVLDSD GSFFLYSKLTVDKSRWQQGNVFSCSVMHEALHNHYTQKSLSLSPGKGGGGSGGGGSGGG GSGGGGSQVQLVQSGAEVKKPGASVKVSCKASGYTFTSYYIHWVRQAPGQGLEWIGCIYPG NVNTNYNEKFKDRATLTVDTSISTAYMELSRLRSDDTAVYFCTRSHYGLDWNFDVWGQGTTV TVSS | 214 |
| | 2nd | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCGGGGSGGGGSGGGGSGGGGSQAV VTQEPSLTVSPGGTVTLTCRSSTGAVTTSNYANWVQQKPGQAPRGLIGGTNKRAPWTPARF SGSLLGGKAALTITGAQAEDEADYYCALWYSNLWVFGGGTKLTVLGGGGSDKTHTCPPCPA PELLGPSVFLFPPKPKDTLMISRTPEVTCVVVDVSHEDPEVKFNWYVDGVEVHNAKTKPREE QYNSTYRVVSVLTVLHQDWLNGKEYKCKVSNKALGAPIEKTISKAKGQPREPQVYTLPPSRE EMTKNQVSLSCAVKGFYPSDIAVEWESNGQPENNYKTTPPVLDSDGSFFLVSKLTVDKSRW QQGNVFSCSVMHEALHNRFTQKSLSLSPGKGGGGSGGGGSGGGGSGGGGSDIQMTQSPS SLSASVGDRVTITCHASQNIYVWLNWYQQKPGKAPKLLIYKASNLHTGVPSRFSGSGSGTDF TLTISSLQPEDFATYYCQQGQTYPYTFGGGTKVEIK | 218 |
| | 3rd | *see above* | 511 |
| Ly3152 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS VAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGGSGG GGSEVQLVESGGGLVQPGGSLRLSCAASGFTFSTYAMNWVRQAPGKGLEWVGRIRSKYNN YATYYADSVKGRFTISRDDSKNSLYLQMNSLKTEDTAVYYCVRHGNFGNSYVSWFAYWGQG TLVTVSSGGGGSDKTHTCPPCPAPELLGPSVFLFPPKPKDTLMISRTPEVTCVVVDVSHEDPE VKFNWYVDGVEVHNAKTKPREEQYNSTYRVVSVLTVLHQDWLNGKEYKCKVSNKALGAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 189 |
| | 2nd | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APELLGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALGAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGK | 142 |
| | 3rd | *see above* | 113 |
| | 4th | *see above* | 122 |
| Ly3169 | 1st | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSE VQLVESGGGLVQPGGSLRLSCAASGFTFSTYAMNWVRQAPGKGLEWVGRIRSKYNNYATYY ADSVKGRFTISRDDSKNSLYLQMNSLKTEDTAVYYCVRHGNFGNSYVSWFAYWGQGTLVTV SSGGGGSDKTHTCPPCPAPELLGPSVFLFPPKPKDTLMISRTPEVTCVVVDVSHEDPEVKFN WYVDGVEVHNAKTKPREEQYNSTYRVVSVLTVLHQDWLNGKEYKCKVSNKALGAPIEKTISK AKGQPREPQVYTLPPSREEMTKNQVSLWCLVKGFYPSDIAVEWESNGQPENNYKTTPPVLD SDGSFFLYSKLTVDKSRWQQGNVFSCSVMHEALHNHYTQKSLSLSPGKGGGGSGGGGSGG GGSGGGGSQVQLVQSGAEVKKPGASVKVSCKASGYTFTSYYIHWVRQAPGQGLEWIGCIYP GNVNTNYNEKFKDRATLTVDTSISTAYMELSRLRSDDTAVYFCTRSHYGLDWNFDVWGQGT TVTVSS | 148 |
| | 2nd | EVQLVESGGGLVQPGGSLRLSCAASGFNIKDTYMHWVRQAPGKGLEWVARIDPANGNSKYA DSVKGRFTISADTSKNTAYLQMNSLRAEDTAVYYCAPFGYYVSDYAMAYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCHASQNIYVWLNWYQQKPGKAPKLLIYKASNLHTGVPSRFSGSGSGTD FTLTISSLQPEDFATYYCQQGQTYPYTFGGGTKVEIK | 152 |
| | 3rd | *see above* | 110 |
| Ly3190 | 1st | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS VAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGGSGG GGSEVQLVESGGGLVQPGGSLRLSCAASGFTFSTYAMNWVRQAPGKGLEWVGRIRSKYNN YATYYADSVKGRFTISRDDSKNSLYLQMNSLKTEDTAVYYCVRHGNFGNSYVSWFAYWGQG TLVTVSSGGGGSDKTHTCPPCPAPELLGPSVFLFPPKPKDTLMISRTPEVTCVVVDVSHEDPE VKFNWYVDGVEVHNAKTKPREEQYNSTYRVVSVLTVLHQDWLNGKEYKCKVSNKALGAPIE KTISKAKGQPREPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPGKGGGGSGGG GSGGGGSGGGGSQVQLVQSGAEVKKPGASVKVSCKASGYTFTSYYIHWVRQAPGQGLEWI GCIYPGNVNTNYNEKFKDRATLTVDTSISTAYMELSRLRSDDTAVYFCTRSHYGLDWNFDVW GQGTTVTVSS | 188 |
| | 2nd | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCGGGGSGGGGSGGGGSGGGGSQA VVTQEPSLTVSPGGTVTLTCRSSTGAVTTSNYANWVQQKPGQAPRGLIGGTNKRAPWTPAR FSGSLLGGKAALTITGAQAEDEADYYCALWYSNLWVFGGGTKLTVLGGGGSDKTHTCPPCP APELLGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALGAPIEKTISKAKGQPREPQVYTLPPSR EEMTKNQVSLSCAVKGFYPSDIAVEWESNGQPENNYKTTPPVLDSDGSFFLVSKLTVDKSRW QQGNVFSCSVMHEALHNRFTQKSLSLSPGKGGGGSGGGGSGGGGSGGGGSDIQMTQSPS SLSASVGDRVTITCHASQNIYVWLNWYQQKPGKAPKLLIYKASNLHTGVPSRFSGSGSGTDF TLTISSLQPEDFATYYCQQGQTYPYTFGGGTKVEIK | 141 |
| | 3rd | *see above* | 113 |
| | 4th | *see above* | 122 |
| Ly3196 | 1st | EVQLVESGGGLVQPGGSLRLSCAASGFNIKDTYIHWVRQAPGKGLEWVARIYPTNGYTRYAD SVKGRFTISADTSKNTAYLQMNSLRAEDTAVYYCSRWGGDGFYAMDYWGQGTLVTVSSASV AAPSVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGSGGG GSEVQLVESGGGLVQPGGSLRLSCAASGFTFSTYAMNWVRQAPGKGLEWVGRIRSKYNNY ATYYADSVKGRFTISRDDSKNSLYLQMNSLKTEDTAVYYCVRHGNFGNSYVSWFAYWGQGT LVTVSSGGGGSDKTHTCPPCPAPELLGPSVFLFPPKPKDTLMISRTPEVTCVVVDVSHEDPEV KFNWYVDGVEVHNAKTKPREEQYNSTYRVVSVLTVLHQDWLNGKEYKCKVSNKALGAPIEK TISKAKGQPREPQVYTLPPSREEMTKNQVSLWCLVKGFYPSDIAVEWESNGQPENNYKTTPP VLDSDGSFFLYSKLTVDKSRWQQGNVFSCSVMHEALHNHYTQKSLSLSPGKGGGGSGGGG SGGGGSGGGGSQVQLVQSGAEVKKPGASVKVSCKASGYTFTSYYIHWVRQAPGQGLEWIG CIYPGNVNTNYNEKFKDRATLTVDTSISTAYMELSRLRSDDTAVYFCTRSHYGLDWNFDVWG QGTTVTVSS | 143 |
| | 2nd | EVQLVESGGGLVQPGGSLRLSCAASGFTFTDYTMDWVRQAPGKGLEWVADVNPNSGGSIY NQRFKGRFTLSVDRSKNTLYLQMNSLRAEDTAVYYCARNLGPSFYFDYWGQGTLVTVSSAS TKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCGGGGSGGGGSGGGGSGGGGSQ AVVTQEPSLTVSPGGTVTLTCRSSTGAVTTSNYANWVQQKPGQAPRGLIGGTNKRAPWTPA RFSGSLLGGKAALTITGAQAEDEADYYCALWYSNLWVFGGGTKLTVLGGGGSDKTHTCPPC PAPELLGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVYTLPPS REEMTKNQVSLSCAVKGFYPSDIAVEWESNGQPENNYKTTPPVLDSDGSFFLVSKLTVDKSR WQQGNVFSCSVMHEALHNRFTQKSLSLSPGKGGGGSGGGGSGGGGSGGGGSDIQMTQSP SSLSASVGDRVTITCHASQNIYVWLNWYQQKPGKAPKLLIYKASNLHTGVPSRFSGSGSGTD FTLTISSLQPEDFATYYCQQGQTYPYTFGGGTKVEIK | 184 |
| | 3rd | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGQGTKVEIKSSASTKGPSVFPLAPSSKS TSGGTAALGCLVKDYFPEPVTVSWNSGALTSGVHTFPAVLQSSGLYSLSSVVTVPSSSLGTQ TYICNVNHKPSNTKVDKKVEPKSC | 123 |
| | 4th | see above | 112 |
| Ly531 | 1st | DIQLTQSPASLAVSLGQRATISCKASQSVDYDGDSYLNWYQQIPGQPPKLLIYDASNLVSGIPP RFSGSGSGTDFTLNIHPVEKVDAATYHCQQSTEDPWTFGGGTKLEIKGGGGSGGGGSGGG GSQVQLQQSGAELVRPGSSVKISCKASGYAFSSYWMNWVKQRPGQGLEWIGQIWPGDGDT NYNGKFKGKATLTADESSSTAYMQLSSLASEDSAVYFCARRETTTVGRYYYAMDYWGQGTT VTVSSGGGGSDIKLQQSGAELARPGASVKMSCKTSGYTFTRYTMHWVKQRPGQGLEWIGYI NPSRGYTNYNQKFKDKATLTTDKSSSTAYMQLSSLTSEDSAVYYCARYYDDHYCLDYWGQG TTLTVSSVEGGSGGSGGSGGSGGVDDIQLTQSPAIMSASPGEKVTMTCRASSSVSYMNWYQ QKSGTSPKRWIYDTSKVASGVPYRFSGSGSGTSYSLTISSMEAEDAATYYCQQWSSNPLTFG AGTKLELKHHHHHH | 106 |

The following items are herein described.
Item 1. A multi-specific antibody, comprising a first binding moiety specific to a first target antigen, and a second binding moiety specific to a second target antigen,
   wherein the first target antigen is a first immune cell receptor, which optionally is a first T cell receptor; and the second target antigen is (i) a second immune cell receptor, optionally a second T cell receptor, which is different from the first immune cell receptor, or (ii) a first tumor associated antigen (TAA); optionally wherein the first T cell receptor and/or the second T cell receptor is a T cell activation receptor or a T cell checkpoint receptor; wherein the first binding moiety is a first Fv fragment comprising a first heavy chain variable region (VH) and a first light chain variable region (VL);
   wherein the first VH is linked to a first flexible peptide linker and a first rigid peptide linker; and the first VL is linked to a second flexible peptide linker and a second rigid peptide linker; and
   wherein the first rigid peptide linker and the second rigid peptide linker form one or more disulfide bonds.
Item 2. The multi-specific antibody of item 1, wherein the first flexible peptide linker and the second flexible peptide linker are identical, and/or wherein the first rigid peptide linker and the second rigid peptide linker are identical.
3. The multi-specific antibody of item 1 or item 2, wherein the first flexible peptide linker, the second flexible peptide linker, or both are G/S-rich peptide linkers,
   optionally wherein the G/S-rich peptide linkers comprise the formula of (GXS)n, in which X is an integer between 1-6, inclusive, and n is an integer between 1-10, inclusive.
4. The multi-specific antibody of any one of items 1-3, wherein the first rigid peptide linker, the second rigid peptide linker, or both comprise the amino acid sequence of DKTHTCPPCPAPEAAGP (SEQ ID NO: 21) DKTHTCPPCPAPELLGP (SEQ ID NO: 9), or DKTHTCPPCPAPELLGGP (SEQ ID NO:27), which optionally is linked to a G/S rich peptide linker.
5. The multi-specific antibody of item 4, wherein the first rigid peptide linker, the second rigid peptide linker, or both comprise the motif of (GXS)n, which is connected to the N-terminus of DKTHTCPPCPAPEAAGP (SEQ ID NO:21), DKTHTCPPCPAPELLGP (SEQ ID NO:9), or DKTHTCPPCPAPELLGGP (SEQ ID NO:27), wherein X is an integer between 1-6, inclusive, and n is an integer between 1-10, inclusive.
6. The multi-specific antibody of item 1 or item 2, wherein the first rigid peptide linker, the second rigid peptide linker, or both are a rigid peptide linker listed in Table 1.
7. The multi-specific antibody of any one of items 1-6, wherein the second binding moiety is linked to either the first VH via the first flexible peptide linker, or the first VL via the second flexible peptide linker.
8. The multi-specific antibody of any one of items 1-7, which further comprise a third binding moiety specific to a third target antigen.
9. The multi-specific antibody of item 8, wherein the third binding moiety is identical to the second binding moiety and the third target antigen is identical to the second target antigen.
10. The multi-specific antibody of item 8, wherein the third binding moiety is different from the second binding moiety; and optionally wherein the third target antigen is a second TAA.
11. The multi-chain antibody of any one of items 8-10, wherein the second binding moiety is linked to the first VH via the first flexible peptide linker, and wherein the third binding moiety is linked to the first VL via the second flexible peptide linker.
12. The multi-specific antibody of any one of items 7-11, wherein the first VH is further linked to a first Fc fragment via the first rigid peptide linker, and wherein the first VL is further linked to a second Fc fragment via the second rigid peptide linker; each Fc fragment comprising a CH2 domain and a CH3 domain, optionally wherein the Fc fragment(s) is derived from an IgG1 molecule.
13. The multi-specific antibody of item 12, wherein the first Fc fragment and the second Fc fragment comprise mutations in the CH3 domains that enhance heterodimerization over homodimerization of the first and second Fc fragments as relative to the wild-type counterpart and/or reduce protein A binding.
14. The multi-specific antibody of item 13, wherein the mutations are knob-in-hole mutations, charged mutations, or ZW1 mutations.
15. The multi-specific antibody of any one of items 12-14, wherein the first Fc fragment, the second Fc fragment, or both comprise one or more mutations that alter binding activity to an Fc receptor as relative to the wild-type counterpart.
16. The multi-specific antibody of any one of items 12-15, wherein the first Fc fragment, the second Fc fragment, or both comprise an amino acid substitution at one or more of positions 239, 265, 297, 329, 330, and 332,
   optionally wherein the first Fc fragment, the second Fc fragment, or both comprise
   (i) an amino acid substitution at position 329, which optionally is P329G,
   (ii) amino acid substitutions at positions 265 and 297, which optionally are D265A and N297A, and/or
   (iii) amino acid substitutions at positions 239, 330, and 332, which optionally are S239D, A330L and I332E.
17. The multi-specific antibody of any one of items 7-16, wherein the second binding moiety and/or the third binding moiety are in single-chain variable fragment (scFv) format, in a single-domain antibody format, which optionally is a single-domain antibody format, in Fab format, or in cross Fab format; optionally wherein the single-domain antibody format is a heavy chain antibody fragment (VHH).
18. The multi-specific antibody of any one of items 7-17, wherein the second binding moiety is an Fab fragment comprising a first VH-CH1 fragment and a first VL-CL fragment, or wherein the second binding moiety is a cross Fab fragment comprising a first VH-CL fragment and a first VL-CH1 fragment; and optionally
   wherein the third binding moiety is an Fab fragment comprising a second VH-CH1 fragment and a second VL-CL fragment or wherein the third binding moiety is a cross Fab fragment comprising a second VH-CL fragment and a second VL-CH1 fragment.
19. The multi-specific antibody of item 18, which comprises:
   (a) a first polypeptide comprising, from N-terminus to C-terminus, the first VH-CH1 or VH-CL fragment of the second binding moiety, the first flexible peptide linker, the first VH, the first rigid peptide linker, and the first Fc fragment;
   (b) a second polypeptide comprising, from N-terminus to C-terminus, the second VH-CH1 or VH-CL fragment of the third binding moiety, the second flexible peptide linker, the first VL, the second rigid peptide linker, and the second Fc fragment;
   (c) a third polypeptide comprising the first VL-CL or VL-CH1 fragment of the second binding moiety; and
   (d) a fourth polypeptide comprising the second VL-CL or VL-CH1 fragment of the third binding moiety.
20. The multi-specific antibody of item 19, wherein the third polypeptide and the fourth polypeptide are identical.
21. The multi-specific antibody of any one of items 12-16, further comprising a fourth binding moiety specific to a fourth antigen, wherein the fourth binding moiety is a second Fv fragment comprising a second VH and a second VL; wherein the second VH is linked to the first Fc fragment via a first peptide linker and the second VL is linked to the second Fc fragment via a second peptide linker; optionally wherein the first peptide linker is identical to the second peptide linker.
22. The multi-specific antibody of item 21, wherein the first peptide linker, the second peptide linker, or both are G/S-rich peptide linkers,
   optionally wherein the G/S-rich peptide linkers comprise the formula of (GXS)n, in which X is an integer between 1-6, inclusive, and n is an integer between 1-10, inclusive
23. The multi-specific antibody of item 21 or item 22, further comprising a third peptide linker and a fourth peptide linker connected to the second VH and second VL, respectively, optionally wherein the third peptide linker and the fourth peptide linker are a pair of rigid peptide linkers, optionally identical, that form one or more disulfide bonds.
24. The multi-specific antibody of item 23, wherein the third peptide linker, the fourth peptide linker, or both comprise the amino acid sequence of DKTHTCPPCPAPEAAGP (SEQ ID NO: 21), DKTHTCPPCPAPELLGP (SEQ ID NO: 9), DKTHTCPPCPAPELLGGP (SEQ ID NO:27), which optionally is linked to a G/S rich peptide linker.
25. The multi-specific antibody of item 23 or item 24, wherein the third peptide linker, the fourth peptide linker, or both comprise the motif of (GXS)n, which is connected to the N-terminus of DKTHTCPPCPAPEAAGP (SEQ ID NO:21), DKTHTCPPCPAPELLGP (SEQ ID NO:9), or DKTHTCPPCPAPELLGGP (SEQ ID NO:27), wherein X is an integer between 1-6, inclusive, and n is an integer between 1-10, inclusive.
26. The multi-specific antibody of any one of items 21-25, wherein the fourth target antigen is a third immune receptor, optionally a third T cell receptor, which preferably is a T cell activation receptor or a T cell checkpoint receptor; optionally wherein the third immune receptor is different from the first immune receptor and/or the second immune receptor.
27. The multi-specific antibody of any one of items 21-25, wherein the fourth target antigen is a third TAA, which optionally is different from either the first TAA or the second TAA.
28. The multi-specific antibody of any one of items 21-27, comprising:
   (a) a first polypeptide comprising, from N-terminus to C-terminus, the first VH-CH1 or VH-CL fragment of the second binding moiety, the first flexible peptide linker, the first VH, the first rigid peptide linker, the first Fc fragment, the first peptide linker, the second VH, and optionally the second peptide linker;
   (b) a second polypeptide comprising, from N-terminus to C-terminus, the second VH-CH1 or VH-CL fragment of the third binding moiety, the second flexible peptide linker, the first VL, the second rigid peptide linker, the second Fc fragment; the third peptide linker, and second VL, and optionally the fourth peptide linker;
   (c) a third polypeptide comprising the first VL-CL fragment or the first VL-CH1 of the second binding moiety; and
   (d) a fourth polypeptide comprising the second VL-CL or VL-CH1 fragment of the third binding moiety.
29. The multi-specific antibody of item 28, wherein the third polypeptide is identical to the fourth polypeptide.
30. The multi-specific antibody of any one of items 1-5, which further comprises a first heavy chain constant region fragment linked to the first flexible peptide linker, and a second heavy chain constant region fragment linked to the second flexible peptide linker, optionally wherein the first and/or the second heavy chain constant region fragment is derived from an IgG1 molecule;
   wherein each of the first and second heavy chain constant region fragment comprises a hinge domain, a CH2 domain, and a CH3 domain, and
   wherein the second binding moiety is linked to either the first heavy chain constant region fragment or the second heavy chain constant region fragment.
31. The multi-specific antibody of item 30, which further comprises a third binding moiety specific to a third target antigen, wherein the second binding moiety is linked to the first heavy chain constant region fragment, and the third binding moiety is linked to the second heavy chain constant region fragment.
32. The multi-specific antibody of item 31, wherein the third target antigen is identical to the second target antigen, optionally wherein the third binding moiety is identical to the second binding moiety.
33. The multi-specific antibody of item 31 or item 32, wherein the third binding moiety is different from the second binding moiety; and optionally wherein the third target antigen is a second TAA.
34. The multi-specific antibody of any one of items 31-33, wherein the first heavy chain constant region fragment and the second heavy chain constant region fragment comprise mutations in the CH3 domains that enhance heterodimerization over homodimerization of the first and second Fc fragments as relative to the wild-type counterpart and/or reduce protein A binding.
35. The multi-specific antibody of item 33, wherein the mutations are knob-in-hole mutations, charged mutations, or ZW1 mutations.
36. The multi-specific antibody of any one of items 30-35, wherein the first heavy chain constant region fragment, the second heavy chain constant region fragment, or both comprise one or more mutations that alter binding activity to an Fc receptor as relative to the wild-type counterpart.
37. The multi-specific antibody of any one of items 30-36, wherein the first heavy chain constant region fragment, the second heavy chain constant region fragment, or both comprise (i) a deletion at one or more of positions 236-238, and/or (ii) an amino acid substitution at one or more of positions 239, 265, 297, 329, 330, and 332,
   optionally wherein the first heavy chain constant region fragment, the second heavy chain constant region fragment, or both comprise
   (i) a deletion at the position 237,
   (ii) at least two amino acid substitutions selected from the group consisting of L234A, L235A and P329G,
   (iii) a deletion at position 237 and amino acid substitutions of D265A and N297A, iv) amino acid substitutions of S239D, A330L and I332E, and/or
   v) a deletion at the position 237 and the amino acid substitution P329G.
38. The multi-specific antibody of any one of items 31-37, wherein the second binding moiety and/or the third binding moiety are in single-chain variable fragment (scFv) format, in a single-domain antibody format, which optionally is a single-domain antibody format, in Fab format, or in cross Fab format; optionally wherein the single-domain antibody fragment is a heavy chain antibody fragment (VHH).
39. The multi-specific antibody of any one of items 32-38, wherein the second binding moiety is an Fab fragment comprising a first VH-CH1 fragment and a first VL-CL fragment, or wherein the second binding moiety is a cross Fab fragment comprising a first VH-CL fragment and a first VL-CH1 fragment; and optionally
   wherein the third binding moiety is an Fab fragment comprising a second VH-CH1 fragment and a second VL-CL fragment, or wherein the third binding moiety is a cross Fab fragment comprising a second VH-CL fragment and a second VL-CH1 fragment.
40. The multi-specific antibody of item 39, which comprises:
   (a) a first polypeptide comprising, from N-terminus to C-terminus, the first VH-CH1 or VH-CL fragment of the second binding moiety, the first heavy chain constant region fragment, the first flexible peptide linker, the first VH, and the first rigid peptide linker;
   (b) a second polypeptide comprising, from N-terminus to C-terminus, the second VH-CH1 or VH-CL fragment of the third binding moiety, the second heavy chain constant region fragment, the second flexible peptide linker, and the first VL, the second rigid peptide linker;
   (c) a third polypeptide comprising the first VL-CL or VL-CH1 fragment of the second binding moiety; and
   (d) a fourth polypeptide comprising the second VL-CL or VL-CH1 fragment of the third binding moiety.
41. The multi-specific antibody of item 40, wherein the third polypeptide and the fourth polypeptide are identical.
42. The multi-specific antibody of item 1, which is one of the multi-specific antibodies listed in Table 2.
43. A multi-specific antibody, comprising a first binding moiety specific to a first target antigen, and a second binding moiety specific to a second target antigen,
   wherein the first target antigen is a first immune cell receptor, which optionally is a first T cell receptor, preferably a T cell activation receptor or a T cell checkpoint receptor;
   wherein the second target antigen is a second immune cell receptor or a first tumor associated antigen (TAA), optionally wherein the second immune cell receptor is a second T cell receptor, preferably a second T cell activation receptor or a second T cell checkpoint receptor, wherein the second immune cell receptor is different from the first immune cell receptor;
   wherein the first binding moiety is a first Fv fragment comprising a first heavy chain variable region (VH) and a first light chain variable region (VL);
   wherein the first VH is linked to a first peptide linker and a first heavy chain constant region fragment; and the first VL is linked to a second peptide linker and a second heavy chain constant region fragment; and
   wherein the second binding moiety is connected to the first binding moiety via the first heavy chain constant region fragment or the second heavy chain constant region fragment, each of the first heavy chain constant region and the second heavy chain constant region comprises a hinge domain, a CH2 domain, and a CH3 domain; optionally wherein the first and/or the second heavy chain constant region fragment is derived from an IgG1 fragment.
44. The multi-specific antibody of item 43, wherein the first peptide linker, the second peptide linker, or both are G/S-rich peptide linkers,
   optionally wherein the G/S-rich peptide linkers comprise the formula of (GXS)n, in which X is an integer between 1-6, inclusive, and n is an integer between 1-10, inclusive.
45. The multi-specific antibody of item 43 or item 44, which further comprises a third binding moiety specific to a third target antigen.
46. The multi-specific antibody of item 45, wherein the third target antigen is identical to the second target antigen, optionally wherein the third binding moiety is identical to the second binding moiety.
47. The multi-specific antibody of item 45, wherein the third binding moiety is different from the second binding moiety; and optionally wherein the third target antigen is a second TAA.
48. The multi-specific antibody of any one of items 45-47, wherein the first heavy chain constant region fragment and the second heavy chain constant region fragment comprise mutations in the CH3 domains that enhance heterodimerization over homodimerization of the first and second Fc fragments as relative to the wild-type counterpart and/or reduce protein A binding.
49. The multi-specific antibody of item 48, wherein the mutations are knob-in-hole mutations, charged mutations, or ZW1 mutations.
50. The multi-specific antibody of any one of items 45-47, wherein the first heavy chain constant region, the second heavy chain constant region, or both comprise one or more mutations that alter binding activity to an Fc receptor as relative to the wild-type counterpart.
51. The multi-specific antibody of any one of items 45-50, wherein the first heavy chain constant region fragment, the second heavy chain constant region fragment, or both comprise (i) a deletion at one or more of positions 236-238, and/or (ii) an amino acid substitution at one or more of positions 239, 265, 297, 329, 330, and 332,
   optionally wherein the first heavy chain constant region fragment, the second heavy chain constant region fragment, or both comprise
   (i) a deletion at the position 237,
   (ii) at least two amino acid substitutions selected from the group consisting of L234A, L235A and P329G,
   (iii) a deletion at position 237 and amino acid substitutions of D265A and N297A, iv) amino acid substitutions S239D, A330L and I332E, and/or
   v) a deletion at the position 237 and the amino acid substitution P329G.
52. The multi-specific antibody of any one of items 45-51, wherein the second binding moiety and/or the third binding moiety are in single-chain variable fragment (scFv) format, in a single-domain antibody format, which optionally is a heavy-chain only (VHH) format, in Fab format, or in cross Fab format.
53. The multi-specific antibody of any one of items 1-52, wherein the first immune receptor, optionally the second immune receptor and/or the third immune receptor, is selected from the group consisting of CD3, CD28, PD-1, PD-L1, CD47, and a member of the tumor necrosis factor receptor superfamily (TNFRSF), optionally wherein the member of the TNFRSF family is selected from the group consisting of FAS, TNFRSF12A, 4-1BB/CD137, TNFRSF13B, TNFRSF13C, CD27/TNFRSF7, CD30/TNFRSF8, CD40/TNFRSF5, DR3/TNFRSF25, DR4/TNFRSF10A, DR5/TNFRSF10B, DR6/TNFRSF21, GITR/TNFRSF18, HVEM/TNFRSF14, LTBR, OX40/TNFRSF4, TROY/TNFRSF19, RELT/TNFRSF19L, TNFRSF12A, TNFRSF13B, TL1A/TNFSF15, TNFRSF17, TNFRSF1A, TNFRSF11B, RANK/TNFRSF11A, TNFRSF11B, NGFR, EDA2R, and TNFRSF1B.
54. The multi-specific antibody of any one of items 1-53, which binds at least:
   (a) CD3 and CD28,
   (b) CD3 and CD137,
   (c) CD137 and PD-1,
   (d) CD40 and PD-1,
   (e) CD40 and PD-L1,
   (f) CD137 and GITR,
   (g) CD137 and PD-L1,
   (h) CD137 and CD40,
   (i) CD137 and OX40,
   (j) CD3 and PD-1,
   (k) CD3 and PD-L1, or
   (l) CD3 and CTLA4.
55. The multi-specific antibody of any one of items 1-49, wherein the first TAA, optionally the second and third TAAs, is selected from the group consisting of B7H3, CD19, CD20, PSMA, HER2, CEA, BCMA, P53mut, DLL3, MET, and EGFR.
56. The multi-specific antibody of any one of items 1-50, which binds:
   (1) B7H3, CD3, and CD137;
   (2) CD19, CD3, and CD137;
   (3) B7H3, CD3, and CD28;
   (4) CD19, CD3, and CD28;
   (5) B7H3, CD137, and PD-1;
   (6) B7H3, CD40, and PD-1;
   (7) PMSA, CD3, and CD137;
   (8) B7H3 and CD3;
   (9) B7H3 and CD137;
   (10) CD19 and CD3;
   (11) CD19 and CD137;
   (12) B7H4 and CD40.
   (13) HER2, CD3, and CD137;
   (14) CEA, CD3, and CD137;
   (15) BCMA, CD3, and CD137;
   (16) P53mutant, CD3, and CD137;
   (17) PD-1 and CD137;
   (18) PD-1 and CD40;
   (19) B7H3 and CD40;
   (20) PD-L1 and CD40;
   (21) PD-L1 and CD3;
   (22) PD-L1, CD3, and CD137;
   (23) PD-L1, CD3, and CD28;
   (24) PD-L1, CD137, and B7H3;
   (25) PD-L1, CD40, and B7H3;
   (26) PD-1, CD40, and CD137;
   (27) PD-1, CD137, and GITR;
   (28) CD19, CD20, CD3, and CD137;
   (29) CEA and CD3;
   (30) CEA and CD137;
   (31) P53mutant and CD3;
   (32) P53mutant and CD137;
   (33) PD-L1, CD40, and CD137;
   (34) PD-L1 and CD137;
   (35) MET, EGFR, and CD47;
   (36) BCMA and CD3;
   (37) BCMA and CD137;
   (38) PSMA and CD3;
   (39) HER2 and CD3;
   (40) HER2 and CD40;
   (41) HER2 and CD137;
   (42) PSMA and CD137;
   (43) HER2, MET, CD3, and CD137;
   (44) MAGE-A4, CD3, and CD137;
   (45) PRAME, CD3, and CD137;
   (46) HER2, MET and CD3;
   (47) MAGE-A4 and CD3;
   (48) PRAME and CD3;
   (49) HER2, MET, and CD47;
   (50) B7H3, CD3, and PD-1,
   (51) B7H3, CD3, and PD-L1, or
   (52) B7H3, CD3, and CTLA4.
   (53) PSMA, CD3 and CD137;
   (54) PSMA, CD3 and CD28;
   (55) HER2, CD3 and CD137;
   (56) HER2, CD3 and CD28;
   (57) CEA, CD3 and CD137;
   (58) CEA, CD3 and CD28;
   (59) BCMA, CD3 and CD137;
   (60) BCMA, CD3 and CD28; or
   (61) CD19, CD19, CD3 and CD28.
57. The multi-specific antibody of any one of items 1-56, wherein the multi-specific antibody comprises the same heavy chain complementary determining regions (CDRs) and the same light chain CDRs as those in one or more of the parent antibodies listed in Table 1; optionally wherein the multi-specific antibody comprises the same VH and VL as those in the one or more parent antibodies.
58. The multi-specific antibody of any one of items 1-57, which is bivalent, trivalent or tetravalent.
59. A nucleic acid or a nucleic acid set, which collectively encodes the multi-specific antibody setting forth in any one of items 1-58.
60. The nucleic acid or the nucleic acid set of item 59, which is an expression vector or an expression vector set.
61. A host cell, comprising the nucleic acid or the nucleic acid set of item 59 or item 60.
62. The host cell of item 61, which is a mammalian host cell.
63. A method for producing a multi-specific antibody, comprising:
   (i) culturing the host cell of item 61 or item 62 under conditions allowing for expression of the antibody; and
   (ii) harvesting the antibody thus produced.
64. A pharmaceutical composition comprising a multi-specific antibody set forth in any one of items 1-58 or a nucleic acid or nucleic acid set encoding such, and a pharmaceutically acceptable carrier.
65. A method for modulating immune responses in a subject, the method comprising administering to a subject in need thereof an effective amount of the multi-specific antibody of any one of items 1-58, a nucleic acid(s) encoding such, or a pharmaceutical composition comprising the antibody or the encoding nucleic acid(s).
66. The method of item 65, wherein the subject is a human patient having or suspected of having cancer.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

## Claims

1. A multi-specific antibody, comprising:
(a) a first binding moiety specific to a first target antigen,
(b) a second binding moiety specific to a second target antigen,
(c) a third binding moiety specific to a third target antigen, the third binding moiety being identical to the second binding moiety; and
(d) a fourth binding moiety specific to a fourth target antigen;
wherein the first target antigen is a first immune cell receptor; the second target antigen is (i) a second immune cell receptor, which is different from the first immune cell receptor, or (ii) a first tumor associated antigen (TAA); the third target antigen is identical to the second target antigen; and the fourth target antigen is a third immune cell receptor, which is different from the first and second immune cell receptors;
wherein the first binding moiety is a first Fv fragment comprising a first heavy chain variable region (V_{H}) and a first light chain variable region (V_{L});
wherein the first V_{H} is linked to a first flexible peptide linker and a first rigid peptide linker; and the first V_{L} is linked to a second flexible peptide linker and a second rigid peptide linker; optionally wherein the first rigid peptide linker is identical to the second rigid peptide and the first flexible peptide linker is identical to the second flexible peptide liner;
wherein the first rigid peptide linker and the second rigid peptide linker form one or more disulfide bonds;
wherein the second binding moiety is linked to either the first V_{H} via the first flexible peptide linker,
wherein the third binding moiety is linked to the first V_{L} via the second flexible peptide linker;
wherein the first V_{H} is further linked to a first Fc fragment via the first rigid peptide linker, and the first V_{L} is further linked to a second Fc fragment via the second rigid peptide linker; each Fc fragment comprising a CH2 domain and a CH3 domain, optionally wherein the Fc fragment(s) is derived from an IgG1 molecule;
wherein the fourth binding moiety is a second Fv fragment comprising a second V_{H} and a second V_{L}; wherein the second V_{H} is linked to the first Fc fragment via a first peptide linker and the second V_{L} is linked to the second Fc fragment via a second peptide linker; optionally
wherein the first peptide linker is identical to the second peptide linker; and
wherein the multi-specific antibody binds CEA, CD3, and CD137.

2. The multi-specific antibody of claim 1, wherein the first flexible peptide linker, the second flexible peptide linker, or both are G/S-rich peptide linkers,
optionally wherein the G/S-rich peptide linkers comprise the formula of (G_{X}S)ₙ, in which X is an integer between 1-6, inclusive, and n is an integer between 1-10, inclusive; and/or wherein the first rigid peptide linker, the second rigid peptide linker, or both comprise the amino acid sequence of DKTHTCPPCPAPEAAGP (SEQ ID NO: 21) DKTHTCPPCPAPELLGP (SEQ ID NO: 9), or DKTHTCPPCPAPELLGGP (SEQ ID NO:27);
optionally wherein the first rigid peptide linker, the second rigid peptide linker, or both comprise the motif of (G_{X}S)_{n,} which is connected to the N-terminus of DKTHTCPPCPAPEAAGP (SEQ ID NO:21), DKTHTCPPCPAPELLGP (SEQ ID NO:9), or DKTHTCPPCPAPELLGGP (SEQ ID NO:27), wherein X is an integer between 1-6, inclusive, and n is an integer between 1-10, inclusive.

3. The multi-specific antibody of claim 1 or claim 2, wherein the first Fc fragment, the second Fc fragment, or both comprise one or more mutations that alter binding activity to an Fc receptor as relative to the wild-type counterpart; optionally wherein the first Fc fragment, the second Fc fragment, or both comprise an amino acid substitution at one or more of positions 239, 265, 297, 329, 330, and 332;
optionally wherein the first Fc fragment, the second Fc fragment, or both comprise
(i) an amino acid substitution at position 329, which optionally is P329G,
(ii) amino acid substitutions at positions 265 and 297, which optionally are D265A and N297A, and/or
(iii) amino acid substitutions at positions 239, 330, and 332, which optionally are S239D, A330L and I332E.

4. The multi-specific antibody of any one of claims 1-3, wherein the second binding moiety is an Fab fragment comprising a first VH-CH1 fragment and a first VL-CL fragment, or wherein the second binding moiety is a cross Fab fragment comprising a first VH-CL fragment and a first VL-CH1 fragment;
and optionally wherein the third binding moiety is an Fab fragment comprising a second VH-CH1 fragment and a second VL-CL fragment or wherein the third binding moiety is a cross Fab fragment comprising a second VH-CL fragment and a second VL-CH1 fragment.

5. The multi-specific antibody of any one of claims 1-4, comprising:
(a) a first polypeptide comprising, from N-terminus to C-terminus, the VH-CH1 or VH-CL fragment of the second binding moiety, the first flexible peptide linker, the first V_{H}, the first rigid peptide linker, the first Fc fragment, the first peptide linker, and the second V_{H};
(b) a second polypeptide comprising, from N-terminus to C-terminus, the VH-CH1 or VH-CL fragment of the third binding moiety, the second flexible peptide linker, the first V_{L}, the second rigid peptide linker, the second Fc fragment; the second peptide linker, and the second V_{L};
(c) a third polypeptide comprising the VL-CL fragment or the VL-CH1 of the second binding moiety; and
(d) a fourth polypeptide comprising the VL-CL or VL-CH1 fragment of the third binding moiety;
wherein the third polypeptide is identical to the fourth polypeptide.

6. The multi-specific antibody of any one of claims 1-5, wherein the multi-specific antibody comprises the same heavy chain complementary determining regions (CDRs) and the same light chain CDRs as those in:
(a) the VH of SEQ ID NO: 38 and the VL of SEQ ID NO: 39;
(b) the VH of SEQ ID NO: 80 and the VL of SEQ ID NO:81; and
(c) the VH of SEQ ID NO: 54 and the VL of SEQ ID NO: 55.

7. The multi-specific antibody of claim 6, wherein the multi-specific antibody comprises:
(a) a VH comprising SEQ ID NO: 38 and a VL comprising SEQ ID NO: 39;
(b) a VH comprising SEQ ID NO: 80 and a VL comprising SEQ ID NO: 81; and
(c) a VH comprising SEQ ID NO: 54 and a VL comprising SEQ ID NO: 55.

8. The multi-specific antibody of claim 1, wherein:
(a) the first V_{H} comprises the amino acid sequence of SEQ ID NO: 38 and the first V_{L} comprises the amino acid sequence of SEQ ID NO: 39;
(b) the second V_{H} comprises the amino acid sequence of SEQ ID NO: 80 and the second V_{L} comprises the amino acid sequence of SEQ ID NO: 81; and
(c) the VH in the VH-CH1 chain comprises the amino acid sequence of SEQ ID NO: 54 and the VL in the VL-CL chain comprises the amino acid sequence of SEQ ID NO: 55;
wherein the first rigid peptide linker, the second rigid peptide linker, or both comprise the amino acid sequence of DKTHTCPPCPAPEAAGP (SEQ ID NO: 21) DKTHTCPPCPAPELLGP (SEQ ID NO: 9), or DKTHTCPPCPAPELLGGP (SEQ ID NO:27);
wherein the first rigid peptide linker and the second rigid peptide linker further comprise the motif of GGGGS (SEQ ID NO: 3), which is connected to the N-terminus of the first rigid peptide linker and the second rigid peptide linker; and
wherein the first flexible linker, the second flexible linker, the first peptide linker, and the second peptide linker are each a G/S rich peptide linker of GGGGSGGGGSGGGGSGGGGS (SEQ ID NO: 6).

9. A nucleic acid or a nucleic acid set, which collectively encodes the multi-specific antibody setting forth in any one of claims 1-8; which optionally is an expression vector or an expression vector set.

10. A host cell, comprising the nucleic acid or the nucleic acid set of claim 9; which optionally is a mammalian host cell.

11. A method for producing a multi-specific antibody, comprising:
(i) culturing the host cell of claim 10 under conditions allowing for expression of the antibody; and
(ii) harvesting the antibody thus produced.

12. A pharmaceutical composition comprising a multi-specific antibody set forth in any one of claims 1-8 or a nucleic acid or nucleic acid set encoding such, and a pharmaceutically acceptable carrier.

13. A multi-specific antibody of any one of claims 1-8, a nucleic acid(s) encoding such, or a pharmaceutical composition comprising the antibody or the encoding nucleic acid(s) for use in a method for modulating immune responses in a subject in need thereof.

14. A multi-specific antibody of any one of claims 1-8, a nucleic acid(s) encoding such, or a pharmaceutical composition comprising the antibody or the encoding nucleic acid(s) for use in treating cancer in a human patient having or suspected of having cancer.
